# EUROPEAN PATENT APPLICATION

(11) **EP 4 834 382 A1**
(43) Date of publication of application: **04.11.2026**
(21) Application number: 24911522.1
(22) Date of filing: 27.12.2024
(51) Int. Cl.: A61M 1/06

(54) **BREAST PUMP, STORAGE MEDIUM, DETECTION METHOD, AND CONTROL METHOD AND APPARATUS**

(30) Priority: 28.12.2023 WO PCT/CN2023/142819; 28.12.2023 CN 202311847927; 28.12.2023 CN 202311853384; 28.12.2023 CN 202311853326; 28.12.2023 CN 202311850098; 28.12.2023 CN 202311853323; 28.12.2023 CN 202311871063; 28.12.2023 CN 202311837671; 28.12.2023 CN 202311845035; 28.12.2023 CN 202311845027; 28.12.2023 CN 202311850727; 28.12.2023 CN 202311847991; 28.12.2023 CN 202311870807; 28.12.2023 CN 202311870914; 28.12.2023 CN 202311850115; 28.12.2023 CN 202311837664; 28.12.2023 CN 202311849944; 28.12.2023 CN 202311850134; 28.12.2023 CN 202311849958; 28.12.2023 CN 202311850710
(71) Applicant: Shenzhen Lute Jiacheng Supply Chain Management Co., Ltd., Shenzhen, Guangdong 518000 (CN)
(72) Inventor: XIE, Guangbao, Shenzhen, Guangdong 518000 (CN); TANG, Jiyan, Shenzhen, Guangdong 518000 (CN); WEI, Feng, Shenzhen, Guangdong 518000 (CN); CHEN, Xiaozhuan, Shenzhen, Guangdong 518000 (CN)
(74) Representative: Santarelli
(86) International application number: PCT/CN2024/143316
(87) International publication number: WO 2025/140606

(57) **Abstract**

This application relates to a breast pump, a storage medium, a detection method, and a control method and device. The breast pump includes one or both of a detection unit and a control unit. One end of a breast milk collection portion is attached to a breast, and another end of the breast milk collection portion communicates with a milk storage container portion. The milk storage container portion is configured to store breast milk collected by the breast milk collection portion, and the breast milk collection portion forms an internal cavity with an interior of the milk storage container portion for circulation or storage of the breast milk. The negative pressure mechanism is configured to directly or indirectly apply negative pressure to the internal cavity, causing the breast milk to be expressed to the milk storage container portion for storage. The detection unit is configured to acquire a detection parameter detected by the detection unit. The control unit is arranged on the breast pump. This application utilizes the detection unit and the control unit to perform intelligent control on the breast pump, to resolve at least one of problems of breast milk spillage, limited functionality, and improper mounting through intelligent control during use of the breast pump.

## Description

### TECHNICAL FIELD

The present disclosure relates to the field of maternal and infant products, and in particular, to a breast pump, a storage medium, a detection method, and a control method and device.

### BACKGROUND

With the development of society, people's material life has become increasingly abundant, and the pace of life accelerated as well. Reproduction is an indispensable activity for human continuation. However, in the fast-paced society, many working women find it difficult to breast-feed infants according to infants' needs. Moreover, with improvements in living conditions, the volume of breast milk produced by women during the lactation period often exceeds demand. Thus, expressing and storing breast milk enable prompt breast-feeding of infants and allow surplus breast milk to be stored. This ensures that infants can receive adequate breast milk even when a mother's milk production decreases or a mother is unable to breast-feed her infant in person.

Breast pumps, as portable and convenient devices for milk expression, are receiving increasing attention. Their functions and structures are continuously being refined to ensure safer and more efficient operation while improving user experience.

Some models of breast pumps, designed as more discreet expressing devices, allow milk expression under the cover of clothing, making them suitable for a wider range of scenarios and offering effective privacy protection. However, while breast pumps protect user privacy, they make it inconvenient for users to observe the volume of breast milk expressed by the breast pump or to monitor or learn the current state of the breasts in a timely manner. As a result, during the milk expressing, users cannot learn the volume of breast milk expressed or select a proper milk expressing mode, and problems such as breast milk spilling from the milk storage container frequently occur. Since it is often difficult for the human body to maintain the same posture throughout the expressing process, and individual body habits vary, the milk bowl is prone to tilting or shaking. This causes the breast milk inside the milk bowl to slant or form fluctuating waves relative to the milk storage container, increasing the risk of spillage of the breast milk from the milk bowl.

In the prior art, most breast pumps operate in relatively fixed modes with limited functionality, making it difficult to adapt to users' demand for diverse modes.

In addition, breast pumps in the prior art are prone to problems such as malfunction or milk leakage due to improper mounting.

The breast pumps in the prior art, due to their single mode, cannot make adaptive adjustments based on the human breast-feeding status, resulting in low milk expressing efficiency and requiring improvements.

Therefore, specific detection means is needed to resolve at least one of the above problems.

### BRIEF SUMMARY

In view of the deficiencies in the prior art, this application provides a breast pump, a storage medium, a detection method, and a control method and device, helping resolve at least one of the problems of breast milk spillage, failure to acquire a breast milk volume, limited functionality, inefficient milk expressing, and improper mounting in the background.

To resolve the above problems, the present disclosure provides following technical solutions:

The present disclosure provides a breast pump, including a breast milk collection portion, a milk storage container portion, and a negative pressure mechanism, and further including a detection unit and a control unit. One end of the breast milk collection portion is attached to a breast, and another end of the breast milk collection portion communicates with the milk storage container portion.

The milk storage container portion is configured to store breast milk collected by the breast milk collection portion, and the breast milk collection portion forms an internal cavity with an interior of the milk storage container portion for circulation or storage of the breast milk. The negative pressure mechanism is configured to directly or indirectly apply negative pressure to the internal cavity, causing the breast milk to be expressed to the milk storage container portion for storage. The detection unit is configured to acquire a detection parameter. The control unit is configured to perform a selection operation based on the detection parameter. The detection parameter includes at least one of following: a signal indicating whether a component of the breast pump is properly mounted, data on a volume of breast milk expressed by the breast milk collection portion in a single expressing operation, breast milk flow rate data, a current milk expressing status, data information of a volume of expressed breast milk, breast impedance data, breast hardness data, a user parameter, indication information indicating whether the breast milk stored in the breast pump is expressed from a left breast or a right breast, preset user parameter information, a startup signal, weaning indication information, a weaning parameter, breast status data, breast milk flow rate status data, preset breast pump operating parameter information, or milk expressing status data.

Optionally, the breast milk collection portion includes a flange detachably mounted on the milk storage container portion.

The detection unit includes a first detection unit configured to generate a first signal when the flange is not properly mounted on the milk storage container portion; and the control unit is configured to control, based on the first signal, the breast pump to operate.

Optionally, the breast pump further includes a diaphragm mounted on the flange and detachably mounted on the milk storage container portion along with the flange; the detection unit further includes a second detection unit configured to generate a second signal when the diaphragm is not properly mounted on the milk storage container portion along with the flange; and the control unit is configured to control, based on the second signal, the breast pump to operate.

Optionally, the breast pump further includes a main unit, where the negative pressure mechanism and the control unit are arranged inside the main unit, and the main unit is detachably mounted on the milk storage container portion; the detection unit further includes a third detection unit configured to generate a third signal when the main unit is not properly mounted on the milk storage container portion; and the control unit is configured to control, based on the third signal, the breast pump to operate.

Optionally, the detection unit further includes a fourth detection unit configured to generate a fourth signal when the one end of the breast milk collection portion does not meet an attachment condition with the breast; and the control unit is configured to perform following control based on the fourth signal: controlling the negative pressure mechanism to stop operating; and controlling a power source of the breast pump to turn off when a duration of the fourth signal exceeds a preset duration threshold; or controlling the negative pressure mechanism to resume operation when the duration of the fourth signal does not exceed the preset duration threshold.

Optionally, the breast pump further includes a measurement container configured to receive breast milk expressed by the breast milk collection portion in at least a single expressing operation.

The detection unit includes a fifth detection unit configured to detect a volume of the breast milk expressed by the breast milk collection portion in at least a single expressing operation and that is received in the measurement container; and the control unit is configured to control a processing task of the breast pump based on the volume of the breast milk expressed by the breast milk collection portion in at least a single expressing operation, where the controlling a processing task of the breast pump includes adjusting an operating parameter of the negative pressure mechanism and/or adjusting an operating mode of the breast pump.

Optionally, the control unit is configured to: acquire a user parameter; determine an operating parameter of the negative pressure mechanism during operation based on the user parameter; and control the negative pressure mechanism to operate based on the operating parameter, where the user parameter includes a pressure tolerance, and the operating parameter includes a milk expressing intensity and a milk expressing duration, where the milk expressing intensity is positively correlated with the pressure tolerance, and the milk expressing duration is negatively correlated with the pressure tolerance.

Optionally, the breast pump further includes an input unit electrically connected to the control unit and configured to generate the user parameter in response to an input instruction of a user; and/or the breast pump further includes a communication unit electrically connected to the control unit and configured to receive the user parameter from an external terminal.

Optionally, the breast pump further includes a display unit electrically connected to the control unit, where the display unit includes a display region configured to display the user parameter and/or a display region configured to display the operating parameter.

Optionally, the breast pump further includes a control interface, where the control interface includes a first display region and a second display region, where the first display region is configured to display information indicating whether breast milk is expressed from the left breast, and the second display region is configured to display information indicating whether breast milk is expressed from the right breast; and the control unit is configured to: control the first display region to display a first display state and the second display region to display a second display state when the breast pump operates on the right breast, and control the first display region to display a third display state and the second display region to display a fourth display state when the breast pump operates on the left breast, where the first display state is different from the third display state, and the second display state is different from the fourth display state.

Optionally, the first display region is located on a right side of the second display region; and the control unit is configured to: control the first display region to shrink and the second display region to expand when the breast pump operates on the right breast, causing the first display region to form the first display state and the second display region to form the second display state; and control the first display region to expand and the second display region to shrink when the breast pump operates on the left breast, causing the first display region to form the third display state and the second display region to form the fourth display state.

Optionally, the first display region is located above the second display region; and the control unit is configured to: control the first display region to display a third color and the second display region to display a fourth color when the breast pump operates on the right breast, causing the first display region to form the first display state and the second display region to form the second display state; and control the first display region to display the fourth color and the second display region to display the third color when the breast pump operates on the left breast, causing the first display region to form the third display state and the second display region to form the fourth display state.

Optionally, the breast pump further includes a lactation stimulation assembly embedded in the breast milk collection portion, where the control unit is configured to control the lactation stimulation assembly to perform lactation stimulation on the breast.

Optionally, the lactation stimulation assembly includes a stimulation unit electrically connected to the control unit, where the control unit is configured to generate a corresponding startup signal in response to a startup instruction input by the user or based on detection data of a sensor, and control the stimulation unit to start operating in a preset mode, where the preset mode includes at least a first mode for stimulating the breast to heat up.

Optionally, the stimulation unit includes an infrared generator, and the control unit is configured to control, after receiving the corresponding startup signal, the infrared generator to radiate infrared rays to the breast to stimulate the breast to heat up; and the stimulation unit further includes a massage assembly arranged on the breast milk collection portion.

Optionally, the lactation stimulation assembly includes a plurality of oscillators, where the plurality of oscillators are embedded in an inner side wall of the breast milk collection portion in a preset arrangement, causing the plurality of oscillators to contact the breast and perform lactation stimulation on the breast under the control of the control unit when the breast milk collection portion is attached to the breast.

Optionally, the detection unit includes a sixth detection unit configured to detect a current milk expressing status of the user.

The control unit is configured to: generate a mode adjustment signal based on the current milk expressing status and a preset milk expressing status of the user; determine a milk expressing mode based on the mode adjustment signal; and control the breast pump to perform milk expressing in the milk expressing mode.

Optionally, the breast pump further includes a triggering unit configured to generate weaning indication information, where the control unit is configured to control the breast pump to operate in a weaning mode based on the weaning indication information, where the weaning mode is used to assist a mother in ceasing milk secretion.

Optionally, the triggering unit is a physical key, a toggle, or a knob mounted on a housing; and/or the triggering unit is a touch key, and the housing includes a control interface, where the touch key is arranged on the control interface.

Optionally, the internal cavity includes an inflow path for breast milk circulation, causing the breast milk to flow from the breast milk collection portion to the milk storage container portion; and the detection unit includes a breast milk flow rate detection unit configured to detect a breast milk flow rate on the inflow path.

Optionally, the breast pump further includes a diaphragm mounted on and communicated with the milk storage container portion, where the breast milk collection portion has a milk expressing channel communicated with the milk storage container portion to form the inflow path; and the breast milk flow rate detection unit is arranged adjacent to the diaphragm, and is configured to detect a flow rate of breast milk entering the milk storage container portion after passing through a side of the diaphragm.

Optionally, the milk storage container portion is provided with a support engaged with the diaphragm to mount the diaphragm on the milk storage container portion.

At least one mounting position is arranged on the support, where the breast milk flow rate detection unit is mounted in the mounting position.

Optionally, the breast milk flow rate detection unit is configured to receive a light ray reflected during breast milk expressing and generate a detection signal corresponding to a light intensity of the light ray; and the control unit is configured to determine a breast milk flow rate based on the light intensity corresponding to the detection signal.

Optionally, the breast pump has a transparent side wall, where the breast milk flow rate detection unit is configured to receive a light ray from the transparent side wall during breast milk expressing and generate a detection signal corresponding to a light intensity of the light ray.

Optionally, the control unit is configured to determine a breast milk flow rate corresponding to the light intensity based on the light intensity corresponding to the detection signal and a preset mapping relationship between a light intensity and a breast milk flow rate.

Optionally, the control unit is further configured to: acquire, in real time, a current milk expressing flow rate of the breast pump driven at a current milk expressing frequency; determine whether the current milk expressing flow rate is less than a current preset flow rate threshold; and stop, when it is determined that the current milk expressing flow rate is less than the current preset flow rate threshold, driving the breast pump at the current milk expressing frequency, and output a lactation stimulation and milk expression-mixed control mode to drive the breast pump based on the current milk expressing flow rate, causing the breast pump to perform lactation stimulation and milk expression on the user; or stop, when it is determined that the current milk expressing flow rate is greater than or equal to the current preset flow rate threshold, driving the breast pump at the current milk expressing frequency, and output a target milk expressing frequency based on a preset milk expressing frequency adjustment manner to drive the breast pump to operate.

Optionally, the detection unit further includes a breast milk volume detection unit configured to detect data information of a breast milk volume expressed by the breast pump; and the control unit is configured to determine a breast milk volume based on the data information of the breast milk volume.

Optionally, a side wall of the milk storage container portion has N capacity positions having increasing capacities from a first capacity position to an N^{th} capacity position, where N is a positive integer; the breast milk volume detection unit includes at least one detection element arranged at each capacity position; and the control unit is configured to determine a current breast milk volume in the milk storage container portion based on a detection signal of the detection element.

Optionally, the breast milk volume detection unit includes: a flow rate sensor arranged in a nipple channel and configured to measure flow velocity data of the breast milk expressed by the breast pump, where the breast milk expressed by the breast pump is transferred to the milk storage container portion through the nipple channel; and a timing subunit configured to record an operating duration of the negative pressure mechanism and generate timing data, where the control unit is configured to calculate a volume of the breast milk expressed by the breast pump as the breast milk volume based on the flow velocity data and the timing data.

Optionally, the breast milk volume detection unit includes a liquid level detection unit configured to detect a liquid level of the milk storage container portion; and the control unit is configured to determine the breast milk volume in the milk storage container portion based on at least liquid level data from the liquid level detection unit.

Optionally, the breast milk flow rate detection unit unit further includes an angle detection unit configured to detect tilting data of the milk storage container portion, where the angle detection unit is a gyroscope; and the control unit is configured to determine the breast milk volume in the milk storage container portion based on the liquid level data from the liquid level detection unit and the tilting data from the angle detection unit.

Optionally, the liquid level detection unit includes at least one through-beam sensor, including an emitter and a receiver respectively mounted on two opposite sides of the milk storage container portion.

Optionally, the breast milk volume detection unit includes a weight detection module, and the data information of the breast milk volume is breast milk weight data.

Optionally, the milk storage container portion is hung on a housing of the breast pump; and the breast milk volume detection unit is arranged at a joint of the milk storage container portion and the housing to acquire the data information of the breast milk volume after breast milk enters the milk storage container portion.

Optionally, the housing is provided with a supporting portion having a hanging port, and the milk storage container portion is provided with a hanging portion; and the milk storage container portion is hung on the housing through support of the supporting portion for the hanging portion.

Optionally, the hanging portion is arranged at an inlet of the milk storage container portion, and the hanging portion is flange-shaped; an inlet diameter of the milk storage container portion is less than or equal to a diameter of the hanging port; and a diameter of the hanging portion is greater than the diameter of the hanging port, the breast milk volume detection unit is located between the supporting portion and the hanging portion, one end of the breast milk volume detection unit is connected to the supporting portion, and another end of the detection unit is connected to the hanging portion.

Optionally, the breast milk volume detection unit includes at least one elastic member and at least one distance sensor, where the elastic member and the distance sensor are both located between the supporting portion and the hanging portion, and the distance sensor is connected to the control unit; one end of the elastic member is connected to the supporting portion, and another end of the elastic member is connected to the hanging portion; and the distance sensor is configured to detect distance data between the supporting portion and the hanging portion, where the data information of the breast milk volume is the distance data.

Optionally, the breast milk flow rate detection unit includes at least one elastic member and at least one capacitive sensor, where the elastic member and the capacitive sensor are both located between the supporting portion and the hanging portion, and the capacitive sensor is connected to the control unit; one end of the elastic member is connected to the supporting portion, and another end of the elastic member is connected to the hanging portion; and a distance change between the hanging portion and the supporting portion causes a change in capacitance data of the capacitive sensor, where the data information of the breast milk volume is the capacitance data.

Optionally, the breast milk flow rate detection unit includes at least one pressure sensor, where the pressure sensor is connected to the control unit and is configured to detect pressure data between the hanging portion and the supporting portion, where the data information of the breast milk volume is the pressure data.

Optionally, the breast milk flow rate detection unit includes an air chamber assembly and an air pressure sensor, where the air chamber assembly is provided with a closed space, and abuts against both the supporting portion and the hanging portion, where a distance change between the supporting portion and the hanging portion causes a change in a volume of the closed space; and the air pressure sensor is configured to detect air pressure data of the air chamber assembly, where the data information of the breast milk volume data is the air pressure data.

Optionally, the breast pump further includes a breast massage device that includes an attachment portion and at least one electrode portion, where the attachment portion is configured to be attached to the breast, and is arranged on an end of the breast milk collection portion close to the breast, and at least part of the attachment portion forms a part of the breast milk collection portion or is configured to be detachably mounted on or attached to the breast milk collection portion; and the at least one electrode portion is connected to the attachment portion, is directly in contact with a breast surface or is indirectly in contact with the breast surface through a conductive portion on the attachment portion, and is configured to perform electrical stimulation and massage on the breast and/or detect an impedance of the breast.

Optionally, the breast massage device further includes an electrical stimulation unit connected to the electrode portion and including at least an electrical stimulation control circuit, where the electrical stimulation control circuit is configured to actively or passively apply electrical stimulation to the breast surface.

Optionally, the detection unit includes an impedance detection unit connected to the electrode portion and including at least an impedance detection circuit, where the impedance detection circuit is configured to actively or passively detect breast impedance data; and/or a flow rate detection unit configured to detect a breast milk flow rate, where the control unit is configured to adjust an operating parameter of the electrical stimulation unit based on the breast impedance data and/or the breast milk flow rate.

Optionally, the attachment portion includes: an attachment layer, where the attachment portion is attached to the breast through the attachment layer; and a structural layer, where the structural layer and the attachment layer form a double-layer structure having an intermediate accommodating space, and the attachment layer is made of a flexible material, and at least one massage member is arranged in the accommodating space.

Optionally, the detection unit includes: an impedance detection unit configured to detect breast impedance data; and/or a hardness detection unit configured to detect breast hardness data, where the control unit is configured to control the breast pump to operate in different modes based on the breast impedance data and/or the breast hardness data.

Optionally, the breast pump further includes a prompt module configured to generate prompt information based on impedance data information or hardness data information.

The present disclosure provides a method for a breast pump, including: acquiring a parameter related to the breast pump, where the parameter related to the breast pump includes at least a detection parameter detected by a detection unit; and performing a preset operation based on the parameter, where the detection parameter includes at least one of following: a signal indicating whether a component of the breast pump is properly mounted, data on a volume of breast milk expressed by a breast milk collection portion in a single expressing operation, breast milk flow rate data, a current milk expressing status, data information of a volume of expressed breast milk, breast impedance data, breast hardness data, a user parameter, indication information indicating whether the breast milk stored in the breast pump is expressed from a left breast or a right breast, preset user parameter information, a startup signal, weaning indication information, a weaning parameter, breast status data, breast milk flow rate status data, preset breast pump operating parameter information, or milk expressing status data.

Optionally, the acquiring a detection parameter detected by a detection unit includes: acquiring a first signal generated by a first detection unit when improper mounting of a flange is detected; and the performing a preset operation based on the parameter includes: based on the first signal, controlling to automatically generate first prompt information and/or stop milk expressing.

Optionally, the acquiring a detection parameter detected by a detection unit further includes: acquiring a second signal generated by a second detection unit when improper mounting of a main unit is detected; and the performing a preset operation based on the parameter further includes: based on the second signal, controlling to automatically generate second prompt information and/or stop milk expressing.

Optionally, the acquiring a detection parameter detected by a detection unit includes: acquiring a third signal generated when a diaphragm is not properly mounted; and the performing a preset operation based on the parameter further includes: based on the third signal, controlling the breast pump to operate.

Optionally, the acquiring a detection parameter detected by a detection unit includes: acquiring feature triggering information associated with a triggering status of a detection element; and the performing a preset operation based on the parameter includes: determining current breast milk volume information based on the feature triggering information and a preset relationship, where the preset relationship is used to represent a mapping relationship and/or a function relationship between feature triggering information and breast milk volume information.

Optionally, before the step of acquiring feature triggering information associated with a triggering status of a detection element, the method further includes: detecting an operating parameter of the detection element when the detection element senses breast milk in a milk storage container portion; and determining, when an operating parameter of any detection element is greater than or equal to a preset trigger threshold, that the detection element is triggered.

Optionally, when the detection element is a capacitive sensor, the operating parameter is a capacitance of the detection element, and the preset trigger threshold is a preset capacitance threshold.

Optionally, the acquiring a detection parameter detected by a detection unit includes: measuring a volume of breast milk expressed by the breast pump in at least a single expressing operation; and the performing a preset operation based on the parameter includes: determining a current breast milk volume expressed by the breast pump based on a total of the volumes of breast milk expressed by the breast pump in at least respective single expressing operations.

Optionally, the volume of the breast milk expressed by the breast pump in at least a single expressing operation is a volume of breast milk that is obtained by the breast pump in a single expressing operation and that is received between two adjacent expressing operations of a negative pressure mechanism of the breast pump.

Optionally, the data information of the breast milk volume includes liquid level data information of the milk storage container portion, and the acquiring a detection parameter detected by a detection unit includes: acquiring liquid level data from a liquid level detection unit, where the liquid level detection unit includes at least one through-beam sensor; and the performing a preset operation based on the parameter includes determining a breast milk volume in the milk storage container portion based on the liquid level data.

Optionally, the data information of the breast milk volume further includes tilting data of the milk storage container portion, and the acquiring a parameter related to the breast pump includes: acquiring tilting data from an angle detection unit; and the performing a preset operation based on the parameter includes: determining the breast milk volume in the milk storage container portion based on the liquid level data and the tilting data.

Optionally, the acquiring a detection parameter detected by a detection unit includes: determining to enter a breast milk volume measurement procedure when a tilting angle and a motion status of the milk storage container portion meet a breast milk volume measurement condition; and acquiring breast milk volume measurement reference data, where the breast milk volume measurement reference data includes the liquid level data and/or the tilting data of the milk storage container portion; and the performing a preset operation based on the parameter includes: determining the breast milk volume in the milk storage container portion based on the breast milk volume measurement reference data.

Optionally, the data information of the volume of the expressed breast milk includes a detection signal used to represent an intensity of light reflected during breast milk expressing; the acquiring a parameter related to the breast pump includes: acquiring the detection signal; and the performing a preset operation based on the parameter includes: determining a breast milk flow rate based on the light intensity corresponding to the detection signal.

Optionally, the determining a breast milk flow rate based on the light intensity corresponding to the detection signal includes: determining a breast milk flow rate corresponding to the light intensity based on the light intensity corresponding to the detection signal and a preset mapping relationship between a light intensity and a breast milk flow rate.

Optionally, the method further includes: determining a current breast milk volume in the breast pump based on the breast milk flow rate and a current milk expressing duration.

Optionally, the determining a current breast milk volume in the breast pump based on the breast milk flow rate and a current milk expressing duration includes: counting milk expressing durations having a same breast milk flow rate; determining a plurality of breast milk volumes based on each same breast milk flow rate and a corresponding milk expressing duration; and using a total of the plurality of breast milk volumes as the current breast milk volume in the breast pump.

Optionally, the acquiring a detection parameter detected by a detection unit includes: acquiring breast impedance data and/or breast hardness data; and the performing a preset operation based on the detection parameter includes: controlling the breast pump to operate in different modes based on the breast impedance data and/or the breast hardness data.

Optionally, the acquiring a detection parameter detected by a detection unit includes: acquiring the data information of the breast milk volume after breast milk enters the milk storage container portion; and the performing a preset operation based on the detection parameter includes: determining breast milk volume data of the milk storage container portion based on the data information of the breast milk volume.

Optionally, the data information of the breast milk volume is one or more of distance data, capacitance data, pressure data, or air pressure data.

Optionally, before the step of acquiring detection data of the milk storage container portion after breast milk enters the milk storage container portion, the method further includes a following step: calibrating the detection data.

Optionally, the step of acquiring detection data of the milk storage container portion after breast milk enters the milk storage container portion includes a following step: acquiring two or more pieces of detection data at a same time point after breast milk enters the milk storage container portion.

Optionally, the parameter related to the breast pump further includes a control parameter that includes one or more of following: a volume of breast milk expressed by the breast pump in a single operation, a user parameter, indication information indicating whether the breast milk stored in the breast pump is expressed from a left breast or a right breast, preset user parameter information, a startup signal, a volume of the breast milk stored in the milk storage container portion, current milk expressing flow rate data information, a current milk expressing status, a signal indicating whether a component of the breast pump is properly mounted, weaning indication information, a weaning parameter, breast status data, or breast milk flow rate status data; and the performing a preset operation based on the parameter includes: performing the preset operation based on the control parameter.

Optionally, the acquiring a control parameter includes: measuring a volume of breast milk expressed by the breast pump in at least a single expressing operation; and the performing the preset operation based on the control parameter includes: controlling a processing task of the breast pump based on a volume of breast milk obtained in at least a single expressing operation within a preset time, where the controlling a processing task of the breast pump includes adjusting an operating parameter of the negative pressure mechanism and/or adjusting an operating mode of the breast pump.

Optionally, the acquiring a control parameter includes: acquiring the user parameter, where the user parameter includes a pressure tolerance; and the performing the preset operation based on the control parameter includes: determining an operating parameter of the breast pump during operation based on the user parameter, where the operating parameter includes a milk expressing parameter that includes a milk expressing intensity and a milk expressing duration, where the milk expressing intensity is positively correlated with the pressure tolerance, and the milk expressing duration is negatively correlated with the pressure tolerance; and controlling the breast pump to operate based on the operating parameter.

Optionally, the operating parameter further includes at least one of a milk expressing frequency or a lactation stimulation parameter, and the determining an operating parameter of the breast pump during operation based on the user parameter includes: determining the milk expressing frequency of the breast pump during operation based on the pressure tolerance, where the pressure tolerance is positively correlated with the milk expressing frequency; or determining the operating parameter of the breast pump during operation based on the lactation stimulation parameter.

Optionally, the determining an operating parameter of the breast pump during operation based on the user parameter includes: acquiring a reference operating parameter; determining a deviation operating parameter based on the user parameter; and determining the operating parameter of the breast pump during operation based on the reference operating parameter and the deviation operating parameter.

Optionally, the acquiring a control parameter includes: acquiring indication information; and the performing the preset operation based on the control parameter includes: controlling display states of a first display region and a second display region based on the indication information, where the first display region is controlled to display a first display state and the second display region is controlled to display a second display state when the breast pump operates on the right breast, and the first display region is controlled to display a third display state and the second display region is controlled to display a fourth display state when the breast pump operates on the left breast, where the first display state is different from the third display state, and the second display state is different from the fourth display state.

Optionally, the acquiring indication information includes: acquiring the indication information sent by a terminal in communication with the breast pump; or the acquiring indication information includes: acquiring detection information detected by a detection sensor, where the detection sensor is configured to detect an operating position of the breast pump; and determining the indication information based on the detection information.

Optionally, when the first display region is located on a right side of the second display region, the controlling display states of a first display region and a second display region based on the indication information includes: controlling the first display region to shrink and the second display region to expand when it is determined based on the indication information that the breast pump operates on the right breast, causing the first display region to form the first display state and the second display region to form the second display state; and controlling the first display region to expand and the second display region to shrink when it is determined based on the indication information that the breast pump operates on the left breast, causing the first display region to form the third display state and the second display region to form the fourth display state.

Optionally, when the first display region is located above the second display region, the controlling display states of a first display region and a second display region based on the indication information includes: controlling the first display region to display the third color and the second display region to display the fourth color when it is determined based on the indication information that the breast pump operates on the right breast, causing the first display region to form the first display state and the second display region to form the second display state; and controlling the first display region to display the fourth color and the second display region to display the third color when it is determined based on the indication information that the breast pump operates on the left breast, causing the first display region to form the third display state and the second display region to form the fourth display state.

Optionally, the acquiring a control parameter includes: receiving the preset user parameter information of a current user; and the performing the preset operation based on the control parameter includes: inputting the preset user parameter information into a milk expression control model for matching, to obtain an initial milk expression control mode; and outputting initial control data information based on the initial milk expression control mode, to drive, based on the initial control data information, a target breast pump to operate.

Optionally, the method further includes: receiving historical milk expressing data information of the current user; generating a target milk expression control mode based on the historical milk expressing data information and the initial milk expression control mode; and outputting target control data information based on the target milk expression control mode, to drive, based on the target control data information, the target breast pump to operate.

Optionally, the historical milk expressing data information is data information collected in the initial milk expression control mode; and the generating a target milk expression control mode based on the historical milk expressing data information and the initial milk expression control mode includes: acquiring an average volume of expressed milk based on a plurality of pieces of historical milk expressing data information; and determining whether the average volume of expressed milk is within or greater than a milk expressing volume range corresponding to the initial milk expression control mode; and determining that the target milk expression control mode is the same as the initial milk expression control mode if the average volume of expressed milk is within or greater than the milk expressing volume range corresponding to the initial milk expression control mode; or modifying the initial milk expression control mode based on a preset adjustment mechanism to generate the target milk expression control mode.

Optionally, the preset user parameter information includes one or more of age data information, breast size data information, usage season information, or usage region information; and the preset user parameter information is received by a device through manual input or selection performed by the current user on the device.

Optionally, the acquiring a control parameter includes: generating a corresponding startup signal in response to a startup instruction input by a user or based on the detection data of the detection sensor; and the performing the preset operation based on the control parameter includes: starting, based on the startup signal, a stimulation unit to operate in a preset mode, where the preset mode includes at least a first mode for stimulating the breast to heat up.

Optionally, the starting, based on the startup signal, a stimulation unit to operate in a preset mode includes: controlling an infrared generator of the stimulation unit to start operating to enter the first mode; or controlling the stimulation unit to enter a second mode, where in the second mode, the infrared generator stops operating, and a massage assembly of the stimulation unit starts operating to massage the breast; or controlling the stimulation unit to enter a third mode, where in the third mode, the infrared generator and the massage assembly of the stimulation unit start to operate to massage the breast while stimulating the breast to heat up.

Optionally, the breast pump further includes a flow rate sensor configured to detect a flow rate during breast milk expressing by the breast pump, and the generating a corresponding startup signal based on detection data of the detection sensor includes: generating a first startup signal when the flow rate is less than a first flow rate threshold and greater than or equal to a second flow rate threshold; generating a second startup signal when the flow rate is less than the second flow rate threshold and greater than or equal to a third flow rate threshold; or generating, by the flow rate sensor, a third startup signal when the flow rate is less than the third flow rate threshold; and the starting, based on the startup signal, a stimulation unit to operate in a preset mode includes: controlling, based on the first startup signal, an infrared generator of the stimulation unit to start operating to enter the first mode; controlling, based on the second startup signal, the stimulation unit to enter a second mode, where in the second mode, the infrared generator stops operating, and a massage assembly of the stimulation unit starts operating to massage the breast; or controlling, based on the third startup signal, the stimulation unit to enter a third mode, where in the second mode, the infrared generator and the massage assembly of the stimulation unit start to operate to massage the breast while stimulating the breast to heat up.

Optionally, after the starting, based on the startup signal, a stimulation unit to operate in a preset mode, the method further includes:

acquiring a continuous operating duration of the stimulation unit; determining whether the continuous operating duration is greater than a preset duration threshold; and controlling, after it is determined that the continuous operating duration is greater than the preset duration threshold, an infrared generator and/or a massage assembly of the stimulation unit in an operating state to stop operating, and starting the negative pressure mechanism of the breast pump to perform milk expressing.

Optionally, after the starting, based on the startup signal, a stimulation unit to operate in a preset mode, the method further includes: controlling a temperature sensor to detect a temperature of a breast surface; determining whether the temperature is greater than a preset temperature threshold; and controlling, after it is determined that the temperature of the breast surface is greater than the preset temperature threshold, an infrared generator and/or a massage assembly of the stimulation unit in an operating state to stop operating, and starting the negative pressure mechanism of the breast pump to perform milk expressing.

Optionally, the acquiring a control parameter includes: controlling a first measurement unit to measure a liquid level of the milk storage container portion; controlling a second measurement unit to measure a tilting angle of the milk storage container portion; and determining the volume of the breast milk stored in the milk storage container portion based on the liquid level and the tilting angle; and the performing the preset operation based on the control parameter includes: performing the preset operation based on the volume of the breast milk stored in the milk storage container portion.

Optionally, the determining the volume of the breast milk stored in the milk storage container portion based on the liquid level and the tilting angle includes: determining a first volume of the breast milk stored in the milk storage container portion based on the liquid level; determining a correction parameter based on the liquid level and the tilting angle; and correcting the first volume based on the correction parameter, to obtain a second volume of the breast milk stored in the milk storage container portion.

Optionally, the determining the volume of the breast milk stored in the milk storage container portion based on the liquid level and the tilting angle includes: acquiring a calculation formula, where the calculation formula is determined based on a capacity and a shape of the milk storage container portion; and determining the volume of the breast milk stored in the milk storage container portion based on the liquid level, the tilting angle, and the calculation formula.

Optionally, the method further includes: controlling the second measurement unit to measure a tilting direction of the milk storage container portion; and the determining the volume of the breast milk stored in the milk storage container portion based on the liquid level and the tilting angle includes: determining the volume of the breast milk stored in the milk storage container portion based on the liquid level, the tilting angle, and the tilting direction.

Optionally, the determining the volume of the breast milk stored in the milk storage container portion based on the liquid level, the tilting angle, and the tilting direction includes: determining a region in a first plane of the tilting direction; determining a calculation formula based on the region in the first plane of the tilting direction; and determining the volume of the breast milk stored in the milk storage container portion based on the liquid level, the tilting angle, and the calculation formula.

Optionally, the determining the volume of the breast milk stored in the milk storage container portion based on the liquid level, the tilting angle, and the tilting direction includes: determining a region in a first plane of the tilting direction; determining a correspondence table based on the region in the first plane of the tilting direction; searching the correspondence table for a correction parameter based on the liquid level and the tilting angle; determining a first volume of the breast milk stored in the milk storage container portion based on the liquid level; and correcting the first volume based on the correction parameter, to obtain a second volume of the breast milk stored in the milk storage container portion.

Optionally, the acquiring a control parameter includes: acquiring, in real time, a current milk expressing flow rate of the breast pump driven at a current milk expressing frequency; and the performing the preset operation based on the control parameter includes: determining whether the current milk expressing flow rate is less than a current preset flow rate threshold; and stopping, when it is determined that the current milk expressing flow rate is less than the current preset flow rate threshold, driving the breast pump at the current milk expressing frequency, and outputting a lactation stimulation and milk expression-mixed control mode to drive the breast pump based on the current milk expressing flow rate data information, causing the breast pump to perform lactation stimulation and milk expression on the user; or stopping, when it is determined that the current milk expressing flow rate is greater than or equal to the current preset flow rate threshold, driving the breast pump at the current milk expressing frequency, and outputting a target milk expressing frequency based on a preset milk expressing frequency adjustment manner to drive the breast pump to operate.

Optionally, the outputting a target milk expressing frequency based on a preset milk expressing frequency adjustment manner to drive the breast pump to operate includes: setting a milk expressing frequency adjustment factor; calculating an adjusted milk expressing frequency based on the milk expressing frequency adjustment factor and the current milk expressing frequency; updating the current milk expressing frequency to the adjusted milk expressing frequency and applying the adjusted milk expressing frequency to the breast pump to obtain adjusted milk expressing flow rate data information; continuously updating the current milk expressing frequency to obtain a plurality of pieces of adjusted milk expressing flow rate data information; and acquiring a current milk expressing frequency as the target milk expressing frequency if the adjusted milk expressing flow rate data information no longer increases with adjustment of the current milk expressing frequency.

Optionally, the lactation stimulation and milk expression-mixed control mode includes a plurality of levels of lactation stimulation and milk expression-mixed control modes; and the outputting a lactation stimulation and milk expression-mixed control mode to drive the breast pump based on the current milk expressing flow rate data information, causing the breast pump to perform lactation stimulation and milk expression on the user includes: acquiring a lactation stimulation and milk expression-mixed control mode of a corresponding level based on the current milk expressing flow rate data information; and driving the breast pump based on the lactation stimulation and milk expression-mixed control mode of the corresponding level, causing the breast pump to perform lactation stimulation and milk expression on the user.

Optionally, the acquiring a control parameter includes: determining a current milk expressing status of the user; and the performing the preset operation based on the control parameter includes: generating a mode adjustment signal based on the current milk expressing status and a preset milk expressing status of the user, to adjust a milk expressing mode of the breast pump.

Optionally, the determining a current milk expressing status of the user includes: determining the current milk expressing status of the user based on a milk expressing flow rate and breast feature information of the user, where the breast feature information includes at least one of following: breast plumpness; breast composition; or breast hardness.

Optionally, the generating a mode adjustment signal based on the current milk expressing status and a preset milk expressing status of the user, to adjust a milk expressing mode of the breast pump includes: determining a breast milk volume status based on milk expressing efficiency corresponding to the current milk expressing status of the user and milk expressing efficiency corresponding to the preset milk expressing status; and determining the mode adjustment signal based on the breast milk volume status.

Optionally, the method includes a following step: the acquiring a control parameter includes: acquiring a first signal generated when it is detected that a breast shield is not attached to the breast; and the performing the preset operation based on the control parameter includes: controlling the negative pressure mechanism to stop operating and/or generating prompt information and/or turning off the breast pump based on the first signal.

Optionally, the acquiring a control parameter includes: determining an operating mode of the breast pump when an operation instruction is received; acquiring a number of completed weaning days when the operating mode is a weaning mode, where the weaning mode is used to assist a mother in ceasing milk secretion; and the performing the preset operation based on the control parameter includes: determining an operating parameter based on the number of completed weaning days; and performing milk expressing based on the operating parameter.

Optionally, the determining an operating parameter based on the number of completed weaning days includes: determining a total number of completed milk expressing operations and/or a volume of breast milk obtained in a single expressing operation in a day based on the number of completed weaning days.

Optionally, the performing milk expressing based on the operating parameter includes: acquiring a number of completed milk expressing operations in the day; and performing milk expressing based on the volume of breast milk obtained in a single expressing operation when the number of completed milk expressing operations in the day is less than the total number of completed milk expressing operations.

Optionally, the acquiring a control parameter includes: receiving weaning indication information; and the performing the preset operation based on the control parameter includes: operating in the weaning mode based on the weaning indication information.

Optionally, the weaning indication information is triggered and generated by a triggering unit, or is sent by the terminal in communication with the breast pump.

Optionally, the method further includes: receiving configuration information; and configuring a mode parameter of the weaning mode based on the configuration information, where the mode parameter includes a total number of weaning days and/or a daily operating parameter.

Optionally, the daily operating parameter includes at least one of a daily volume of expressed breast milk, a volume of breast milk obtained in a single expressing operation, or a daily number of milk expressing operations.

Optionally, the acquiring a control parameter includes: acquiring breast status data and/or breast milk flow rate status data; and the performing the preset operation based on the control parameter includes: determining a breast state based on the breast status data and/or the breast milk flow rate status data; and starting a proper milk expressing procedure based on the breast state.

Optionally, a step of acquiring a breast state includes: acquiring breast impedance data; comparing the breast impedance data with an impedance threshold; and determining a breast state, where if the breast impedance data is less than the impedance threshold, it is determined that the breast state is milk-sufficiency, or if the breast impedance data is greater than or equal to the impedance threshold, it is determined that the breast state is milk-deficiency.

Optionally, a step of acquiring a breast milk flow rate state includes: acquiring breast milk flow rate data in a fluid pathway of the breast pump; comparing a breast milk flow rate with a flow rate threshold; and determining a breast milk flow rate state, where if the breast milk flow rate is greater than or equal to a first flow rate threshold and less than a second flow rate threshold, it is determined that the breast milk flow rate state is normal; if the breast milk flow rate is less than or equal to the first flow rate threshold, it is determined that the breast milk flow rate state is low; or if the breast milk flow rate is greater than or equal to the second flow rate threshold, it is determined that the breast milk flow rate state is high.

Optionally, the starting a proper milk expressing procedure includes initiating a stimulation mode, initiating a breast unclogging mode, initiating an expressing intensity matching mode, or initiating a relief mode.

The present disclosure provides a breast pump, including: at least one processor; and a memory, communicatively connected to the at least one processor, where the memory stores instructions executable by the at least one processor, where the instructions are executed by the at least one processor, causing the at least one processor to perform the method in the above implementations.

The present disclosure provides a breast pump, including a breast milk collection portion, a milk storage chamber, and a negative pressure mechanism, and further including at least one detector and a control unit, where one end of the breast milk collection portion is attached to a breast, and another end of the breast milk collection portion communicates with the milk storage chamber through a nipple channel; the milk storage chamber is configured to store breast milk collected by the breast milk collection portion; the negative pressure mechanism is configured to directly or indirectly apply negative pressure to the nipple channel or the milk storage chamber, causing breast milk to be expressed to the milk storage chamber for storage; the detector is configured to acquire a detection parameter; the control unit is configured to perform an operation based on the detection parameter; and the detection parameter includes at least one of following: a signal indicating whether a component of the breast pump is properly mounted, data on a volume of breast milk expressed by the breast milk collection portion in a single expressing operation, breast milk flow rate data, a current milk expressing status, data information of a volume of expressed breast milk, breast impedance data, breast hardness data, a user parameter, indication information indicating whether the breast milk stored in the breast pump is expressed from a left breast or a right breast, preset user parameter information, a startup signal, weaning indication information, a weaning parameter, breast status data, breast milk flow rate status data, preset breast pump operating parameter information, or milk expressing status data.

Optionally, the detector includes a breast milk flow rate detector configured to detect a flow rate of the breast milk along a flow path in the breast pump.

Optionally, an inflow channel for breast milk circulation is provided between the breast milk collection portion and the milk storage chamber, causing the breast milk to flow from the breast milk collection portion into the milk storage chamber; and the detector includes a breast milk flow rate detector configured to detect a breast milk flow rate on the inflow channel.

Optionally, the inflow channel includes a check valve arranged between the breast milk collection portion and the milk storage chamber, and the breast milk flow rate detector is arranged on the check valve to detect a flow rate of breast milk passing through the check valve.

Optionally, the check valve includes a first valve portion and a second valve portion communicated with the first valve portion, where the first valve portion has a first opening communicated with the nipple channel and a second opening communicated with the second valve portion, where the first valve portion of the check valve is configured to collect a breast milk flow of a milk expressing channel through the first opening, and allow breast milk with a predetermined outflow characteristic to flow into the second valve portion through the second opening; and a flow rate detection element is arranged between the first valve portion and the second valve portion, and is configured to detect a flow rate of the breast milk with the predetermined outflow characteristic.

Optionally, the first valve portion and the second valve portion are integrally formed, and are combined into a figure-8-shaped structure.

Optionally, the flow rate detection element includes a gravity sensor arranged on the second valve portion and configured to detect a change in gravity of the second valve portion to determine the flow rate of the breast milk with the predetermined outflow characteristic.

Optionally, the control unit is further configured to: acquire, in real time, a current milk expressing flow rate of the breast pump driven at a current milk expressing frequency; stop, when the current milk expressing flow rate is less than a current preset flow rate threshold, driving the breast pump at the current milk expressing frequency, and output a lactation stimulation and milk expression-mixed control mode to drive the breast pump based on the current milk expressing flow rate, causing the breast pump to perform lactation stimulation and milk expression on a user; or stop, when the current milk expressing flow rate is greater than or equal to the current preset flow rate threshold, driving the breast pump at the current milk expressing frequency, and output a target milk expressing frequency based on a preset milk expressing frequency adjustment manner to drive the breast pump to operate.

Optionally, the detector further includes a breast milk volume detector configured to detect data information of a volume of acquired breast milk expressed by the breast pump, and the control unit is configured to determine a breast milk volume based on the data information of the breast milk volume.

Optionally, the breast milk volume detector includes a liquid level detector configured to detect a liquid level of the milk storage chamber; and the control unit is configured to determine a breast milk volume in the milk storage chamber based on at least liquid level data from the liquid level detector.

Optionally, the breast milk flow rate detector further includes an angle detector configured to detect tilting data of the milk storage chamber; and the control unit is configured to determine the breast milk volume in the milk storage chamber based on the liquid level data from the liquid level detector and the tilting data from the angle detector.

Optionally, the angle detector is a gyroscope or a three-axis acceleration sensor.

Optionally, the detector further includes: an impedance detector configured to detect breast impedance data; and/or a hardness detector configured to detect breast hardness data, where the control unit is configured to control the breast pump to operate in different modes based on the breast impedance data and/or the breast hardness data.

Optionally, the breast pump further includes a prompt module configured to generate prompt information based on impedance data information or hardness data information.

The present disclosure provides a method for a breast pump, including: acquiring a parameter related to the breast pump, where the parameter related to the breast pump includes at least a detection parameter detected by a detector and/or a control parameter acquired by a control unit; and performing a preset operation based on the detection parameter and/or the control parameter, where the detection parameter and/or the control parameter includes one or more of following: a signal indicating whether a component of the breast pump is properly mounted, data on a volume of breast milk expressed by the breast milk collection portion obtained in a single expressing operation, breast milk flow rate data, a current milk expressing status, data information of a volume of expressed breast milk, breast impedance data, breast hardness data, a user parameter, indication information indicating whether the breast milk stored in the breast pump is expressed from a left breast or a right breast, preset user parameter information, a startup signal, weaning indication information, a weaning parameter, breast status data, breast milk flow rate status data, or milk expressing status data.

Optionally, the data information of the breast milk volume includes liquid level data information of a milk storage chamber, and the acquiring a detection parameter detected by a detector includes: determining a breast milk volume in the milk storage chamber based on the liquid level data; and the data information of the breast milk volume further includes tilting data of the milk storage chamber, and the acquiring a parameter related to the breast pump includes: acquiring tilting data from an angle detector; and the performing a preset operation based on the parameter includes: determining the breast milk volume in the milk storage chamber based on the liquid level data and the tilting data.

Optionally, the acquiring a detection parameter detected by a detector includes: determining to enter a breast milk volume measurement procedure when a tilting angle and a motion status of the milk storage chamber meet a breast milk volume measurement condition; and acquiring breast milk volume measurement reference data, where the breast milk volume measurement reference data includes the liquid level data and/or the tilting data of the milk storage chamber; and the performing a preset operation based on the parameter includes: determining the breast milk volume in the milk storage chamber based on the breast milk volume measurement reference data.

Optionally, the step of determining to enter a breast milk volume measurement procedure includes a following step: determining a vibration status of the breast pump and/or determining a tilting status of the breast pump.

Optionally, the acquiring a detection parameter detected by a detector includes: acquiring breast impedance data and/or breast hardness data; and the performing a preset operation based on the detection parameter includes: controlling the breast pump to operate in different modes based on the breast impedance data and/or the breast hardness data.

Optionally, includes: acquiring a breast pump parameter; and acquiring an initial milk expression control mode based on the breast pump parameter.

Optionally, controlling the breast pump to operate based on the initial milk expression control mode.

Optionally, generating a target milk expression control mode based on the historical milk expressing data information and the initial milk expression control mode; and outputting target control data information based on the target milk expression control mode, to drive, based on the target control data information, the target breast pump to operate.

Optionally, receiving historical milk expressing data information of a current user; generating a target milk expression control mode based on the historical milk expressing data information and the initial milk expression control mode; and outputting target control data information based on the target milk expression control mode, to drive, based on the target control data information, the target breast pump to operate.

Optionally, the historical milk expressing data information is data information collected in the initial milk expression control mode; and the generating a target milk expression control mode based on the historical milk expressing data information and the initial milk expression control mode includes: acquiring an average volume of expressed milk based on a plurality of pieces of historical milk expressing data information; and determining whether the average volume of expressed milk is within or greater than a milk expressing volume range corresponding to the initial milk expression control mode; and determining that the target milk expression control mode is the same as the initial milk expression control mode if the average volume of expressed milk is within or greater than the milk expressing volume range corresponding to the initial milk expression control mode; or modifying the initial milk expression control mode based on a preset adjustment mechanism to generate the target milk expression control mode.

Optionally, the preset user parameter information includes one or more of age data information, breast size data information, usage season information, or usage region information; and the preset user parameter information is received by a device through manual input or selection performed by the current user on the device.

Optionally, the acquiring a breast pump parameter includes: obtaining a liquid level in the milk storage chamber measured by a first measurement unit; obtaining a tilting angle in the milk storage chamber measured by a second measurement unit; and determining a volume of breast milk stored in the milk storage chamber based on the liquid level and the tilting angle.

Optionally, the determining a volume of breast milk stored in the milk storage chamber based on the liquid level and the tilting angle includes: determining a first volume of the breast milk stored in the milk storage chamber based on the liquid level; determining a correction parameter based on the liquid level and the tilting angle; and correcting the first volume based on the correction parameter, to obtain a second volume of the breast milk stored in the milk storage chamber.

Optionally, the determining a volume of breast milk stored in the milk storage chamber based on the liquid level and the tilting angle includes: acquiring a calculation formula, where the calculation formula is determined based on a capacity and a shape of the milk storage chamber; and determining the volume of the breast milk stored in the milk storage chamber based on the liquid level, the tilting angle, and the calculation formula.

Optionally, controlling the second measurement unit to measure a tilting direction of the milk storage chamber, where the determining a volume of breast milk stored in the milk storage chamber based on the liquid level and the tilting angle includes: determining the volume of the breast milk stored in the milk storage chamber based on the liquid level, the tilting angle, and the tilting direction.

Optionally, the determining the volume of the breast milk stored in the milk storage chamber based on the liquid level, the tilting angle, and the tilting direction includes: determining a region in a first plane of the tilting direction; determining a calculation formula based on the region in the first plane of the tilting direction; and determining the volume of the breast milk stored in the milk storage chamber based on the liquid level, the tilting angle, and the calculation formula.

Optionally, the determining the volume of the breast milk stored in the milk storage chamber based on the liquid level, the tilting angle, and the tilting direction includes: determining a region in a first plane of the tilting direction; determining a correspondence table based on the region in the first plane of the tilting direction; searching the correspondence table for a correction parameter based on the liquid level and the tilting angle; determining a first volume of the breast milk stored in the milk storage chamber based on the liquid level; and correcting the first volume based on the correction parameter, to obtain a second volume of the breast milk stored in the milk storage chamber.

Optionally, the acquiring a detection parameter includes: acquiring current milk expressing flow rate data of the breast pump in real time; and the performing a preset operation based on the detection parameter includes: stopping, when the current milk expressing flow rate is less than a current preset flow rate threshold, driving the breast pump at a current milk expressing frequency, and outputting a lactation stimulation and milk expression-mixed control mode to drive the breast pump based on current milk expressing flow rate data information, causing the breast pump to perform lactation stimulation and milk expression on the user; or stopping, when the current milk expressing flow rate is greater than or equal to the current preset flow rate threshold, driving the breast pump at the current milk expressing frequency, and outputting a target milk expressing frequency based on a preset milk expressing frequency adjustment manner to drive the breast pump to operate.

Optionally, the outputting a target milk expressing frequency based on a preset milk expressing frequency adjustment manner to drive the breast pump to operate includes: setting a milk expressing frequency adjustment factor; calculating an adjusted milk expressing frequency based on the milk expressing frequency adjustment factor and the current milk expressing frequency; updating the current milk expressing frequency to the adjusted milk expressing frequency and applying the adjusted milk expressing frequency to the breast pump to obtain adjusted milk expressing flow rate data information; continuously updating the current milk expressing frequency to obtain a plurality of pieces of adjusted milk expressing flow rate data information; and acquiring a current milk expressing frequency as the target milk expressing frequency if the adjusted milk expressing flow rate data information no longer increases with adjustment of the current milk expressing frequency.

Optionally, the lactation stimulation and milk expression-mixed control mode includes a plurality of levels of lactation stimulation and milk expression-mixed control modes; and the outputting a lactation stimulation and milk expression-mixed control mode to drive the breast pump based on the current milk expressing flow rate data information, causing the breast pump to perform lactation stimulation and milk expression on the user includes: acquiring a lactation stimulation and milk expression-mixed control mode of a corresponding level based on the current milk expressing flow rate data information; and driving the breast pump based on the lactation stimulation and milk expression-mixed control mode of the corresponding level, causing the breast pump to perform lactation stimulation and milk expression on the user.

Optionally, the acquiring a control parameter includes: determining current milk expressing status data of the user; and the performing a preset operation based on the control parameter includes: generating a mode adjustment signal based on the current milk expressing status data and preset milk expressing status data of the user, to adjust a control mode of the breast pump.

Optionally, the determining a current milk expressing status of the user includes: determining the current milk expressing status of the user based on a breast milk flow rate state and breast status data, where the breast status data includes at least one of following: breast plumpness; breast composition; or breast hardness.

Optionally, the generating a mode adjustment signal based on the current milk expressing status and a preset milk expressing status of the user, to adjust a milk expressing mode of the breast pump includes: determining a breast milk volume status based on milk expressing efficiency corresponding to the current milk expressing status of the user and milk expressing efficiency corresponding to the preset milk expressing status; and determining the mode adjustment signal based on the breast milk volume status.

Optionally, the acquiring a control parameter includes: acquiring breast status data and/or breast milk flow rate status data; and the performing a preset operation based on the control parameter includes: determining a breast pump operating mode and/or a milk expressing procedure based on the breast status data and/or the breast milk flow rate status data.

Optionally, the performing a preset operation based on the control parameter includes: determining a milk expressing status based on the breast status data and/or the breast milk flow rate status data; and determining the breast pump operating mode and/or the milk expressing procedure based on the milk expressing status.

Optionally, the step of acquiring breast status data includes: acquiring breast impedance data; comparing the breast impedance data with an impedance threshold; and determining a breast state, where if the breast impedance data is less than the impedance threshold, it is determined that the breast state is milk-sufficiency, or if the breast impedance data is greater than or equal to the impedance threshold, it is determined that the breast state is milk-deficiency.

Optionally, the step of acquiring breast milk flow rate status data includes: acquiring breast milk flow rate data in the breast milk flow rate detector, where if a breast milk flow rate is greater than or equal to a first flow rate threshold and less than a second flow rate threshold, it is determined that a breast milk flow rate state is normal; if the breast milk flow rate is less than or equal to the first flow rate threshold, it is determined that the breast milk flow rate state is low; or if the breast milk flow rate is greater than or equal to the second flow rate threshold, it is determined that the breast milk flow rate state is high.

Optionally, the starting a proper milk expressing procedure includes initiating a stimulation mode, initiating a breast unclogging mode, initiating an expressing intensity matching mode, or initiating a relief mode.

The present disclosure provides a computer-readable storage medium, storing an executable program, where the executable program is executed by a processor to implement the method in the above implementations.

This application achieves following beneficial effects:
(1) Problems such as breast milk leakage caused by improper mounting of breast pump accessories are reduced.
(2) j
(3) Breast milk volume detection is achieved.
(4) Breast milk volume detection accuracy is improved.
(5) Breast milk flow rate monitoring solutions are provided.
(6) Intelligent control or switching is achieved.
(7) Users are allowed to customize expressing data based on personal needs or preferences.
(8) Breast state monitoring is achieved.
(8) The detection unit and the control unit are utilized to perform intelligent control on the breast pump, to resolve at least one of problems of breast milk spillage, limited functionality, and improper mounting through intelligent control during use of the breast pump.

### BRIEF DESCRIPTION OF THE DRAWINGS

FIG. 1 is a schematic structural diagram of a breast pump according to an embodiment of the present disclosure;
FIG. 2 is a schematic structural diagram of a breast pump according to an embodiment of the present disclosure;
FIG. 3 is another schematic structural diagram of a breast pump according to an embodiment of the present disclosure;
FIG. 4 is another schematic structural diagram of a breast pump according to an embodiment of the present disclosure;
FIG. 5 is another schematic structural diagram of a breast pump according to an embodiment of the present disclosure;
FIG. 6 is another schematic structural diagram of a breast pump according to an embodiment of the present disclosure;
FIG. 7 is a schematic diagram of a control principle of a detection method according to an embodiment of the present disclosure;
FIG. 8 is a schematic diagram of a control method of the detection method according to an embodiment of the present disclosure;
FIG. 9 is another schematic diagram of a control method of the detection method according to an embodiment of the present disclosure;
FIG. 10 is an application environment diagram according to an embodiment of this application;
FIG. 11 is an application environment diagram according to an embodiment of this application;
FIG. 12 is a schematic flowchart of a detection method;
FIG. 13 is a schematic flowchart of steps of triggering a detection element according to an embodiment of this application;
FIG. 14 is a schematic flowchart of steps of triggering a detection element according to an embodiment of this application;
FIG. 15 is a schematic flowchart of steps of calculating breast milk volume information according to an embodiment of this application;
FIG. 16 is a schematic flowchart of steps of calculating breast milk volume information according to an embodiment of this application;
FIG. 17 is a schematic flowchart of steps of calculating breast milk volume information according to an embodiment of this application;
FIG. 18 is a structural block diagram of a breast milk flow rate detection unit according to an embodiment of this application;
FIG. 19 is a structural block diagram according to an embodiment of this application;
FIG. 20 is a schematic structural diagram according to an embodiment of this application;
FIG. 21 is a flowchart of a detection method according to an embodiment of the present disclosure;
FIG. 22 is another flowchart of a detection method according to an embodiment of the present disclosure;
FIG. 23 is a flowchart of a control method according to an embodiment of the present disclosure;
FIG. 24 is a schematic structural diagram of a breast pump according to an embodiment of the present disclosure;
FIG. 25 is another schematic structural diagram of a breast pump according to an embodiment of the present disclosure;
FIG. 26 is a structural block diagram of a breast pump according to an embodiment of the present disclosure;
FIG. 27 is a schematic structural diagram of a breast pump according to an embodiment of this application;
FIG. 28 is a schematic structural diagram of a breast pump according to an embodiment of this application;
FIG. 29 is a schematic structural diagram of a terminal according to an embodiment of this application;
FIG. 30 is a schematic flowchart of a control method according to an embodiment of this application;
FIG. 31 is a schematic structural diagram of a breast pump according to this application;
FIG. 32 is a schematic diagram of a control interface of a breast pump according to an embodiment of this application;
FIG. 33 is a schematic diagram of a control interface of a breast pump according to an embodiment of this application;
FIG. 34 is a schematic diagram of a control interface of a breast pump according to an embodiment of this application;
FIG. 35 is a schematic diagram of a control interface of a breast pump according to an embodiment of this application;
FIG. 36 is a schematic diagram of a control interface of a breast pump according to an embodiment of this application;
FIG. 37 is a schematic diagram of a control interface of a breast pump according to an embodiment of this application;
FIG. 38 is a schematic diagram of a control interface of a breast pump according to an embodiment of this application;
FIG. 39 is a schematic structural diagram of a breast pump according to this application;
FIG. 40 is a schematic structural diagram of a terminal device according to an embodiment of this application;
FIG. 41 is a schematic structural diagram of a system according to an embodiment of this application;
FIG. 42 is a schematic flowchart of a control method according to an embodiment of this application;
FIG. 43 is a schematic flowchart of a specific embodiment of a control method according to an embodiment of the present disclosure;
FIG. 44 is a schematic flowchart of a specific embodiment of a control method according to an embodiment of the present disclosure;
FIG. 45 is a schematic flowchart of a substep of a fifth step in FIG. 44;
FIG. 46 is a schematic diagram of a breast shield of a breast pump according to the present disclosure;
FIG. 47 is a module diagram of a breast pump according to the present disclosure;
FIG. 48 is a schematic flowchart of a control method according to the present disclosure;
FIG. 49 is a schematic flowchart of a control method according to the present disclosure;
FIG. 50 is a schematic flowchart of a control method according to an embodiment of this application;
FIG. 51 is a schematic flowchart of a breast milk volume measurement method for a breast pump according to an embodiment of this application;
FIG. 52 is a schematic structural diagram of a milk storage container portion according to an embodiment of this application;
FIG. 53 is a schematic diagram of region division according to an embodiment of this application;
FIG. 54 is a schematic flowchart of a specific embodiment of a control method according to an embodiment of the present disclosure;
FIG. 55 is a schematic diagram of compositional structures of a specific embodiment of a breast pump according to an embodiment of the present disclosure;
FIG. 56 is a schematic diagram of a specific embodiment of a mounting position structure of the lactation stimulation assembly in a breast pump according to an embodiment of the present disclosure;
FIG. 57 is a flowchart of a control method according to an embodiment of this specification;
FIG. 58 is a flowchart of a method for generating a mode adjustment signal according to an embodiment of this specification;
FIG. 59 is a schematic structural diagram of an expressing assembly according to an embodiment of this specification;
FIG. 60 is a schematic structural exploded view of the expressing assembly in FIG. 59;
FIG. 61 is a structural diagram of a breast pump according to the present disclosure;
FIG. 62 is a structural diagram of a breast pump according to the present disclosure;
FIG. 63 is a structural diagram of a breast pump according to the present disclosure;
FIG. 64 is a schematic structural diagram of controlling an electronic component of a breast pump according to the present disclosure;
FIG. 65 is a flowchart of a method for controlling a breast pump to operate according to the present disclosure;
FIG. 66 is a flowchart of a method for controlling a breast pump to operate according to the present disclosure;
FIG. 67 is a schematic flowchart of a control method according to this application;
FIG. 68 is a schematic diagram of a relationship between a number of days in a weaning mode and a daily total volume of expressed breast milk according to an embodiment of this application;
FIG. 69 is a detailed schematic flowchart of a fourth step according to a twelfth embodiment;
FIG. 70 is a flowchart of a control method according to an embodiment of this application;
FIG. 71 is a schematic structural diagram of a system according to an embodiment of this application;
FIG. 72 is a schematic diagram of a first embodiment of a touch key according to an embodiment of this application;
FIG. 73 is a schematic diagram of a second embodiment of a touch key according to an embodiment of this application;
FIG. 74 is a schematic diagram of a control interface according to an embodiment of this application;
FIG. 75 is a schematic flowchart of a control method according to an embodiment of this application;
FIG. 76 is a three-dimensional diagram of a breast pump according to an embodiment of the present disclosure;
FIG. 77 is a cross-sectional view of a breast pump according to an embodiment of the present disclosure;
FIG. 78 is another structural cross-sectional view of a breast pump according to an embodiment of the present disclosure;
FIG. 79 is another structural cross-sectional view of a breast pump according to an embodiment of the present disclosure;
FIG. 80 is another structural cross-sectional view of a breast pump according to an embodiment of the present disclosure;
FIG. 81 is another structural cross-sectional view of a breast pump according to an embodiment of the present disclosure;
FIG. 82 is another structural cross-sectional view of a breast pump according to an embodiment of the present disclosure;
FIG. 83 is an exploded view of a breast pump according to an embodiment of the present disclosure;
FIG. 84 is an exploded view of a breast pump according to an embodiment of the present disclosure;
FIG. 85 is a schematic diagram of a breast pump according to an embodiment of the present disclosure;
FIG. 86 is a schematic diagram of a breast pump according to an embodiment of the present disclosure;
FIG. 87 is a cross-sectional view of a breast pump according to an embodiment of the present disclosure;
FIG. 88 is a cross-sectional view of a breast pump in a tilted state according to an embodiment of the present disclosure;
FIG. 89 is a diagram of a control module of a breast milk flow rate detection unit according to an embodiment of the present disclosure;
FIG. 90 is a schematic logic diagram according to an embodiment of the present disclosure;
FIG. 91 is a schematic logic diagram according to an embodiment of the present disclosure;
FIG. 92 is a logical schematic diagram according to an embodiment of the present disclosure;
FIG. 93 is a schematic logic diagram according to an embodiment of the present disclosure;
FIG. 94 is a schematic logic diagram according to an embodiment of the present disclosure;
FIG. 95 is a specific schematic structural diagram of a breast pump according to an embodiment of this specification;
FIG. 96 is a flowchart of a detection method according to an embodiment of this specification;
FIG. 97 is a flowchart of a method for determining a current breast milk volume in a breast pump according to an embodiment of this specification;
FIG. 98 is a flowchart of another method for determining a current breast milk volume in a breast pump according to an embodiment of this specification;
FIG. 99 is a three-dimensional schematic structural diagram of a breast pump according to an embodiment of the present disclosure;
FIG. 100 is a three-dimensional schematic structural diagram of a breast pump according to an embodiment of the present disclosure;
FIG. 101 is a cross-sectional view of a breast pump according to an embodiment of the present disclosure;
FIG. 102 is a cross-sectional view of an attachment portion of a breast massage device according to an embodiment of the present disclosure;
FIG. 103 is a diagram of a control module of the breast massage device according to an embodiment of the present disclosure;
FIG. 104 is a first logic diagram of a control method according to an embodiment of the present disclosure;
FIG. 105 is a second logic diagram of the control method according to an embodiment of the present disclosure;
FIG. 106 is a third logic diagram of the control method according to an embodiment of the present disclosure;
FIG. 107 is a fourth logic diagram of the control method according to an embodiment of the present disclosure;
FIG. 108 is a structural block diagram of a detection unit according to an embodiment of the present disclosure;
FIG. 109 is a schematic structural diagram of a wearable device according to an embodiment of the present disclosure;
FIG. 110 is a schematic structural diagram of a breast pump according to an embodiment of the present disclosure;

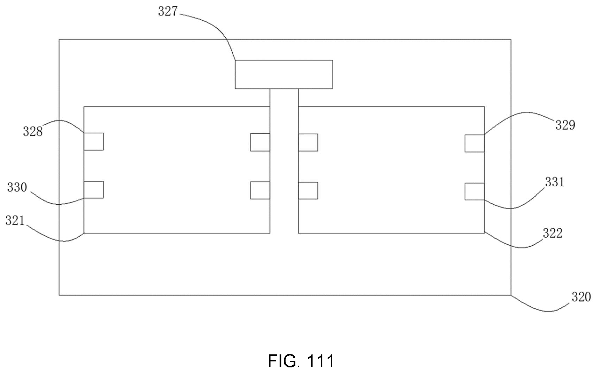

FIG. 111 is a structural module diagram of the wearable device according to the present disclosure;
FIG. 112 is a method flowchart of a control method according to an embodiment of the present disclosure;
FIG. 113 is a method flowchart of a control method according to an embodiment of the present disclosure;

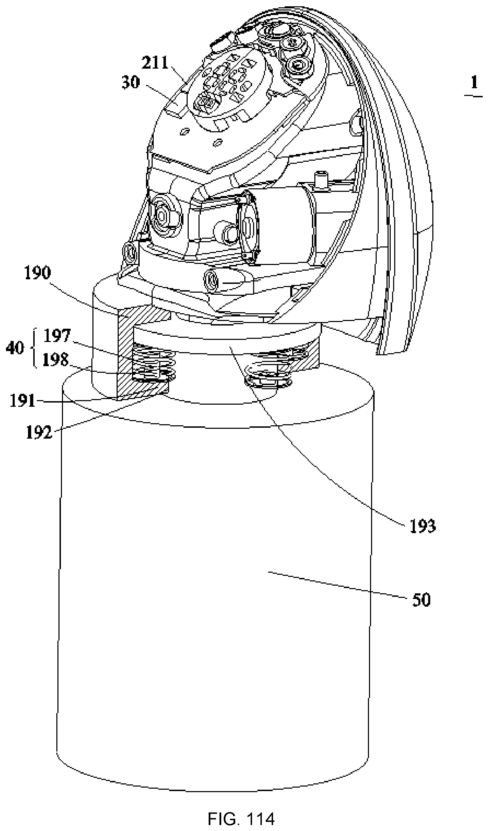

FIG. 114 is a three-dimensional schematic structural diagram of a breast pump according to an embodiment of the present disclosure;

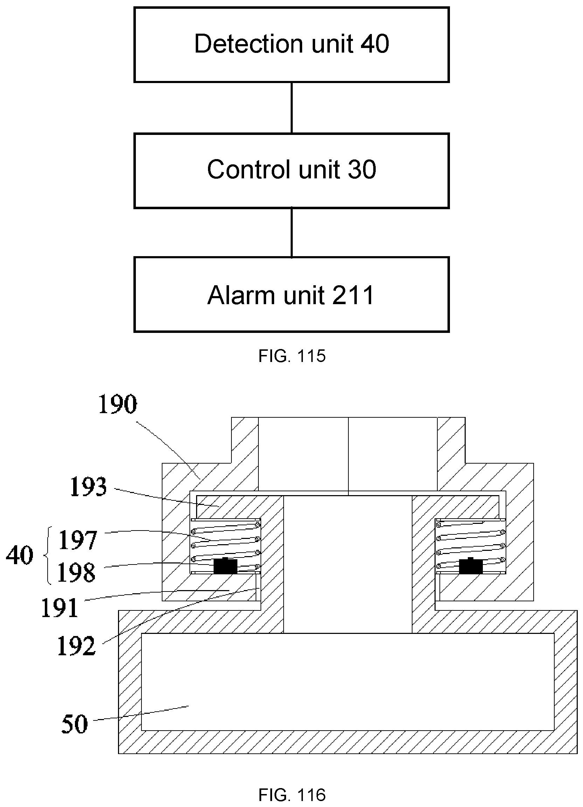

FIG. 115 is a diagram of a control module according to an embodiment of the present disclosure;
FIG. 116 is a cross-sectional view of a detection unit of a breast pump with a distance sensor according to an embodiment of the present disclosure;

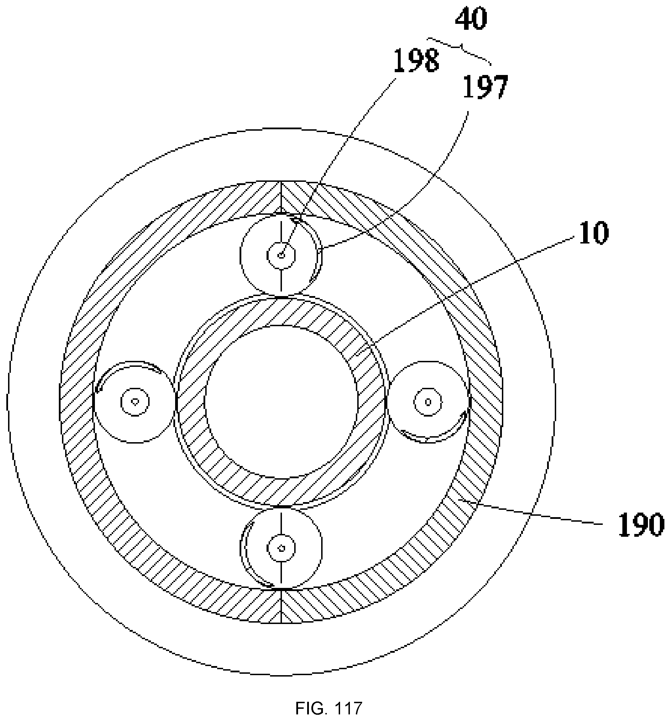

FIG. 117 is a cross-sectional view of a detection unit of a breast pump with a distance sensor according to an embodiment of the present disclosure;

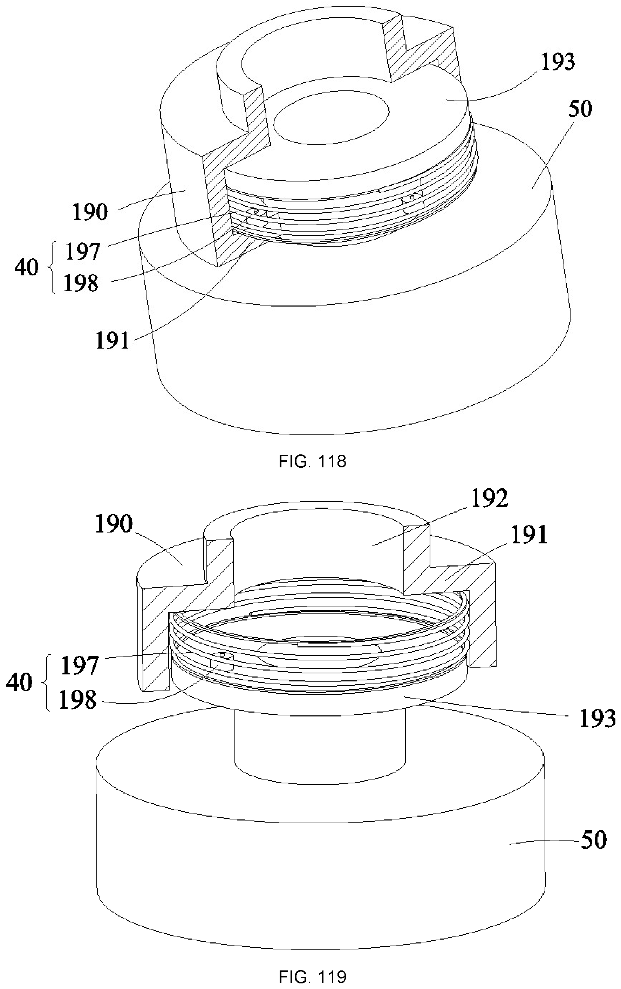

FIG. 118 is a three-dimensional cross-sectional view of a detection unit of a breast pump with a distance sensor according to an embodiment of the present disclosure;
FIG. 119 is a three-dimensional cross-sectional view of a detection unit of a breast pump with a distance sensor according to an embodiment of the present disclosure;

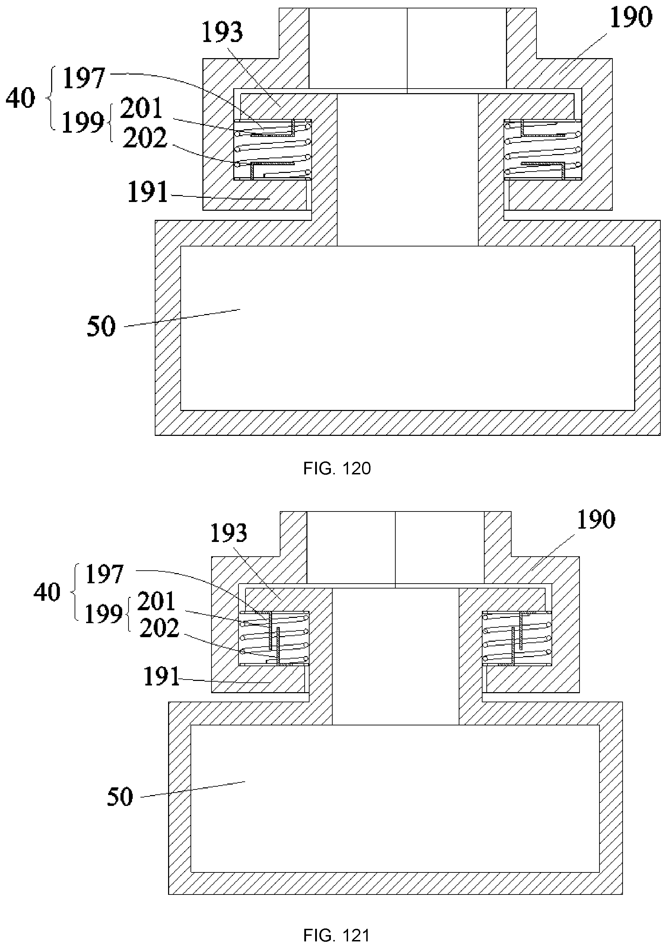

FIG. 120 is a cross-sectional view of a detection unit of a breast pump with a capacitive sensor according to an embodiment of the present disclosure;
FIG. 121 is a cross-sectional view of a detection unit of a breast pump with a capacitive sensor according to an embodiment of the present disclosure;

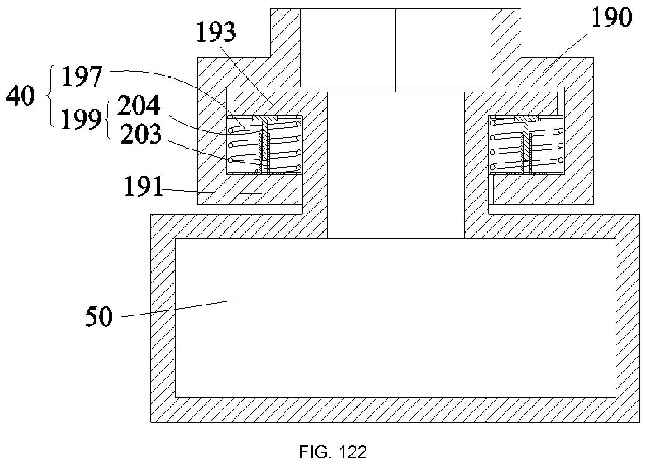

FIG. 122 is a cross-sectional view of a detection unit of a breast pump with a capacitive sensor according to an embodiment of the present disclosure;

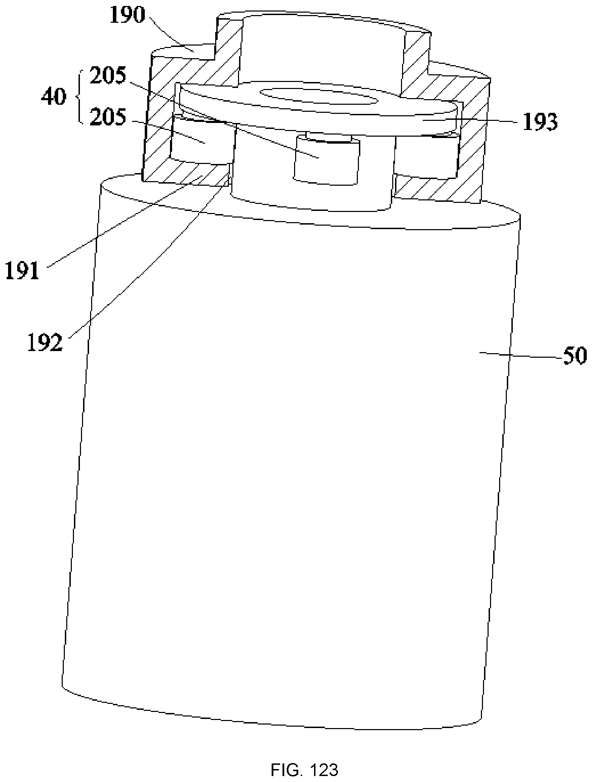

FIG. 123 is a three-dimensional cross-sectional view of a detection unit of a breast pump with a pressure sensor according to an embodiment of the present disclosure;

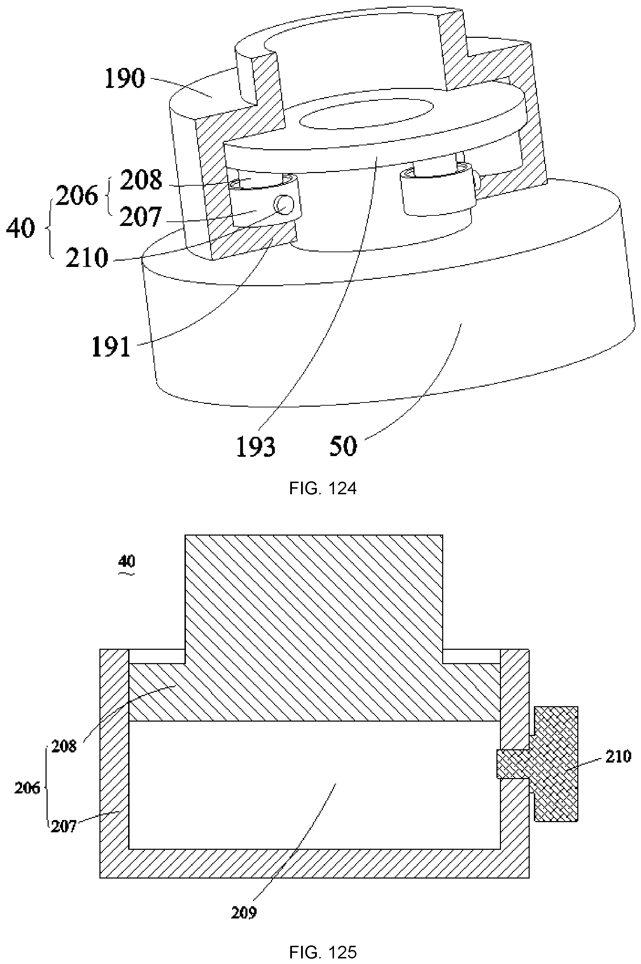

FIG. 124 is a three-dimensional cross-sectional view of a detection unit of a breast pump with an air pressure sensor according to an embodiment of the present disclosure;
FIG. 125 is a cross-sectional view of a detection unit with an air pressure sensor according to an embodiment of the present disclosure;

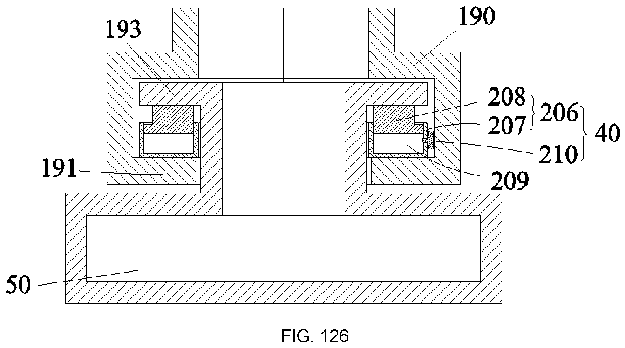

FIG. 126 is a cross-sectional view of a detection unit of a breast pump with an air pressure sensor according to an embodiment of the present disclosure;

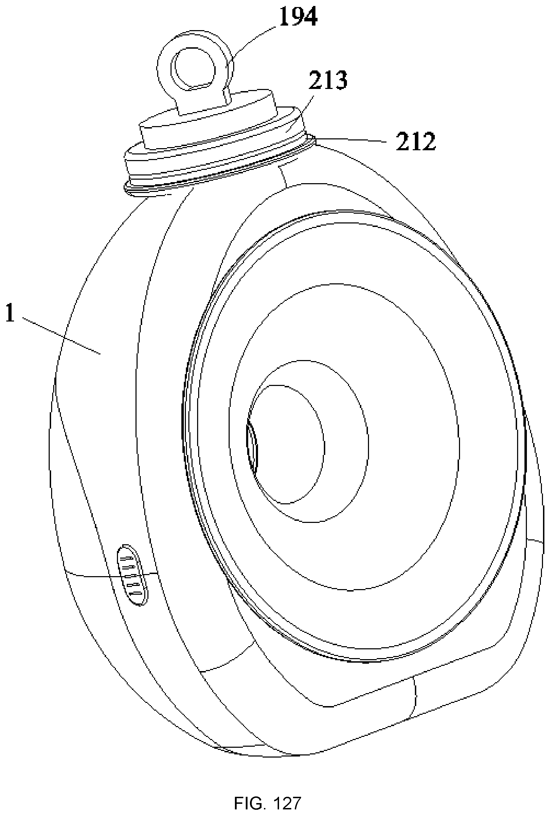

FIG. 127 is a three-dimensional schematic structural diagram of a breast pump according to an embodiment of the present disclosure;

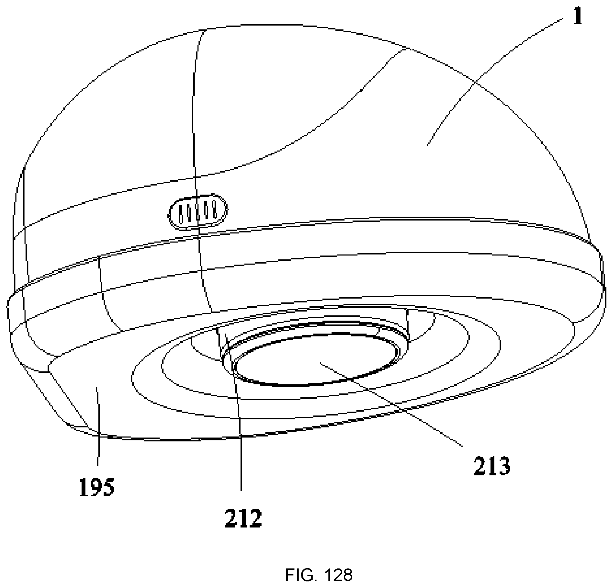

FIG. 128 is a three-dimensional schematic structural diagram of a breast pump according to an embodiment of the present disclosure;

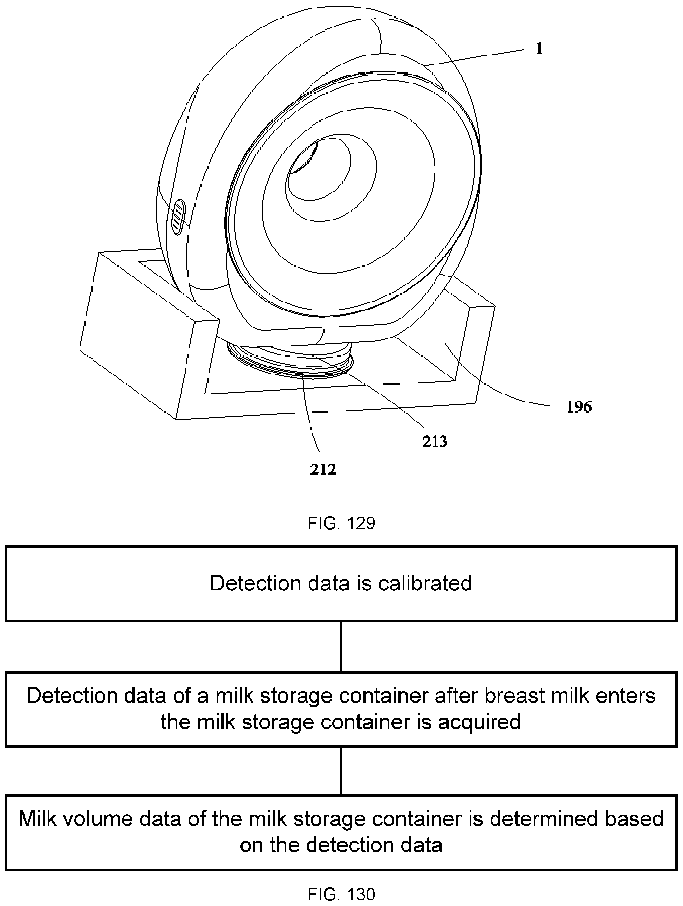

FIG. 129 is a three-dimensional schematic structural diagram of a breast pump according to an embodiment of the present disclosure;
FIG. 130 is a flowchart of steps of a detection method according to an embodiment of the present disclosure;

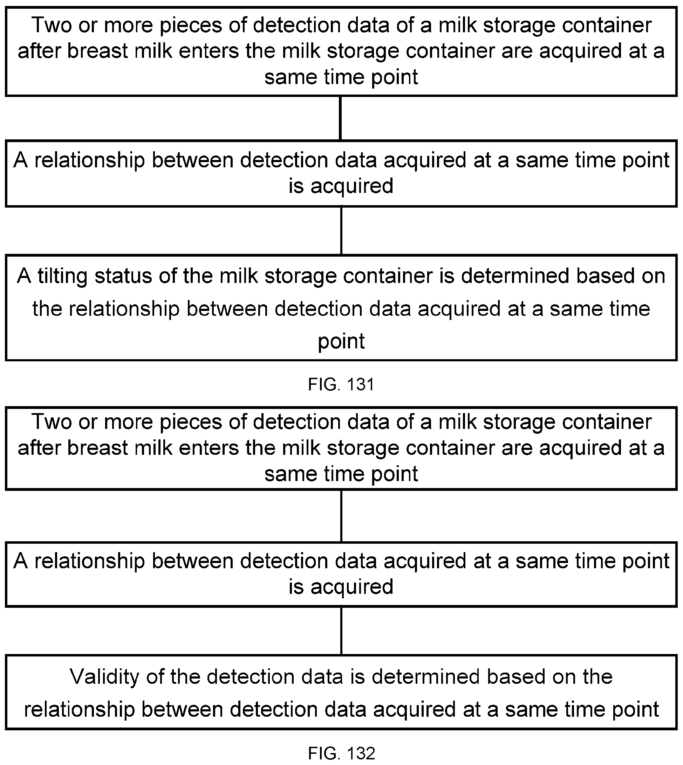

FIG. 131 is a flowchart of steps of determining a tilted state of a milk storage container portion according to an embodiment of the present disclosure;
FIG. 132 is a flowchart of steps of determining validity of detection data according to an embodiment of the present disclosure;

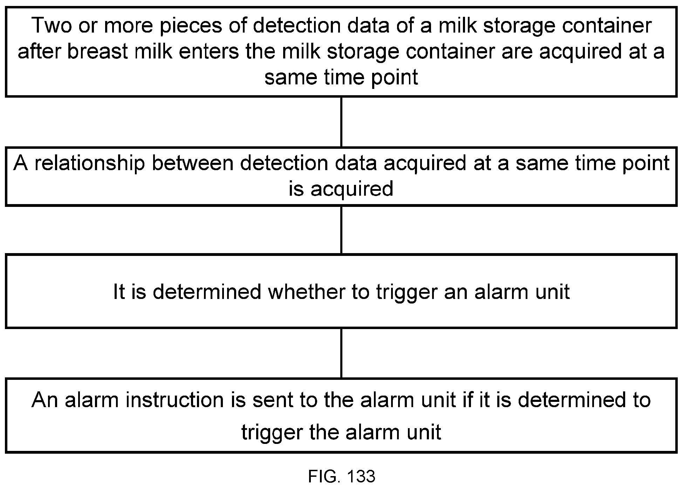

FIG. 133 is a flowchart of steps of determining to trigger an alarm unit according to an embodiment of the present disclosure;

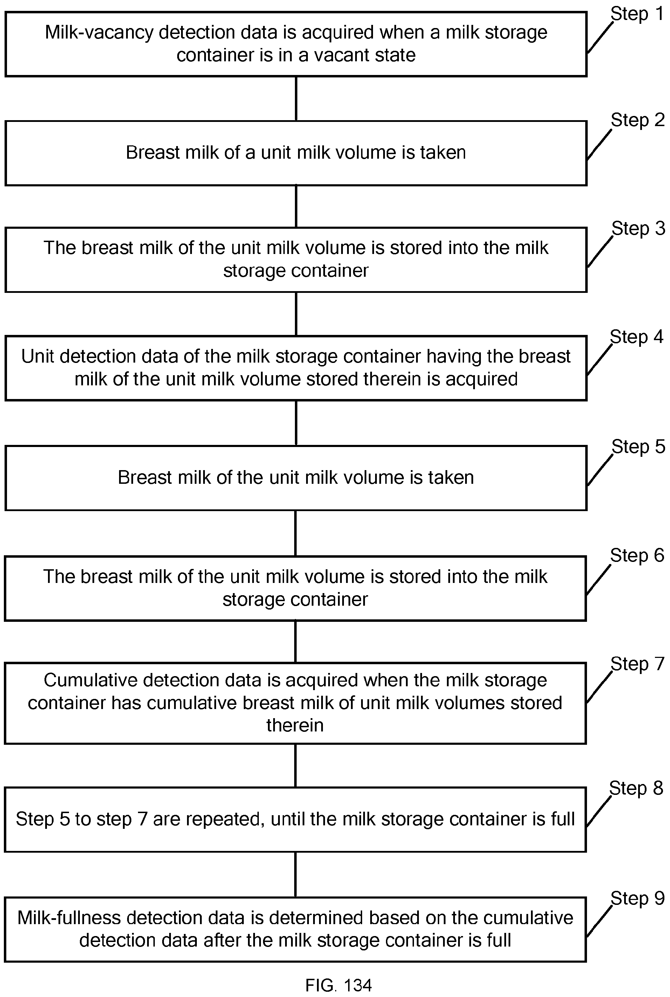

FIG. 134 is a flowchart of steps of calibrating detection data according to an embodiment of the present disclosure;
FIG. 135 is a flowchart of steps of calibrating detection data according to an embodiment of the present disclosure;
FIG. 136 is a structural block diagram of a detection device according to an embodiment of the present disclosure;
FIG. 137 is a structural block diagram of a control device according to an embodiment of the present disclosure;
FIG. 138 is a schematic structural diagram of a breast pump according to an embodiment of the present disclosure; and
FIG 139 is a schematic structural diagram of a storage structure according to an embodiment of the present disclosure.

### DETAILED DESCRIPTION OF THE DRAWINGS AND THE PRESENTLY PREFERRED EMBODIMENTS

Technical solutions in the embodiments of this application are clearly and completely described below with reference to drawings in the embodiments of this application. Apparently, the described embodiments are merely some rather than all of the embodiments of this application. All other embodiments obtained by a person of ordinary skill in the art based on the embodiments of this application without making creative efforts shall fall within the protection scope of this application.

In addition, the terms "first" and "second" are merely used for description, and cannot be understood as indicating or implying relative importance or implying a quantity of indicated technical features. Therefore, a feature defined with "first" or "second" may explicitly or implicitly include one or more features. In the description of the present disclosure, "a plurality of" means two or more, unless explicitly and specifically defined otherwise.

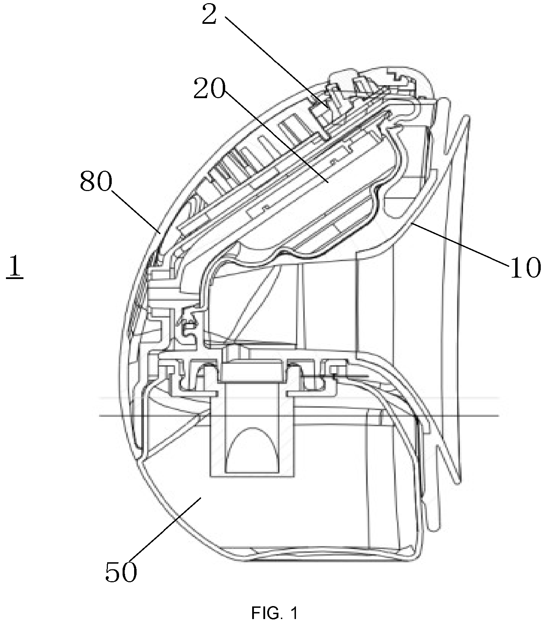

Referring to FIG. 1, a breast pump 1 in FIG. 1 includes a milk storage container portion 50, a negative pressure mechanism 20, and a breast milk collection portion 10.

The breast milk collection portion is also referred to as a breast shield 10.

In some implementations, the breast shield 10 may include a flange 11, and may further include a diaphragm support integrally formed with the flange. The diaphragm support is configured to support a diaphragm. The flange may include one end attached to a breast, and may further include one end in communication with the milk storage container portion or a milk storage chamber. Preferably, the end in communication with the milk storage container portion or the milk storage chamber may be a nipple channel.

In some implementations, one end of the breast milk collection portion is attached to the breast, and another end is in fluid communication with the milk storage container portion. The milk storage container portion is configured to store breast milk collected by the breast milk collection portion, and the breast milk collection portion forms an internal cavity with an interior of the milk storage container portion for circulation or storage of the breast milk.

The negative pressure mechanism can directly or indirectly apply negative pressure to the internal cavity, causing the breast milk to be expressed to the milk storage container portion for storage.

In some implementations, the milk storage container portion 50 may also be referred to as a milk storage chamber, and may include forms such as a milk lid, a milk bowl, and a bottle. This is not limited in this application.

In some implementations, the breast pump further includes a detection unit 40 (not shown in the figure, which may be a detector) and a control unit 30 (not shown in the figure). The detection unit is configured to obtain a detection parameter. The control unit is configured to control the breast pump.

In some implementations, the detection unit 40 is mounted on the breast pump 1, and can detect, sense, or acquire one or more of a mounting status, distance sensing, a volume, a flow rate, a weight, a pressure, a liquid level, a breast status parameter, a preset user parameter, or a historical data parameter.

In some implementations, the detection unit, such as an optical detection unit, needs to be mounted on the breast pump. Preferably, the detection unit needs to be mounted on a part not in contact with breast milk, for example, may be arranged inside a housing 80 or a main unit 2 (not shown in the figure). More preferably, the detection unit needs to be mounted on an outer wall, for example, on an outer wall of the milk storage container portion, or on an outer wall of the main unit, or on a non-breast milk side indirectly applying negative pressure.

In some implementations, the main unit 2 includes a control unit 30 and a negative pressure mechanism 20.

In the negative pressure mechanism 20 of the breast pump 1 in this application, a power structure configured to generate negative pressure includes but is not limited to a piezoelectric pump, a diaphragm pump, a hydraulic pump, or a mechanical pump. The negative pressure mechanism 20 may directly generate negative pressure in the breast shield through communication with the breast shield, or may indirectly generate negative pressure in the breast shield 10 by transmitting the negative pressure to a liquid barrier such as a diaphragm or an airbag. The breast milk is drawn into the milk storage container portion 50 for storage through the negative pressure.

In some implementations, the negative pressure mechanism may apply negative pressure manually or automatically.

In some implementations, the above expression "generate negative pressure in the breast shield through communication with the breast shield" means that an air passage from a negative pressure driving portion communicates with the internal cavity, and the above expression "indirectly generate negative pressure by transmitting the negative pressure to a liquid barrier" means that the negative pressure driving mechanism acts via a deformable diaphragm, a bladder, or a breathable yet liquid-impermeable barrier material positioned between milk and the negative pressure driving member.

In some implementations, the internal cavity 70 (not shown in the figure) may be an internal cavity formed by direct communication between the breast shield 10 and the milk storage container portion 50, or may be a cavity formed by communication between the two through other tubes.

In some implementations, the breast pump further includes a control system. The control system may be mounted on the main unit, or may be a separate handheld remote control, allowing a user to directly control the breast pump to operate.

In some implementations, the breast pump further includes a power supply module. The power supply module may be a storage battery, a dry cell, a wire, or the like.

For ease of explanation, in some implementations, the detection unit 40 is also referred to as a detection element 40, and the breast milk collection portion 10 is also referred to as a breast shield 10.

### First embodiment

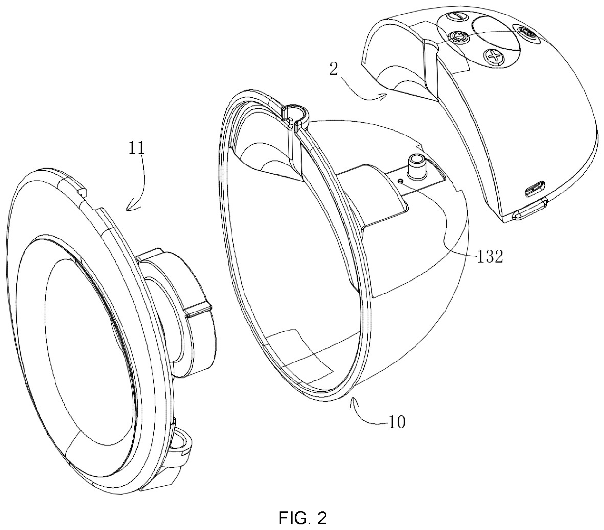

In this embodiment, as shown in FIG. 2, the breast pump 1 includes main components such as a milk storage container portion 50, a flange 11, and a main unit 2. Generally, both the main unit 2 and the flange 11 are arranged to be detachable from the milk storage container portion 50, to help a mother store breast milk in the milk storage container portion 50 and clean the milk storage container portion 50 and the flange 11 in a timely manner after milk expressing, achieving maximum convenience during use. Among user groups of such products, the primary users are postpartum mothers, who may have various roles such as working mothers or stay-at-home mothers. The usage of such products is often affected by environments. For instance, working mothers may face constraints like time pressure or workplace conditions during use, while stay-at-home mothers may face constraints such as infant crying or household chores in daily life. In such environments, mothers are prone to distraction or emotional instability during use of the breast pump 1, resulting in improper mounting of the breast pump 1. This may further cause breast milk to flow into the main unit 2, damaging the main unit 2, or may lead to breast milk spillage that contaminates the breast milk, stains clothing, and creates numerous inconveniences for the users.

An embodiment of this application provides a detection method for proper mounting and a breast pump 1 using the method. It is determined based on detection signals from a plurality of detection units whether the flange 11, the diaphragm 60, and the main unit 2 are properly mounted on the milk storage container portion 50, to ensure that the breast pump 1 can be properly mounted without being affected by external factors, thereby improving operating stability of the breast pump 1, and ensuring higher convenience and better experience for users during use.

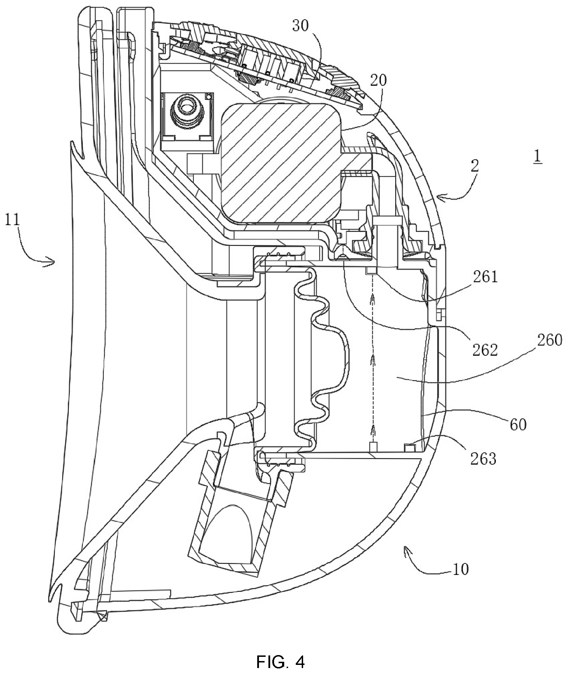

Referring to FIG. 2 to FIG. 6, the breast pump 1 provided in this embodiment includes a milk storage container portion 50, a main unit 2, a flange 11, and a diaphragm 60 mounted on the flange 11. Both the main unit 2 and the flange 11 are detachably mounted on the milk storage container portion 50. The milk storage container portion 50 is configured to hold expressed breast milk. The main unit 2 is configured to generate a suction force, control a magnitude of the suction force, an expressing speed, and a milk expressing time, record expressing data, and the like. The diaphragm 60 is configured to generate a suction force in the breast pump 1. The diaphragm 60 moves back and forth, to generate negative pressure inside the breast pump 1, so as to pump milk from a breast. In addition, during the milk expressing, the diaphragm 60 can prevent the milk from flowing back to the breast, ensuring smooth milk expressing. In this embodiment, the main unit 2 includes a negative pressure mechanism 20 configured to generate negative pressure through expressing and a control unit 30 configured to control the negative pressure mechanism 20. The negative pressure mechanism 20 is configured to generate a suction force on a breast of a user through the flange 11, pump breast milk, and discharge the breast milk into the milk storage container portion 50. The flange 11 is configured to connect to the milk storage container portion 50 and fasten the milk storage container portion 50. During the use of the breast pump 1, the flange 11 is snugly attached to the breast, to ensure that no gap exists between a nipple and the milk storage container portion 50. The main unit 2 generates a suction force to allow milk in the breast to be expressed through the flange 11 and collected in the milk storage container portion 50. Since the flange 11 is a part to be in indirect contact with the nipple and the breast, the flange 11 in this embodiment is made of soft, non-toxic, and non-irritating materials to reduce friction and irritation to the nipple and the breast.

Referring to FIG. 2 to FIG. 8, preferably, in the above embodiment, a first detection unit 131 is arranged between the flange 11 and the milk storage container portion 50. The first detection unit 131 is configured to generate a first signal when the flange 11 is not properly mounted. It may be understood that this process needs to be implemented after the main unit 2 is mounted on the milk storage container portion 50. The determination regarding whether the flange 11 is properly mounted on the milk storage container portion 50 may be performed through the control unit 30 of the main unit 2. Before the flange 11 is mounted on the milk storage container portion 50, a preset sensor performs signal sensing for the control unit 30, so that the control unit 30 skips detecting the first signal in this process. Furthermore, when the sensor detects that the flange 11 reaches the milk storage container portion 50 and mounting starts, the control unit 30 starts to detect the first signal. When detecting the generation of the first signal, the control unit 30 may automatically generate and send first prompt message to prompt the user to remount the flange 11 and stop the milk expressing.

It may be understood that the overall operation of the breast pump 1 in this embodiment is controlled by the control unit 30 in the main unit 2. The control unit 30 may be a central control unit (CPU), an application specific integrated circuit (ASIC), or one or more integrated circuits configured to implement the embodiments of the present disclosure. This is not limited herein.

It may be understood that, the preset sensor that performs signal sensing for the control unit 30 to enable the control unit 30 to determine whether the flange 11 is in contact with the milk storage container portion 50 to initiate mounting on or removal from the milk storage container portion 50 in the above solution may be achieved through various types of sensors such as an ultrasonic sensor or a photoelectric sensor. When the flange 11 is completely removed from the milk storage container portion 50, the sensor may perform signal sensing for the control unit 30, so that the control unit 30 skips detecting the first signal. That is to say, the control unit 30 skips detecting the first signal either when the flange 11 is not mounted on the milk storage container portion 50 or is completely removed from the milk storage container portion 50 and is no longer in contact with the milk storage container portion 50. Details are not described herein.

Referring to FIG. 2 to FIG. 6, preferably, in the above embodiment, the breast pump 1 further includes a second detection unit 132 and a third detection unit 133. The second detection unit 132 is configured to generate a second signal when the main unit 2 is not properly mounted. The third detection unit 133 is configured to generate a third signal when the flange 11 is not properly mounted. The control unit 30 is further configured to detect the second signal and the third signal, and automatically generate prompt information and/or stop the milk expressing and/or automatically turn off the main unit 2. It may be understood that the implementation of the above process follows the same principle as determining whether the flange 11 is properly mounted on the milk storage container portion 50. Specifically, a preset sensor performs signal sensing for the control unit 30. When the main unit 2 is not mounted on the milk storage container portion 50 or is completely separated from the milk storage container portion 50, the control unit 30 skips detecting the second signal. Details are not described herein.

In addition, that the third detection unit 133 is configured to generate a third signal when the diaphragm 60 is not properly mounted may be understood as follows: When the flange 11 is not properly mounted on the milk storage container portion 50, triggering the first signal, the diaphragm 60 mounted on the flange 11 is consequently also improper, triggering the third detection unit 133 to generate the third signal.

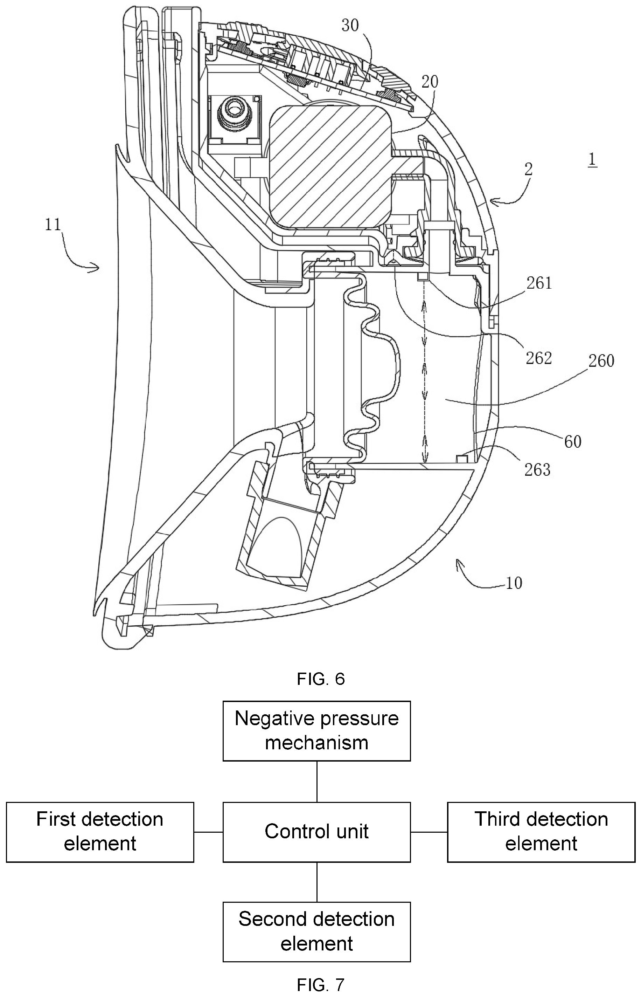

Referring to FIG. 7 to FIG. 8, preferably, in the above embodiment, two assembly options are provided for the user to assemble the breast pump 1. One is to first mount the main unit 2 on the milk storage container portion 50 and then mount the flange 11 on the milk storage container portion 50, to complete the assembly of the breast pump 1. The other is to first mount the flange 11 on the milk storage container portion 50 and then mount the main unit 2 on the milk storage container portion 50, to complete the assembly of the breast pump 1. Both assembly solutions require the control unit 30 to detect the first signal, the second signal, and the third signal. During operation of the breast pump 1 after startup, the control unit 30 of the main unit 2 detects whether the first signal, the second signal, and the third signal can be obtained. If the first signal and the second signal cannot be obtained, it is determined that the main unit 2 is properly mounted on the milk storage container portion 50 and that the flange 11 is properly mounted on the milk storage container portion 50. In this case, the operation can continue.

Preferably, if the control unit 30 obtains the first signal, it is determined that the flange 11 is not properly mounted on the milk storage container portion 50. In this case, the control unit 30 sends first prompt information to prompt the user to remount the flange 11 and stop the milk expressing. Furthermore, if the control unit 30 obtains the second signal, it is determined that the main unit 2 is not properly mounted on the milk storage container portion 50 and that the flange 11 is not mounted on the milk storage container portion 50. In this case, the control unit 30 automatically generates and sends second prompt information to prompt the user to remount the main unit 2 and stop the milk expressing. If the control unit 30 obtains the first signal and then obtains the second signal, it is determined that neither the main unit 2 nor the flange 11 is properly mounted on the milk storage container portion 50, and prompts need to be given sequentially.

Moreover, in the above process, the generation of the first signal indicates that the flange 11 is not properly mounted. Therefore, the diaphragm 60 mounted on the flange 11 is also not properly mounted, triggering the third detection unit 133 to generate the third signal. In this case, the control unit 30 detects the first signal and the third signal. When the control unit 30 detects the third signal, it indicates that the flange 11 is not properly mounted on the milk storage container portion 50, and therefore the diaphragm 60 mounted on the flange 11 is also not properly mounted. The control unit 30 automatically generates and sends prompt information, and automatically turns off the main unit 2. This scenario is applied to the use of the breast pump. Details are described later.

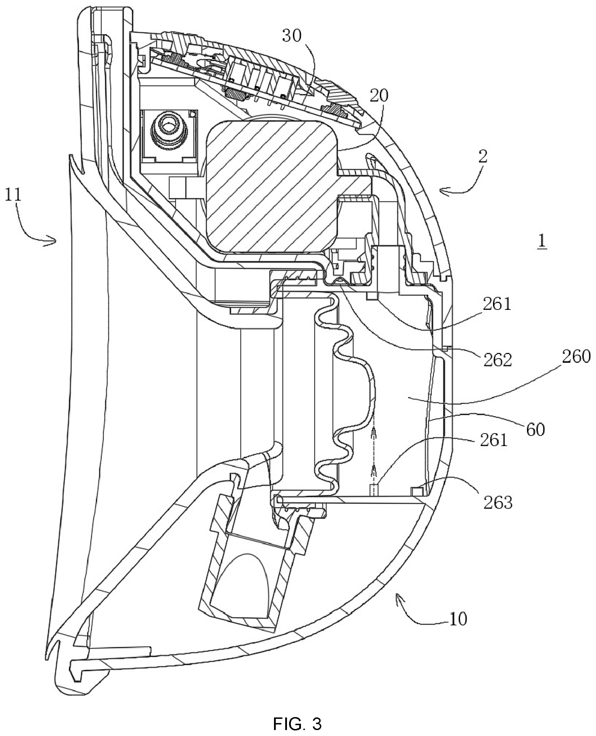

Referring to FIG. 3 to FIG. 6, based on the above solution, it may be understood that, the flange 11 in this embodiment may be used as a lid on a side of the milk storage container portion 50. The flange 11 is provided with a milk shield, and the flange 11 is mounted on the milk storage container portion 50 to achieve fastening of the milk storage container portion 50. However, during actual use, the user may accidentally touch the flange 11 when using the breast pump 1, causing the flange 11 to deviate on the milk storage container portion 50, resulting in improper mounting. This also results in improper mounting of the diaphragm 60 on the flange 11. In this case, air leakage easily occurs in the milk storage container portion 50. In addition, in another scenario, during normal use, a user may remove the flange 11 from the milk storage container portion 50 to retrieve the milk without first turning off the main unit 2, leaving an opening of the milk storage container portion 50 exposed. Moreover, both scenarios may cause the breast milk to spill out of the milk storage container portion 50, resulting in breast milk contamination and easy staining of clothes. Moreover, when the flange 11, together with the diaphragm 60, is removed from the milk storage container portion 50, due to lack of blocking by the diaphragm 60 in the milk storage container portion, the milk is expressed back into the main unit 2, causing damage to the main unit 2. Therefore, the third detection unit 133 is arranged, to prevent this problem.

In another embodiment, the breast pump further includes a fourth detection unit configured to generate a fourth signal when the diaphragm 60 is not properly mounted. It may be understood that, in a scenario, after the flange 11 is properly mounted on the milk storage container portion 50, if the diaphragm 60 is not properly mounted, the mounting between the diaphragm 60 and the milk storage container portion 50 remains improper after the flange 11 is properly mounted on the milk storage container portion 50, triggering the fourth detection unit to generate the fourth signal. It may be understood that, in the previous embodiment, the third detection unit 133 is configured to generate the third signal when the diaphragm 60 is not properly mounted. Therefore, in this embodiment, the fourth detection unit is equivalent to the third detection unit 133 in the previous embodiment. Details are not described herein. For structural implementation, reference may be made to FIG. 3 to FIG. 6. Furthermore, when the fourth detection unit generates the fourth signal, the control unit 30 detects both the first signal and the fourth signal. When the control unit 30 detects the fourth signal, it indicates that the flange 11 is not properly mounted on the milk storage container portion 50, which also results in improper mounting of the diaphragm 60 on the flange 11. In this case, the control unit 30 automatically generates and sends fifth prompt information, stops milk expressing, and automatically turns off the main unit 2 to prevent milk backflow.

Specifically, when the control unit 30 detects the third signal, it indicates that the breast pump 1 has encountered one of the above two scenarios. The control unit 30 is configured to assign a signal level to the third signal, categorizing the third signal as either "A" or "B". By determining whether the third signal is at the level "A" or "B", the control unit 30 executes different corresponding mechanisms. When the control unit 30 detects that the third signal is at the level "A", it indicates that the user may accidentally touch the flange 11 and cause the flange 11 to deviate on the milk storage container portion 50, causing improper mounting of the flange 11. This also results in improper mounting of the diaphragm 60 mounted on the flange 11, causing air leakage in the milk storage container portion 50. In this case, the control unit 30 automatically generates and sends third prompt information to prompt the user of the air leakage of the milk storage container portion 50 and stops the milk expressing, and then turns off the main unit 2 to prevent the breast milk from spilling out or entering the main unit 2. In addition, when the control unit 30 detects that the third signal is at the level "B", it indicates that the user removes the flange 11 from the milk storage container portion 50 for further operations such as breast milk retrieval without first turning off the main unit 2. In this case, the user removes the flange 11 together with the diaphragm 60 from the milk storage container portion 50, leaving the opening of the milk storage container portion 50 exposed. In this case, the control unit 30 automatically generates and sends fourth prompt information to prompt the user that the main unit 2 is still on, and simultaneously turns off the main unit 2 to prevent the breast milk from spilling out or entering the main unit 2.

In an embodiment, the first detection unit 131 includes a Hall sensor, which is arranged between the flange 11 and the milk storage container portion 50 to detect whether the flange 11 is properly mounted on the milk storage container portion 50.

Referring to FIG. 3 to FIG. 4, specifically, the first detection unit 131 includes a sensing element arranged at a bottom position of the milk storage container portion 50 away from a mounting position of the main unit 2, and a triggering element arranged at a top position of the milk storage container portion 50 close to the mounting position of the main unit 2. In this embodiment, the triggering element is a magnet, and the sensing element is a Hall sensor arranged on the milk storage container portion 50. The triggering element and the sensing element are arranged opposite each other on the milk storage container portion 50, enabling the Hall sensor to generate varying Hall signals, i.e., the first signal, based on magnetic field changes of the magnet. When the flange 11 is properly mounted on the milk storage container portion 50, a side of the flange 11 directly blocks the triggering element, causing a change in the magnetic signal emitted by the triggering element to the sensing element. When the first detection unit 131 detects that the change in the magnetic signal matches a preset magnetic change value existing when the flange 11 is properly mounted, it indicates that the flange 11 is properly mounted. When the flange 11 is not properly mounted on the milk storage container portion 50, leaving the magnet exposed, the magnetic signal emitted by the triggering element to the sensing element remains unchanged. In this case, the first detection unit 131 sends the first signal. There are various ways to determine relative positions of the flange 11 and the milk storage container portion 50 through the Hall sensor and the magnet, which are described by way of example only herein, and this embodiment of this application is not limited thereto.

In another embodiment, the first detection unit 131 includes a photoelectric sensor, which is arranged between the main unit 2 and the flange 11 also to detect whether the flange 11 is properly mounted on the milk storage container portion 50.

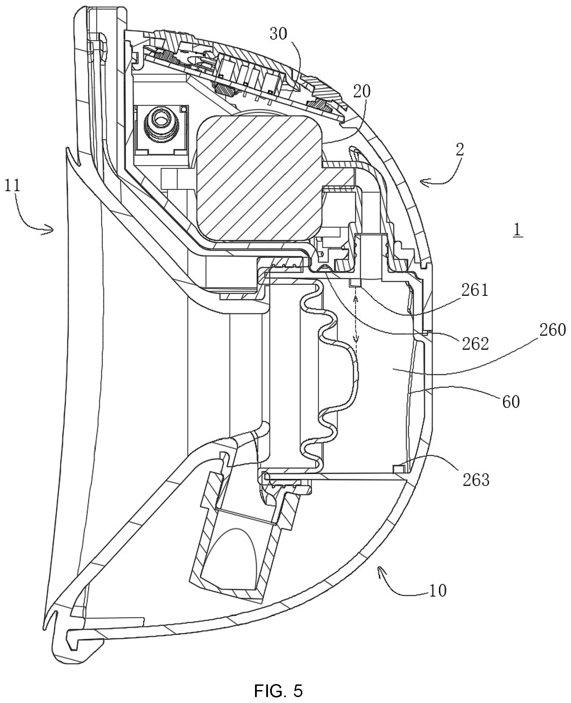

Referring to FIG. 5-5, specifically, an expression "between the main unit 2 and the flange 11" includes that "between the flange 11 and the milk storage container portion 50". It may be understood that photoelectric sensors applicable in this embodiment include various types of sensors such as a light-shielding photoelectric sensor, a laser sensor, and a transmissive photoelectric sensor. Furthermore, when a light-shielding photoelectric sensor is used, the light-shielding photoelectric sensor may be mounted on the milk storage container portion 50, and a volume of a light ray (in candelas) shielded by the flange 11 when properly mounted is preset. A volume of a light ray shielded by the flange is detected, to determine a position of the flange 11 on the milk storage container portion 50, so as to determine whether the flange 11 is properly mounted. When a candela value of the light ray shielded by the flange 11 on the milk storage container portion 50 matches the preset value, it indicates that the flange 11 is properly mounted. When a laser sensor is used, the laser sensor may be mounted on the milk storage container portion 50 at a position where the flange 11 passes, and a light ray reflection time existing when the flange 11 is properly mounted is preset. A change in a light ray reflection time caused by a change in a light ray reflection length existing after the flange 11 is mounted on the milk storage container portion 50 is detected, to detect whether the flange 11 is properly mounted. When the light ray reflection time changed by the flange 11 matches the preset value, it indicates that the flange 11 is properly mounted. When a transmissive photoelectric sensor is used, intensity of a light ray passing through the flange 11 is detected, to determine whether the flange 11 is properly mounted.

There are various ways to determine relative positions of the flange 11 and the milk storage container portion 50 through the photoelectric sensor, which are described by way of example only herein, and this embodiment of this application is not limited thereto. Therefore, the first detection unit 131 and the second detection unit 132 described above may be implemented by magnetic induction, photoelectric induction, mechanical switch-type configurations. These are merely examples, and this embodiment of this application is not limited thereto.

In an embodiment, the second detection unit 132 includes a photoelectric sensor arranged between the main unit 2 and the milk storage container portion 50, to detect whether the main unit 2 is properly mounted on the milk storage container portion 50.

The mounting position, the operating principle, and the like of the first detection unit 131 are described above. The second detection unit 132 is similar to the first detection unit 131, except that the second detection unit 132 is arranged between the main unit 2 and the milk storage container portion 50 to detect whether the main unit 2 is properly mounted on the milk storage container portion 50. Reference may be made to the description of the first detection unit 131. Details are not described herein.

In an embodiment, each of the third detection unit 133 and the fourth detection unit include a pressure sensor. The pressure sensor is arranged on the diaphragm 60 between the milk storage container portion 50 and the flange 11, to timely prompt the user and automatically activate a response mechanism when the flange 11 is not properly mounted or deviates on the milk storage container portion 50 during use and when the flange 11 is directly removed from the milk storage container portion 50.

Specifically, during the mounting, the pressure sensor is arranged on the milk storage container portion 50 and located in a negative pressure chamber 260 formed between the milk storage container portion 50 and the diaphragm 60. It may be understood that the negative pressure chamber 260 formed between the milk storage container portion 50 and the diaphragm 60 has different air pressure states during normal operation and during shutdown. The pressure sensor detects airtightness of the negative pressure chamber 260. When the diaphragm 60 is not properly mounted on the milk storage container portion 50, the pressure sensor detects that air pressure in the negative pressure chamber 260 does not reach a preset standard, thus determining that the negative pressure chamber 260 is not in a sealed state, and further determining that the diaphragm 60 is not properly determined. Therefore, the pressure sensor feeds back a fourth signal. The control unit 30 automatically generates and sends fifth prompt information and stops the milk expressing after receiving the fourth signal.

In another embodiment, the fourth detection unit further includes a touch sensor. When the diaphragm is not properly mounted, the touch sensor sends the fourth signal to the control unit 30, and the control unit 30 proceeds to a next operation. Details are not described herein.

Specifically, during the use, the pressure sensor is arranged on the milk storage container portion 50 and located in the negative pressure chamber 260 formed between the milk storage container portion 50 and the diaphragm 60. It may be understood that the negative pressure chamber 260 formed between the milk storage container portion 50 and the diaphragm 60 has different air pressure states during normal operation and during shutdown. The pressure sensor detects the airtightness of the negative pressure chamber 260. When the flange 11 deviates on the milk storage container portion 50 or when the flange 11 is directly removed from the milk storage container portion 50, the diaphragm 60 moves together with the flange 11 and encounters improper mounting. The pressure sensor detects an air pressure change in the negative pressure chamber 260 and feeds back a third signal. The control unit 30, when detecting the third signal, assigns a signal level "A" or a signal level "B" to the third signal based on an intensity of the signal fed back by the pressure sensor when detecting an air pressure value in the negative pressure chamber 260. The level "A" signal represents a weak signal, indicating that the air pressure inside the negative pressure chamber 260 is still within a specific range and is less than an external air pressure. This indicates that the negative pressure chamber 260 is leaking gas, further indicating that the flange 11 is still on the milk storage container portion 50. In this case, the control unit 30 automatically generates and sends third prompt information to prompt the user that the diaphragm 60 is not properly mounted and the main unit 2 is not turned off, stops the milk expressing operation, and turns off the main unit 2, to prevent the breast milk from spilling out or being expressed back into the main unit 2. Furthermore, the level "B" signal represents a strong signal, indicating that the pressure sensor detects that the air pressure in the negative pressure chamber 260 significantly changes per unit time, which is substantially the same as the external air pressure. This further indicates that the user removes the flange 11 from the milk storage container portion 50 for further operations such as milk retrieval without first turning off the main unit 2, leaving the opening of the milk storage container portion 50 exposed. In this case, the control unit 30 automatically generates and sends fourth prompt information to prompt the user that the diaphragm 60 is not properly mounted and the main unit 2 is not turned off, and turns off the main unit 2. Because the intensity level "B" of the third signal indicates that the diaphragm 60 is removed from the milk storage container portion 50 along with the flange 11, the breast pump 1 needs to take automatic measures in a timely manner. Therefore, the control unit 30 needs to directly turn off the main unit 2 while sending the fourth prompt information, to prevent the breast milk from spilling out or entering the main unit 2.

Exemplarily, the third signal is emitted by the third detection unit 133 located in the milk storage container portion 50. As a pressure sensor, the third detection unit 133 needs to detect an air pressure change in the negative pressure chamber 260 in a fixed time period, to send a signal of an accurate level signal, so as to prevent an excessively large air pressure change in the negative pressure chamber 260 caused by long-time air leakage due to long-time improper mounting as a result of the user touching the flange 11 and causing deviation of the flange 11. In other words, the air pressure change caused by the long-time air leakage in the negative pressure chamber 260 is equivalent to the air pressure change caused by the user removing the diaphragm 60 from the milk storage container portion 50 along with the flange 11, which may cause the control unit 30 to erroneously determine the signal level of the third signal. Therefore, the control unit 30 is designed to detect the third signal after a preset duration since the air pressure change in the milk storage container portion 50. The preset duration may be two seconds, three seconds, or the like, which is not limited in this embodiment of this application. In addition, an air pressure change per preset duration is measured to send third signals of different levels, to ensure accuracy of the third signal.

To ensure accuracy of the fourth signal, the control unit is designed to detect the fourth signal based on the time preset for the third signal in the previous embodiment. Details are not described herein.

Referring to FIG. 7, in the above embodiment, optionally, the negative pressure mechanism 20 in the main unit 2 is electrically connected to the control unit 30, to be controlled by the control unit 30 to perform expressing operations. It may be understood that, when third prompt information or fourth prompt information appears and the main unit 2 is simultaneously turned off, the control unit 30 controls the negative pressure mechanism 20 to close, to prevent the breast milk from entering the negative pressure mechanism 20 through a suction port of the negative pressure mechanism 20 and subsequently entering a casing of the main unit 2 through the negative pressure mechanism 20. Further, the milk pump 1 further includes a three-way connector, with one end connected to the negative pressure mechanism 20, another end connected to the flange 11, and a last end connected to a bowl body.

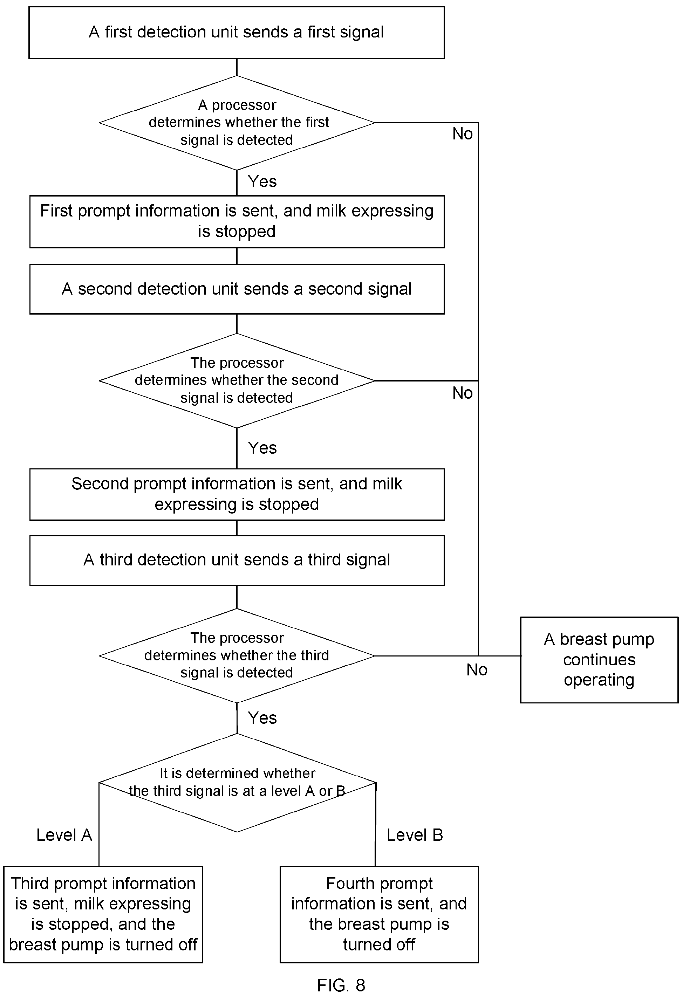

A proper mounting detection method for the breast pump in the embodiments of this application is described in detail below based on the above description of FIG. 2 to FIG. 6. Exemplarily, refer to FIG. 7 to FIG. 8. FIG. 8 is a flowchart of a proper mounting detection method according to an embodiment of this application. This embodiment provides a detailed description of the proper mounting detection method from a perspective of detecting whether the flange is properly mounted, and implements the method through the control unit of the main unit. This embodiment includes following steps:
First step (S1): It is determined whether a first signal is detected. When the first signal is detected, step S6 is performed. When no first signal is detected, step S5 is performed.

Second step: It is determined whether a second signal is detected. When the second signal is detected, step S7 is performed. When no second signal is detected, step S3 is performed.

Third step: It is determined whether a third signal is detected. When the third signal is detected, step S4 is performed. When no third signal is not detected, step S5 is performed.

Fourth step: A signal level of the third signal is detected. When the signal level of the third signal is detected as the level "A", step S8 is performed. When the signal level of the third signal is detected as the level "B", step S9 is performed.

Fifth step: The breast pump operates normally.

Sixth step: First prompt information is automatically generated and sent to prompt the user to remount the flange and stop the milk expressing operation.

Seventh step: Second prompt information is automatically generated and sent to prompt the user to remount the main unit, and the milk expressing is stopped.

Eighth step: Third prompt information is automatically generated and sent to prompt the user that the negative pressure chamber is leaking air and the main unit is not turned off, the milk expressing is stopped, and the main unit is turned off.

Ninth step: Fourth prompt information is automatically generated and sent to prompt the user that the negative pressure chamber is leaking air and the main unit is not turned off, and the main unit is turned off.

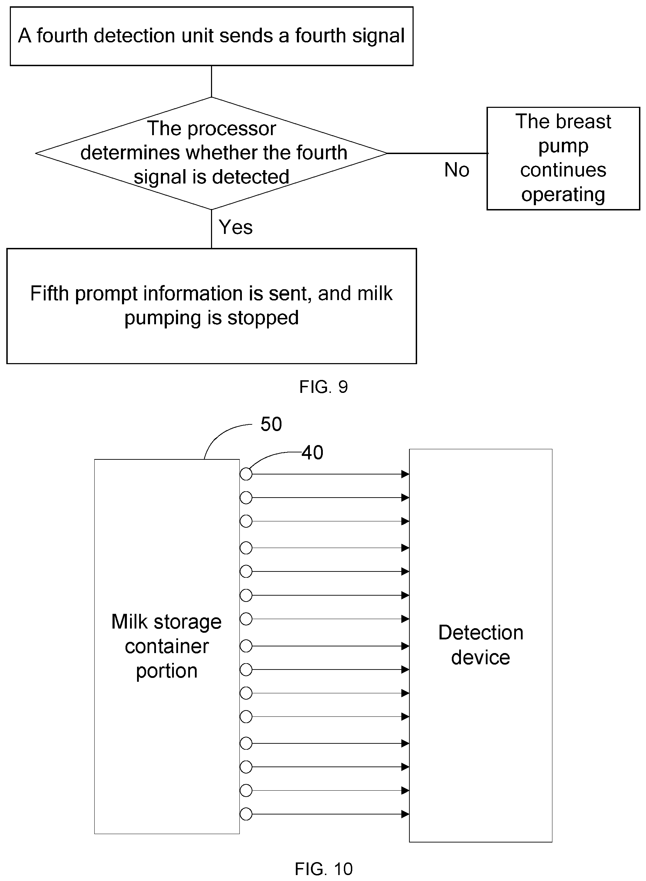

FIG. 9 is a flowchart of an improper mounting detection method for a diaphragm according to an embodiment of this application. This embodiment provides a detailed description of the proper mounting detection method from a perspective of detecting whether the diaphragm is properly mounted, and implements the method through the control unit of the main unit. In FIG. 9, this embodiment includes following steps:

First step (S1): It is determined whether a fourth signal is detected. When the fourth signal is detected, step S3 is performed. When no fourth signal is detected, step S2 is performed.

Second step: The breast pump 1 operates normally.

Third step: Fifth prompt information is automatically generated and sent to prompt the user to remount the diaphragm, and the milk expressing is stopped.

Preferably, in the above solution, the first prompt information, the second prompt information, the third prompt information, and the fourth prompt information may be sent to the user through voice broadcasting, subtitle display, vibration, or the like. When the operation of the breast pump includes tasks such as milk expressing, massage, and cleaning, the normal operation of the breast pump refers to continuing the above functions. When detecting that the flange, the main unit, and the diaphragm are not properly mounted, the control unit controls the air pump to stop expressing milk without affecting other normal operations. This is not limited in this embodiment of this application.

### Second embodiment

A breast milk volume detection provided in an embodiment of this application may be applied to an application environment shown in FIG. 10. A milk storage container portion 50 is configured to accommodate breast milk. A plurality of detection elements 40 configured to sense breast milk may be mounted on the milk storage container portion 50. Specifically, when breast milk reaches an arrangement position of any detection element 40, the detection element 40 can be triggered by sensing the breast milk. A detection device may perform the above breast milk volume detection method based on a triggering status of the detection element 40, thereby achieving measurement of a breast milk volume in the milk storage container portion 50.

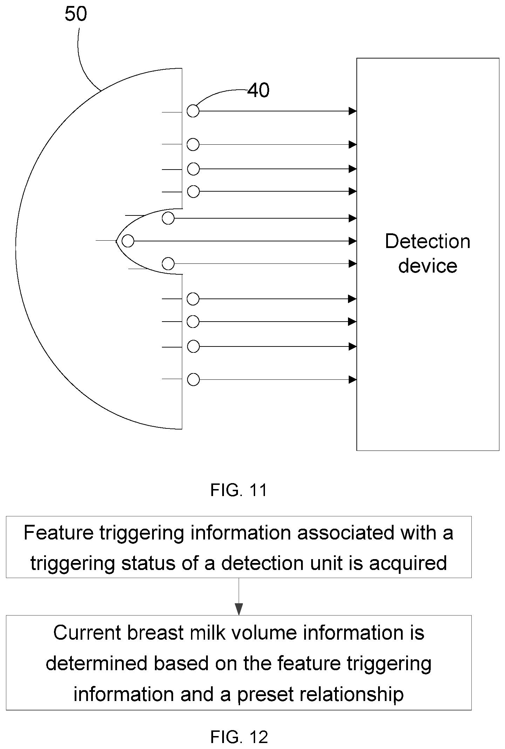

In this embodiment of this application, as shown in FIG. 10 and FIG. 12, a breast milk volume detection method is provided, including: acquiring a detection parameter detected by the detection unit; and performing a preset operation based on the detection parameter. A description is provided by using an example in which the method is applied to a breast milk flow rate detection unit 40 in FIG. 10. The method includes following steps:
Step 202: Feature triggering information associated with a triggering status of a detection element is acquired.

The association between the feature triggering information and the triggering status of the detection element 40 may be a direct association relationship or an indirect association relationship. The "direct association relationship" means that the feature triggering information is directly correlated with the triggering status of the detection element 40. For example, the feature triggering information includes but is not limited to at least one of a number of triggered detection elements 40 or a number of non-triggered detection elements 40. In addition, the "indirect association relationship" means that a triggered detection element 40 has preset association information while and the feature triggering information is correlated with the preset association information. For example, the preset association information includes but is not limited to at least one of triggering channel information corresponding to each detection element 40, information about a position of each detection element 40 on the milk storage container portion 50, or capacity information corresponding to each detection element 40.

Step 204: Current breast milk volume information is determined based on the feature triggering information and a preset relationship.

The preset relationship is used to represent a mapping relationship and/or a function relationship between feature triggering information and breast milk volume information. The "mapping relationship" refers to a one-to-one correspondence between feature triggering information and breast milk volume information. That is, one piece of feature triggering information corresponds to one piece of preset breast milk volume information. the "function relationship " refers to a preset function relationship between feature triggering information and breast milk volume information. That is, feature triggering information may be substituted into the preset function relationship to calculate breast milk volume information.

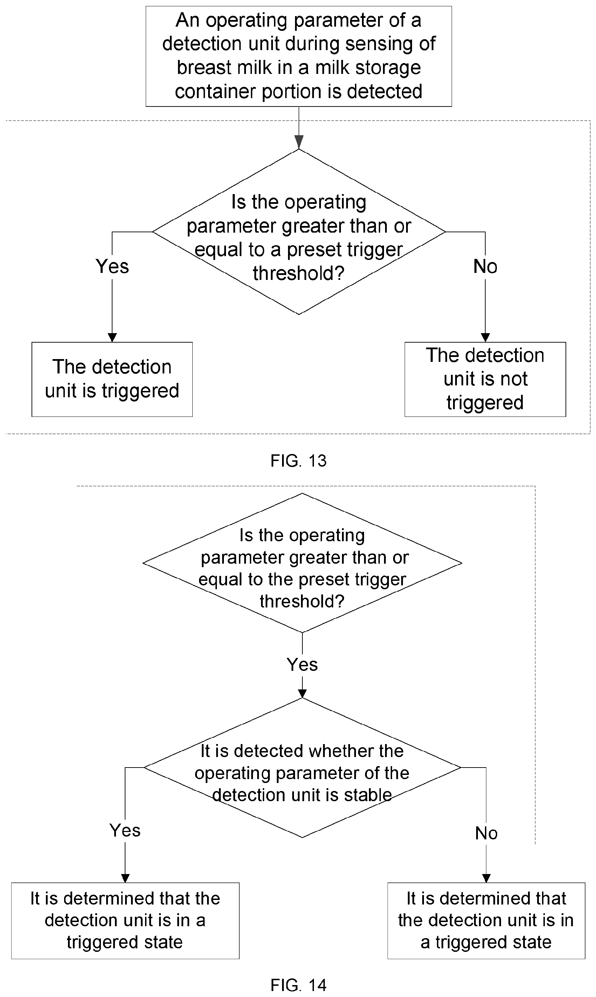

According to the breast milk volume detection method, the feature triggering information associated with the triggering status of the detection element 40 is obtained, and the current breast milk volume information is determined based on the feature triggering information and the preset relationship, so as to determine the breast milk volume in the milk storage container portion 50. Because the detection element 40 can be triggered during breast milk expressing, real-time online breast milk volume detection can be achieved through the above method. As shown in FIG. 10 and FIG. 12 to FIG. 13, in an embodiment, before step 202 described above, the method further includes following steps:
Step 302: An operating parameter of the detection unit 40 during sensing of breast milk in the milk storage container portion 50 is detected.

The detection element 40 includes but is not limited to a capacitive sensor or a photoelectric sensor, specifically, any one of a capacitive electrode, a photoelectric sensor, an infrared sensor, or a microwave detection sensor. Certainly, other types of sensors that can detect presence of breast milk when the breast milk reaches a position of the detection element 40 and be triggered to change the operating parameter are also feasible. The operating parameter includes any one of a capacitance, a current value, or a voltage value.

Step 304: An operating parameter of each detection element 40 is compared with a trigger threshold, it is determined whether the operating parameter of each detection element 40 is greater than or equal to the preset trigger threshold, and when the operating parameter of any detection element 40 is greater than or equal to the preset trigger threshold, it is determined that the detection element 40 is triggered, or otherwise, it is determined that the detection element 40 is not triggered.

The setting of the trigger threshold is not limited. Specifically, the trigger threshold is preset based on the operating parameter corresponding to detection of breast milk by the detection element 40. For example, in some embodiments, the trigger threshold may be a minimum value of the operating parameter corresponding to the detection of breast milk by the detection element 40. Certainly, in some other embodiments, the trigger threshold may be a rated value at which the detection element 40 operates normally when triggered.

For ease of understanding, a detailed description is provided below by using an example in which the detection element 40 is a capacitor plate.

In the above steps, when the detection element 40 is a capacitor plate, the operating parameter is a capacitance of the detection element 40, and the preset trigger threshold is a preset capacitance threshold.

Specifically, when breast milk reaches an arrangement position of any capacitor plate, a capacitance of the capacitor plate changes due to the presence of the breast milk. Therefore, in step 302, the capacitance of the capacitor plate is detected in real time, and then in step 304, the capacitance of each capacitor plate is compared with the preset capacitance threshold in real time. Based on a comparison result, a real-time triggering status of each capacitor can be determined. Certainly, it should be noted that, the capacitor electrode may be a standardized capacitor electrode or a non-standardized capacitor electrode.

As shown in FIG. 10 and FIG. 12 to FIG. 14, based on steps 302 to 304 described above, in order to improve accuracy of detecting the triggering status of the detection element 40, following steps may be provided during step 304:
Step 402: When the operating parameter of any detection element 40 is greater than or equal to the preset trigger threshold, stability of the operating parameter of the detection element 40 is detected.

The "stability" refers to a degree of stability of the operating parameter of the detection element 40. When the detection element 40 can stably sense breast milk, the operating parameter of the detection element 40 is a value that tends to be stable or fluctuates within an allowable range, which does not fluctuate or does not fluctuate beyond an allowable fluctuation threshold. In this case, the operating parameter of the detection element 40 is determined to be in a stable state. Conversely, when the operating parameter of the detection element 40 fluctuates beyond the allowable fluctuation threshold, the operating parameter of the detection element 40 is determined to be in an unstable state.

In step 402, taking a time as a criterion for determining stability of the operating parameter of the detection element 40 as an example, in some embodiments, specifically, step 402 includes following substeps:
(1). When the operating parameter of any detection element 40 is greater than or equal to the trigger threshold, a duration in which the operating parameter is greater than or equal to the trigger threshold is detected.

The "duration" refers a length of time the operating parameter of the detection element 40 is maintained in a state of being greater than the trigger threshold.

(2). When the duration is greater than a preset time, it is determined that the operating parameter of the detection element 40 is in a stable state.

The preset time is not limited, and may be specifically set based on actual usage. For example, the preset time may be three seconds to ten seconds. In an embodiment, a preset time of five seconds is used as an example. When the operating parameter of any detection element 40 can remain greater than or equal to the trigger threshold for more than five seconds, that is, the duration is greater than five seconds, the operating parameter of the detection element 40 is in a stable state. Conversely, if the duration is less than or equal to five seconds, the operating parameter of the detection element 40 is in an unstable state.

Step 404: When the operating parameter of the detection element 40 is in a stable state, it is determined that the detection element 40 is in a triggered state.

It should be noted that, the milk storage container portion 50 has N capacity positions. A capacity position is a virtual position defined on the milk storage container portion 50 according to positions corresponding to different capacities, eliminating a need for scale lines on the milk storage container portion 50. The N capacity positions have increasing capacities from a first capacity position to an N^{th} capacity position. At least one detection element 40 is arranged in each capacity position.

The capacity of the milk storage container portion 50 is not limited. For example, in some embodiments, the capacity of the milk storage container portion 50 is 150 ml. If the capacity of the milk storage container portion 50 is divided into 15 equal parts, the milk storage container portion 50 has 15 capacity positions. Specifically, a first capacity position corresponds to a position corresponding to a capacity of 10 ml of the milk storage container portion 50, a second capacity position corresponds to a position corresponding to 20 ml, and so on, and a 15^{th} capacity position corresponds to a position corresponding to 150 ml.

It is worth mentioning that each capacity position is defined as a virtual spatial position on the corresponding capacity level of the milk storage container portion. On the milk storage container portion, the capacity position may be represented by a physical capacity scale line, or the capacity position of the corresponding capacity level may be determined based on a liquid level of a different capacity level injected into the milk storage container portion. For example, in an embodiment in which the milk storage container portion is provided with capacity scale lines, a plurality of scale markings corresponding to different capacity levels are arranged on the capacity scale lines, and a position of each scale mark may be considered as a capacity position. In an embodiment in which the milk storage container portion is provided with no capacity scale lines, when a specific volume of liquid is injected, a liquid level displayed on the milk storage container portion may be considered as a capacity position. For example, if 10 ml of liquid is injected into the milk storage container portion, a liquid position of the 10 ml of liquid on the milk storage container portion may be considered as a capacity position corresponding to the capacity level of 10 ml.

It should be noted that the above embodiment is described in detail based on the milk storage container portion in FIG. 10. The capacity positions of the milk storage container portion in FIG. 10 are distributed at equal intervals in an extending direction of the milk storage container portion. Certainly, the capacity positions of the milk storage container portion may alternatively be arranged at unequal intervals. As shown in FIG. 11, capacity positions of a milk storage container portion 50a are arranged at unequal intervals. Therefore, a plurality of detection elements 40a are arranged at unequal intervals on the milk storage container portion 50a.

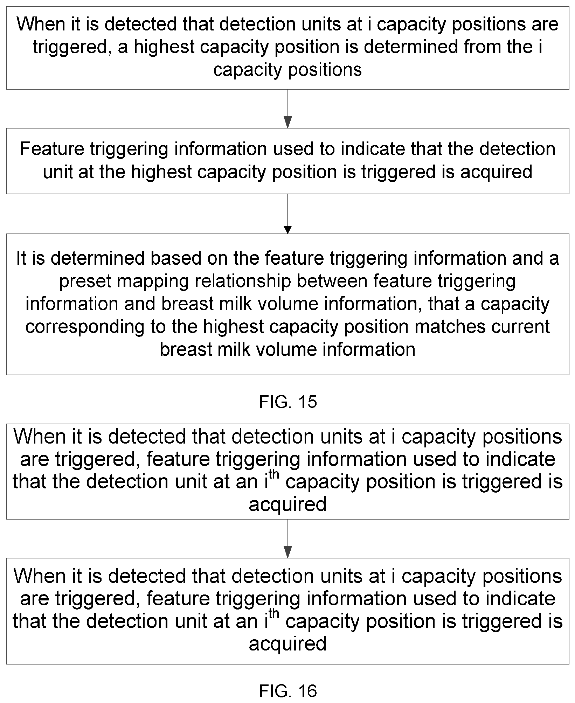

As shown in FIG. 10, FIG. 12, and FIG. 15, based on the arrangement of the capacity positions, in an embodiment, step 202 described above specifically includes following steps:
Step 502: When it is detected that detection elements 40 at i capacity positions are triggered, a highest capacity position is determined from the i capacity positions, where i is a natural number and 1≤i≤N.

Specifically, for example, the above milk storage container portion 50 has a capacity of 150 ml and 15 capacity positions. Assuming that all of the detection elements 40 on the milk storage container portion 50 are intact, if 100 ml of breast milk is stored in the milk storage container portion 50, the detection elements 40 at a first capacity position to a tenth capacity position are triggered. In this case, i=10, and a highest capacity position corresponding to a highest capacity value may be selected from the ten capacity positions. For example, if a capacity value corresponding to the tenth capacity position is the highest, the tenth capacity position corresponding to a capacity of 100 ml is determined as the highest capacity position.

Step 504: Feature triggering information used to indicate that the detection element 40 at the highest capacity position is triggered is acquired.

Specifically, for example, the tenth capacity position is used as the highest capacity position. In this case, feature triggering information of the detection element 40 triggered at the tenth capacity position is acquired, which is associated with the capacity of 100 ml corresponding to the tenth capacity position at which the detection element 40 is located.

Step 204 described specifically includes a following step:
Step 506: It is determined based on the feature triggering information and a preset mapping relationship between feature triggering information and breast milk volume information, that the capacity corresponding to the highest capacity position matches the current breast milk volume information.

Specifically, for example, when the feature triggering information indicates that the detection element 40 corresponding to the tenth capacity position is triggered, it is determined based on the tenth capacity position corresponding to the detection element 40 and the preset mapping relationship between feature triggering information and breast milk volume information that the capacity of 100 ml corresponding to the tenth capacity position matches the current breast milk volume information. That is, a detection result is that the current breast milk volume of the milk storage container portion 50 is 100 ml.

It should be noted that, in addition to the scenario where all of the detection elements 40 on the milk storage container portion 50 are intact, steps 502 to 506 described above are also applicable when some of the detection elements 40 on the milk storage container portion 50 are damaged. For example, if the detection element 40 at the tenth capacity position is damaged and therefore cannot be triggered, in step 502, when the milk storage container portion 50 has 110 ml of breast milk, the detection elements 40 at the first capacity position to a ninth capacity position and the detection element 40 at an eleventh capacity position are triggered, that is, a total of ten capacity positions are triggered. Among the ten capacity positions, the eleventh capacity position corresponds to a highest capacity value. Therefore, the eleventh capacity position corresponding to a capacity of 110 ml is determined as the highest capacity position. Therefore, in subsequent steps 504 to 506, the detection element 40 at the eleventh capacity position is triggered for calculation. A final detection result is that the current breast milk volume of the milk storage container portion 50 is 110 ml. As shown in FIG. 10, FIG. 3, and FIG. 7, in another embodiment, step 202 includes following steps:

Step 602: When it is detected that the detection elements 40 at the i capacity positions are triggered, feature triggering information used to indicate that the detection element 40 at an i^{th} capacity position is triggered is acquired, where i is a natural number and 1≤i≤N.

Specifically, for example, the above milk storage container portion 50 has a capacity of 150 ml and 15 capacity positions. Assuming that all of the detection elements 40 on the milk storage container portion 50 are intact, if 100 ml of breast milk is stored in the milk storage container portion 50, the detection elements 40 at a first capacity position to a tenth capacity position are triggered. In this case, i=10, and the feature triggering information of the detection element 40 triggered at the tenth capacity position is acquired. The feature triggering information is associated with the capacity of 100 ml corresponding to the tenth capacity position at which the detection element 40 is located.

Step 204 includes a following step:
Step 604: It is determined that based on the feature triggering information and the preset mapping relationship between feature triggering information and breast milk volume information, that the capacity corresponding to the i^{th} capacity position matches the current breast milk volume information.

Specifically, for example, when the feature triggering information indicates that the detection element 40 corresponding to the tenth capacity position is triggered, it is determined based on the tenth capacity position corresponding to the detection element 40 and the preset mapping relationship between feature triggering information and breast milk volume information that the capacity of 100 ml corresponding to the tenth capacity position matches the current breast milk volume information. That is, a detection result is that the current breast milk volume of the milk storage container portion 50 is 100 ml.

As shown in FIG. 10, FIG. 12, and FIG. 17, in another embodiment, only one detection element is arranged at each capacity position. In this case, step 202 includes following steps:

Step 702: It is detected that a number of triggered detection elements 40 is i, where i is a natural number and 1≤i≤N.

Specifically, when the milk storage container portion 50 contains 130 ml of breast milk, the detection elements 40 at the first capacity position to a thirteenth capacity position are triggered. That is, the number of triggered detection elements 40 is 13. In this case, i=13

Step 704: Feature triggering information indicating the number i of triggered detection elements 40 is acquired.

Specifically, for example, the milk storage container portion 50 contains 130 ml of breast milk. In this case, featuring trigger information indicating a number i=13 of triggered detection elements 40 is acquired.

Step 204 includes a following step:
Step 706: Current breast milk volume information matching a capacity corresponding to the i triggered detection elements 40 is calculated based on the feature triggering information and a function relationship between feature triggering information and breast milk volume information.

Specifically, for example, the number of triggered detection elements 40 indicated by the feature triggering information is i=13. A calculation is performed based on a function relationship of Q=i×a, where Q is the capacity corresponding to the i triggered detection elements 40, i is the number of triggered detection elements 40, and a is a capacity interval between two adjacent capacity positions. Herein, for example, the milk storage container portion 50 has a capacity of 150 ml and 15 capacity positions, and a=10 ml. In this case, Q=13×10 ml=130 ml. Current breast milk volume information matching the capacity of 130 ml corresponding to the 13 triggered detection elements 40 is obtained. That is, a detection result is that the current breast milk volume of the milk storage container portion 50 is 130 ml. It is worth mentioning that a manner of triggering a detection element is not limited. In addition to the above embodiment of determining whether the detection element triggered by automatically monitoring an operating parameter of the detection element, in some other embodiments, the detection element may be triggered manually.

In some embodiments, the method further includes:
displaying the current breast milk volume information and/or outputting the current breast milk volume information to an external terminal device.

Specifically, a manner of feeding back the breast milk volume information to the user is not limited, including but not limited to the following two manners:
(1). In addition to calculating the breast milk volume information, the breast milk flow rate detection unit in the above method can further directly display the breast milk volume information through a display module arranged on the breast milk flow rate detection unit, for viewing by the user.
(2). The breast milk flow rate detection unit can further output the current breast milk volume information to an external terminal device. The terminal device may be provided with a dedicated application (APP), allowing the user to view the breast milk volume information on a corresponding user interface (UI) of the APP.

It should be understood that although the steps in the flowcharts in the above embodiments are shown in sequence as indicated by the arrows, these steps are not necessarily performed in sequence as indicated by the arrows. The execution order of these steps is not strictly limited, and these steps may be performed in other orders, unless clearly described otherwise. Moreover, at least some of the steps in the flowcharts in the above embodiments may include a plurality of steps or stages. These steps or stages are unnecessarily performed at the same time, but may be executed at different times. These steps or stages are unnecessarily performed sequentially, but may be performed alternately with other steps or at least some of the steps or stages of other steps.

Based on the same inventive concept, an embodiment of this application further provides a breast milk flow rate detection unit configured to implement the breast milk volume detection method described above. An implementation solution provided by the device for resolving the problem is similar to the implementation described in the above method. Therefore, for specific limitations on one or more embodiments of the breast milk flow rate detection unit provided below, reference may be made to the limitations on the breast milk flow rate detection method above. Details are not described herein.

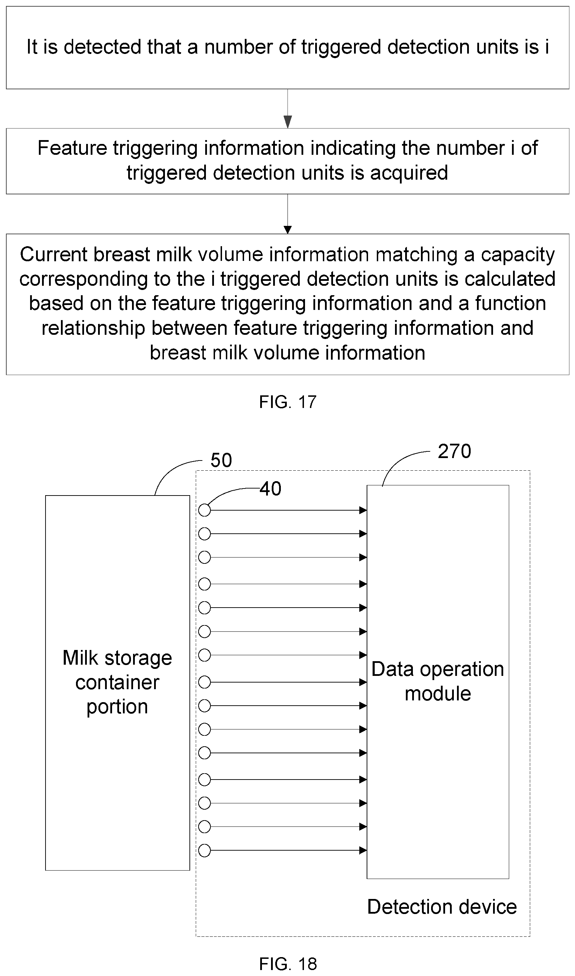

In an embodiment, as shown in FIG. 10 and FIG. 18, a breast milk volume detection device 100 is provided, including a detection element 40 and a data operation module 270.

The detection element 40 is configured to sense breast milk in the milk storage container portion; and

The data operation module 270 is configured to: acquire feature triggering information associated with a triggering status of the detection element 40, and determine current breast milk volume information based on the feature triggering information and a preset relationship, where the preset relationship is used to represent a mapping relationship and/or a function relationship between feature triggering information and breast milk volume information, and the breast milk volume information is used to represent a current breast milk volume accommodated in the milk storage container portion.

In an embodiment, the data operation module is further configured to: detect an operating parameter of any detection element 40 when sensing the breast milk in the milk storage container portion, and determine, when the operating parameter of any detection element 40 is greater than a preset trigger threshold, that the detection element 40 is triggered.

In an embodiment, the data operation module is further configured to: detect, when the operating parameter of any detection element is greater than the preset trigger threshold, stability of the operating parameter of the detection element, and determine that the detection element is in a triggered state when the operating parameter of the detection element is in a stable state.

In an embodiment, the data operation module is further configured to: detect, when the operating parameter of any detection element is greater than the trigger threshold, a duration in which the operating parameter is greater than the trigger threshold; and determine that the detection element is in a stable state when the duration is greater than a preset time.

In addition, a side wall of the milk storage container portion has N capacity positions having increasing capacities from a first capacity position to an N^{th} capacity position, and at least one detection element 40 is arranged at each capacity position.

The data operation module is further configured to: determine a highest capacity position from i capacity positions when it is detected that detection elements 40 at the i capacity positions are triggered, where i is a natural number and 1≤i≤N; obtain feature triggering information indicating that a detection element 40 at the highest capacity position is triggered; and determine, based on the feature triggering information and a preset mapping relationship between feature triggering information and breast milk volume information, that a capacity corresponding to the highest capacity position matches the current breast milk volume information.

In an embodiment, the milk storage container portion has N capacity positions having increasing capacities from a first capacity to an N^{th} capacity position. At least one detection element 40 is arranged in each capacity position.

The data operation module is further configured to: acquire, when detection elements 40 at i capacity positions are triggered, feature triggering information used to indicate that a detection element 40 at an ith capacity position is triggered is acquired, where i is a natural number and 1≤i≤N; and determine, based on the feature triggering information and the preset mapping relationship between feature triggering information and breast milk volume information, that a capacity corresponding to the i^{th} capacity position matches the current breast milk volume information.

In addition, the milk storage container portion has N capacity positions having increasing capacities from a first capacity position to an N^{th} capacity position, and one detection element 40 is arranged at each capacity position.

The data operation module is further configured to: detect that a number of triggered detection elements 40 is i, where i is a natural number and 1≤i≤N; obtain feature triggering information representing the number i of triggered detection elements 40; and calculate, based on the feature triggering information and the function relationship between feature triggering information and breast milk volume information, current breast milk volume information matching a capacity corresponding to the i triggered detection elements 40.

All or some of the modules in the above breast milk flow rate detection unit may be implemented by software, hardware, or a combination thereof. The above modules may be built in or independent of a processor of a computer device in a form of hardware, or may be stored in a memory of the computer device in a form of software, for invoke by the processor to perform the operations corresponding to the above modules.

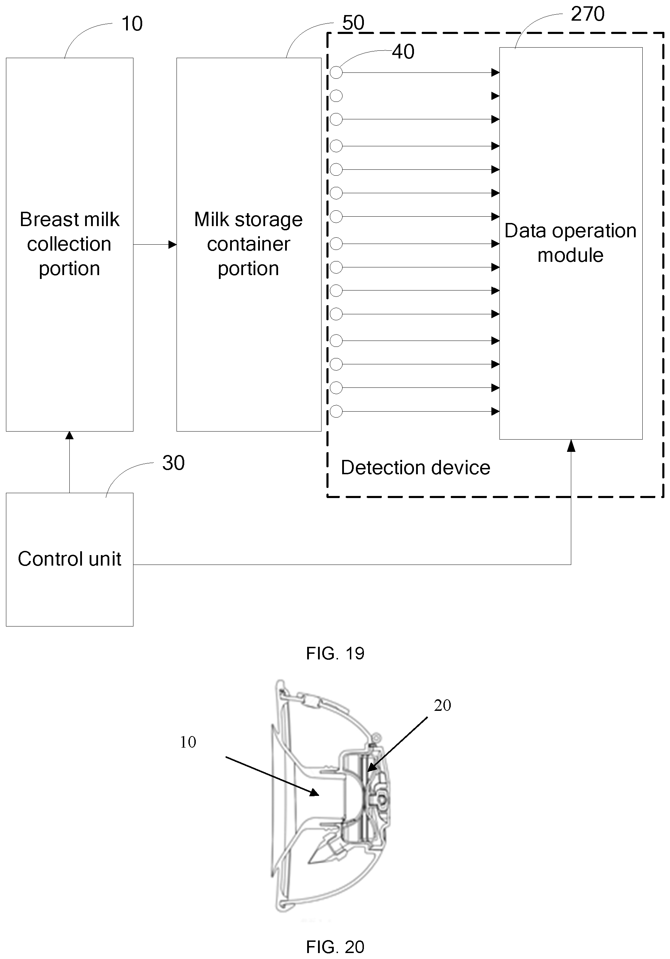

In an embodiment, as shown in FIG. 19 to FIG. 20, a breast pump 900 is provided, including a breast milk collection portion 10, a milk storage container portion 50, the above detection device, and a control unit 30.

The breast milk collection portion 10 is configured to pump milk from a breast of a user.

The milk storage container portion 50 communicates with the breast milk collection portion and configured to store the breast milk.

The above detection device is configured to detect a volume of the breast milk stored in the milk storage container portion 50.

The control unit 30 is electrically connected to the breast milk collection portion 10 and the detection device respectively, and is configured to control the breast milk collection portion 10 to squeeze the breast of the user, to cause the breast to secrete milk, and is further configured to control the detection device to detect a volume of breast milk flowing into the milk storage container portion 50.

Specifically, the breast milk collection portion 10 further includes a breast shield (not shown in the figure) and a negative pressure mechanism (not shown in the figure). The breast shield is configured to accommodate a women's breast to perform a milk expressing operation. The negative pressure mechanism is configured to form negative pressure under control of the control unit to squeeze the breast of the user, so as to cause the breast to secrete milk, and pump the breast milk.

It is worth mentioning that, in some embodiments, the control unit 30 and the data operation module 270 of the detection device may be integrated onto a same circuit board (that is, a main board). Certainly, in other embodiments, the two may be located on different circuits.

### Third embodiment

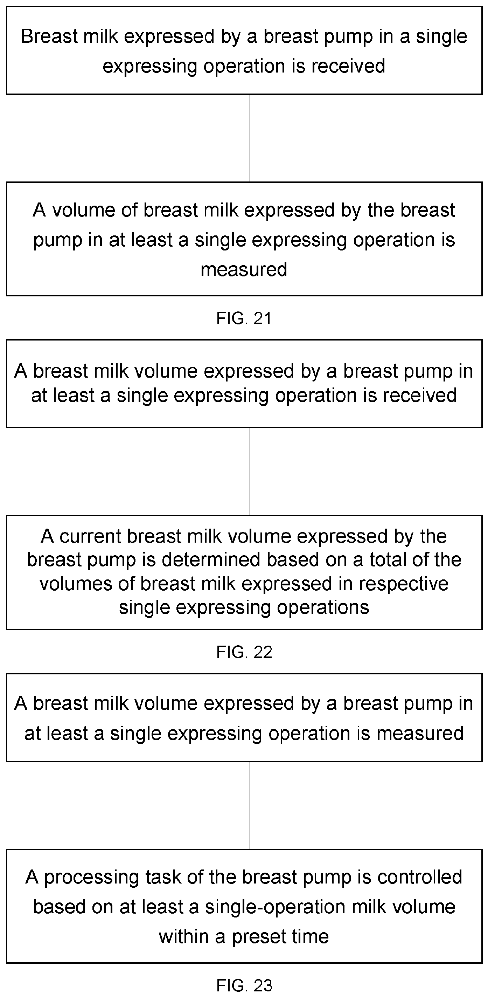

As shown in FIG. 21, the present disclosure further provides a breast milk volume detection method, applied to a breast pump and including: acquiring a detection parameter detected by a detection unit; and performing a preset operation based on the detection parameter.

In this embodiment, the acquiring a detection parameter detected by a detection unit includes: receiving breast milk expressed by the breast pump in a single expressing operation, measuring a volume of breast milk expressed by the breast pump in at least a single expressing operation, and obtaining a breast milk volume detection parameter.

In this embodiment, the breast pump utilizes negative pressure generated by a negative pressure mechanism to pump breast milk. The negative pressure mechanism operates by alternating between expressing and pressure release in a cyclic manner: one expressing operation followed by one pressure release. The breast milk obtained in a single expressing operation refers to breast milk obtained by the negative pressure mechanism of the breast pump from one expressing operation. The breast milk obtained in a single expressing operation of the negative pressure mechanism can instantly reflect a milk expressing status of the breast of the user in a current operating mode of the breast pump. In a specific embodiment of the present disclosure, the breast milk expressed by the breast pump in a single expressing operation may be received once, or may be received two or three times.

In a specific embodiment of the present disclosure, that the breast milk obtained in a single expressing operation refers to breast milk obtained by the negative pressure mechanism of the breast pump from one expressing operation means that the breast milk obtained in a single expressing operation is obtained between two adjacent expressing operations of the negative pressure mechanism of the breast pump, or may be understood as that the breast milk that is obtained by the breast pump in a single expressing operation and that is received between two adjacent expressing operations of the negative pressure mechanism of the breast pump, where a time of the receipt is controlled between two adjacent expressing operations of the negative pressure mechanism.

In this embodiment, a volume of breast milk expressed by the breast pump in at least a single expressing operation is measured. Specifically, a detection unit such as a capacitive sensor, a resistive sensor, or a photoelectric sensor is utilized to measure a volume, a weight, or other parameters of the breast milk obtained in a single expressing operation. Specifically, the volume of the breast milk obtained in a single expressing operation is referred to as a single-operation breast milk volume. Single-operation breast milk volume information includes a single-operation breast milk volume value, information about a relationship between a single-operation breast milk volume value and a time, or scattered information of the single-operation breast milk volume value.

In a specific embodiment of the present disclosure, after the volume of the breast milk obtained in a single expressing operation is measured, the breast milk obtained in a single expressing operation is released for collection into the milk storage container portion. Breast milk is released after each measurement is completed for a next measurement.

As shown in FIG. 22, in the present disclosure, based on the measurement of the volume of the breast milk that is obtained by the breast pump in at least a single expressing operation and that is received, a total of the volumes of breast milk obtained in at least respective single expressing operations (a total breast milk volume) is calculated. Specifically, the volume of the breast milk obtained in a single expressing operation is measured, and then a current breast milk volume expressed by the breast pump (a total breast milk volume) is determined based on a total volume of breast milk obtained in all single expressing operations.

The breast milk volume detection in this embodiment of the present disclosure is based on the measurement of breast milk obtained in a single expressing operation, specifically, measurement of a volume of breast milk obtained in a single expressing operation. Therefore, an obtained value of the total breast milk volume is very accurate with few errors.

As shown in FIG. 23, a control method for a breast pump according to an embodiment of the present disclosure includes following:
A volume of breast milk that is obtained by the breast pump in a single expressing operation and that is received is measured. In a specific embodiment of the present disclosure, specifically, a volume of breast milk obtained by the negative pressure mechanism of the breast pump from one expressing operation is measured. The volume of the breast milk obtained in a single expressing operation is a single-operation breast milk volume.

A processing task of the breast pump is controlled based on at least a single-operation breast milk volume (a volume of breast milk obtained in a single expressing operation) within a preset time.

Information about the at least a single-operation breast milk volume includes a single-operation breast milk volume value, information about a relationship between a single-operation breast milk volume and a time, and scattered information of the single-operation breast milk volume value. The at least a single-operation breast milk volume within the preset time refers to a single-operation breast milk volume value, information about a relationship between a single-operation breast milk volume and a time, and scattered information of the single-operation breast milk volume value within a fixed time period, such as one day, two days, three days, or scattered time points within a fixed time period.

In this embodiment of the present disclosure, the processing task of the breast pump includes adjusting an operating parameter of the negative pressure mechanism of the breast pump and adjusting an operating mode of the breast pump.

The intelligent control method for a breast pump in this embodiment of the present disclosure is to intelligently control the breast pump to operate based on the volume of the breast milk obtained in a single expressing operation (a single-operation breast milk volume). Because the breast milk obtained in a single expressing operation is the breast milk obtained by the negative pressure mechanism from one expressing operation, information about the volume of the breast milk obtained in a single expressing operation can instantly reflect a milk expressing status of the breast of the user in a current operating mode of the breast pump. The information about the single breast milk volume includes a single-operation breast milk volume value, information about a relationship between a single-operation breast milk volume value and a time, and the scattered information of the single-operation breast milk volume value (in this specification, the single-operation breast milk volume refers to a volume of breast milk obtained in a single expressing operation). For example, if the volume of the breast milk obtained in a single expressing operation is large, it indicates that a current physical status of the user adapts to a current operating task of the breast pump. For example, if the volume of the breast milk obtained in a single expressing operation is small, it indicates that the current physical status of the user does not adapt to the current operating task of the breast pump. The breast pump can intelligently adjust the operating parameter of the negative pressure mechanism or the operating mode of the breast pump to enable the user to successfully pump breast milk under the operating task adapting to the breast pump.

The information about the relationship between a single-operation breast milk volume value and a time provides feedback on an operating status of the negative pressure mechanism during use of the breast pump by the user within a fixed time period. Based on this information, the operating parameter of the negative pressure mechanism of the breast pump, such as a duration of a single expressing operation of the negative pressure mechanism, a negative pressure or vacuum level of a single expressing operation, a peak negative pressure or peak vacuum level of a single expressing operation, and a pumping cycle or frequency may be adjusted. If the single-operation breast milk volume is consistently large within the fixed time period, the negative pressure or vacuum level of a single expressing operation is increased, or the duration of a single expressing operation of the negative pressure mechanism is reduced, or the expressing frequency is increased, or the expressing time is reduced, or the like.

The scattered information of the single-operation breast milk volume value reflects an operating status of the negative pressure mechanism at scattered time points during the use of the breast pump by the user. This can represent a milk expressing status of the mother at scattered time points, thereby intelligently prompting the user of an optimal time to use the breast pump. For example, based on a magnitude of a single-operation breast milk volume value at each of 8 a.m., 9 a.m., and 10 a.m., an intelligent prompt is sent to the user that an optimal time to use the breast pump is a breast milk expressing time line in the morning. Alternatively, a current single-operation breast milk volume value at 8 a.m is compared with a historical single-operation breast milk volume value at 8 a.m. If the current single-operation breast milk volume is larger, it indicates that a current processing effect of the breast pump during use by the user is good. If the current single-operation breast milk volume value at 8 a.m is less than the historical single-operation breast milk volume value at 8 a.m, it indicates that the current processing effect of the breast pump during the use by the user is not good. Therefore, the processing task of the breast pump needs to be adjusted. That is, the operating mode of the breast pump needs to be adjusted.

In the present disclosure, the processing task of the breast pump is controlled based on information about the volume of the breast milk obtained in a single expressing operation, to promote use of the information about the volume of the breast milk obtained in a single expressing operation and enhance intelligent use of the breast pump. As the volume of the breast milk obtained in a single expressing operation can indicate the current status of the user in a timely and real-time manner, the intelligent control method for a breast pump of the present disclosure is more intelligent, advanced, prompt, and effective, improving user experience.

In some embodiments of the present disclosure, the operating frequency of the negative pressure mechanism of the breast pump is adjusted based on a relationship between a single-operation breast milk volume value and a preset breast milk volume threshold. The preset breast milk volume threshold may include a single breast milk volume threshold or a plurality of breast milk volume thresholds. A plurality of breast milk volume thresholds may be set based on actual usage, to adapt to different levels of processing tasks of the breast pump.

In this embodiment of the present disclosure, the preset breast milk volume threshold includes a first breast milk volume threshold and a second breast milk volume threshold, both of which may be set based on an average volume of breast milk obtained from the mother in a single expressing operation. For ease of description, in the present disclosure, exemplarily, the first breast milk volume threshold is greater than the average volume of the breast milk obtained from the mother in a single expressing operation, and the second breast milk volume threshold is less than the average volume of the breast milk obtained from the mother in a single expressing operation.

The average volume of the breast milk obtained from the mother in a single expressing operation may alternatively be obtained by calculating an average of a plurality of measurements of a volume of expressed breast milk obtained through the breast milk volume detection method in the embodiments of the present disclosure.

The average single breast milk expressing volume of the mother may alternatively be estimated in advance based on an average daily breast milk secretion volume of the lactating mother, a daily number of breast-feeding operations, and a number of milk expressing operations during single-operation use of the breast pump, which is preset by the control unit of the breast pump. For instance, a normal lactating mother secretes 600 mL to 1500 mL of breast milk a day, with a daily breast-feeding frequency of 4 to 6, and each use of the breast pump involves 100 to 150 expressing operations (that is, a number of adjacent expressing operations of the negative pressure mechanism 20). In this case, an estimated average single breast milk expressing volume of the mother is 0.7 mL to 3.75 mL. Exemplarily, the second breast milk volume threshold may be set to at 2 mL, and the first breast milk volume threshold may be set at 4 mL.

The operating frequency of the negative pressure mechanism of the breast pump is adjusted based on the relationship between a single-operation breast milk volume value and a preset breast milk volume threshold. A specific operation is as follows:
When it is determined that the single-operation breast milk volume value exceeds the first breast milk volume threshold, it indicates abundant milk production from the breast (such as a gushing flow), which signifies good milk flow. In this case, the expressing frequency may be increased to complete the breast milk expressing quickly, to shorten an operating time of the breast pump.

When it is determined that the single-operation breast milk volume value falls between the second breast milk volume threshold and the first breast milk volume threshold, the current operating frequency of the negative pressure mechanism of the breast pump is maintained.

In some embodiments of the present disclosure, controlling the processing task of the breast pump based on the information about the volume of the breast milk obtained in a single expressing operation further includes:
adjusting the operating mode of the breast pump based on the relationship between a single-operation breast milk volume value and the preset breast milk volume threshold, where the operating mode of the breast pump includes a milk expressing mode and a lactation stimulation mode.

A specific operation is as follows:
When it is determined that the single-operation breast milk volume value is less than the second breast milk volume threshold, the breast pump is adjusted from the milk expressing mode to the lactation stimulation mode, to start the lactation stimulation mode.

Furthermore, in some embodiments of the present disclosure, the preset breast milk volume threshold is set to only a single value, namely, a third breast milk volume threshold, which is close to the average single breast milk expressing volume of the mother.

When it is determined that the single-operation breast milk volume value is less than the third breast milk volume threshold, the breast pump is adjusted from the milk expressing mode to the lactation stimulation mode, to start the lactation stimulation mode. In addition, when it is determined that the single-operation breast milk volume exceeds the third breast milk volume threshold, the lactation stimulation mode is ended, the milk expressing mode is started, and a power of an air pump is adjusted, to ensure that a current power of the negative pressure mechanism is appropriate for milk expressing of the breast of the user.

The preset breast milk volume threshold of the present disclosure may be set to a plurality of values, and the operating mode of the breast pump is intelligently adjusted based on comparison between the single-operation breast milk volume and the plurality of values. In this embodiment of the present disclosure, the lactation stimulation mode includes one or more of following manners: massage on the breast, electrical stimulation on the breast, and thermal stimulation on the breast.

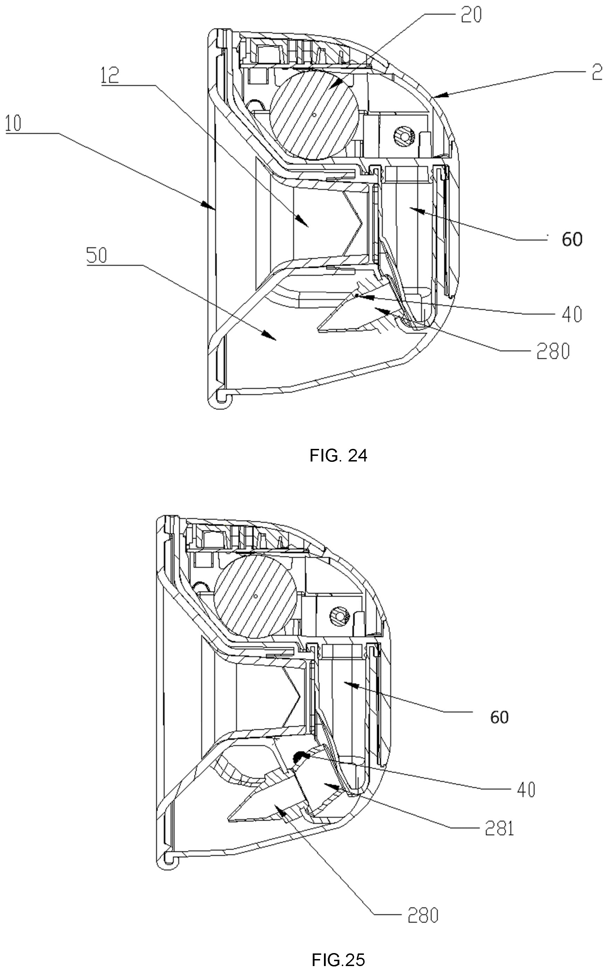

As shown in FIG. 24, a breast pump 1 according to an embodiment of the present disclosure includes a main unit 2, a breast milk collection portion 20, a measurement container, a detection unit 40, and a milk storage container portion 50.

The main unit 2 includes a negative pressure mechanism 20 configured to generate negative pressure and a control unit 30. The negative pressure mechanism 20 operates by alternating between expressing and pressure release in a cyclic manner: one expressing operation followed by one pressure release. The control unit 30 is an art well-known to those skilled in the art, which is not described in detail in the present disclosure.

The breast milk collection portion 20 is configured to come into contact with a breast of a user and pump breast milk under the negative pressure of the negative pressure mechanism 20.

The measurement container is configured to receive the breast milk from the breast milk collection portion 20. In this embodiment of the present disclosure, the measurement container receives breast milk expressed by the breast milk collection portion 20 in a single expressing operation, releases the breast milk obtained in a single expressing operation after a measurement operation is completed, and receives another piece of breast milk obtained in a single expressing operation, and so on.

The detection unit 40 is configured to detect a single-operation breast milk volume of the breast milk that is obtained by the breast milk collection portion 20 in a single expressing operation and that is received in the measurement container.

The milk storage container portion 50 is detachably connected to the breast milk collection portion 20, and is configured to hold breast milk released from the measurement container. The milk storage container portion 50 may be made of hard or soft materials, and a shape of the container is fixed or irregular. For example, in this embodiment of the present disclosure, the container is bowl-shaped.

The control unit 30 is electrically connected to the detection unit 40, and the control unit 30 is configured to receive information about the single-operation breast milk volume and control a processing task of the breast pump. In this embodiment, the information about the single-operation breast milk volume includes a single-operation breast milk volume value, information about a relationship between a single-operation breast milk volume and a time, and scattered information of the single-operation breast milk volume value.

In this embodiment of the present disclosure, the breast milk collection portion 20 includes a breast shield 21 and a diaphragm 60.

The breast shield 21 is configured to be attached to the breast of the user.

The diaphragm 60 is arranged opposite the breast shield 21 and communicates with the measurement container.

In this embodiment of the present disclosure, the measurement container includes a one-way valve 280, which is used as the measurement container. The one-way valve receives the breast milk expressed by the breast milk collection portion 20 in a single expressing operation, releases the breast milk obtained in a single expressing operation after a measurement operation is completed, and receives another piece of breast milk obtained in a single expressing operation, and so on.

In this embodiment of the present disclosure, the one-way valve 280 is mounted inside the milk storage container portion, and is connected to the diaphragm 60.

As shown in FIG. 25, in some other embodiments of the present disclosure, the breast milk collection portion 20 includes a breast shield 21, a diaphragm 60, and a one-way valve 280. The diaphragm 60 is arranged opposite the breast shield 21. The one-way valve 280 communicates with the diaphragm 60 and the measurement container 800.

The breast shield 21 is configured to be attached to the breast of the user. The expressed breast milk reaches the diaphragm 60 along a nipple channel and enters the measurement container 800. In this embodiment of the present disclosure, the measurement container is a conduit 281. That is, in the present disclosure, the conduit 281 receives breast milk expressed by the breast milk collection portion 20 in a single expressing operation, releases the breast milk obtained in a single expressing operation after a measurement operation is completed, and receives another piece of breast milk obtained in a single expressing operation, and so on.

In this embodiment of the present disclosure, the conduit 281 is fixed to an inner wall of the milk storage container portion 50 through a support. Alternatively, the conduit 281 may be directly arranged the inner wall of the milk storage container portion 50.

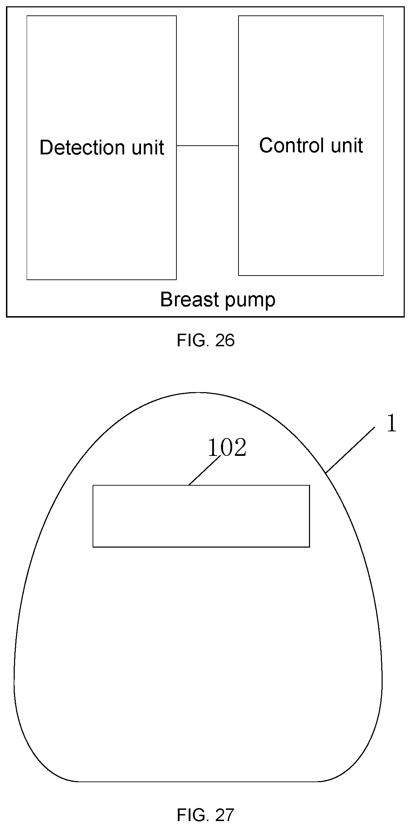

As shown in FIG. 26, in the breast pump in this embodiment of the present disclosure, the detection unit 40 is electrically connected to the control unit to feed back the information about the single-operation breast milk volume to the control unit. The detection unit 40 includes a capacitive sensor, a resistive sensor, or a photoelectric sensor.

The capacitive sensor, the resistive sensor, or the photoelectric sensor is mounted on the one-way valve 280 or the conduit 281.

In some embodiments of the present disclosure, when the measurement container is a one-way valve 280, the capacitive sensor, the resistive sensor, or the photoelectric sensor is arranged on the one-way valve 280.

In some embodiments of the present disclosure, when the measurement container is a conduit 281, the capacitive sensor, the resistive sensor, or the photoelectric sensor is arranged on the conduit 281.

A measurement principle of the capacitive sensor is to obtain a pressure parameter (a single-operation breast milk volume) based on a change in a capacitance. A specific structure of the capacitive sensor is well-known to those skilled in the art. Therefore, details are not described in the present disclosure.

A measurement principle of the resistive sensor is to obtain a pressure parameter (a single-operation breast milk volume) based on a change in a resistance. A specific structure of the capacitive sensor is well-known to those skilled in the art. Therefore, details are not described in the present disclosure.

A measurement principle of the photoelectric sensor is to obtain a volume or a height of breast milk in the measurement container based on a change in a light signal. A specific structure of the photoelectric sensor is well-known to those skilled in the art. Therefore, details are not described in the present disclosure.

### Fourth embodiment

A breast pump 1 further includes a control unit 30. The control unit 30 is electrically connected to a breast shield 10, and is configured to: acquire a user parameter; determine an operating parameter of the breast shield 10 during operation based on the user parameter; and control the breast shield 10 to operate based on the operating parameter, where the user parameter includes a pressure tolerance, and the operating parameter includes a milk expressing intensity and a milk expressing duration, where the milk expressing intensity is positively correlated with the pressure tolerance, and the milk expressing duration is negatively correlated with the pressure tolerance.

Referring to FIG. 27, as shown in FIG. 27, FIG. 27 is a first schematic structural diagram of a breast pump according to this application. In FIG. 27, the breast pump 1 includes an input unit 102 configured to input a user parameter.

Furthermore, the control unit 20 is electrically connected to the input unit 102, and the input unit 102 is configured to generate the user parameter in response to an input instruction of a user.

Through the input unit, the user can directly input the user parameter through the breast pump, and adjust an operating parameter of the breast pump, to cause the operating parameter of the breast pump to be more in line with a need of the user.

In some implementations, a housing further includes a display unit 101 electrically connected to the control unit 20. The display unit 101 includes at least one of a first display region 106 or a second display region 107. The first display region 106 is configured to display the user parameter, and the second display region 107 is configured to display the operating parameter.

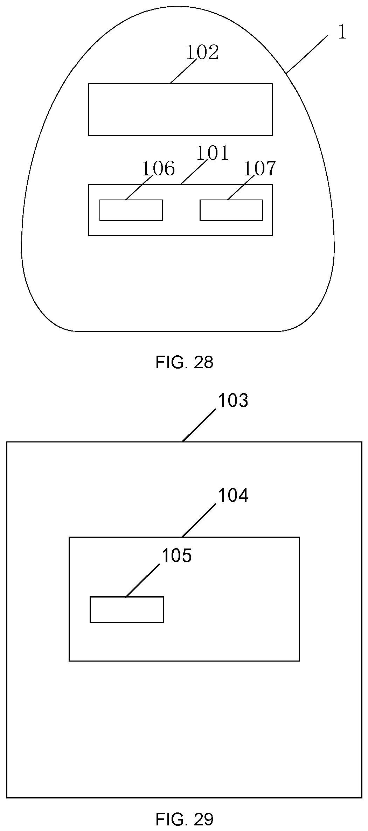

In some implementations, the display unit 101 exists independently of the input unit. For reference, refer to FIG. 28, FIG. 28 is a second schematic structural diagram of a breast pump according to this application. As shown in FIG. 28, in FIG. 28, the breast pump in FIG. 28 is provided with both an input unit 102 and a display unit 101.

In some implementations, the display unit 101 and the input unit 102 are integrated into a same panel, defining the combination of the display unit 101 and the input unit 102 as an operation panel. In this case, the user may input a user parameter through the operation panel and view the first display region and/or the second display region through the operation panel.

In some implementations, the breast pump further includes a communication unit electrically connected to the control unit. The communication unit is configured to establish communication with an external terminal. The control unit is connected to the communication unit.

In some implementations, the breast pump includes both a communication unit and an input unit.

Arranging the communication unit on the breast pump to communicate with the external terminal allows the user to set a user parameter through a mobile APP and the like, to improve convenience of using the breast pump.

In some implementations, the user parameter includes at least one of an age, a breast size, or a pressure tolerance.

In some implementations, the operating parameter includes at least one of a lactation stimulation parameter or a milk expressing parameter.

In some implementations, the lactation stimulation parameter includes at least one of a lactation stimulation frequency, a lactation stimulation intensity, or a lactation stimulation duration.

Increasing any one of the lactation stimulation frequency, the lactation stimulation intensity, or the lactation stimulation duration can help a mother secrete more breast milk, to increase a breast milk volume expressed during subsequent milk expressing.

Referring to FIG. 29, FIG. 29 is a schematic structural diagram of a terminal according to an embodiment of this application. As shown in FIG. 29, a terminal 103 includes a control interface 104.

In some implementations, the control interface 104 includes an input control 105 configured to input the user parameter.

In some implementations, when the user parameter includes a plurality of sub-parameters, the control interface includes a plurality of input controls 105.

For example, when the user parameter includes an age, a breast size, and a pressure tolerance, the control interface includes three input controls 105 respectively configured to input the age, the breast size, and the pressure tolerance.

In some implementations, the control interface 104 further includes a third display region configured to display a current operating parameter of the breast pump.

In some implementations, the control interface 104 further includes a fourth display region configured to display an operating parameter corresponding to the user parameter after the user inputs the user parameter.

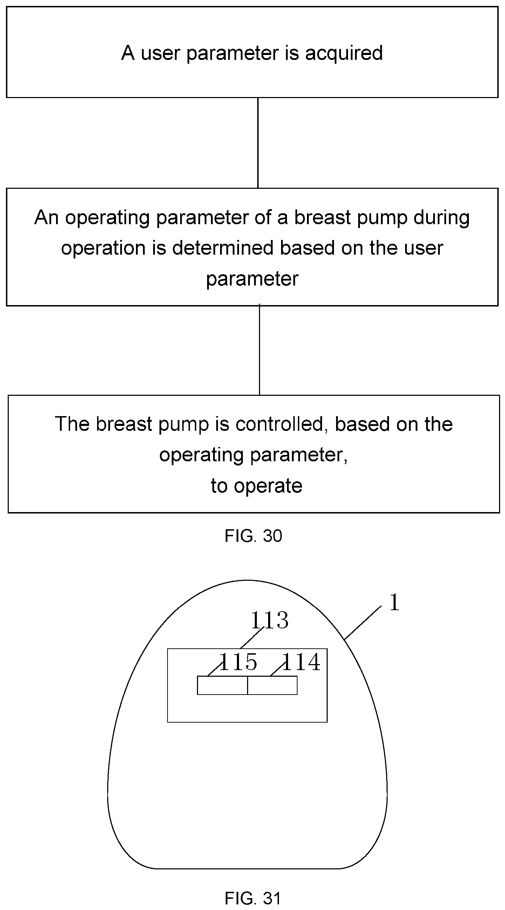

Referring to FIG. 30, FIG. 30 is a schematic flowchart of a control method according to an embodiment of this application. As shown in FIG. 30, the operation control method for a breast pump includes a first step to a third step.

First step: A user parameter is acquired, where the user parameter includes a pressure tolerance.

In some implementations, the first step includes following steps.
(1). The user parameter generated by the input unit of the breast pump in response to the input instruction of the user is acquired.
(2). Alternatively, the user parameter sent by the external terminal is acquired.

Second step: An operating parameter of the breast pump during operation is determined based on the user parameter, where the operating parameter includes a milk expressing parameter that includes a milk expressing intensity and a milk expressing duration, where the milk expressing intensity is positively correlated with the pressure tolerance, and the milk expressing duration is negatively correlated with the pressure tolerance.

In some implementations, the user parameter includes at least one of an age, a breast size, or a pressure tolerance.

In some implementations, the operating parameter includes at least one of a lactation stimulation parameter or a milk expressing parameter.

In some implementations, the lactation stimulation parameter includes at least one of a lactation stimulation frequency, a lactation stimulation intensity, or a lactation stimulation duration.

Increasing any one of the lactation stimulation frequency, the lactation stimulation intensity, or the lactation stimulation duration can help a mother secrete more breast milk, to increase a breast milk volume expressed during subsequent milk expressing.

In some implementations, the milk expressing parameter includes at least one of a milk expressing frequency, a milk expressing intensity, or a milk expressing duration.

Increasing any one of the milk expressing frequency, the milk expressing intensity, or the milk expressing duration can allow the breast pump to pump more breast milk when the milk expressing is ended.

In some implementations, the breast pump has a reference operating parameter. If the breast pump does not obtain the user parameter, that is, if the user does not input the user parameter, the breast pump operates based on the reference operating parameter.

Furthermore, the reference operating parameter includes at least one of a default lactation stimulation parameter or a default milk expressing parameter.

Exemplarily, the operating parameter is a lactation stimulation parameter. Since an older age is generally associated with lower breast milk secretion, to ensure an adequate volume of expressed breast milk, if the user is of an age greater than the default lactation stimulation parameter, at least one of the lactation stimulation frequency, the lactation stimulation intensity, or the lactation stimulation duration is increased, to increase a breast milk volume expressed during subsequent milk expressing.

Furthermore, if the user is of an age less than the default lactation stimulation parameter, at least one of the lactation stimulation frequency, the lactation stimulation intensity, or the lactation stimulation duration is reduced, to prevent an excessive volume of breast milk from being expressed during subsequent milk expressing, thereby preventing hindering a future weaning process of the mother.

Exemplarily, the operating parameter is a breast size. Since a small breast size is generally associated with lower breast milk secretion, to ensure an adequate volume of expressed breast milk, if the breast size is less than the default lactation stimulation parameter, at least one of the lactation stimulation frequency, the lactation stimulation intensity, or the lactation stimulation duration is increased, to increase a breast milk volume expressed during subsequent milk expressing.

Furthermore, if the breast size is greater than the default lactation stimulation parameter, at least one of the lactation stimulation frequency, the lactation stimulation intensity, or the lactation stimulation duration is reduced, to prevent an excessive volume of breast milk from being expressed during subsequent milk expressing, thereby preventing hindering a future weaning process of the mother.

For example, a larger pressure tolerance may withstand a larger lactation stimulation pressure. In this case, if the pressure tolerance is greater than the default lactation stimulation parameter, at least one of the lactation stimulation frequency or the lactation stimulation intensity is increased, and the lactation stimulation duration is reduced, to complete the lactation stimulation more quickly and reduce a time to use the breast pump for a user with a large pressure tolerance.

Furthermore, if the pressure tolerance is less than the default lactation stimulation parameter, at least one of the lactation stimulation frequency or the lactation stimulation intensity is reduced, and the lactation stimulation duration is increased, to complete the lactation stimulation with a gentler pressure, thereby preventing the user from feeling pain during the use of the breast pump.

Exemplarily, the operating parameter is a milk expressing parameter. Since an older age is generally associated with lower breast milk secretion, to ensure an adequate volume of expressed breast milk, If the user is of an older age greater than the default user parameter, at least one of the milk expressing frequency, the milk expressing intensity, or the milk expressing duration is increased, to increase a breast milk volume expressed during subsequent milk expressing.

Furthermore, if the user is of an age less than the default user parameter, at least one of the milk expressing frequency, the milk expressing intensity, or the milk expressing duration is reduced, to prevent an excessive volume of breast milk from being expressed during milk expressing, thereby preventing hindering a future weaning process of the mother.

Exemplarily, the operating parameter is a breast size. Since a small breast size is generally associated with lower breast milk secretion, to ensure an adequate volume of expressed breast milk, if the breast size is less than the default user parameter, at least one of the milk expressing frequency, the milk expressing intensity, or the milk expressing duration is increased, to increase a breast milk volume expressed during milk expressing.

Furthermore, if the breast size is greater than the default user parameter, at least one of the milk expressing frequency, the milk expressing intensity, or the milk expressing duration is reduced, to prevent an excessive volume of breast milk from being expressed during subsequent milk expressing, thereby preventing hindering a future weaning process of the mother.

For example, a larger pressure tolerance may withstand a larger milk expressing pressure. If the pressure tolerance is larger than the default milk expressing parameter, at least one of the milk expressing frequency or the milk expressing intensity is increased, and the milk expressing duration is reduced, to complete the milk expressing more quickly and reduce a time to use the breast pump for a user with a large pressure tolerance.

Furthermore, if the pressure tolerance is less than the default user parameter, at least one of the milk expressing frequency or the milk expressing intensity is reduced, and the milk expressing duration is increased, to complete the milk expressing with a gentler pressure, thereby preventing the user from feeling pain during the use of the breast pump.

In some implementations, the user parameter does not need to be compared with the default user parameter. Instead, after the user parameter is acquired, a corresponding operating parameter may be queried from a pre-stored correspondence table between a user parameter and an operating parameter based on the user parameter.

For example, after the pressure tolerance is acquired, a corresponding milk expressing intensity and milk expressing duration are queried from a correspondence table between a pressure tolerance and a milk expressing intensity and a milk expressing duration based on the pressure tolerance.

In some implementations, the pre-stored correspondence table between a user parameter and an operating parameter is obtained through testing based on a correlation between user parameters and operating parameters in the table.

In some implementations, the milk expressing parameter further includes a milk expressing frequency, and the determining an operating parameter of the breast pump during operation based on the user parameter includes:
determining the milk expressing frequency of the breast pump during operation based on the pressure tolerance, where the pressure tolerance is positively correlated with the milk expressing frequency.

In some implementations, the milk expressing frequency is directly queried from the correspondence table between a pressure tolerance and a milk expressing frequency based on the pressure tolerance.

In some implementations, the pressure tolerance is compared with a default pressure tolerance in the default user parameter, and a final milk expressing frequency is obtained through adjustment of a default milk expressing frequency based on a comparison result.

In some implementations, the operating parameter further includes a lactation stimulation parameter, and the determining an operating parameter of the breast pump during operation based on the user parameter includes:
determining the operating parameter of the breast pump during operation based on the lactation stimulation parameter.

In some implementations, the lactation stimulation parameter includes a lactation stimulation duration and a lactation stimulation frequency, and the determining the operating parameter of the breast pump during operation based on the lactation stimulation parameter includes:

determining a lactation stimulation duration and a lactation stimulation frequency of the breast pump during operation based on the pressure tolerance, where the lactation stimulation intensity is positively correlated with the pressure tolerance, and the lactation stimulation duration is negatively correlated with the pressure tolerance.

In some implementations, the second step includes following steps.
(1). A reference operating parameter is acquired.
(2). A deviation operating parameter is determined based on the user parameter.
(3). The operating parameter of the breast pump during operation is determined based on the reference operating parameter and the deviation operating parameter.

In some implementations, the deviation operating parameter is a coefficient, and the reference operating parameter multiplied by the deviation operating parameter equals the operating parameter of the breast pump during operation.

In some implementations, age-based user parameters are defined by ranges, with different age ranges corresponding to different operating parameters, that is, coefficients.

As an example, the user parameter is defined to include ages, and the operating parameter includes lactation stimulation intensities. The ages are divided into ranges of 18 to 25, 25 to 35, and above 35. The reference operating parameter of the breast pump corresponds to the age range of 25 to 35. If the age range of the user parameter is 18 to 25, it is determined that the deviation operating parameter is 0.9. In this case, a lactation stimulation intensity of the breast pump during operation equals a reference lactation stimulation intensity corresponding to the reference operating parameter multiplied by 0.9. If the age range of the user parameter is above 35, it is determined that the deviation operating parameter is 1.1. In this case, a lactation stimulation intensity of the breast pump during operation equals the reference lactation stimulation intensity corresponding to the reference operating parameter multiplied by 1.1.

In some implementations, the deviation operating parameter is a deviation value, and the reference operating parameter plus the deviation value equals the operating parameter of the breast pump during operation.

As an example, the user parameter is defined to include ages, and the operating parameter includes lactation stimulation intensities. The ages are divided into ranges of 18 to 25, 25 to 35, and above 35. The reference operating parameter of the breast pump corresponds to the age range of 25 to 35. If the age range of the user parameter is 18 to 25, it is determined that the deviation operating parameter is -0.005 MPa. In this case, a lactation stimulation intensity of the breast pump during operation is equal to the reference lactation stimulation intensity corresponding to the reference operating parameter plus - 0.005 MPa. If the age range of the user parameter is above 35, it is determined that the deviation operating parameter is +0.005 MPa. In this case, a lactation stimulation intensity of the breast pump during operation is the reference lactation stimulation intensity corresponding to the reference operating parameter plus +0.005 MPa.

In some implementations, the operating parameter corresponding to a pressure tolerance is defined by levels, with different levels corresponding to different coefficients or deviation values.

In some implementations, the operating parameter corresponding to a breast size is defined by ranges, with different size ranges corresponding to different coefficients or deviation values.

Furthermore, for specific embodiments corresponding to a breast size and a pressure tolerance, reference may be made to the embodiment corresponding to an age, which are not described in detail herein.

Third step: The breast pump is controlled to operate based on the operating parameter.

In some implementations, the negative pressure mechanism is a power mechanism of the breast pump, and the control unit controls the negative pressure mechanism to operate based on the operating parameter, causing the breast pump to operate based on the operating parameter.

In some implementations, the breast pump includes a display unit, including at least one of a first display region or a second display region. After the determining an operating parameter of the breast pump based on the user parameter, the method further includes:
controlling the first display region to display the user parameters; and/or
controlling the second display region to display the operating parameter.

Controlling the first display region to display the user parameter and/or controlling the second display region to display the operating parameter allows the users to directly learn a current status of the breast pump.

### Fifth embodiment

Referring to FIG. 31, FIG. 31 is a schematic structural diagram of a breast pump according to this application. As shown in FIG. 31, a breast pump 1 further includes a control interface 113. The control interface 113 includes a first display region 114 and a second display region 115 configured to display information indicating whether breast milk is expressed from a left breast or a right breast. When the breast pump operates on the right breast, the first display region displays a first display state, and the second display region displays a second display state. When the breast pump operates on the left breast, the first display region displays a third display state, and the second display region displays a fourth display state. The first display state is different from the third display state, and the second display state is different from the fourth display state.

In some implementations, a display parameter included in the above display state in this embodiment of this application includes but is not limited to an aspect ratio, a color, a brightness, an area, a shape, a position, and a text indicator of the display region, where the text includes "Left" or "Right", or "L" or "R".

Exemplarily, the first display state and the third display state each may include the text "Right" or "R", and the second display state and the fourth display state each includes the text "Left" or "L", indicating not only through the area but also through the text whether the breast milk stored in the breast pump is expressed from the left breast or the right breast.

Certainly, this application is not limited thereto. Each display state may further incorporate parameters such as an aspect ratio, a color, a brightness, an area, a shape, and a position, so that the control interface of the breast pump can more clearly indicate whether the breast milk is expressed from the left breast or the right breast.

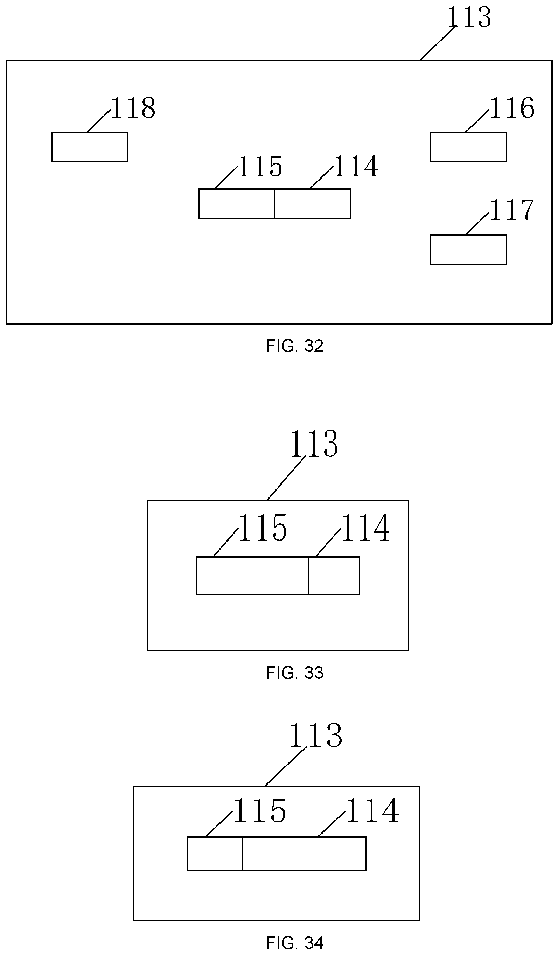

Referring to FIG. 32, FIG. 32 is a schematic diagram of a control interface of a breast pump according to an embodiment of this application. As shown in FIG. 32, a control interface 113 includes a first display region 114 and a second display region 115.

In some implementations, the control interface 113 further includes one or more of a third display region 116, a fourth display region 117, or a fifth display region 118. Details are provided in following embodiments, which are not described herein.

In some implementations, the first display region 114 is located on a right side of the second display region 115. When the breast pump operates on a right breast, the first display region 114 is controlled to shrink and the second display region 115 is controlled to expand, so that the first display region forms a first display state and the second display region forms a second display state. Referring to FIG. 33, FIG. 33 is a schematic diagram of a control interface 113 formed after the first display region 114 is shrunk and the second display region 115 is expanded.

When the breast pump operates on a right breast of a person wearing the breast pump, that is, when breast milk is expressed from the right breast, the breast pump is located on a left-hand side of a viewer when directly viewing the person wearing the breast pump. In this embodiment of this application, because the second display region 115 is expanded and the first display region 114 is shrunk when the breast milk is expressed from the right breast, when the user views the control interface 113 on the breast pump after milk expressing ends, a first display state on the first display region and a second display state on the second display region are the same as the states observed when the breast pump is worn on the right breast and viewed directly by a person (when the breast pump is worn on the right breast, the breast pump is located on the left-hand side of the person when viewed directly by the person, and in this case, the second display region on the left side is larger). This allows the user to quickly recognize that the breast milk is expressed from the right breast, helping the user determine a usage status of the breast pump.

In some implementations, the first display region 114 is located on the right side of the second display region 115. When the breast pump operates on the left breast, the first display region 114 is expanded and the second display region 115 is shrunk, so that the first display region 114 forms the third display state and the second display region 115 forms the fourth display state. Referring to FIG. 34, FIG. 34 is a schematic diagram of a control interface 113 formed after the first display region 114 is expanded and the second display region 115 is shrunk.

When the breast pump operates on a left breast of a person wearing the breast pump, that is, when breast milk is expressed from the right breast, the breast pump is located on a right-hand side of a viewer when directly viewing the person wearing the breast pump. In this embodiment of this application, because the first display region 114 is expanded and the second display region 115 is shrunk when the breast milk is expressed from the left breast, when the user views the control interface 113 on the breast pump after milk expressing ends, a first display state on the first display region and a second display state on the second display region are the same as the states observed when the breast pump is worn on the left breast and viewed directly by a person (when the breast pump is worn on the left breast, the breast pump is located on the right-hand side of the person when viewed directly by the person, and in this case, the first display region on the left side is larger). This allows the user to quickly recognize that the breast milk is expressed from the right breast, helping the user determine a usage status of the breast pump.

In some implementations, the first display region 114 includes a text "Left" or "L", which scales with an area change of the first display region 114 and always remain centered within the first display region 114. Similarly, the second display region 115 includes a text "Right" or "R", which scales with an area change of the second display region 115 and always remain centered within the second display region 115.

In some implementations, when the breast pump is not worn on the user, the first display region 114 and the second display region 115 have a same aspect ratio and area.

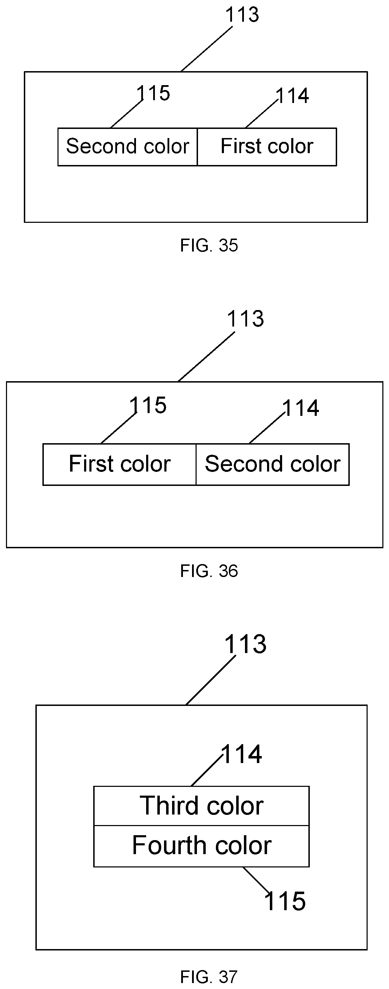

In some implementations, the first display region is located on the right side of the second display region. When the breast pump operates on the right breast, the first display region displays a first color and the second display region displays a second color, so that the first display region forms the first display state and the second display region forms the second display state. Referring to FIG. 35, FIG. 35 is a schematic diagram of a control interface 113 formed after the first display region 114 displays the first color and the second display region 115 displays the second color.

In some implementations, when the breast pump operates on the left breast, the first display region displays the second color and the second display region displays the first color, so that the first display region forms the third display state and the second display region forms the fourth display state. Referring to FIG. 36, FIG. 36 is a schematic diagram of a control interface 113 formed after the first display region 114 displays the second color and the second display region 115 displays the first color.

In some implementations, the first color and the second color are high-contrast colors, with the first color being brighter and more vivid than the second color. For example, the first color is white and the second color is black.

In some implementations, the brighter and more vivid one of the first color and the second color is used to indicate a corresponding position of breast milk in the breast pump. For example, when the color of the first display region is brighter and more vivid, it indicates that the breast milk in the breast pump is expressed from the left breast, and when the color of the second display region is brighter and more vivid, it indicates that the breast milk is expressed from the right breast.

Through displaying of different colors in the first display region and the second display region, a user can quickly determine a wearing status of the breast pump based on the color of the first display region and the color of the second display region.

In some implementations, when the breast pump is not worn on the user, the first display region 114 and the second display region 115 display a same color.

In some implementations, shapes of the first display region 114 and the second display region 115 are not limited to a rectangle shown in FIG. 32, FIG. 33, FIG. 34, FIG. 35, and FIG. 36. Therefore, in addition to an area change, the first display region 114 and the second display region 115 may further include a shape change.

In some implementations, a larger one of the first display region and the second display region is crescent-shaped, resembling "" or "", while a smaller one is circular, resembling "∘", with the crescent-shaped region semi-surrounding the circular region.

In some implementations, in addition to an area change, the first display region 114 and the second display region 115 further include a brightness change. A larger one of the first display region 114 and the second display region 115 exhibits a higher brightness, while a smaller one has a lower brightness.

In some implementations, the first display region 114 is not located on the right side of the second display region 115, but above the second display region 115.

When the breast pump has a small width, utilization of an available space of the breast pump can be maximized.

In some implementations, when the breast pump operates on the right breast, the first display region 114 displays a third color and the second display region 115 displays a fourth color, so that the first display region 114 forms the first display state and the second display region 115 forms the second display state.

Referring to FIG. 37, FIG. 37 is a schematic diagram of a control interface 113 formed after the first display region 114 displays the third color and the second display region 115 displays the fourth color.

When the breast pump operates on the left breast, the first display region 114 displays the fourth color and the second display region displays the third color, so that the first display region 42 forms the third display state and the second display region forms the fourth display state.

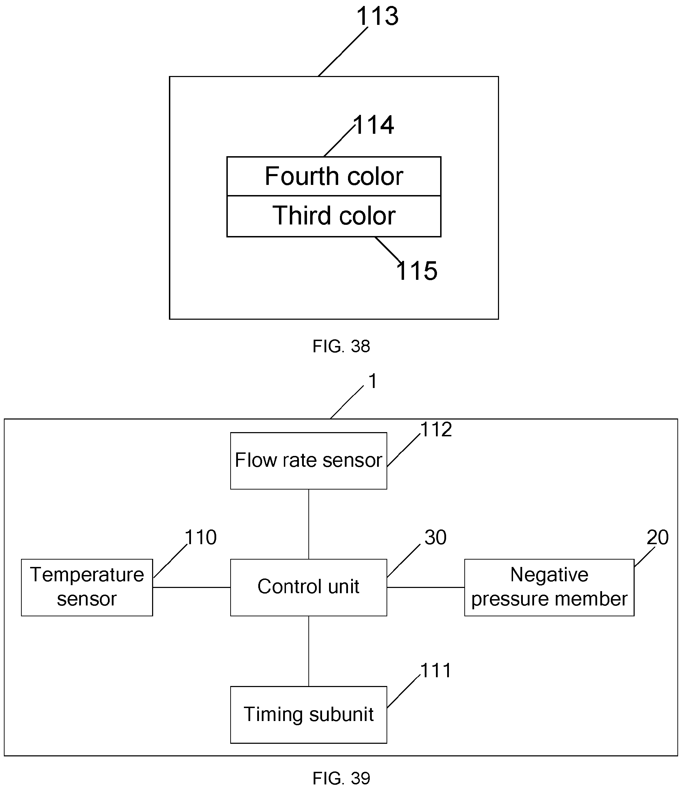

Referring to FIG. 38, FIG. 38 is a schematic diagram of a control interface 113 formed after the first display region 114 displays the fourth color and the second display region 115 displays the third color.

In some implementations, the third color and the fourth color are high-contrast colors. For example, the third color is white and the fourth color is black.

In some implementations, the third color is defined as white and the fourth color as black. When the first display region is white, it indicates that the breast milk in the breast pump is expressed from the right breast, and when the second display region is white, it indicates that the breast milk is expressed from the left breast.

In this way, the user can quickly determine a wearing position of the breast pump by observing a position of the third fourth color or the fourth color, and the first display region and the second display region are arranged vertically, which can save horizontal space and facilitate arrangement of more additional display regions.

In some implementations, shapes of the first display region 114 and the second display region 115 are not limited to a rectangle shown in FIG. 37 and FIG. 38. The shapes of the first display region 114 and the second display region 115 are not limited in this application.

In some implementations, the wearing position of the breast pump may be represented by brightness changes of the first display region 114 and the second display region 115.

For example, when the breast pump operates on the left breast, the brightness of the first display region 114 is greater than the brightness of the second display region 115, and when the breast pump operates on the right breast, the brightness of the first display region 114 is less than the brightness of the second display region 115.

In some implementations, when the breast pump operates on the right breast, in addition to color changes, the first display region 114 is expanded and the second display region 115 is shrunk, so that the first display region 114 forms the first display state and the second display region 115 forms the second display state.

In some implementations, when the breast pump operates on the left breast, in addition to color changes, the first display region 114 is shrunk and the second display region 115 is expanded, so that the first display region 114 forms the third display state and the second display region 115 forms the fourth display state.

Changing both colors and areas of the first display region 114 and the second display region 115 when the breast pump is worn increases a visual difference between the first display region 114 and the second display region 115, making it easier for the user to determine the wearing position of the breast pump and reducing a determination time for the user.

In some implementations, the area changes of the first display region 114 and the second display region 115 are achieved through aspect ratio changes.

In some implementations, the first display region 114 and the second display region 115 are defined to display initial colors in an initial state. When a display region changes from an initial color to a third color, a width of the display region remains unchanged while a length of the display region is increased, thereby increasing the area. When a display region changes from the initial color to a fourth color, a width of the display region remains unchanged while a length of the display region is reduced, thereby reducing the area.

In some implementations, the first display region 114 and the second display region 115 are defined to display initial colors in an initial state. When a display region changes from an initial color to a third color, a length of the display region remains unchanged while a width of the display region is increased, thereby increasing the area. When a display region changes from the initial color to a fourth color, a length of the display region remains unchanged while a width of the display region is reduced, thereby reducing the area.

Referring then to FIG. 39, FIG. 39 is a schematic structural diagram of a third embodiment of a breast pump according to an embodiment of this application.

As shown in FIG. 39, in some implementations, the breast pump further includes a temperature sensor 110 arranged in the milk storage container portion 50 and configured to measure a temperature of the breast milk in the milk storage container portion 50. The control interface further includes a third display region 116 configured to display the measured temperature of the temperature sensor.

In some implementations, the temperature sensor 110 is connected to the control unit 30. The control unit 30 is configured to receive temperature measurement information from the temperature sensor 110 and display the temperature measurement information on the third display region 116.

Through the temperature sensor 110 arranged in the milk storage container portion 50, the temperature of the breast milk stored in the milk storage container portion 50 can be monitored, to prevent the breast milk stored in the milk storage container portion 50 from spoiling as a result of an excessively high temperature.

In some implementations, breast milk cannot be stored at a room temperature for too long. Exemplarily, colostrum (milk expressed within 6 days postpartum) can be stored at a room temperature of 27°C to 32°C for 12 hours at a room temperature. Mature breast milk (milk expressed after 6 days postpartum) can be stored at a room temperature of 15°C for 24 hours, at a room temperature of 19°C to 22°C for 10 hours, and at a room temperature of 25°C for 6 hours. Therefore, the breast pump may be further provided with a timing device configured to record a storage time of the breast milk in the milk storage container portion 50, determine, based on the temperature detected by the temperature sensor, a maximum breast milk storage time corresponding to the current temperature, and send a prompt to the user when the storage time of the breast milk in the milk storage container portion 50 exceeds the maximum storage time corresponding to the temperature indicated by the temperature sensor.

In some implementations, a manner of sending the prompt to the user includes but is not limited to following: the breast pump sends voice messages, or the breast pump sends prompt information to a terminal communicatively connected to the breast pump, such as a mobile phone connected to the breast pump via Bluetooth, to prevent breast milk from spoiling as a result of prolonged storage.

In some implementations, the breast pump further includes a control unit 30 connected to the negative pressure mechanism 20 and configured to control an operating status of the negative pressure mechanism 20 to facilitate usage of the breast pump by the user.

In some implementations, the breast pump further includes a timing subunit 111 configured to record an operating duration of the negative pressure mechanism.

In some implementations, the control interface further includes a fourth display region 117 in FIG. 32, which is configured to display a recorded time of the timing subunit when the negative pressure mechanism starts operating.

In some implementations, the control unit 30 is configured to receive timing information from the timing subunit 111 and display the timing information on the fourth display region 117.

Displaying the recorded time of the timing subunit allows the user to determine an approximate volume of breast milk currently being expressed by the breast pump based on the recorded time, which facilitates the usage of the breast pump, and can prevent discomfort to the wearer as a result of prolonged operation of the breast pump.

In some implementations, the breast pump further includes a flow rate sensor 112. Because breast milk expressed by the breast pump is transferred to the milk storage container portion through the nipple channel, the flow rate sensor 112 is arranged in the nipple channel. The control unit is further connected to the flow rate sensor 112 and the timing subunit 111. The control unit 30 is configured to calculate the volume of the breast milk expressed by the breast pump based on flow rate data of the flow rate sensor 112 and the timing data of the timing subunit.

In some implementations, the control unit 30 is further configured to display, on the fifth display region 118, the calculated volume of the breast milk expressed by the breast pump.

In some implementations, the control interface includes a fifth display region 118 configured to display the volume of the breast milk expressed by the breast pump.

Arranging the flow rate sensor to calculate the volume of the breast milk expressed by the breast pump in combination with the timing subunit allows the user to the learn current expressed breast milk volume. This prevents situations where daily expressed breast milk falls short of baby's needs.

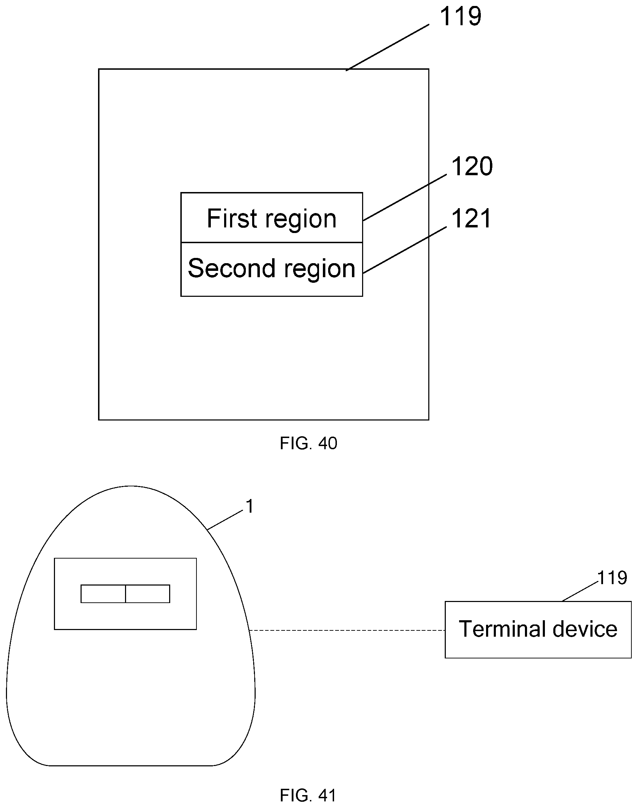

Referring then to FIG. 40, FIG. 40 is a schematic structural diagram of a terminal device according to an embodiment of this application. The terminal device 119 is configured to be communicatively connected to the breast pump. As shown in FIG. 40, the terminal device 119 includes a control interface that includes a first region 120 and a second region 121 configured to display information indicating whether breast milk is expressed from a left breast or a right breast. The first region 120 is located above the second region 121.

In some implementations, information such as the breast milk temperature and the breast milk volume described above can be further viewed on the control interface of the terminal device 119.

In some implementations, information such as a name and an age of the breast pump wearer and a wearing time of the breast pump can be further viewed on the control interface of the terminal device 119.

Referring then to FIG. 41, FIG. 41 is a schematic structural diagram of a system according to an embodiment of this application. As shown in FIG. 41, the system includes the breast pump 1 and the terminal device 119 described above. The breast pump 1 communicates with the terminal device 119.

In some implementations, the breast pump 1 communicates with the terminal device 119 through wired communication, for example, through a type-c data cable. A specific manner of the wired communication is not limited in this application.

In some implementations, the breast pump 1 communicates with the terminal device 119 through wireless communication, for example, through Bluetooth. A specific manner of the wireless communication is not limited in this application.

In some implementations, the breast pump 1 may send various information to the terminal device 119, to display information including but not limited to a breast milk temperature, a recorded time of the timing subunit, a breast milk volume expressed by the breast pump in the terminal device 119.

In this way, a user can remotely view a usage status of the breast pump, facilitating usage by the user.

In some implementations, the terminal features a first key. When the first button is triggered, the terminal sends third information to the breast pump to indicate that the breast pump is operating on a right breast, so that the first display region displays a first display state and the second display region displays a second display state.

The terminal features a second key. When the second button is triggered, the terminal sends fourth information to the breast pump to indicate that the breast pump is operating on a left breast, so that the first display region displays a third display state and the second display region displays a fourth display state.

In this way, the user can control the breast pump remotely, or in other words, control the breast pump without touching the breast pump, so that the user can use the breast pump in regions with insufficient privacy.

In some implementations, the terminal further features a third key configured to control an operating status of the negative pressure mechanism to control the breast pump whether to perform milk expressing.

In some implementations, the milk storage container portion of the breast pump is provided therein with a cooling unit and a temperature sensor. The terminal further features a fourth key configured to control the cooling unit whether to perform cooling. This allows the user to operate the cooling unit through the fourth key when the user notices that a temperature of the breast milk in the milk storage container portion is excessively high, thereby regulating the temperature of the breast milk and prolonging a storage time of the breast milk.

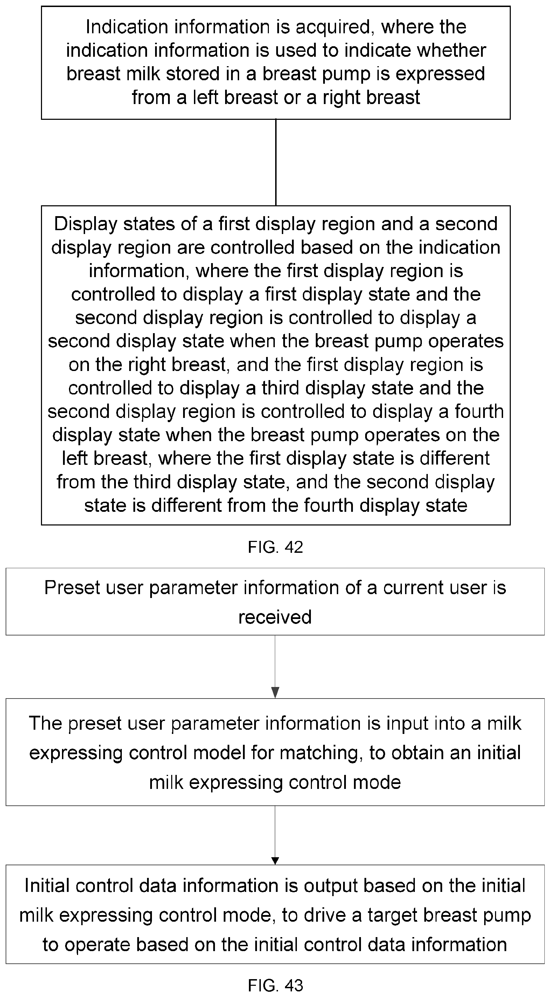

Referring to FIG. 42, FIG. 42 is a schematic flowchart of a control method according to an embodiment of this application. As shown in FIG. 42, the control method includes a first step to a second step.

First step: Indication information is acquired, where the indication information is used to indicate whether breast milk stored in a breast pump is expressed from a left breast or a right breast.

In some implementations, the step of acquiring indication information includes following steps.
(1). The indication information sent by a terminal in communication with the breast pump is acquired.

In some implementations, the terminal sends indication information to the breast pump to indicate whether the breast milk stored in the breast pump is expressed from the left breast or the right breast, for example, the third information and the fourth information described above.

In some implementations, the breast pump features two keys respectively configured to record breast milk expressing from the left breast and breast milk expressing from the right breast. The indication information is generated through triggering of the keys.

In some implementations, the acquiring indication information includes following steps:
(1). Detection information detected by a detection sensor is acquired, where the detection sensor is configured to detect an operating position of the breast pump.
(2). The indication information is determined based on the detection information.

In some implementations, the breast pump may be worn inside a compatible bra. A detection element may be arranged on the bra. The detection sensor determines the operating position of the breast pump by sensing the element.

In some implementations, one side of the bra, for example, the left breast, is provided with a detection element, while the other side, for example, the right breast, of the bra is not provided with a detection element. The detection sensor generates detection information by detecting presence or absence of the detection element.

Exemplarily, when the detection sensor is worn on the left breast, first detection information is detected, and when the detection sensor is worn on the right breast, second detection information is detected, so that the breast pump determines the operating position based on the detection information.

In some implementations, both sides of the bra are provided with detection elements that have different labels. The detection sensor generates detection information by detecting the labels on the detection elements

In some implementations, the detection sensor includes a Hall sensor or a radio frequency identification device (RFID) reader, which is not limited in this application.

By detecting detection information through the detection sensor, the breast pump can learn a current operating position and control display states of the first display region and the second display region to record a breast milk expressing position, to facilitate usage by the user.

Second step: Display states of a first display region and a second display region are controlled based on the indication information, where the first display region is controlled to display a first display state and the second display region is controlled to display a second display state when the breast pump operates on the right breast, and the first display region is controlled to display a third display state and the second display region is controlled to display a fourth display state when the breast pump operates on the left breast, where the first display state is different from the third display state, and the second display state is different from the fourth display state.

In some implementations, when the first display region is located on a right side of the second display region, the step of controlling display states of a first display region and a second display region based on the indication information includes following steps:
(1). The first display region is controlled to shrink and the second display region is controlled to expand when it is determined based on the indication information that the breast pump operates on the right breast, causing the first display region to form the first display state and the second display region to form the second display state.
(2). The first display region is controlled to expand and the second display region is controlled to shrink when it is determined based on the indication information that the breast pump operates on the left breast, causing the first display region to form the third display state and the second display region to form the fourth display state.

In some implementations, when the first display region is located on the right side of the second display region, the step of controlling display states of a first display region and a second display region based on the indication information includes following steps:
(1). The first display region is controlled to display a first color and the second display region is controlled to display a second color when it is determined based on the indication information that the breast pump operates on the right breast, causing the first display region to form the first display state and the second display region to form the second display state.
(2). The first display region is controlled to display the second color and the second display region is controlled to display the first color when it is determined based on the indication information that the breast pump operates on the left breast, causing the first display region to form the third display state and the second display region to form the fourth display state.

In some implementations, when the first display region is located above the second display region, the step of controlling display states of a first display region and a second display region based on the indication information includes following steps:
(1). The first display region is controlled to display the third color and the second display region is controlled to display the fourth color when it is determined based on the indication information that the breast pump operates on the right breast, causing the first display region to form the first display state and the second display region to form the second display state.
(2). The first display region is controlled to display the fourth color and the second display region to display the third color when it is determined based on the indication information that the breast pump operates on the left breast, causing the first display region to form the third display state and the second display region to form the fourth display state.

In some implementations, the display control method for a breast pump provided in this embodiment of this application further includes following steps:
(1). The first display region is controlled to expand and the second display region is controlled to shrink when it is determined based on the indication information that the breast pump operates on the right breast, causing the first display region to form the first display state and the second display region to form the second display state.
(2). The first display region is controlled to shrink and the second display region is controlled to expand when it is determined based on the indication information that the breast pump operates on the left breast, causing the first display region to form the third display state and the second display region to form the fourth display state.

### Sixth embodiment

Referring to FIG. 43, an embodiment of the present disclosure provides a control method, including a first step to a third step.

First step: Preset user parameter information of a current user is received.

As different users have different preset user parameter information, receiving the preset user parameter information of the current user can achieve recommendation of a proper milk expression control mode for the current user based on the preset user parameter information of the current user.

Second step: The preset user parameter information is input into a milk expression control model for matching, to obtain an initial milk expression control mode.

The milk expression control model stores a large amount of data acquired based on different preset user parameter information, and defines a plurality of preset milk expression control modes corresponding to a plurality of types of preset user parameter information. Therefore, after the preset user parameter information of the current user is received, a proper initial milk expression control mode can be provided for the current user. However, it should be noted that, although the initial control mode is determined through data matching based on a current physiological parameter of the current user, the initial control mode may not necessarily represent an optimal milk expression control mode for a specific user, and instead, is a relatively proper control mode for an initial stage of using the breast pump by the current user.

Third step: Initial control data information is output based on the initial milk expression control mode, to drive, based on the initial control data information, a target breast pump to operate.

After the corresponding initial control mode is obtained through the second step, an intelligent mobile terminal outputs corresponding initial control data information based on the initial control mode. Therefore, the current user may control the target breast pump to operate on the intelligent mobile terminal based on the initial control data information provided by the intelligent mobile terminal, or directly input the relevant initial control data information on a control interface of the target breast pump to control the target breast pump to operate.

According to the control method provided in this embodiment of the present disclosure, the preset user parameter information is received from the current user, matching is performed in the milk expression control model based on the preset user parameter information to obtain the initial milk expression control mode adapting to the preset user parameter information, and the initial control data information is output based on the initial milk expression control mode to drive the target breast pump to operate. This resolves technical problems of low milk expressing efficiency and poor user experience as a result of the breast pump device being unable to provide different breast pump control modes based on preset user parameter information of different users.

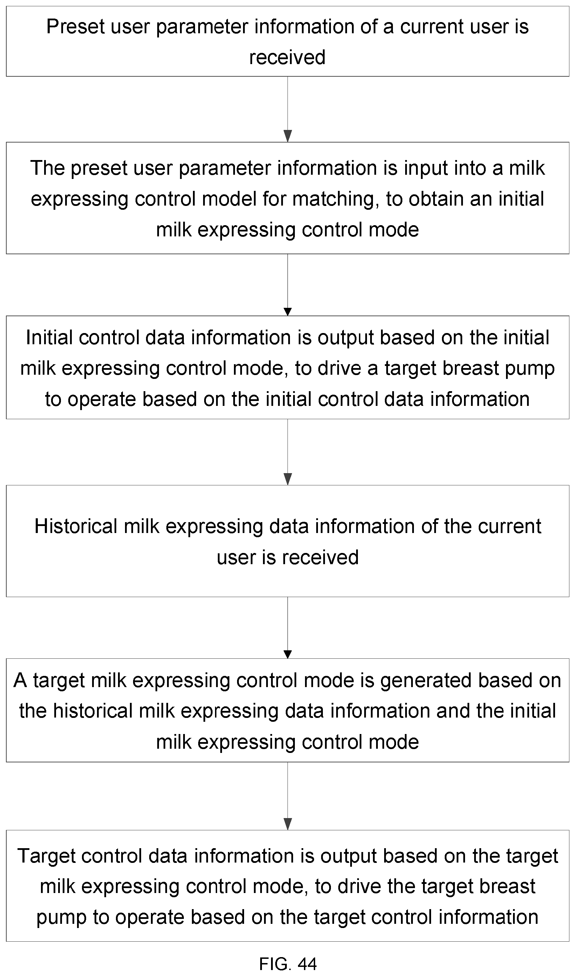

Referring to FIG. 44, in some embodiments, the initial control mode is a control mode that is directly output after matching in the breast pump control model based on the preset user parameter information of the current user. However, a volume of milk expressed by the breast pump is not only affected by the preset user parameter information, but also by other factors during actual use, such as dietary habits and sleep habits of the current user, environmental factors, and other physiological parameters (such as female's menstrual period). Therefore, after the corresponding initial milk expression control mode is obtained based on the preset user parameter information, adjustments need to be made based on an actual usage status of the user, to obtain a target milk expression control mode adapting to the current user. Therefore, in this embodiment, the control method further includes a fourth step to a sixth step.

Fourth step: Historical milk expressing data information of the current user is received.

In order to provide the current user with a breast pump control mode adapting to the current user, historical milk expressing data information when the current user uses the breast pump for milk expressing needs to be first acquired.

Fifth step: A target milk expression control mode is generated based on the historical milk expressing data information and the initial milk expression control mode.

After the historical milk expressing data information is obtained, it needs to be determined based on the historical milk expressing data information whether the initial milk expression control mode is an optimal milk expression control mode for the current user. If the initial milk expression control mode is not an optimal milk expression control mode for the current user, the initial milk expression control mode needs to be adjusted based on the historical milk expressing data information, to obtain a target milk expression control mode adapting to the preset user parameter information of the current user.

Sixth step: Target control data information is output based on the target milk expression control mode, to drive the target breast pump to operate based on the target control information.

After the target milk expression control mode is obtained through the fifth step, the intelligent mobile terminal outputs the corresponding target control data information based on the target control mode. Therefore, the current user may control the target breast pump to operate on the intelligent mobile terminal based on the target control data information provided by the intelligent mobile terminal, or may directly input relevant target control data information on the control interface of the target breast pump to control the target breast pump to operate.

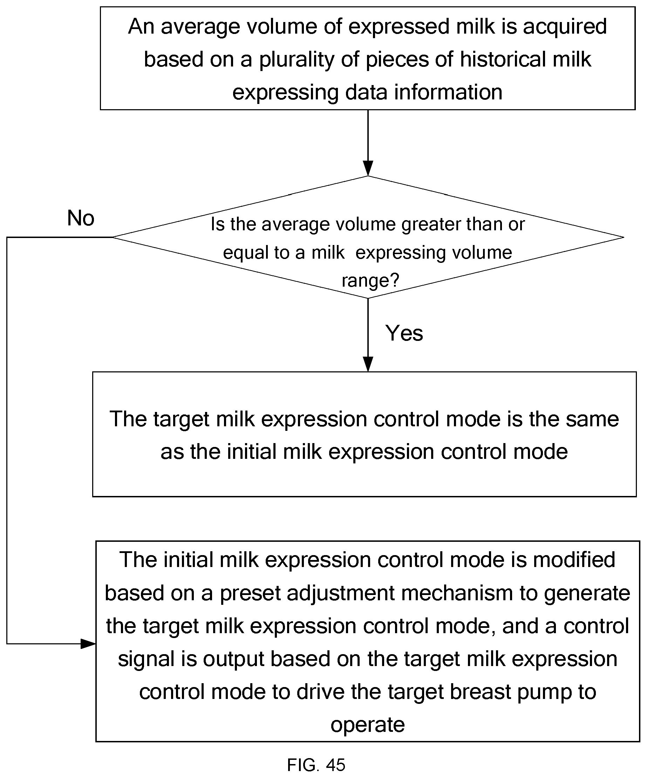

Referring to FIG. 45, in some embodiments, the actual usage of the breast pump by the current user during milk expressing may, in all likelihood, not strictly conform to the initial milk expression control mode in the third step. Therefore, after the historical milk expressing data information of the current user is received through the fourth step in the above embodiment, the method further includes screening the historical milk expressing data information, so that the historical milk expressing data information after the screening corresponds to milk expressing data information acquired in the initial milk expression control mode. In this embodiment, the step of generating a target milk expression control mode based on the historical milk expressing data information and the initial milk expression control mode in the fifth step in the above embodiment includes following substeps:
(1). An average volume of expressed milk is acquired based on a plurality of pieces of historical milk expressing data information.

The acquired average volume of expressed milk is calculated based on a preset number of pieces of historical milk expressing data information. A specific preset number may be adaptively set based on different application scenarios. Generally, an excessively small preset number easily leads to insufficient accuracy in results obtained in subsequent steps, while an excessively large preset number leads to delays in providing the user with a proper target milk expression control mode. Therefore, the preset number (corresponding to "a plurality of" in this step) is set to five to ten.

(2). It is determined whether the average volume of expressed milk is within or greater than a milk expressing volume range corresponding to the initial milk expression control mode.

The milk expressing volume range corresponding to the initial milk expression control mode is a volume of breast milk expressed by the breast pump in a single expressing operation in the initial milk expression control mode. The initial milk expression control mode obtained based on the preset user parameter information corresponds to a preset milk expressing volume range. Milk expressing volume ranges corresponding to different milk expression control modes may be the same, different, or partially the same. For example, a milk expression control mode has a milk expressing volume range of 100 ml to 120 ml, and another milk expression control mode has a milk expressing volume range of 115 ml to 130 ml. These milk expressing volume ranges each are an interval set based on a large amount of preset user parameter information after the preset user parameter information is acquired, or is an interval scientifically calculated based on preset user parameter information.

If the average volume of expressed milk is within or greater than the milk expressing volume range corresponding to the initial milk expression control mode, a following step is performed.

(3). It is determined that the target milk expression control mode is the same as the initial milk expression control mode.

If in step (2), the average of a plurality of breast milk volumes obtained by the current user by using the breast pump in the initial milk expression control mode is within or greater than the milk expressing volume range corresponding to the initial milk expression control mode, it indicates that proper milk expressing efficiency and a proper milk expressing volume adapting to the preset user parameter information of the current user can be achieved when the current user uses the breast pump device in the initial milk expression control mode. Therefore, the initial milk expression control mode is recommended as the target milk expression control mode to the current user, so that the current user can use the breast pump based on the target milk expression control mode.

Otherwise, a following step is performed.

(4). The initial milk expression control mode is modified based on a preset adjustment mechanism to generate the target milk expression control mode.

If the breast milk volume average obtained by the current user after performing milk expressing by using the breast pump in the initial milk expression control mode is less than the milk expressing volume range corresponding to the initial milk expression control mode, it indicates that the initial milk expression control mode does not fully adapt to the current user. Therefore, the initial milk expression control mode needs to be modified to output a target milk expression control mode that adapts to the current user, to improve milk expressing efficiency for the current user.

In some embodiments, the target milk expression control mode includes target control data information, including a target milk expressing pressure, a target milk expressing time point, and a target milk expressing duration. The target milk expressing pressure is a milk expressing pressure generated for driving the breast pump to operate, which is a pressure value or a pressure range. The target milk expressing time point refers to one or more specific time points within a day, which may be a time point within a time period, for example, a time period from 9:00 a.m. and 10:00 a.m. in a day. The target milk expressing duration is a duration from starting using the breast pump within the time point to completing a current milk expressing process. In this embodiment, if the breast milk volume average (an actual value) obtained by the current user by performing milk expressing in the initial milk expression control mode through the above embodiment is less than the milk expressing volume range (a preset value) corresponding to the initial milk expression control mode, it indicates that the initial milk expression control mode needs to be adjusted to obtain a target milk expression control mode adapting to the current user. In this case, step (4) of modifying the initial milk expression control mode based on a preset adjustment mechanism to generate the target milk expression control mode in the above embodiment includes following steps:
(41). An initial milk expressing pressure, an initial milk expressing time point, and an initial milk expressing duration corresponding to the initial milk expression control mode are acquired.

In this embodiment, the initial pressure is adjusted to adjust the initial milk expression control mode. This adjustment manner includes any one of following:
(42). A preset pressure adjustment value is added to the initial milk expressing pressure to obtain a target milk expressing pressure, the initial milk expressing time point is used as a target milk expressing time point, and the initial milk expressing duration is used as the target milk expressing duration.

This adjustment manner focuses solely on modifying the initial pressure, while the target milk expressing time point and the target milk expressing duration still adopt the initial milk expressing time point and the initial milk expressing duration corresponding to the initial milk expression control mode. This adjustment manner, by increasing the milk expressing pressure, increases efficiency of milk expressing by the current user by using the breast pump. The preset pressure adjustment value needs to be set to a proper value, and cannot be set excessively high to avoid causing harm to the current user. A step-by-step adjustment manner may be implemented. Specifically, the preset pressure adjustment value is initially set to a relatively small value and then gradually increased. Finally, after confirmation by the current user, the adjusted pressure value is used as the target milk expressing pressure.

Alternatively,
(43). A preset pressure adjustment value is added to the initial milk expressing pressure to obtain a target milk expressing pressure, a preset time point adjustment value is added to the initial milk expressing time point to obtain a target milk expressing time point, and the initial milk expressing duration is used as the target milk expressing duration.

This adjustment manner adjusts both the initial milk expressing pressure and the initial milk expressing time point to obtain the corresponding target milk expressing pressure and target milk expressing time point, while using the initial milk expressing duration as the target milk expressing duration.

Alternatively,
(44). A preset pressure adjustment value is added to the initial milk expressing pressure to obtain a target milk expressing pressure, the initial milk expressing time point is used as a target milk expressing time point, and a preset duration adjustment value is added to the initial milk expressing duration to obtain a target milk expressing duration.

This adjustment manner adjusts the initial milk expressing pressure and the initial milk expressing duration to obtain the corresponding target milk expressing pressure and target milk expressing duration, while using the initial milk expressing time point as the target milk expressing duration.

Alternatively,
(45). A preset pressure adjustment value is added to the initial milk expressing pressure to obtain a target milk expressing pressure, a preset time point adjustment value is added to the initial milk expressing time point to obtain a target milk expressing time point, and a preset duration adjustment value is added to the initial milk expressing duration to obtain a target milk expressing duration.

This adjustment manner adjusts all of the initial milk expressing pressure, the initial milk expressing time point, and the initial milk expressing duration to obtain the corresponding target milk expressing pressure, target milk expressing time point, and target milk expressing duration.

In some embodiments, the initial milk expressing pressure is used as the target milk expressing pressure, and one or more of the initial milk expressing time point or the initial milk expressing duration are adjusted, to obtain a target milk expression control mode. Specifically, this adjustment manner includes any one of following:

(46). The initial milk expressing pressure is used as the target milk expressing pressure, a preset time point adjustment value is added to the initial milk expressing time point to obtain a target milk expressing time point, and the initial milk expressing duration is used as the target milk expressing duration.

This adjustment manner adjusts the initial milk expressing time point to obtain the corresponding target milk expressing time point, while using the initial milk expressing pressure as the target milk expressing pressure and the initial milk expressing duration as the target milk expressing duration.

Alternatively,
(47). The initial milk expressing pressure is used as the target milk expressing pressure, a preset time point adjustment value is added to the initial milk expressing time point to obtain a target milk expressing time point, and a preset duration adjustment value is added to the initial milk expressing duration to obtain a target milk expressing duration.

This adjustment manner adjusts both the initial milk expressing time point and the initial milk expressing duration to obtain the corresponding target milk expressing time point and target milk expressing duration, while using the initial milk expressing pressure as the target milk expressing pressure.

Alternatively,
(48). The initial milk expressing pressure is used as the target milk expressing pressure, the initial milk expressing time point is used as the target milk expressing time point, and a preset duration adjustment value is added to the initial milk expressing duration to obtain a target milk expressing duration.

This adjustment manner adjusts the initial milk expressing duration to obtain the corresponding target milk expressing duration, while using the initial milk expressing pressure as the target milk expressing pressure and the initial milk expressing time point as the target milk expressing time point.

In some embodiments, during the modification of the initial milk expression control mode based on the preset adjustment mechanism to generate the target milk expression control mode in the above embodiment, the initial milk expressing pressure, the adjustments to the initial milk expressing pressure, the initial milk expressing time point, or the initial milk expressing duration are all carried out incrementally. Then, after a target milk expressing mode is obtained each time, a volume of milk expressed in a current target milk expressing mode is compared with the milk expressing volume range corresponding to the initial milk expression control mode to determine whether the volume of milk meets a requirement, so as to determine whether to modify the initial milk expression control mode in another adjustment manner to generate a final target milk expression control mode. Furthermore, in some cases, if the milk volume obtained from operating the breast pump in all target milk expression control modes obtained from modifying the initial milk expression control mode in the above embodiments is still not greater than or within the milk expressing volume range corresponding to the initial milk expression control mode, milk expressing volumes are ranked in descending order and a target milk expression control mode corresponding to a largest milk expressing volume is used as the final target milk expression control mode.

In some embodiments, after the target milk expression control mode corresponding to the current user is obtained through any one of the above embodiments, the target milk expression control mode is bound to the current user. Specifically, after the current user logs, through identity authentication, in to an application on the intelligent mobile device for controlling the breast pump, the application assigns a unique identity (ID) to the current user, and then bind the obtained target milk expression control mode with the unique ID. Even if the user changes the smart mobile terminal or the breast pump, the user can still obtain the target milk expression control mode adapting to the user by logging in to the application for controlling the breast pump.

In some embodiments, since the milk expressing volume during the operation of the breast pump is affected by factors such as age information and breast size information of the current user, usage season information, and usage region information, the preset user parameter information described in the above embodiments includes one or more of following: age information and breast size information of the current user, usage season information, and usage region information. Typically, in order to provide the user with a more accurate breast pump control mode, the preset user parameter information includes all of the age information, the breast size information, the usage season information, and the usage region information. Therefore, the breast pump control model stores a plurality of initial breast pump control modes corresponding to combinations of a plurality of pieces of age information, a plurality of pieces of breast size information, a plurality of pieces of usage season information, and a plurality of pieces of usage region information. Classification of usage regions is typically based on countries where the target breast pump is used, and corresponding breast pump control modes are results determined from analysis and screening based on a large amount of data acquired from preset user parameter information. In this embodiment, due to variations in usage region and usage time of the breast pump, corresponding breast pump control modes are set for different usage regions or usage seasons. This means that even for users with a same age and a same breast size in a same season, corresponding initial milk expression control modes differ across regions. Alternatively, even for users with a same age and a same breast size in a same region, corresponding initial milk expression control modes may differ across seasons (such as winter and summer). In a specific example, after the current user manually inputs preset user parameter information through a device (such as a smartphone or a pad) connected to the target breast pump, or selects and inputs the preset user parameter information through a selection interface provided by the device, the device performs matching based on the received preset user parameter information in the breast pump control mode to obtain a corresponding initial milk expression control mode, and then displays corresponding initial control data information on the interface of the device based on the initial control mode, so that the user controls the target breast pump to operate based on the initial control data information. In addition, the device further provides a control interface of the target breast pump. The user may control the target breast pump to operate based on the initial control data information.

### Seventh embodiment

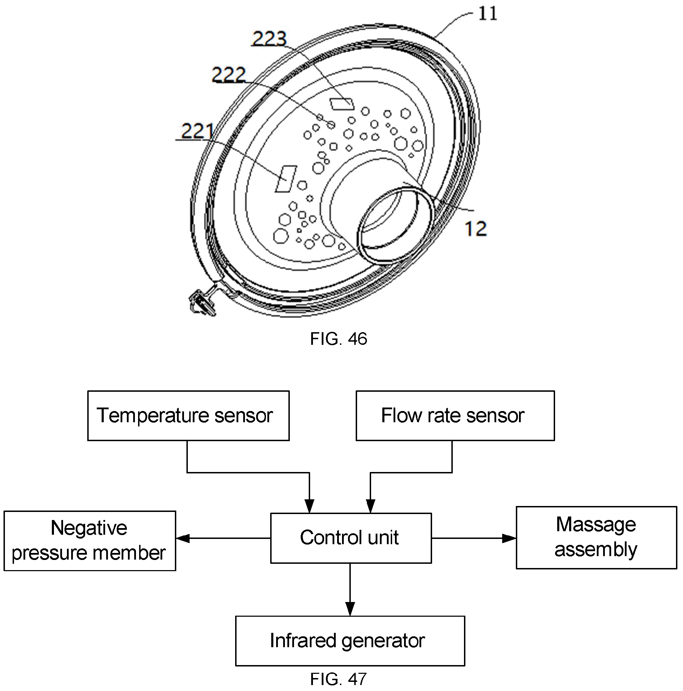

As shown in FIG. 46 to FIG. 47, during use of a breast pump of the present disclosure, a breast is accommodated in and snugly attached to a breast shield 10. When a negative pressure mechanism 20 operates, milk is expressed from the breast under negative pressure generated by the negative pressure mechanism 20 and stored in the milk storage container portion 50 through the nipple channel 12. The breast pump of the present disclosure is further provided with a stimulation unit 220 electrically connected to a control unit and controlled by the control unit. The control unit may generate a corresponding startup signal in response to a startup instruction input by the user or based on detection data from the sensor, and start the stimulation unit to operate in a preset mode based on the startup signal, where the preset mode includes at least a first mode for stimulating the breast to heat up, thereby unclogging blocked breast ducts and triggering the breast into milk-ejection reflex.

In some implementations, the breast pump further includes a housing, on which at least one key is arranged. The user may trigger the generation of the startup instruction by triggering the key.

In some implementations, the breast pump further includes a communication unit, through which the breast pump can communicate with an external terminal. The user may input a startup instruction on the external terminal, and the startup instruction is sent to the control unit of the breast pump through the communication unit to be received by the control unit.

Specifically, in some embodiments, the stimulation unit 220 includes an infrared generator 221, and the control unit is configured to control, after receiving the corresponding startup signal, the infrared generator to radiate infrared rays to the breast to stimulate the breast to heat up. A heating technology of the infrared generator works by emitting infrared rays onto a to-be-heated object. Except for rays that are reflected or transmitted, other rays are absorbed by the object and converted into thermal motion of material molecules, thereby heating the object.

Furthermore, in some embodiments, a wavelength of a far-infrared ray emitted by the infrared generator 221 is 4 µm to 14 µm. Human skin contains about 70% water, which is an efficient absorber of far-infrared rays. Therefore, an absorption spectrum of the breast for far-infrared rays closely resembles an absorption spectrum of water, exhibiting two absorption peaks of 2.5 µm to 4 µm and 5.6 µm to 10 µm. Therefore, far-infrared rays with wavelengths of 4 µm to 14 µm can be well absorbed by the breast.

In some implementations, the wavelength of the far-infrared ray emitted by the infrared generator 221 is 4 µm, 9 µm, or 14 µm.

Furthermore, the infrared generator 221 is arranged on a side of the breast shield 10 facing away from the breast. The breast shield 10 is snugly attached to skin on a breast surface. Arranging the infrared generator 221 on the breast shield 10 can bring a far-infrared ray emission source closer to the breast. In addition, the infrared generator 221 is mounted on the flange 11 of the breast shield 10 to prevent discomfort as a result of direct contact with skin.

Furthermore, in some embodiments, the breast pump is further provided with a temperature sensor 223 configured to detect a temperature of the breast surface. The temperature sensor 223 is electrically connected to the control unit. When the infrared generator 221 starts operating, the temperature sensor 223 also starts operating in real time. When the temperature sensor 223 detects that the temperature of the breast surface is higher than a specific value, the control unit controls the infrared generator 221 to stop operating, to prevent burns as a result of an excessively high temperature of the breast.

Optionally, the temperature sensor 223 includes a contact temperature sensor 223 and a non-contact temperature sensor 223. The contact temperature sensor 223 is located on a surface of the flange 11 and directly contacts the breast, which can effectively detect a temperature of the breast. The non-contact sensor may be arranged on an inner side or an outer side of the flange 11. A type of the temperature sensor 223 is not specifically limited in the present disclosure.

Furthermore, in some embodiments, the breast pump is further provided with a flow rate sensor 224 configured to detect a flow rate during breast milk expressing by the breast pump. The flow rate sensor 224 is electrically connected to the control unit. The flow rate sensor 224 is arranged in the nipple channel 12. Breast milk is acquired by the milk storage container portion 50 through the nipple channel 12. Arranging the flow rate sensor 224 in the nipple channel 12 can accurately detect a flow rate during breast milk acquisition in real time and feed back flow rate information to the control unit. The control unit controls the operating mode of the breast pump based on a magnitude of the flow rate.

Furthermore, in some embodiments, the breast pump further includes a massage assembly 222 arranged on the breast shield 10. The massage assembly 222 is electrically connected to the control unit and is directly controlled by the control unit. Specifically, the massage assembly 222 includes a plurality of protruding structures arranged on the flange 11 and a plurality of oscillators arranged inside the main unit 2. The oscillators are snugly attached to a wall of the flange 11. When the oscillators operate, vibration generated acts on the wall of the flange 11, driving the protruding structures to vibrate, thereby achieving massage on the breast. The massage assembly 222 improves breast blood circulation, promotes breast milk secretion, effectively unclogs breast meridians, and unclogs breast ducts by massaging the breast.

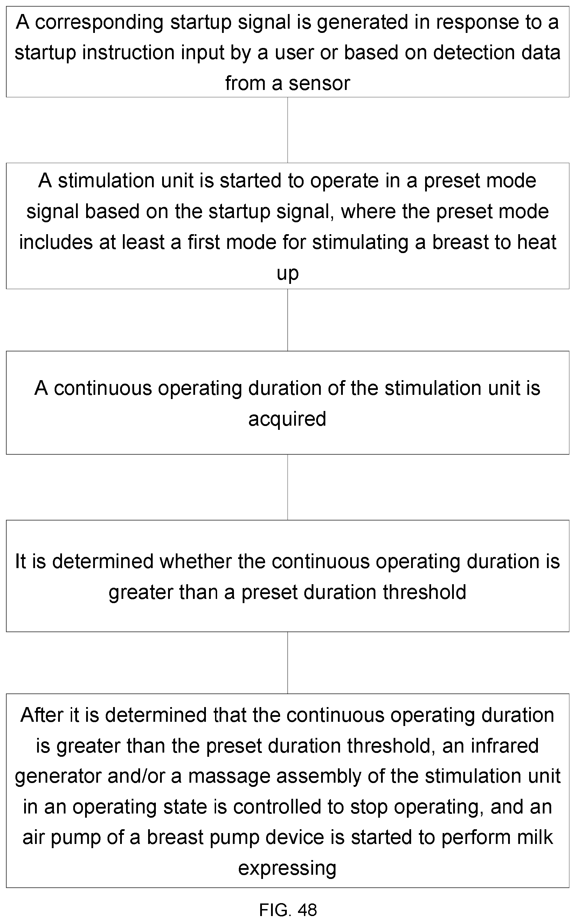

Referring to FIG. 48, as shown in FIG. 48, a control method according to an embodiment of this application includes a first step to a fifth step.

First step: A corresponding startup signal is generated in response to a startup instruction input by a user or based on detection data of a detection sensor.

In some embodiments, the generation of the startup signal may be controlled by the user. The user may send an instruction to a breast pump through a terminal to start a corresponding lactation stimulation mode. The control unit receives the startup signal to start the corresponding lactation stimulation mode. In some instances, the breast pump may be provided with a switch key for the corresponding lactation stimulation mode. When the user turns on the switch key, an instruction is sent to the control unit to start the corresponding lactation stimulation mode. The control unit receives the startup signal and controls the stimulation unit 220 to operate.

In some implementations, the startup signal may be generated through the detection data of the sensor. For example, the startup signal may be generated through the flow rate during breast milk expressing detected by the flow rate sensor or through an ambient temperature detected by an ambient temperature sensor mounted on the housing.

Second step: The stimulation unit is started to operate in a preset mode based on the startup signal, where the preset mode includes at least a first mode for stimulating the breast to heat up.

In some implementations, the preset mode includes at least the first mode for stimulating the breast to heat up, so as to resolve a problem that traditional hot compress methods are prone to scalding skin, thereby more effectively unclogging blocked breast ducts.

In some implementations, the second step includes following steps.
(1). The infrared generator of the stimulation unit is controlled to start operating to enter the first mode.
(2). Alternatively, the stimulation unit is controlled to enter a second mode, where in the second mode, the infrared generator stops operating, and the massage assembly of the stimulation unit starts operating to massage the breast.
(3). Alternatively, the stimulation unit is controlled to enter a third mode, where in the third mode, the infrared generator and the massage assembly of the stimulation unit start to operate to massage the breast while stimulating the breast to heat up.

In some implementations, performing massage through the massage assembly can unclog blocked milk ducts.

In some implementations, the breast pump includes a first key, a second key, and a third key. The user triggers the first key to generate a startup instruction to enter the first mode, triggers the second key to generate a startup instruction to enter the second mode, and triggers the third key to generate a startup instruction to enter the third mode.

In some implementations, the breast pump includes a flow rate sensor, which generates different startup instructions when detecting flow rates in different ranges. This is described in detail below in this specification, and is not described in detail herein.

In some implementations, the breast pump includes an ambient temperature sensor, which generates different startup instructions when detecting ambient temperatures in different ranges.

Exemplarily, when the ambient temperature is less than a first ambient temperature threshold, a startup instruction is generated to enter the third mode; when the ambient temperature is greater than or equal to the first ambient temperature threshold and less than a second ambient temperature threshold, a startup instruction is generated to enter the second mode; or when the ambient temperature is greater than or equal to the second ambient temperature threshold, a startup instruction is generated to enter the first mode.

The ambient temperature sensor may be arranged on the housing to measure a room temperature. When the ambient temperature is low, that is, less than the first ambient temperature threshold, poor blood circulation due to cold weather increases a likelihood of milk duct blockage. In this case, the third mode is started, to perform both breast heating and massage. When the ambient temperature is moderate, that is, greater than or equal to the first ambient temperature threshold and less than the second ambient temperature threshold, only breast massage is performed. When the ambient temperature is high, only breast heating stimulation is performed.

The above manners provide three operating modes for the stimulation unit, for selection by the user based on personal needs or for intelligent selection based on data of the sensor, thereby improving user experience.

In some implementations, the first step includes a following step.

A corresponding startup signal is generated based on the flow rate during breast milk expressing by the breast pump detected by the flow rate sensor.

In some implementations, the step of generating a corresponding startup signal based on the flow rate during breast milk expressing by the breast pump detected by the flow rate sensor includes a following step.
(1). A first startup signal is generated when the flow rate is less than a first flow rate threshold and greater than or equal to a second flow rate threshold.

In this case, the second step includes a following step.

(2). The infrared generator of the stimulation unit is controlled to start operating to enter the first mode based on the first startup signal.

Specifically, during the milk expressing by the breast pump, the flow rate sensor detects the flow rate when the breast milk flows into the milk storage container portion 50. If the flow rate is less than the first flow rate threshold and greater than or equal to the second flow rate threshold, it indicates that the flow rate is high. In this case, only breast heating stimulation is performed, to prevent milk duct blockage.

In some implementations, the step of generating a corresponding startup signal based on the flow rate during breast milk expressing by the breast pump detected by the flow rate sensor includes a following step.
(1). A second startup signal is generated when the flow rate is less than the second flow rate threshold and greater than or equal to a third flow rate threshold.

In this case, the second step includes a following step.

(2). The stimulation unit is controlled to enter the second mode based on the second startup signal, where in the second mode, the infrared generator stops operating, and the massage assembly of the stimulation unit starts operating to massage the breast.

Specifically, during the milk expressing by the breast pump, the flow rate sensor detects the flow rate when the breast milk flows into the milk storage container portion 50. If the flow rate is less than the second flow rate threshold and greater than or equal to the third flow rate threshold, it indicates that the flow rate is moderate. In this case, only massage is performed.

Furthermore, in most cases, stimulating the breast to heat up through the infrared generator is a non-contact manner, which is relatively gentle and provides comfortable user experience. However, this manner takes effect slowly, as this manner requires a specific heating time. If the flow rate is high, indicating a low likelihood of milk duct blockage of the user, only breast heating stimulation is performed. The massage manner using the massage assembly works quickly, but requires direct contact with the breast of the user, which may cause discomfort. Therefore, massage is performed only when the flow rate is moderate.

In some implementations, the step of generating a corresponding startup signal based on the flow rate during breast milk expressing by the breast pump detected by the flow rate sensor includes a following step.
(1). The flow rate sensor generates a third startup signal when the flow rate is less than the third flow rate threshold.

In this case, the second step includes a following step.

(2). The stimulation unit is controlled to enter the third mode based on the third startup signal, where in the third mode, the infrared generator and the massage assembly of the stimulation unit start to operate to massage the breast while stimulating the breast to heat up.

Specifically, during the milk expressing by the breast pump, the flow rate sensor detects the flow rate when the breast milk flows into the milk storage container portion 50. If the flow rate is less than the third flow rate threshold, it indicates that the flow rate is low. In this case, both breast heating stimulation and massage are performed to maximize unclogging of blocked milk ducts, increase a breast milk expressing volume, and prevent insufficient breast milk expressing.

In this embodiment of the present disclosure, the first flow rate threshold, the second flow rate threshold, or the third flow rate threshold are preset to an average flow rate of different women during normal milk expressing with a breast pump, as derived from big data. When the flow rate does not reach the average flow rate, lactation stimulation needs to be performed on the breast before milk expressing. If the flow rate exceeds the average milk volume, the control unit does not start the lactation stimulation mode and remains in the current operation.

In some implementations, the first flow rate threshold depends on the average flow rate.

Exemplarily, the first flow rate threshold is equal to the average flow rate, or is equal to the average flow rate multiplied by a preset scaling factor, such as 0.9 or 1.1.

In this case, when the average flow rate determined from big data changes, the first traffic threshold also changes.

In some implementations, the moderate flow rate described above refers to a flow rate with a moderate difference from the average flow rate, the large flow rate described above refers to a flow rate with a small difference from the average flow rate, and the small flow rate described above refers to a flow rate with a large difference from the average flow rate.

In some implementations, the breast pump can be flexibly switched between different modes, and the current mode needs to be stopped before switching to a new mode. To enhance operational convenience, during the switching between different modes, if functional units that need to be started, such as the infrared generator and the massage assembly, are already in operation, the functional units can simply remain in operation, without a need to turn off and then restart the functional units, thereby avoiding energy wastes.

In some implementations, maximum operation durations of the three modes may be adjusted without a need to be uniform. Once the maximum operation durations are reached, the control unit controls the breast pump to stop the current mode.

In some implementations, the method in this embodiment may only include the first step and the second step.

Third step: A continuous operating duration of the stimulation unit is acquired.

Fourth step: It is determined whether the continuous operating duration is greater than a preset duration threshold.

In some implementations, the preset duration threshold is preset at the factory and may be independently set by a user after leaving the factory.

Fifth step: After it is determined that the continuous operating duration is greater than the preset duration threshold, the infrared generator and/or the massage assembly of the stimulation unit in an operating state is controlled to stop operating, and the negative pressure mechanism of the breast pump is started to perform milk expressing.

In some implementations, the control unit includes a timing module arranged therein, which records the continuous operating time of the stimulation unit.

In this way, milk expressing can be performed after the breast is fully massaged, which can unclog blocked milk ducts in a timely manner to prevent milk duct blockage.

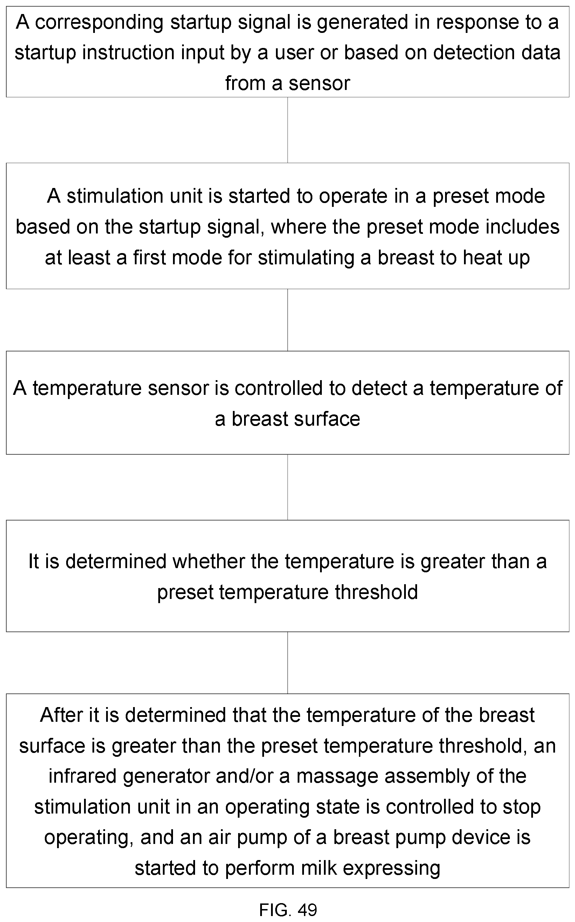

In some implementations, referring to FIG. 49, FIG. 49 is a schematic flowchart of a third embodiment of a control method according to an embodiment of this application. As shown in FIG. 49, the control method includes a sixth step to a tenth step.

Sixth step: A corresponding startup signal is generated in response to a startup instruction input by a user or based on detection data of a detection sensor.

Seventh step: The stimulation unit is started to operate in a preset mode based on the startup signal, where the preset mode includes at least a first mode for stimulating the breast to heat up.

Eighth step: The temperature sensor is controlled to detect a temperature of a breast surface.

Ninth step: It is determined whether the temperature is greater than a preset temperature threshold.

In some implementations, the preset temperature threshold is preset at the factory and may be independently set by a user after leaving the factory.

In some implementations, the preset temperature threshold cannot be excessively high and needs to be lower than a temperature that can burn human skin, for example, not higher than 45°C, to prevent skin burns.

In some implementations, the preset temperature threshold cannot be excessively low, for example, not lower than the normal temperature of human bodies, that is, 36°C, to prevent the stimulation unit from being terminated at the beginning of operation and therefore being unable to unclog blocked milk ducts.

Tenth step: After it is determined that the temperature of the breast surface is greater than the preset temperature threshold, the infrared generator and/or the massage assembly of the stimulation unit in an operating state is controlled to stop operating, and the negative pressure mechanism of the breast pump is started to perform milk expressing.

In some implementations, after it is determined that the temperature of the breast surface is greater than the preset temperature threshold, it is determined that the stimulation unit has sufficiently stimulated the breast and milk expressing can be started.

### Eighth embodiment

In some implementations, the performing a preset operation based on the control parameter includes:
performing the preset operation based on the volume of the breast milk stored in the milk storage container portion.

In some implementations, the performing the preset operation based on the volume of the breast milk stored in the milk storage container portion includes: displaying the volume of breast milk stored in the milk storage container portion on the display interface of the breast pump, and sending information to the terminal to display the volume of breast milk stored in the milk storage container portion.

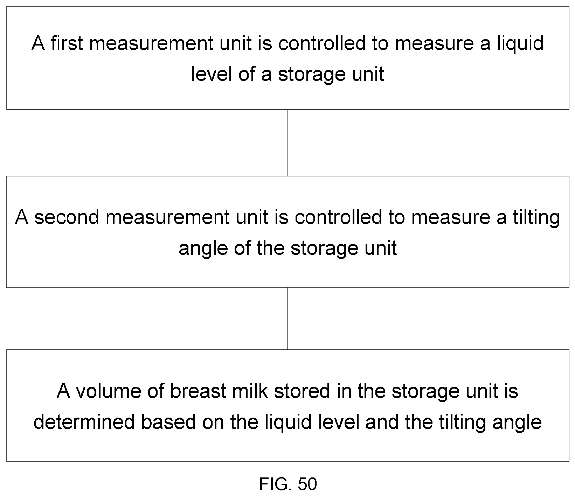

Referring to FIG. 50, FIG. 50 is a schematic flowchart of a control method according to an embodiment of this application. As shown in FIG. 50, the control method includes a first step to a third step.

First step: A first measurement unit is controlled to measure a liquid level of the milk storage container portion.

In some implementations, the first measurement unit is a time of flight (TOF) sensor.

Optionally, the TOF sensor is arranged on a top of the milk storage container portion.

In some implementations, the first measurement unit includes a plurality of liquid level sensors.

Optionally, the plurality of liquid level sensors are arranged at specific intervals on a side wall of the milk storage container portion.

Second step: A second measurement unit is controlled to measure a tilting angle of the milk storage container portion.

In some implementations, the second measurement unit is a three-axis acceleration sensor configured to measure a three-axis acceleration of the milk storage container portion.

In some implementations, a calculation formula for a tilting angle α is as follows: $α = arccos \left(\frac{z}{g}\right) \times \frac{180}{π} .$

z is a z-axis acceleration measured by the three-axis acceleration sensor, and g is a gravitational acceleration.

In some implementations, the second step includes a following step.
(1). The second measurement unit is controlled to measure the tilting angle of the milk storage container portion when the first volume is within a first volume range.

In some implementations, when the volume of the breast milk stored in the milk storage container portion is excessively high or excessively high, measurement errors are minimal, and no volume correction is necessary.

In some implementations, the first volume range is [10 mL, 150 mL].

Third step: The volume of the breast milk stored in the milk storage container portion is determined based on the liquid level and the tilting angle.

In some implementations, the third step includes a first substep (1) to a third substep (3).

First substep (1): A first volume of the breast milk stored in the milk storage container portion is determined based on the liquid level.

In some implementations, the measured liquid level is not a specific value but rather a plurality of values within a range. In particular, when the milk storage container portion is tilted, the liquid surface is also tilted. Therefore, a plurality of liquid levels are measured. In this case, a value may be selected from the liquid level range based on a selection rule and determined as the liquid level for a subsequent calculation step.

In some implementations, the selection rule is to select a minimum, middle, or maximum value within the liquid level range.

It may be understood that different selection rules affect a value of a correction parameter in the third step. Exemplarily, a correction parameter for the minimum value selected from the liquid level range is greater than a correction coefficient for the maximum value selected from the liquid level range.

Second substep (2): A correction parameter is determined based on the liquid level and the tilting angle.

In some implementations, the milk storage container portion is not shaped like a cylinder or cuboid having a linear relationship between a liquid level difference and a breast milk volume difference. The linear relationship between a liquid level difference and a breast milk volume difference means that a breast milk volume increase is consistent when the liquid level increases by a preset height from any height. For example, if the liquid level rises by 2 com from 3 cm to 5 cm, a variation in the volume of the breast milk stored in the milk storage container portion is equal to the variation in the liquid level from 5 cm to 7 cm.

In some implementations, as described above, the milk storage container portion is in a shape having a linear relationship between a liquid level difference and a breast milk volume difference, such as a bowl shape. If the shape is bowl-like, when the liquid level increases by 2 cm from 3 cm to 5 cm, a variation in the volume of the breast milk stored in the milk storage container portion is less than the variation in the liquid level from 5 cm to 7 cm. This is because a liquid level indicates a larger cross-sectional area of the bowl and indicates storage of more breast milk.

Therefore, if the milk storage container portion is in a shape without a linear relationship between a liquid level difference and a breast milk volume difference, the liquid level needs to be considered during calculation of the correction parameter.

In some implementations, when the volume of the breast milk stored in the milk storage container portion is known, the milk storage container portion may be tilted for measurement. A plurality of groups of data is recorded, and then the measured data is statistically analyzed to establish a correspondence table among a liquid level, a tilting angle, and a correction parameter. A unique correction parameter can be determined based on a liquid level and a tilting angle.

Exemplarily, with a maximum storage volume of the milk storage container portion defined as 150 mL and a maximum liquid level defined as 10 cm, a plurality of measurement sets are established, each with a different actual volume of breast milk stored in the milk storage container portion.

For example, an actual volume of breast milk stored in the milk storage container portion in a first measurement group, that is, the second volume, is 20 mL, an actual volume in a second measurement group is 25 mL, and so on, and an actual volume in a twenty-first measurement group is 130 mL. The first measurement group is tilted to 5°, 10°, ..., 45°, 50°, ..., N°, where N is a positive integer greater than 50. A first volume and a liquid level at each tilting angle are recorded separately, and then a correction parameter is calculated based on the second volume and the first volume, and the liquid level, the tilting angle, and the correction parameter at each tilting angle are recorded, and are filled into the correspondence table among a liquid level, a tilting angle, and a correction parameter. The same applies to other measurement groups. Details are not described herein. In this way, the correspondence table among a liquid level, a tilting angle, and a correction parameter.

In the above manner, the correction parameter may be a difference between the second volume and the first volume, or may be a proportional relationship between the second volume and the first volume.

Third substep (3): The first volume is determined based on the correction parameter, to obtain a second volume of the breast milk stored in the milk storage container portion.

Through the correction of the first volume, the tilting angle can be introduced during the calculation of the breast milk volume, thereby improving accuracy of the calculated second volume.

In some implementations, after the third substep (3), the third step further includes a fourth substep (4).

Fourth substep (4): A preset operation is performed when the tilting angle is greater than a preset angle value.

In some implementations, the preset operation includes but is not limited to stopping milk expressing and sending prompt information.

Sending prompt information includes but is not limited to controlling the alarm light to light up, controlling a buzzer to send an alarm, sending a voice prompt, and sending warning information to a terminal in communication with the breast pump, to prompt an APP run on the terminal to pop up a warning window, and so on.

If the tilting angle is excessively large, the breast milk may spill out of the milk storage container portion. In mild cases, this may lead to wastes. More severely, the breast milk may flow back into a negative pressure mechanism 20 of the breast pump, causing damage to the negative pressure mechanism 20. Therefore, in this application, when the tilting angle is greater than a preset value, the preset operation is performed, to prevent breast milk leakage, avoid wastes, and ensure safe operation of the breast pump.

In some implementations, the third step includes a fifth substep (5) to a sixth substep (6).

Fifth substep (5): A calculation formula is acquired, where the calculation formula is determined based on a capacity and a shape of the milk storage container portion.

In some implementations, the calculation formula is established based on data obtained from prior measurements, and milk storage container portions with different capacities and shapes correspond to different calculation formulas.

Exemplarily, with a maximum storage volume of the milk storage container portion defined as 150 mL and a maximum liquid level defined as 10 cm, a plurality of measurement sets are established, each with at least one of a different actual volume of breast milk stored in the milk storage container portion, a different tilting angle, or a different liquid level.

For example, an actual volume of breast milk stored in the milk storage container portion in a first measurement group, that is, the second volume, is 20 mL, an actual volume in a second measurement group is 25 mL, and so on, and an actual volume in a twenty-first measurement group is 130 mL.

The first measurement group is tilted to 5°, 10°, ..., 45°, 50°. A liquid level at each tilting angle is recorded, to obtain data corresponding to a plurality of groups of liquid levels, tilting angles, and actual volumes. The same applies to other measurement groups. Details are not described herein.

A regression parameter is determined based on the data corresponding to the plurality of groups of liquid levels, tilting angles, and actual volumes through multiple linear regression or multiple nonlinear regression, to establish a regression line or a regression curve, which is used as the calculation formula corresponding to the milk storage container portion.

For example, the calculation formula is: volume=A*(liquid level)+B*(tilting angle)+C, where A, B, and C are all regression parameters.

In some implementations, milk storage container portions of different shapes and capacities correspond to different calculation formulas. Specifically, calculation formulas differ not only for milk storage container portions with a same shape but different capacities but also for milk storage container portions with different shapes but a same capacity, as well as for milk storage container portions with different shapes and different capacities.

Furthermore, the milk storage container portions referred to in the statement "milk storage container portions of different shapes and capacities correspond to different calculation formulas" all refer to milk storage container portions without a linear relationship between a liquid level difference and a breast milk volume difference.

It may be understood that, for milk storage container portions that have a linear relationship between a liquid level difference and a breast milk volume difference, for example, milk storage container portions in a shape of a regular cylinder, a calculation formula remains the same. In this case, it is unnecessary to set a plurality of calculation formulas for milk storage container portions of different capacities.

Sixth substep (6): The volume of the breast milk stored in the milk storage container portion is determined based on the liquid level, the tilting angle, and the calculation formula.

In some implementations, the liquid level and the tilting angle may be substituted into the calculation formula, to determine the volume of the breast milk stored in the milk storage container portion.

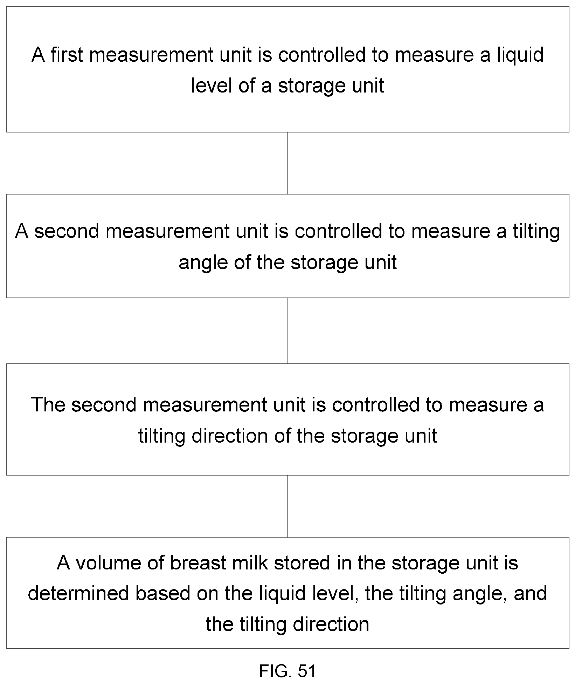

Referring to FIG. 51, FIG. 51 is a schematic flowchart of a breast milk volume measurement method for a breast pump according to an embodiment of this application. As shown in FIG. 51, the control method includes a fourth step to a seventh step.

Fourth step: A first measurement unit is controlled to measure a liquid level of the milk storage container portion.

For details, refer to the above description in this specification. Details are not described herein.

Fifth step: A second measurement unit is controlled to measure a tilting angle of the milk storage container portion.

For details, refer to the above description in this specification. Details are not described herein.

Sixth step: The second measurement unit is controlled to measure a tilting direction of the milk storage container portion.

In some implementations, the second measurement unit is a three-axis acceleration sensor configured to measure a three-axis acceleration of the milk storage container portion.

In some implementations, a calculation formula for the tilting angle β is as follows: $β = arctan \left(\frac{y}{x}\right) \times \frac{180}{π} .$
y is a y-axis acceleration measured by the three-axis acceleration sensor, and x is an x-axis acceleration measured by the three-axis acceleration sensor

Seventh step: The volume of the breast milk stored in the milk storage container portion is determined based on the liquid level, the tilting angle, and the tilting direction.

In some implementations, the irregular shape of the milk storage container portion leads to different liquid levels when the milk storage container portion is tilted by a same angle in different directions, even when the volume of the breast milk stored in the milk storage container portion is consistent.

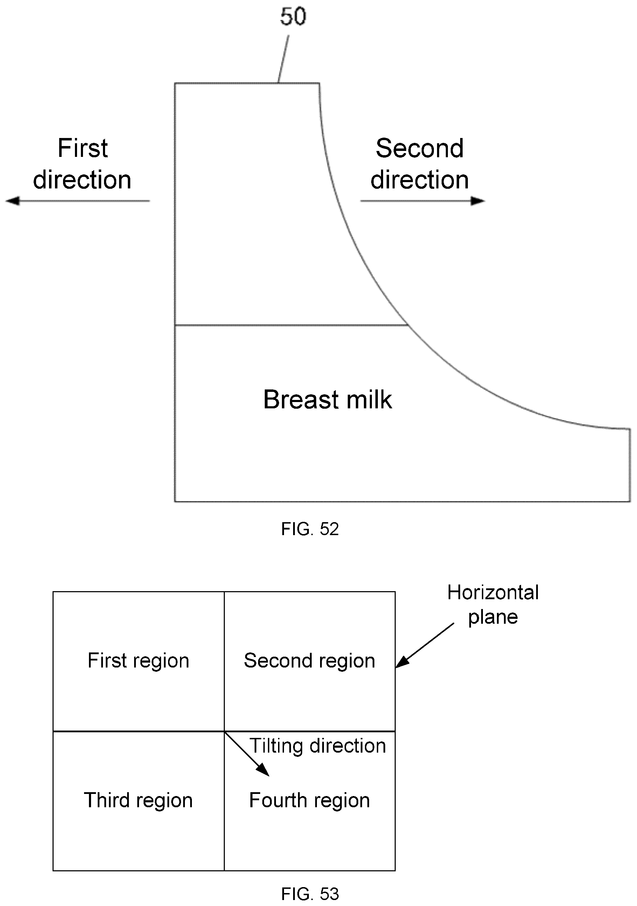

Referring to FIG. 52, FIG. 52 is a schematic structural diagram of a milk storage container portion according to an embodiment of this application. As shown in FIG. 52, when a milk storage container portion 50 in FIG. 52 is tilted in a first direction and a second direction, a same volume of breast milk forms different liquid levels. Therefore, calculating the volume of the breast milk stored in the milk storage container portion further requires taking the tilting direction of the milk storage container portion into consideration.

In some implementations, the seventh step includes following steps.
(1). A region in a first plane of the tilting direction is determined.
(2). A calculation formula is determined based on the region in the first plane of the tilting direction.
(3). The volume of the breast milk stored in the milk storage container portion is determined based on the liquid level, the tilting angle, and the calculation formula.

In some implementations, the tilting direction refers to a direction of tilting on a horizontal plane, and may be determined based on a direction of change in an axis of the milk storage container portion.

Referring to FIG. 53, FIG. 53 is a schematic diagram of region division according to an embodiment of this application; As shown in FIG. 53, a horizontal plane is divided into four regions, each corresponding to a calculation formula. It should be understood that the horizontal plane is divided into at least two regions. A specific number of regions may be selected as needed, which is not limited herein.

In some implementations, the calculation formula needs to be determined through prior measurements. For example, a coordinate system is established on the horizontal plane, with a tilting direction of the second region defined as [0°, 90°]. A plurality of measurements are performed in a direction of 45°, and a calculation formula corresponding to the direction of 45° is then determined as a calculation formula for the entire second region.

For a calculation formula determining method, refer to the above description in this specification. Details are not described herein.

In some implementations, a calculation accuracy of the calculation formula is directly proportional to a number of defined regions.

In some implementations, the seventh step includes following steps.
(1). A region in a first plane of the tilting direction is determined.
(2). A correspondence table is determined based on the region in the first plane of the tilting direction.
(3). The correspondence table is searched for a correction parameter based on the liquid level and the tilting angle.
(4). A first volume of the breast milk stored in the milk storage container portion is determined based on the liquid level.
(5). The first volume is corrected based on the correction parameter, to obtain a second volume of the breast milk stored in the milk storage container portion.

In some implementations, each region within the first plane corresponds to a correspondence table. The correspondence table is used to store the correspondence between a liquid level, a tilting angle, and a correction parameter. A corresponding correction parameter can be queried from the correspondence table based on the liquid level and the tilting angle.

In some implementations, the correspondence table needs to be pre-established. For example, a coordinate system is established on the horizontal plane, with a tilting direction of the second region defined as [0°, 90°]. A plurality of measurements are taken in performed in a direction of 45°, and a correspondence table determined in the direction of 45° is then determined as a correspondence table for the entire second region.

### Ninth embodiment

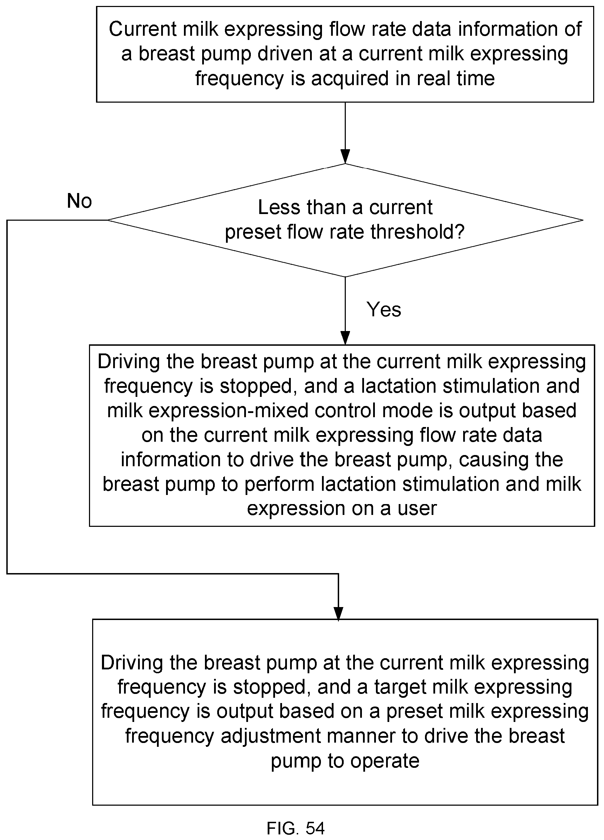

Referring to FIG. 54, an embodiment of the present disclosure provides a control method. During actual use, the control method is applied to a breast pump control unit or to a terminal device connected to a breast pump. The control method includes a first step to a fourth step.

First step: Current milk expressing flow rate data information of the breast pump driven at a current milk expressing frequency is acquired in real time.

A milk expressing frequency refers to a number of times (or expressed as a milk expressing duration) the breast pump performs milk expressing on a user during a pumping cycle. Under different milk expressing frequencies, a flow rate of milk expressing performed by the breast pump on a user is usually inconsistent. In actual use, a relationship between a milk expressing flow rate and a milk expressing frequency is not simply linear, and simply increasing the milk expressing frequency does not necessarily increase the milk expressing flow rate. Therefore, it is necessary to adaptively adjust an operating mode of the breast pump based on the current milk expressing frequency and the current milk expressing flow rate data information.

Second step: It is determined whether the current milk expressing flow rate data information is less than a current preset flow rate threshold.

Different milk expressing frequencies correspond to different preset flow rate thresholds. Therefore, in this step, the current milk expressing flow rate data information is acquired, and is compared with the current preset flow rate threshold. The breast pump can be adjusted to an efficient milk expressing mode based on the current relationship.

If it is determined in the second step that the current milk expressing flow rate data information is less than the current preset flow rate threshold, a following step is performed:
Third step: Driving the breast pump at the current milk expressing frequency is stopped, and a lactation stimulation and milk expression-mixed control mode is output based on the current milk expressing flow rate data information to drive the breast pump, causing the breast pump to perform lactation stimulation and milk expression on the user.

Since it is determined in the second step that the current milk expressing flow rate data information is less than the current preset flow rate threshold, it indicates that a breast milk volume expressed by the breast pump at the current milk expressing frequency is lower than a corresponding current preset flow rate value. Therefore, it is necessary to output a lactation stimulation and milk expression-mixed control mode based on the current milk expressing flow rate data information to drive the breast pump and reduce the current milk expressing frequency, causing the breast pump to perform lactation stimulation and milk expression on the user.

If it is determined in the second step that the current milk expressing flow rate data information is greater than or equal to the current preset flow rate threshold, a following step is performed:
Fourth step: Driving the breast pump at the current milk expressing frequency is stopped, and a target milk expressing frequency is output based on a preset milk expressing frequency adjustment manner to drive the breast pump to operate.

If it is determined in the second step that the current milk expressing flow rate data information is not less than the current preset flow rate threshold, it indicates that the current milk expressing frequency is consistent with the target milk expressing frequency for the user to use the breast pump, or the current milk expressing frequency is lower than the target milk expressing frequency for the user to use the breast pump. Therefore, it is necessary to adjust the current milk expressing frequency based on the preset milk expressing frequency adjustment manner and output the target milk expressing frequency to drive the breast pump, so as to improve current milk expressing efficiency.

In some embodiments, after completion of the third step or the fourth step, a milk expressing frequency is output as the current milk expressing frequency to drive the operation of the breast pump. In this case, the current preset flow rate threshold is correspondingly updated based on the current milk expressing frequency. That is, different current milk expressing frequencies correspond to different current preset flow rate thresholds.

In some embodiments, a default milk expressing frequency or a milk expressing frequency set by the user is used as the current milk expressing frequency to drive the breast pump to perform milk expressing operations. Due to different user parameters or different environmental parameters, the obtained current milk expressing flow rate data information of the breast pump varies. Therefore, adapting to different current milk expressing flow rate data information, a plurality of lactation stimulation and milk expression-mixed control modes of different levels are correspondingly established. In this way, after a lactation stimulation and milk expression-mixed control mode of a corresponding level is obtained based on the current milk expressing flow rate data information, the breast pump can be driven based on the lactation stimulation and milk expression-mixed control mode of the corresponding level.

In some embodiments, one milk pumping cycle for the breast pump includes a milk expressing frequency, a lactation stimulation frequency, and usually, a pause frequency. In this embodiment, different lactation stimulation and milk expression-mixed control mode correspond to different milk expressing frequencies and different lactation stimulation frequencies within a milk pumping cycle. Therefore, after the lactation stimulation and milk expression-mixed control mode of the corresponding level is obtained based on the current milk expressing flow rate data information, the breast pump can be driven with the corresponding milk expressing frequency and lactation stimulation frequency based on the specific current milk expressing flow rate data information. For example, if the current milk expressing flow rate data information is less than the current preset flow rate threshold, a difference between the two is acquired, and a lactation stimulation and milk expression-mixed control mode of a suitable level is output based on the difference. Clearly, a larger difference indicates a smaller milk expressing frequency and a larger lactation stimulation frequency in the lactation stimulation and milk expression-mixed control mode of the suitable level. A smaller difference indicates a larger milk expressing frequency and a smaller lactation stimulation frequency in the lactation stimulation and milk expression-mixed control mode of the suitable level. In this embodiment, a specific relationship between the difference and the milk expressing frequency and the lactation stimulation frequency may be established as an optimal correspondence derived from a plurality of rounds of data acquisition, calculation, and verification.

In some embodiments, the fourth step of outputting a target milk expressing frequency based on a preset milk expressing frequency adjustment manner to drive the breast pump to operate includes a first substep to a fifth substep.

First substep: A milk expressing frequency adjustment factor is set.

In this embodiment, due to a need to increase the current milk expressing frequency in order to improve the milk expressing efficiency of the breast pump, the milk expressing frequency adjustment factor is set to a value greater than 1. Generally, an initial value of the milk expressing frequency adjustment factor is set to a value that is relatively close to 1, for example, a value between 1.05 and 1.30.

Second substep: An adjusted milk expressing frequency is calculated based on the milk expressing frequency adjustment factor and the current milk expressing frequency.

In this embodiment, a multiplication result of the milk expressing frequency adjustment factor and the current milk expressing frequency is acquired as the adjusted milk expressing frequency. For example, a milk pumping cycle is ten minutes. If a milk expressing duration occurs 100 times, a current milk expressing frequency is 10/min. If the milk expressing frequency adjustment factor is 1.1, the adjusted milk expressing frequency is 0.11/min. A milk pumping cycle includes a plurality of milk expressing durations that may be set adaptively. Generally, a milk expressing duration is between 0.1s and 3s, and optionally, between 0.3s and 3 s. For example, a milk expressing duration is 0.3s, 2s, or 3s. Clearly, when a long milk expressing duration is set, the initial default milk expressing frequency of the breast pump is adaptively reduced.

Third substep: The adjusted milk expressing frequency is applied as current milk expressing frequency to the breast pump to obtain adjusted milk expressing flow rate data information.

After the adjusted milk expressing frequency is calculated based on the milk expressing frequency adjustment factor and the current milk expressing frequency in the second substep, the adjusted milk expressing frequency is used to the breast pump to obtain the corresponding adjusted milk expressing flow rate data information.

Fourth substep: The current milk expressing frequency is continuously updated to obtain a plurality of pieces of adjusted milk expressing flow rate data information.

In order to obtain a milk expressing frequency corresponding to higher milk expressing efficiency, it is necessary to gradually increase and update the milk expressing frequency and obtain a plurality of pieces of adjusted milk expressing flow rate data information. After repeated execution of the second substep and the third substep, a plurality of pieces of adjusted milk expressing flow rate data information can be obtained.

Fifth substep: A current milk expressing frequency is acquired as the target milk expressing frequency if the adjusted milk expressing flow rate data information no longer increases with adjustment of the current milk expressing frequency.

Through repeated execution of the second substep to the fourth substep described above, adjusted milk expressing flow rate data information corresponding to different current milk expressing frequencies is obtained. However, as the current milk expressing frequency increases, the acquired adjusted milk expressing flow rate data information stops further increase after reaching a maximum value due to user factors. Therefore, a current milk expressing frequency corresponding to the maximum value is acquired as the target milk expressing frequency to drive the breast pump to operate, so that the breast pump can provide the user with an efficient control method that can achieve adaptive adjustment to an efficient milk expressing mode.

In some embodiments, in response to the breast pump being driven to operate based on the lactation stimulation and milk expression-mixed control mode, the breast pump needs to perform lactation stimulation on the user. In this embodiment, a lactation stimulation assembly is arranged on the breast shield of the breast pump. During lactation stimulation, the breast pump controls the lactation stimulation assembly arranged on the breast shield to operate, to massage and relax the breast of the user, thereby expressing more milk during subsequent milk expressing by the breast pump. In a specific example, the lactation stimulation assembly includes a plurality of vibrators embedded and fixed in the breast shield. This allows the plurality of vibrators to be attached to the breast of the user during use of the breast pump by the user. Therefore, during the lactation stimulation, the plurality of vibrators can be controlled to perform various massage actions such as vibration and rolling on the breast of the user to promote milk flows.

In some embodiments, in order to achieve more accurate and efficient adaptive adjustment of the breast pump to an efficient milk expressing mode, it is necessary to ensure accuracy of the obtained current milk expressing flow rate data information. Therefore, in this embodiment, a flow rate sensor is arranged at a milk expressing channel of the breast pump to acquire the current milk expressing flow rate data information. In a specific example, the flow rate sensor includes a photoelectric sensor. An emitter and a receiver of the photoelectric sensor are respectively arranged on opposite sides of a nipple channel of the breast pump. Therefore, when the breast pump pumps breast milk, the photoelectric sensor can accurately obtain the current milk expressing flow rate data information.

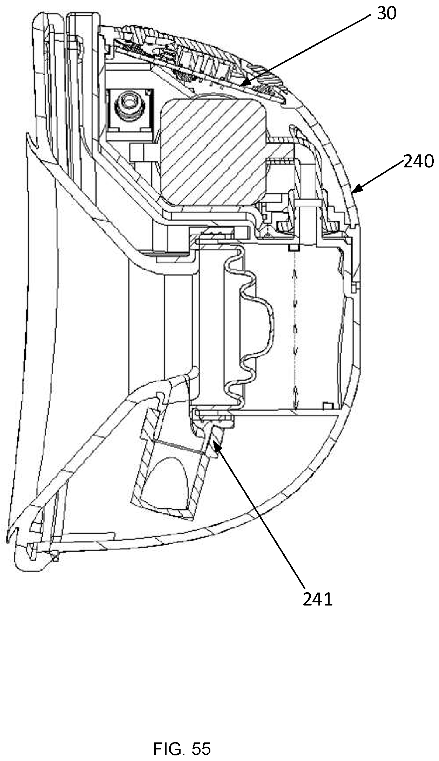

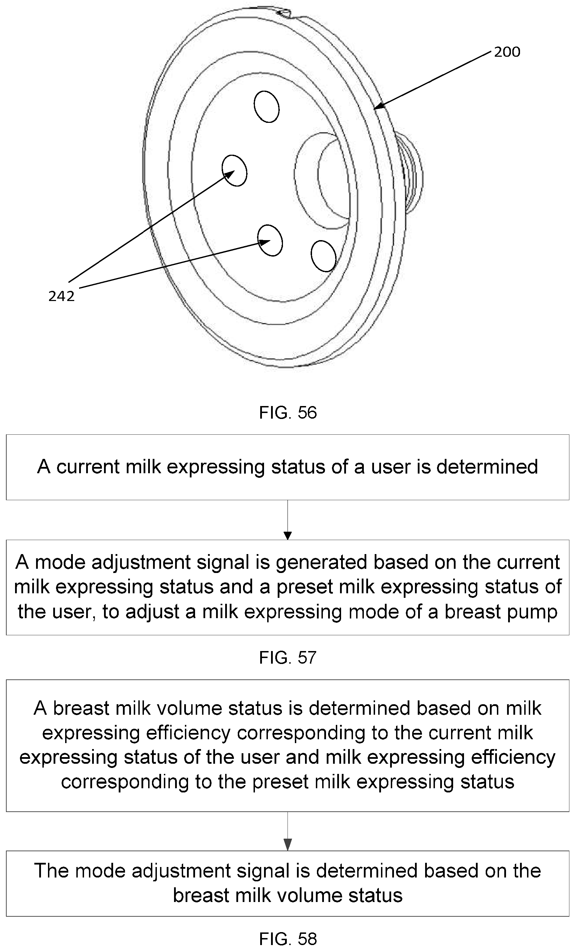

Referring to FIG. 55 and FIG. 56, an embodiment of the present disclosure further provides a breast pump, including a housing 240, a flow rate monitoring assembly 241, a lactation stimulation assembly 242, and a control unit 30. A flow rate monitoring assembly 500 is arranged in the housing 240, and is configured to monitor flow rate data information during breast milk expressing. The lactation stimulation assembly 242 is embedded in a breast shield 10. When the breast shield 10 is attached to a breast for milk expressing, the lactation stimulation assembly 242 is also attached to the breast, which performs milk expressing on a user during operation. The control unit 30 is electrically connected to a negative pressure mechanism 20, the flow rate monitoring assembly 241, and the lactation stimulation assembly 242, and is configured to receive flow rate data information acquired by the flow rate monitoring assembly 241 to perform the control method in any one of the above embodiments, and then output a control signal to drive the negative pressure mechanism 20 and the lactation stimulation assembly 242.

Referring to FIG. 55, in some embodiments, in order to obtain more accurate flow rate data information, the flow rate monitoring assembly 500 in the above embodiments includes a flow rate sensor arranged in a milk expressing channel of the housing 240 to acquire flow rate data information. In a specific example, the flow rate sensor is implemented by a photoelectric sensor. An emitter and a receiver of the photoelectric sensor are respectively arranged on opposite sides of the milk expressing channel. Therefore, when breast milk passes through the milk expressing channel, the photoelectric sensor can accurately monitor the flow rate data information.

Referring to FIG. 56, in some embodiments, the lactation stimulation assembly 242 includes a plurality of oscillators embedded in an inner side wall of the breast shield 10 in a preset arrangement. During use by the user, the breast shield 10 is attached to the breast to perform milk expressing, and the plurality of oscillators are also attached to the breast. Therefore, during lactation stimulation, the control unit 30 controls the plurality of oscillators to operate, to perform lactation stimulation on the breast.

### Tenth embodiment

Referring to a flowchart of a control method in an embodiment of this specification shown in FIG. 57, in some embodiments of this specification, the control method may be applied to a breast pump.

As shown in FIG. 57, a milk expressing mode of the breast pump may be adjusted through following steps:
First step: A current milk expressing status of the user is determined.

Specifically, the current milk expressing status of the user can represent a current volume of milk stored in the breast of the user. For different volumes of milk stored in the breast, corresponding milk expressing modes may be used.

Second step: A mode adjustment signal is generated based on the current milk expressing status and a preset milk expressing status of the user, to adjust a milk expressing mode of the breast pump.

Specifically, different milk expressing modes correspond to different preset milk expressing statuses. Therefore, a mode adjustment signal corresponding to the milk expressing mode may be generated based on a relative relationship between the current milk expressing status and the preset milk expressing status of the user. Through the mode adjustment signal, the breast pump can either change or maintain the current milk expressing mode.

In some examples, the preset milk expressing status may be a specific value or a range of values. A value of a preset milk expressing volume is not specifically limited in this embodiment of this specification.

In some examples, the preset milk expressing status may be set based on a current physical status (which may specifically refer to a breast state) of the user. For example, when the breast state is engorgement, milk-sufficiency, or milk-deficiency, the corresponding preset milk expressing status varies. Therefore, milk expressing statuses of the user in different states during initial use of the breast pump by the user may be statistically analyzed to determine parameters related to the preset milk expressing status in different states, such as milk expressing efficiency and a milk expressing volume.

For example, reference data may be formed based on first three to five operations of milk expressing data of the user, to be used as the milk expressing status.

In some examples, milk expressing volume data of the breast of the user may be acquired from big data. The data may include a volume of milk expressed from each breast-feeding operation, a daily total milk expressing volume, a breast size, a breast shape, and the like. The acquired data is then organized and analyzed, to calculate statistical indicators such as an average, a standard deviation, peak and trough values, and draw trend charts illustrating changes in a milk expressing volume over time. In this way, a model for predicting a milk expressing volume of the breast of the mother can be established. The preset milk expressing status of the user can be determined based on the model.

It may be understood that the above-described method for determining the preset milk expressing status is merely an example and cannot be understood as a limitation on the present disclosure. The above description is used to indicate that the preset milk expressing status of the user may be determined based on existing data. In some other examples, the preset milk expressing status may be customized by the user.

According to the control method in the above example, a mode adjustment signal may be generated based on the current milk expressing status and the preset milk expressing status of the user, and then the milk expressing mode of the breast pump may be adjusted based on the mode adjustment signal. The entire process operates without manual intervention, enabling automatic adjustment of the milk expressing mode of the breast pump, reducing operational complexity, and achieving as complete as possible expressing of milk accumulated in breast ducts.

A breast milk expressing process usually involves expressing milk stored in the breast. Therefore, a current milk expressing volume of the user is closely related to a status of the breast of the user.

Therefore, in some optional examples, the current milk expressing volume of the user may be determined based on a milk expressing flow rate and breast feature information of the user.

Specifically, the breast feature information of the user can represent a volume of milk currently stored in the breast, which defines the current milk expressing volume. The milk expressing flow rate of the user represents a current milk expressing velocity (for example, a volume of milk expressed per unit time). With the milk expressing flow rate and the breast feature information of the user, accuracy of determining the current milk expressing status of the user can be improved.

In some implementations, the breast feature information may include at least one of breast plumpness, breast composition, breast hardness, or a breast milk expressing flow rate.

Breast plumpness refers to a size and a degree of filling of the breast. A higher breast plumpness indicates a larger current milk expressing volume of the user.

In some examples, the breast plumpness may be determined through measurement of breast size changes or measurement of breast volume changes during contraction. Breast size changes may be monitored through impedance, resistance, or acoustic measurement. Breast composition refers to components (such as breast milk and water) currently present in the breast. A higher proportion of milk in the breast composition indicates a larger current milk expressing volume of the user. Breast size changes may be further monitored using imaging technologies or sensors that detect changes in a surface of breast skin.

In some examples, breast composition may be performed through impedance monitoring. Specifically, a larger volume of milk in the breast indicates lower impedance of the breast. Impedance data information of the breast is acquired through a bioelectrical impedance method, and the volume of milk in the breast is determined based on the impedance size.

Breast hardness refers to a degree of hardening of milk in the breast. A higher degree of hardening indicates more milk stored in the breast.

In some examples, the breast hardness may be acquired through a hardness sensor.

In some examples, the breast milk expressing flow may be acquired through a flow rate sensor. The flow rate sensor is preferably arranged on or near a breast milk flow path between the breast shield and the milk storage container portion, and preferably, may be arranged on a duckbill valve. The flow rate sensor used may be an optical, electrical, or acoustic flow rate sensor, or any other suitable type of sensors that can measure a flow rate value, for example, one or more of an illuminance sensor, an infrared sensor, a capacitive sensor, or an ultrasonic sensor.

Determining the current milk expressing status of the user by using at least one of the breast plumpness, the breast composition, and the breast hardness can improve flexibility of acquiring the current milk expressing status of the user, thereby adapting to different application scenarios as needed.

In some other implementations, the current milk expressing volume of the user may be determined through measurement of a rate of breast milk expressing. A specific manner of determining the current milk expressing volume of the user is not limited in this embodiment of this specification.

According to the solution in the above example, the current milk expressing status of the user may be determined based on the milk expressing flow rate and the breast feature information of the user, and a mode adjustment signal for adjusting the milk expressing mode may be generated based on the current milk expressing status and the preset milk expressing status of the user.

In an example, FIG. 58 is a flowchart of a method for generating a mode adjustment signal according to an embodiment of this specification. As shown in FIG. 58, following steps may be specifically performed

Third step: A breast milk volume status is determined based on milk expressing efficiency corresponding to the current milk expressing status of the user and milk expressing efficiency corresponding to the preset milk expressing status.

The milk expressing efficiency refers to a volume of milk expressed from the breast per unit time. In some examples, the volume of the milk expressed from the breast may be acquired through a flow rate sensor, to determine the milk expressing efficiency per unit time.

Specifically, the flow rate sensor may be arranged on the milk flow path bringing the breast shield and the milk storage container portion (for example, a milk storage container) into communication.

Specifically, when breast milk flows through the milk flow path, the flow rate sensor can detect the presence of the breast milk. This detection causes changes in internal parameters (for example, pressure and resistance) of the flow rate sensor, with different milk expressing volumes corresponding to different magnitudes of parameter changes. Therefore, the milk expressing volume can be determined based on the parameter changes of the flow rate sensor.

In some examples, the flow rate sensor may be devices that can measure a milk flow rate, for example, a capacitive sensor, a photoelectric sensor, or a differential pressure sensor.

Specifically, the milk expressing efficiency may be determined based on the current milk expressing status of the user, where the milk expressing efficiency can represent the current milk expressing volume of the breast, and the milk expressing efficiency may be compared with the preset milk expressing efficiency, to determine the breast milk volume status used to indicate a volume of milk currently stored in the breast (for example, the breast milk volume status may be milk sufficiency, milk deficiency, or blocked).

Fourth step: The mode adjustment signal is determined based on the breast milk volume status.

Specifically, the breast milk volume status can represent a current milk volume of the breast, based on which a corresponding mode adjustment signal can be generated to adaptively adjust the milk expressing mode of the breast pump.

In some examples, when it is determined that the breast milk volume status is abundant, a milk expressing mode adjustment signal is generated to adjust the milk expressing mode of the breast pump to the milk expressing mode.

Specifically, when it is determined that the breast milk volume status is abundant, it indicates that a large amount of milk can be expressed from the breast of the user. In this case, a milk expressing mode adjustment signal is generated to adjust the milk expressing mode of the breast pump to the milk expressing mode.

The abundant breast milk state means that the volume of the milk stored in the breast is greater than a preset milk volume and the milk expressing efficiency is higher than preset efficiency.

In some examples, when it is determined that the breast milk volume status is blocked, a lactation stimulation mode adjustment signal is generated to adjust the milk expressing mode of the breast pump to the lactation stimulation mode.

Specifically, when it is determined that the breast milk volume status is blocked, it indicates that only a small amount of milk can be expressed from the breast of the user. Reasons for this phenomenon may include low milk storage in the breast or milk coagulates into hardened lumps and cannot be naturally expressed. In this case, a lactation stimulation mode adjustment signal may be generated to set the milk expressing mode of the breast pump to the lactation stimulation mode, thereby promoting milk secretion.

In some examples, the lactation stimulation mode may be vibration, hot compress, kneading massage, electrical stimulation, or a combination thereof.

In some examples, the user may customize the relevant modes and parameters corresponding to the modes.

In some examples, after a plurality of milk expressing and lactation stimulation operations, the milk in the breast gradually decreases, and even no milk can be expressed. Therefore, the control method in the example of this specification may further include: stopping a current milk expressing process when it is determined that the breast milk volume status is milk-deficiency.

A corresponding milk expressing mode can be determined based on the milk expressing efficiency corresponding to the current milk expressing status of the user and the milk expressing efficiency corresponding to the preset milk expressing status. This helps control the entire milk expressing process and improves user experience.

In some embodiments, the breast milk flow rate is not entirely consistent. For example, in an initial stage, the breast milk flow rate is relatively high, but then gradually decreases over time. Therefore, during breast milk expressing, a milk expressing frequency may be adjusted based on actual milk expressing efficiency.

For example, when the milk expressing mode of the breast pump is adjusted to the milk expressing mode, if it is determined that a difference between the milk expressing efficiency corresponding to the current milk expressing status of the user and the milk expressing efficiency corresponding to the preset milk expressing status is greater than a preset first difference, a frequency adjustment signal is generated to increase the milk expressing frequency of the breast pump.

Specifically, when it is determined that the difference between the milk expressing efficiency corresponding to the current milk expressing status of the user and the milk expressing efficiency corresponding to the preset milk expressing status is greater than the preset first difference, it indicates that the volume of the milk stored in the breast of the user far exceeds the preset milk expressing volume. In this case, the milk expressing frequency may be increased to rapidly pump the breast milk. When it is determined that the difference between the milk expressing efficiency corresponding to the current milk expressing status of the user and the milk expressing efficiency corresponding to the preset milk expressing status is less than the preset first difference, it indicates that the current milk expressing volume is still greater than the preset milk expressing volume. In this case, the generation of the frequency adjustment signal may be stopped, to pump the breast milk at a normal frequency.

In some examples, the milk expressing frequency should be set at a level that does not cause damage to the breast of the user while ensuring relatively comfortable experience for the user during the milk expressing.

In some examples, the preset first difference may be set by the user, or may be a default value preset at the factory, where the default value is a reference value that meets needs of a majority of users.

In some examples, the milk in the breast of the user may be expressed at a consistent milk expressing frequency.

Automatically adjusting the milk expressing frequency based on changes in the current milk expressing efficiency during the milk expressing can improve user experience.

In some embodiments, there may be a case where, despite a plurality of attempts in the milk expressing stimulation mode, some milk remains in the breast. In this case, it may be determined based on actual milk expressing efficiency whether to continue the lactation stimulation task.

For example, if it is determined that the difference between the milk expressing efficiency corresponding to the current milk expressing status of the user and the milk expressing efficiency corresponding to the preset milk expressing status is less than a preset second difference, a driving stop signal is generated, to cause the breast pump to stop the current lactation stimulation task.

Specifically, if it is determined that the difference between the milk expressing efficiency corresponding to the current milk expressing status of the user and the milk expressing efficiency corresponding to the preset milk expressing status is less than the preset second difference, it indicates that the volume of the milk currently stored in the breast of the user is significantly lower than the preset milk expressing volume. Even if the breast pump operates in the lactation stimulation mode, the breast cannot produce more milk. In this case, the driving stop signal may be generated to stop the current lactation stimulation task and milk expressing task.

It should be noted that, the milk expressing mode adjustment scheme in the above example is a dynamic process. For example, in an initial milk expressing stage, if hardened lumps exist in the breast (indicating a blocked state), both the milk expressing volume and the milk expressing efficiency are relatively low, which, for example, are less than the preset milk expressing volume and milk expressing efficiency. In this case, a signal may be generated to switch the breast pump to the lactation stimulation mode. As the stimulation progresses, the hardened lumps gradually dissolve, so that the milk expressing volume slowly increases. When the milk expressing efficiency turns to be greater than the preset milk expressing efficiency, a signal may be generated to switch the breast pump to the milk expressing mode. As the milk expressing proceeds, the milk in the breast gradually decreases, and so does the milk expressing volume. When the milk expressing volume turns to be less than the preset milk expressing volume again, a signal may be generated to switch the breast pump to the lactation stimulation mode, to re-perform lactation stimulation. This cycle repeats until it is determined that the difference between the current milk expressing efficiency and the preset milk expressing efficiency of the user is less than the preset second threshold. In this case, the current lactation stimulation task and milk expressing task are stopped.

During the adjustment of the milk expressing mode of the breast pump, the breast pump pumps breast milk. However, due to a limited capacity of the breast pump, the breast milk may overflow from the breast pump.

To avoid the above case, the milk expressing mode adjustment provided in this embodiment of this specification may further include: acquiring a current breast milk volume in the breast pump, and generating prompt information and/or stopping the current milk expressing task when it is determined that the current breast milk volume in the breast pump is greater than a milk volume threshold.

Specifically, when the current breast milk volume in the breast pump is greater than the breast milk volume threshold, it indicates a possibility of breast milk overflowing from the breast pump. In this case, generating the prompt information can prompt the user to stop the current breast milk expressing process in a timely manner, and/or stopping the current milk expressing task can automatically stop the current breast milk expressing process. Either of the two manners can prevent the breast milk from overflowing from the breast pump and improve user experience.

In some examples, the breast milk volume threshold may be related to the capacity of the breast pump. For example, the breast milk volume threshold may be less than the capacity of the breast pump. The breast milk volume threshold is not limited in this embodiment of this specification, which may be set based on the capacity of the breast pump.

It may be understood that, a plurality of implementations provided in the embodiments of this specification are described above. The optional manners described in the implementations may be combined and cross referenced without conflict, thereby forming a plurality of possible implementations, all of which may be considered as disclosed implementations of this specification.

For example, in some examples, during determining of the current breast milk volume in the breast pump, a signal may be further generated to display the current breast milk volume, to directly provide the user with a specific value of the current breast milk volume.

This specification further provides a milk expressing mode adjustment device corresponding to the above milk expressing mode adjustment method.

The description of the milk expressing mode adjustment device provided below can correspond to and be cross-referenced with the description of the milk expressing mode adjustment method provided above.

The milk expressing mode adjustment device may include a milk expressing status determining unit and a processing unit.

The milk volume determining unit is configured to determine a current milk expressing status of the user.

The processing unit is configured to generate a mode adjustment signal based on the current milk expressing status and a specified milk expressing status of the user, to adjust a milk expressing mode of the breast pump.

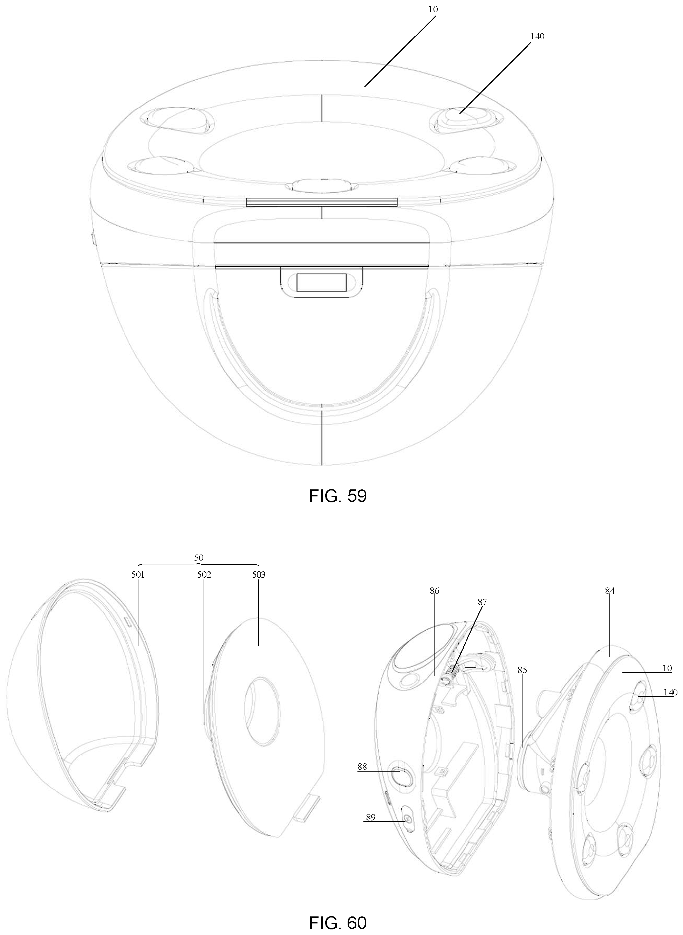

In some examples of this specification, refer to schematic structural diagram of a milk expressing assembly shown in FIG. 59 and FIG. 60. As shown in FIG. 59 and FIG. 60, the expressing assembly may include:

a massage mechanism 140 arranged on the breast shield 10, electrically connected to the milk expressing mode adjustment device, and configured to perform a lactation stimulation operation on the breast of the user when the control unit outputs a lactation stimulation mode adjustment signal.

Specifically, when the breast shield 10 is in contact with the breast of the user, the control unit can determine a current milk expressing status of the user, and then generate a mode adjustment signal based on the current milk expressing status and the preset milk expressing status of the user. For example, a milk expressing mode adjustment signal may be generated to drive the negative pressure mechanism 20 to operate to generate negative pressure. Under the negative pressure, the breast shield 10 can pump milk from the breast, and cause the breast milk to flow into the milk storage container portion 50 through a corresponding tube and be stored in the milk storage container portion 50. For another example, a lactation stimulation mode adjustment signal is generated, to drive the massage mechanism 140 to operate. The massage mechanism 140 can massage the breast of the user. When the control unit determines that the current milk expressing status is greater than the preset milk expressing status, the breast shield can pump milk from the breast.

When a current expressing task is completed or a breast milk volume reaches the breast milk volume threshold, the negative pressure mechanism 20 stops operating, and the negative pressure environment disappears. In this case, the breast shield 10 no longer pumps milk from the breast. In this case, the milk storage container portion 50 may be dismounted for feeding operations, or the breast milk in the milk storage container portion 50 may be stored in other ways. In some examples, the negative pressure mechanism may include an air passage, a vacuum generator (for example, a vacuum pump) configured to provide negative pressure to the air passage, an electromagnetic valve (for example, an electromagnetic valve 87 shown in FIG. 60) configured to open and close the air passage, and a processor configured to control a negative pressure generation process. The processor may be electrically connected to the vacuum generator and the electromagnetic valve.

In some examples of this specification, the milk storage container portion 50 may be a milk bowl or other containers matching a breast pump body for storing liquids. For example, the milk storage container portion may alternatively be a bottle made of glass.

In some examples, as shown in FIG. 60, the milk storage container portion 50 may include three parts: a bowl body 501, a duckbill valve 502, and a bowl cover 503. The bowl body 501 may include a concave milk containing cavity, with an external arc-shaped surface like a breast or a bra, and an opening for sealing by a cover body.

In some optional examples, the bowl body 501 may further include a bottom for placement on a flat surface, and the bottom includes a support flange. The bowl body 501 may further include a fitting protrusion connected to the bowl cover 503, as well as a milk outlet for pouring breast milk.

In some examples, the bowl cover 503 may include a cover plate configured to seal the opening of the bowl body 501. Specifically, the bowl cover 503 includes a flange that extends toward the milk bowl and a sealing edge that extends axially. For example, the bowl cover 503 includes a cylindrical structure extending into the milk bowl cavity, which features a milk expressing channel. One end of the cylindrical structure may be in communication with the one-way duckbill valve 502. Additionally, a splash guard is arranged at a position corresponding to the milk pouring port, and the bottom of the milk bowl includes a fitting groove that can engage with the fitting protrusion on the splash guard.

In some examples, the expressing assembly may include a plurality of massage mechanisms 140 that may be evenly distributed on the breast shield 10. Each massage mechanism 140 may include a plurality of stimulation portion, so that the massage mechanism 140 can effectively perform electrical stimulation and massage on the breast. The electrical stimulation and massage are performed on the breast to stimulate tissue cells of the breast, induce contraction and relaxation in related tissues of the breast, regulate flowing of bodily fluids such as blood and breast milk, stimulate internal neural regulation of the breast, improve an overall status of the breast, promote secretion and flowing of breast milk, and improve functionality of internal tissues of the breast.

It should be noted that the breast pump body provided in this embodiment of this specification may further include other components, such as a diaphragm and a diaphragm support. To facilitate the description of the technical solutions in this specification and to highlight innovative aspects, components of the breast pump body that may be implemented by the related art are omitted in this specification.

For example, in some implementations, as shown in FIG. 59, the expressing assembly may further include a display, which may be arranged on the breast pump body and is configured to display a current expressed breast milk volume.

For example, in some examples, as shown in FIG. 59 and FIG. 60, the expressing assembly may further include a front housing 84 and a rear housing 86. The negative pressure mechanism may be at least partially arranged in the front housing 84. The breast shield 10 and the milk storage container portion 50 may be respectively connected to the front housing 84 and the rear housing 86, to form an integrated wearable unit. The wearable unit may be, or at least partially may be worn within a shoulder strap.

In some examples, in a breast milk flowing direction, the expressing assembly may further include a protruding hole 85 located on the front housing 84.

In some examples, the rear housing 86 may further include components such as a battery, a negative pressure air passage, a circuit board, and a solenoid valve therein.

For another example, in some examples, as shown in FIG. 59 and FIG. 60, the expressing assembly may further include a key 88 arranged on the rear housing 86 and a latch structure 89 that engages with the key 88. When the key 88 is triggered, the latch structure 89 can be unlocked.

In some implementations, the breast pump may be a wearable breast pump. Designing the breast pump as a wearable breast pump facilitates use anytime and anywhere, offering comfortable experience. Moreover, a wearable breast pump provides various advantages such as efficient milk expressing, intelligent control, and fashionable portability, offering the user a more convenient and comfortable option.

### Eleventh embodiment

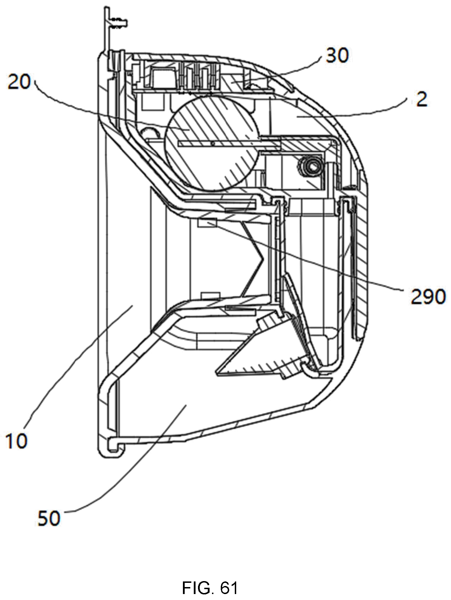

As shown in FIG. 61 to FIG. 64, the present disclosure provides a breast pump. The breast pump includes a milk storage container portion 50, a main unit 2, a breast shield 10, and a power source 297. The milk storage container portion 50 holds milk, and is shaped like a bowl. The main unit has a negative pressure mechanism 20 and a control unit 30 therein. The negative pressure mechanism 20 forms negative pressure, so that milk can be expressed from a human breast. The control unit 30 is configured to control the electronic components to operate. The breast shield 10 covers the human breast and is attached to the breast. In this embodiment, the milk storage container portion 50, the main unit 2, and the breast shield 10 are integrally assembled. The main unit 2 is detachably connected to the milk storage container portion 50, and the breast shield 10 is also detachably connected to the milk storage container portion 50. In other embodiments of the present disclosure, the milk storage container portion, the main unit, and the breast shield may be separate structures. The main unit is connected to the milk storage container portion through a tube, and the breast shield communicates with the milk storage container portion.

During use of the breast pump of the present disclosure, the breast shield 10 is firmly placed over and snugly attached to the human breast, and then the negative pressure mechanism 20 is started. The negative pressure mechanism 20 forms negative pressure, to pump milk from the breast, which flows into the milk storage container portion through a milk expressing channel. To ensure normal operation of the breast pump, the present disclosure is further provided with a detection unit (290, 291, 294) configured to detect whether the breast is attached to or meet an attachment condition with the breast shield 10. The detection unit is connected to the control unit, and generates a first signal and transmits the first signal to the control unit when the breast is not attached to or does not meet the attachment condition with the breast shield. In this embodiment of the present disclosure, the detection unit may be a capacitive sensor 290 configured to detect a change in a capacitance of the breast, a photoelectric sensor 291 configured to detect a change in a distance between the breast and the breast shield, or a pressure sensor 294 configured to detect a change in a pressure between the breast and the breast shield. When the data detected by the capacitive sensor, the photoelectric sensor, or the pressure sensor indicates that the breast shield is not attached to the breast or falls within a data range indicating that the breast is sealed, it is considered that the attachment condition is not met.

In this embodiment of the present disclosure, the control unit 30 is provided with a timing unit 296. When the breast is not fully attached to the breast shield, the detection unit sends a first signal. In this case, the control unit receives the first signal, and sends prompt information. Moreover, the timing unit starts counting, and feeds back a second signal to the control unit. The control unit may adjust an operating status of the negative pressure mechanism or turn off the breast pump based on the feedback from the timing unit. Specifically, when the second signal exceeds a preset duration threshold, the control unit turns off the breast pump, and turns off the power source 297 of the breast pump. When the second signal does not exceed the preset duration threshold, the control unit restarts the negative pressure mechanism, and resumes the task of the negative pressure mechanism existing before the first signal is generated, thereby restoring the breast pump to a normal operating state. In a specific implementation, the preset duration threshold is set to three seconds to five seconds. If the second signal exceeds three seconds to five seconds, the breast pump is turned off in a timely manner, to prevent milk from flowing into the main unit. If the second signal does not exceed three seconds to five seconds, it may be considered that an accidental misoperation is made by a person. In this case, the negative pressure mechanism needs to be restored to operation in a timely manner. For the preset duration threshold, adjustments may be made. Generally, in case of accidental misoperation, a person can react within three seconds to five seconds.

In this embodiment of this application, the negative pressure mechanism features silent operation and high reliability to avoid disturbing a mother and a baby during use. The breast shield is made of soft, non-toxic, and eco-friendly materials to provide a comfortable feel and ensure that no chemical reactions occur when breast milk flows through the breast shield.

In this embodiment of this application, the breast pump is further provided with an alarm device. When detecting that the breast is not attached to the breast shield, the control unit may trigger the alarm device to send prompt signals such as alarm sounds or flashing lights. These prompt signals may prompt the user to adjust the usage status of the breast pump in a timely manner, to prevent the situation that the breast is not attached to the breast shield during operation of the negative pressure mechanism. In addition, the design of the alarm device improves user experience and safety.

In this embodiment of this application, the breast pump is further provided with an intelligent adjustment device, which can automatically adjust a position and a shape of the breast shield based on a breast shape and a breast size of the user, achieving better attachment to the breast. In addition, the device can automatically adjust an operating status and an expressing intensity of the negative pressure mechanism based on an expressing need and an expressing duration of the user, to provide more comfortable and safer expressing experience.

In this embodiment of this application, the breast pump is further provided with a massage function that stimulates acupoints and lymphatic systems around the breast, to promote blood circulation and unclog breast ducts, thereby increasing both a volume and quality of breast milk. The breast pump may be further provided with a heating function, to heat the breast shield and the breast, so that the breast becomes softer to prevent milk duct blockage. These auxiliary functions can further enhance usage comfort and functionality of the breast pump.

As shown in FIG. 61, in the present disclosure, the capacitive sensor 290 is configured to detect a change in a capacitance of the breast, to determine an attachment status between the breast and the breast shield by detecting the capacitance change, thereby controlling the operating status of the breast pump. The capacitive sensor includes at least one pair of electrodes positioned on the breast shield. When the breast is snugly attached to the breast shield, the capacitive sensor can acquire a capacitance. When the breast is not attached to the breast shield, meaning that at least one electrode cannot contact the breast, the capacitance acquired by the sensor is zero or close to zero. This case, the capacitive sensor generates a first signal and transmits the first signal to the control unit. If the capacitance changes detected by the capacitive sensor vary gradually as the breast milk volume decreases, it is considered that the breast is attached to the breast shield, and the capacitive sensor generates no first signal.

In this embodiment, the capacitive sensor may include at least one fixed sensor permanently mounted inside the breast shield. When the breast is attached to the breast shield, the fixed sensor forms a capacitance with the breast. The attachment status between the breast and the breast shield may be determined through measurement of a value of the capacitance.

Based on the above embodiment, one or more mobile sensors may be further added to improve accuracy of the attachment detection. The plurality of mobile sensors can better cover various parts of the breast. These mobile sensors can move relative to the breast shield, to more accurately detecting the attachment status between the breast and the breast shield.

The mobile sensors can adapt to breasts of different sizes. Since a breast size and a breast shape vary from person to person, the plurality of mobile sensors can better meet needs of different users. Through adjustment of the positions of the mobile sensors, more personalized user experience can be provided, and the accuracy of the attachment detection can be improved.

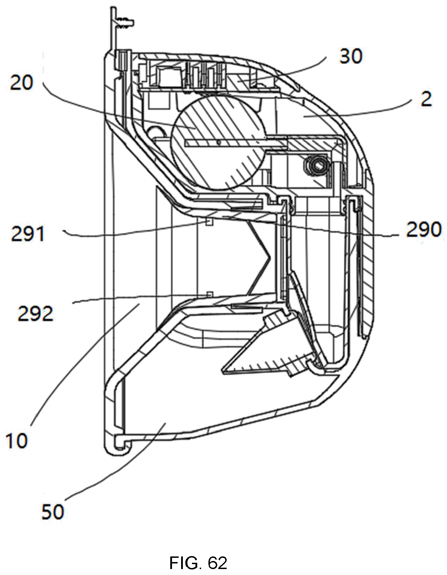

As shown in FIG. 62, in the present disclosure, the detection unit is a photoelectric sensor 291, which may be an infrared sensor or an ultrasonic sensor.

The infrared sensor may include at least one transmitter sensor 291 and at least one receiver sensor 292, which are positioned opposite each other. In this embodiment, the infrared sensor measures a change in a distance between the breast and the breast shield, to determine the attachment status between the breast and the breast shield. For example, when the detected distance change exceeds a specific threshold, for example, when the detected distance change exceeds five millimeters, it may be considered that the breast is not attached to the breast shield. In this case, the infrared sensor sends a first signal to the control unit, and the control unit triggers the breast pump to send a prompt alarm. When the detected distance change does not exceed five millimeters, it may be considered that the breast shield has only undergone a minor adjustment, and therefore there is no need to send the first signal. A specific distance threshold may be adjusted based on actual needs. For example, the specific distance threshold may be adjusted based on factors such as an individual condition of the user and a usage environment. If the user has large breasts, the threshold needs to be appropriately increased to avoid erroneous determination as non-attachment.

In this embodiment, the ultrasonic sensor may include a transmitter and a receiver. The transmitter is configured to receive emit ultrasonic waves, and the receiver is configured to receive reflected ultrasonic waves. The ultrasonic sensor may measure a change in a distance between the transmitter and the receiver, to determine a change in a distance between the breast and the breast shield.

Based on the above embodiments, the infrared sensor may be further provided with one or more compensation sensors, which are configured to receive light rays from a transmitter sensor of the infrared sensor and compensate for detection errors of the infrared sensor. When the breast is not attached to the breast shield, a value of the compensation sensor changes. The change in the value may be measured to compensate for and correct detection results of the infrared sensor.

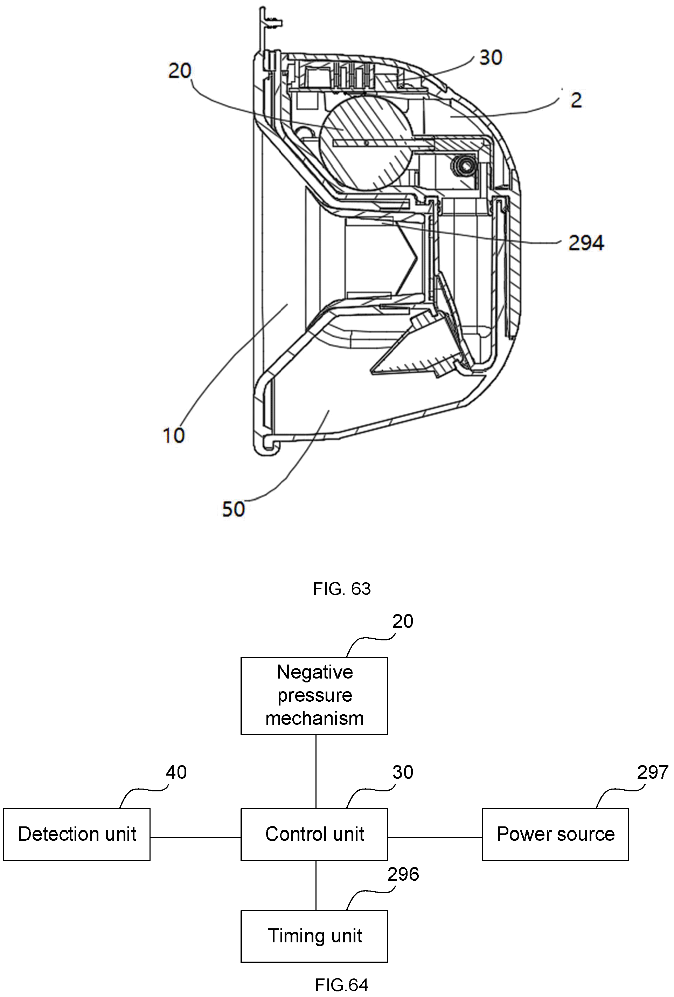

As shown in FIG. 63, in the present disclosure, the detection unit is a pressure sensor 294, which is configure to detect a change in a pressure between the breast and the breast shield. The pressure sensor 294 is arranged on the breast shield and includes at least two contact terminals configured contact the human breast. These contact terminals may be evenly spaced and arranged on a surface of the breast shield or between the breast shield and the flange, forming one or more pressure sensor arrays. When the breast is attached to the breast shield, these contact strips contact the breast surface and sense a pressure from the breast. Values of the pressure sensed by these contact strips may be measured to determine whether the breast is attached to the breast shield. When the breast is snugly attached to the breast shield, the pressure sensor can acquire a value of the pressure between the breast and the breast shield. When the breast is not attached to the breast shield, meaning that the contact terminals cannot contact the breast, and the contact terminals cannot sense the pressure from the breast. In this case, the value of the pressure between the breast and the breast shield is zero or close to zero. The pressure sensor generates a first signal and transmits the first signal to the control unit. The control unit controls the breast pump to send an alarm and controls the negative pressure mechanism to stop operating.

Furthermore, a plurality of additional auxiliary contact strips may be arranged as needed to enhance adaptation to different breast shapes and breast positions. These auxiliary contact strips may be arranged at other positions on the breast shield, for example, at a bottom, to provide more pressure measurement points. Increasing a number and a distribution range of contact strips can improve detection accuracy and reliability of the pressure sensor.

In an embodiment, the breast pump further includes a communication port configured to establish communication with a computer and/or mobile device. The communication port transmits various signals to the computer or the mobile device to perform corresponding operations. For example, the communication port sends prompt or alarm information, to which the computer and/or the mobile device responds by prompting the user to adjust the position of the breast shield in a timely manner.

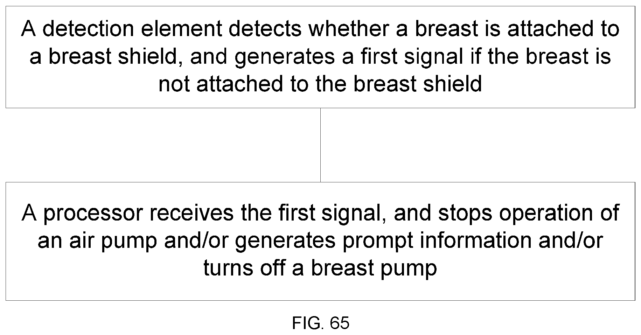

As shown in FIG. 65, an embodiment of this application further provides a method for controlling a breast pump to operate, including: acquiring a control parameter; and performing a preset operation based on the control parameter. Specific steps include following:

The detection unit detects whether the breast is attached to the breast shield, and generates a first signal and sends the first signal to the control unit when detecting non-attachment. The control unit acquires a first signal parameter. In some implementations, during milk expressing by the user by using the breast pump, the breast is attached to the breast shield. However, the user may not remain completely still while wearing the breast pump. For instance, the user may walk or move, which may cause the breast and the breast shield to not be fully attached, leaving gaps. This may lead to milk leakage into the main unit and, in severe cases, damage the breast pump. Therefore, this application incorporates a detection unit to detect whether the breast is attached to the breast shield. In some implementations, a specific form of the first signal is determined based on a specific type of the detection unit, which is not limited in this application. Exemplarily, the first signal may be a current signal or a voltage signal. Furthermore, when the first signal is a voltage signal, the first signal may be a high-level signal. When the detection unit detects that the breast is attached to the breast shield, the detection unit may send a first complementary signal to the control unit. The first complementary signal may be a low-level signal. When receiving the first signal, that is, a high-level signal, the control unit interprets this as an indication that the breast is not attached to the breast shield. Conversely, when receiving the first complementary signal, that is, a low-level signal, the control unit interprets this as an indication that the breast is attached to the breast shield.

The control unit receives the first signal, and stops the operation of the negative pressure mechanism and/or generates prompt information and/or turns off the breast pump. This is equivalent to that the control unit performs the preset operation based on the first signal parameter.

In some implementations, if the control unit receives the first signal, it indicates that the breast is not attached to the breast shield. In this case, continuing the milk expressing operation may cause damage to the breast pump. Therefore, the control unit stops the operation of the negative pressure mechanism and/or generates the prompt information and/or turns off the breast pump.

In some implementations, the prompt information may be voice information, for example, "Please attach to the breast pump".

In some implementations, the breast pump is provided with an alarm unit, for example, a buzzer, and the prompt information may be generated through activation of the alarm unit.

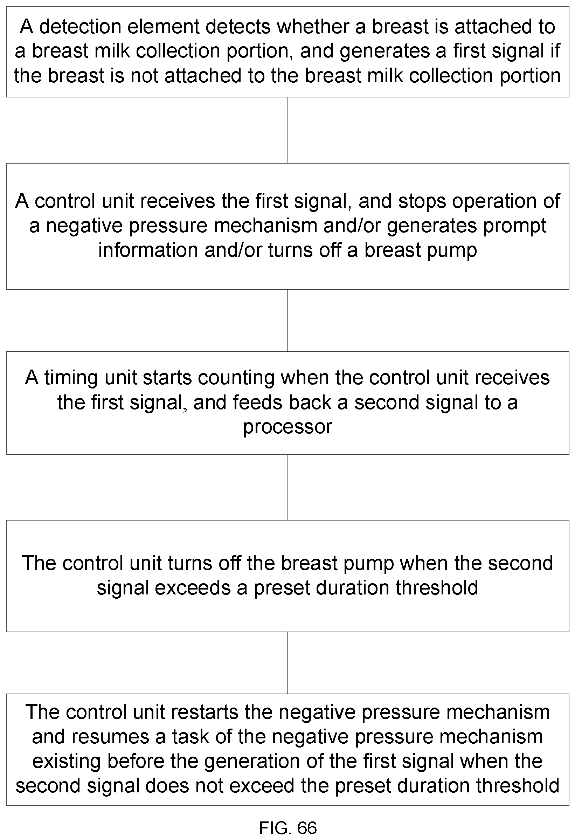

As shown in FIG. 66, in some implementations, the method for controlling a breast pump to operate provided in this embodiment of this application further includes following:

The detection unit detects whether the breast is attached to the breast shield, and generates a first signal when detecting non-attachment.

The control unit receives the first signal, and stops the operation of the negative pressure mechanism and/or generates prompt information and/or turns off the breast pump.

A timing unit starts counting when the control unit receives the first signal and feeds back a second signal to the control unit.

In some implementations, the second signal is used to indicate a recorded duration of the timing unit.

In some implementations, when the detection unit sends the first complementary signal to the control unit, the timing unit stops counting and stops sending the second signal to the control unit.

The control unit turns off the breast pump when the second signal exceeds a preset duration threshold.

In some implementations, when the second signal exceeds the preset duration threshold, it indicates that the breast has not been attached to the breast pump for a long time. This may indicate that the user has finished milk expressing and will not resume shortly, or that the user has forgotten to turn off the device despite not using the breast pump. Therefore, the breast pump is directly turned off.

The control unit restarts the negative pressure mechanism and resumes a task of the negative pressure mechanism existing before the generation of the first signal when the second signal does not exceed the preset duration threshold.

In some implementations, when the second signal does not exceed the preset duration threshold, it indicates that the breast is re-attached to the breast shield, indicating that the user intends to resume milk expressing. In this case, the control unit restarts the negative pressure mechanism and resumes the task of the negative pressure mechanism existing before the generation of the first signal. This can enhance intelligence of the breast pump, and prevent cases where the user needs to initiate a new milk expressing operation due to an unexpected interruption during the milk expressing.

In some implementations, the capacitive sensor detects a change in a capacitance of the breast, and generates the first signal when the capacitance is zero or close to zero, or otherwise, generates no first signal.

In some implementations, since the human body is conductive and air is insulating, whether the breast is attached to the breast shield can be detected by a capacitive sensor. For details, reference may be made to the relevant description in the previous sections of this specification. The details are not described herein.

In some implementations, the photoelectric sensor detects a change in the distance between the breast and the breast shield, and generates a first signal when the distance change exceeds a preset distance threshold, or otherwise, generates no first signal.

In some implementations, to prevent interruptions in milk expressing due to accidental shaking, the first signal is generated only when the distance change exceeds the preset threshold for a predetermined duration. Otherwise, no first signal is generated.

In some implementations, the pressure sensor detects a change in the pressure between the breast and the breast shield, and generates the first signal when a contact terminal senses a pressure of zero or close to zero, or otherwise, generates no first signal. In the present disclosure, determining whether the breast is attached to the breast shield by detecting the pressure change between the breast and the breast shield achieves effective detection at low costs.

In some implementations, when the breast is attached to the breast shield, the pressure sensor is arranged in a direction of gravity of the breast to detect q partial gravity of the breast. When the breast is attached to the breast shield, the pressure sensor can detect a partial weight, and a measured pressure is relatively large. Conversely, when the breast is not attached to the breast shield, the pressure sensor cannot detect the gravity of the breast, and a measured pressure is zero or close to zero.

### Twelfth embodiment

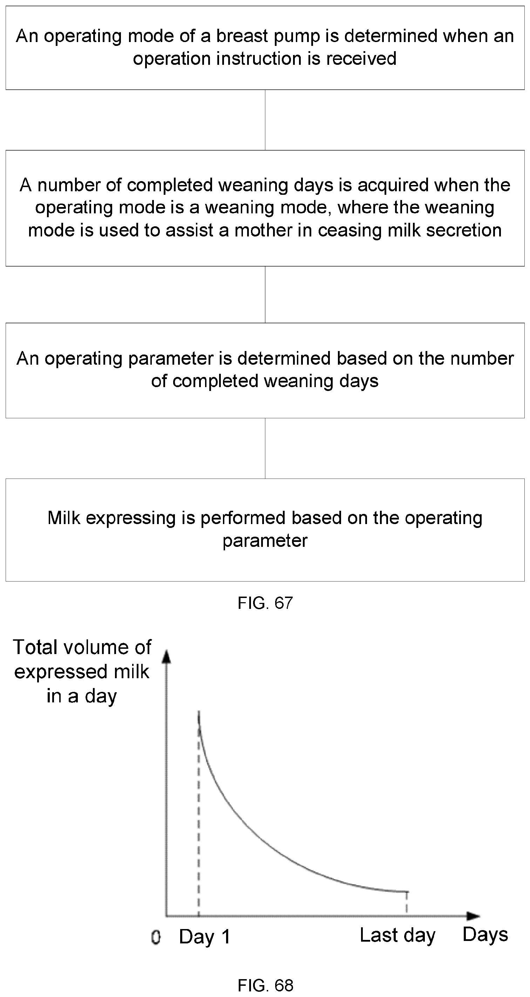

Referring to FIG. 67, FIG. 67 is a schematic flowchart of a control method according to this application. The control method is applied to a breast pump. As shown in FIG. 67, the control method includes a first step and a fourth step.

First step: An operating mode of the breast pump is determined when an operation instruction is received.

In some implementations, the breast pump is provided with a start key, such as a "Start" key or an "ON" key. When the key is triggered, the breast pump receives the operation instruction and starts to perform milk expressing.

In some implementations, the breast pump includes a communication unit, through which the breast pump can communicate with an external terminal. The external terminal may send an operation instruction to the breast pump through the communication unit.

In some implementations, the operating mode of the breast pump includes a normal mode and a weaning mode.

In some implementations, in the normal mode, the breast pump operates with a fixed configuration for milk expressing. In the weaning mode, the breast pump performs milk expressing based on a current weaning progress.

Second step: A number of completed weaning days is acquired when the operating mode is a weaning mode, where the weaning mode is used to assist a mother in ceasing milk secretion.

In some implementations, the weaning mode has following characteristics: lasting for consecutive days, and a daily total volume of expressed breast milk overall exhibiting a declining trend as the days progress.

Referring to FIG. 68, FIG. 68 is a schematic diagram of a relationship between a number of days in a weaning mode and a daily total volume of expressed breast milk according to an embodiment of this application. As shown in FIG. 68, on day 1, a volume of expressed breast milk is largest, while on a final day, a volume of expressed breast milk reaches a lowest level and approaches zero. As the days progress, the daily total volume of expressed breast milk overall exhibits a declining trend.

It may be understood that the relationship between the number of days in the weaning mode and the daily total volume of expressed breast milk shown in FIG. 68, is for illustrative purposes only and does not represent a sole implementation.

In some implementations, the control method provided in this embodiment of this application further includes following steps.
(1). A switching instruction is received.
(2). Switching to the weaning mode is performed based on the switching instruction.

Specifically, if the breast pump is not in the weaning mode, the user may send a switching instruction by triggering a triggering unit on the breast pump or through a terminal, to switch the breast pump from another mode to the weaning mode.

In some implementations, the triggering unit may be a physical unit, such as a switch, a knob, or a key.

In some implementations, the control method provided in this embodiment of this application further includes following steps.
(1). A weaning progress is recorded when the weaning mode is ended, where the weaning mode includes a number of completed weaning days and a number of completed milk expressing operations in a day.
(2). Switching to the weaning mode is performed to operate based on the recorded weaning progress when the switching instruction is received.
Third step: An operating parameter is determined based on the number of completed weaning days.

In some implementations, the operating parameter includes a total number of completed milk expressing operations and a volume of breast milk obtained in a single expressing operation in the day.

In some implementations, the third step includes: determining a total number of completed milk expressing operations and/or a volume of breast milk obtained in a single expressing operation in a day based on the number of completed weaning days.

When the total number of completed milk expressing operations is a preset value, the volume of breast milk obtained in a single expressing operation may be determined based on the number of completed weaning days. When the volume of breast milk obtained in a single expressing operation is a preset value, the total number of completed milk expressing operations in the day may be determined based on the number of completed weaning days.

In some implementations, the total number of completed milk expressing operations in the day refers to a total number of times the breast pump needs to be used in the day, and the volume of breast milk obtained in a single expressing operation refers to a volume of breast milk that needs to be expressed each time the breast pump is used.

In some implementations, the volume of breast milk obtained in a single expressing operation may be replaced by a duration of a single milk expressing operation or other parameters that can affect the volume of breast milk obtained in a single expressing operation.

Fourth step: Milk expressing is performed based on the operating parameter.

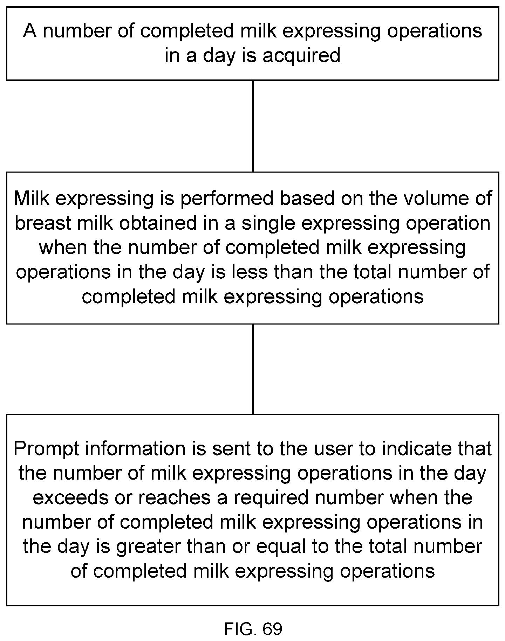

In some implementations, referring to FIG. 69 again, FIG. 69 is a detailed schematic flowchart of the fourth step. The fourth step includes following substeps: a first substep to a second substep.

First substep: A number of completed milk expressing operations in the day is acquired.

In some implementations, in the weaning mode, each time the breast pump completes a milk expressing operation reaching a required breast milk volume, the recorded number of completed milk expressing operations in the day is increased by one.

In some implementations, the breast pump includes a milk storage container portion configured to store the breast milk expressed by the breast pump. A plurality of liquid level sensors are arranged at fixed intervals in the milk storage container portion to measure a liquid level of the breast milk stored in the milk storage container portion. The liquid level of the breast milk stored in the milk storage container portion can indicate a volume of the breast milk stored in the milk storage container portion. Therefore, through sensor data from the liquid level sensors, a breast milk volume expressed during each milk expressing operation can be determined.

In some implementations, in the weaning mode, each time the breast pump completes a milk expressing operation not reaching a required breast milk volume, the breast pump determines a difference between the current milk expressing volume and the required volume, and stops, when a milk expressing volume of a next milk expressing operation reaches the difference, the milk expressing operation and increases the recorded number of completed milk expressing operations in the day by one.

In some implementations, the breast pump synchronizes the number of completed milk expressing operations in the day to a terminal and/or a server.

Second substep: Milk expressing is performed based on the volume of breast milk obtained in a single expressing operation when the number of completed milk expressing operations in the day is less than the total number of completed milk expressing operations.

In some implementations, performing milk expressing based on the volume of breast milk obtained in a single expressing operation means that the breast pump actively stops operating after a breast milk volume expressed by the breast pump reaches the volume of breast milk obtained in a single expressing operation.

In some implementations, the fourth step further includes a third substep. Third substep: Prompt information is sent to the user to indicate that the number of milk expressing operations in the day exceeds or reaches a required number when the number of completed milk expressing operations in the day is greater than or equal to the total number of completed milk expressing operations.

In some implementations, "exceeding the required number" means that the number of milk expressing operations completed by the user exceeds the total number of completed milk expressing operations, and "reaching the required number" means that the number of milk expressing operations completed by the user equals the total number of completed milk expressing operations.

Sending the prompt information to the user when the number of completed milk expressing operations in the day is greater than or equal to the total number of completed milk expressing operations can prevent the milk expressing volume of the user in the day from exceeding the required volume. This avoids problems such as incomplete execution of the weaning plan and reduced effectiveness of the weaning process. Furthermore, sending the prompt information can reduce situations where mothers struggle to persist with weaning due to factors such as baby's crying.

In some implementations, the breast pump includes a voice broadcast assembly, and the third substep includes following steps.
(1). Voice prompt information is sent to the user through the voice broadcast assembly, to indicate whether the number of milk expressing operations in the day exceeds or reaches the required number.
(2). After the voice prompt information is sent, milk expressing is performed based on the volume of breast milk obtained in a single expressing operation if another operation instruction is received, or the breast pump is caused to enter a standby mode if a stop instruction is received.

In some implementations, the voice prompt information may be "Today's expressing task is complete" or "No further expressing is required".

In some implementations, the user may trigger generation of an operation instruction by pressing a start key of the breast pump, for example, the above "Start" key or "ON" key.

In some implementations, the user may trigger generation of a stop instruction by touching and holding the start key of the breast pump, for example, touching and holding the start key of the breast pump for more than 2s.

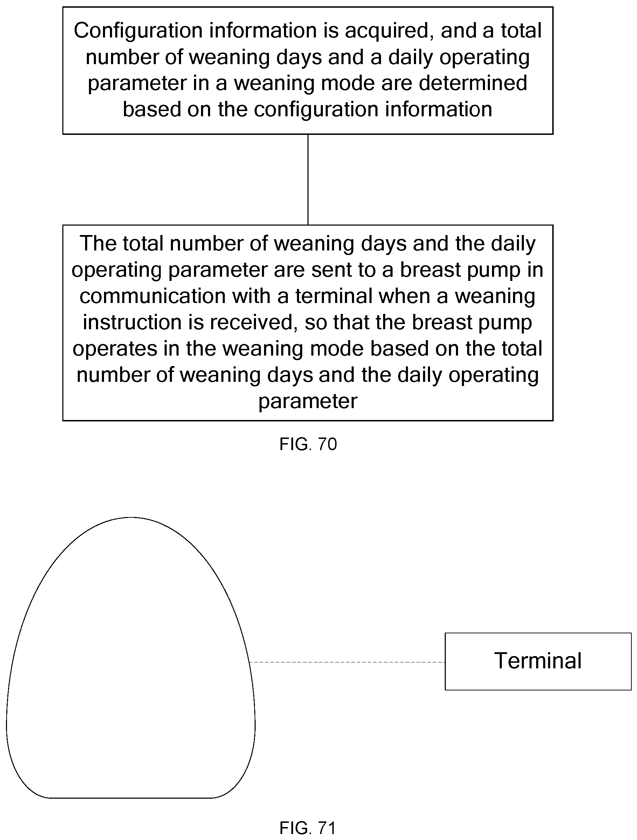

Referring to FIG. 70, FIG. 70 is a flowchart of a control method according to an embodiment of this application. The control method is applied to a terminal, and includes a fifth step to a sixth step.

Fifth step: Configuration information is acquired, and a total number of weaning days and a daily operating parameter in the weaning mode are determined based on the configuration information.

In some implementations, the user may input configuration information by operating the terminal, for example, by operating a mobile APP, computer software, and/or a website.

In some implementations, the terminal may acquire the configuration information from a server.

In some implementations, the terminal may communicate with two breast pumps simultaneously. In this case, the configuration information includes device identifiers of the breast pumps. The terminal then sends the total number of weaning days and the daily operating parameter to the breast pumps corresponding to the device identifiers in the configuration information.

In some implementations, the device identifier is a media access control (MAC) address, a factory serial number, or the like of the breast pump.

Sixth step: The total number of weaning days and the daily operating parameter are sent to the breast pump in communication with the terminal when a weaning indication is received, so that the breast pump operates in the weaning mode based on the total number of weaning days and the daily operating parameter.

In some implementations, the operating parameter includes a daily volume of breast milk obtained in a single expressing operation, which decreases as the number of completed weaning days increases.

Refer to Table 1 below. In Table 1, a total number of weaning days is 30, and as the number of weaning days increases in Table 1, a volume of breast milk obtained in a single expressing operation gradually decreases.

**(Table 1)**

| Weaning day | Total volume of expressed breast milk | Total number of completed milk expressing operations | Volume of breast milk obtained in a single expressing operation |
|---|---|---|---|
| Day 1 | 1000 ml | 5 | 200 ml |
| ... | ... | ... | ... |
| Day 15 | 600 ml | 5 | 120 ml |
| ... | ... | ... | ... |
| Day 30 | 400 ml | 5 | 80 ml |

In some implementations, the operating parameter includes a daily total number of completed milk expressing operations, which decreases as the number of completed weaning days increases.

Refer to Table 2 below. In Table 2, a total number of weaning days is 30, and as the number of weaning days increases in Table 2, a total number of completed milk expressing operations gradually decreases.

**(Table 2)**

| Weaning day | Total volume of expressed breast milk | Total number of completed milk expressing operations | Volume of breast milk obtained in a single expressing operation |
|---|---|---|---|
| Day 1 | 800 ml | 5 | 160 ml |
| ... | ... | ... | ... |
| Day 15 | 640 ml | 4 | 160 ml |
| ... | ... | ... | ... |
| Day 30 | 480 ml | 3 | 160 ml |

In some implementations, the daily total number of completed milk expressing operations and the daily volume of breast milk obtained in a single expressing operation are inversely proportional to a current number of weaning days.

Refer to Table 3 below. In Table 3, a total number of weaning days is 30, and as the number of weaning days increases in Table 3, both a total number of completed milk expressing operations and a daily volume of breast milk obtained in a single expressing operation gradually decrease.

**(Table 3)**

| Weaning day | Total volume of expressed breast milk | Total number of completed milk expressing operations | Volume of breast milk obtained in a single expressing operation |
|---|---|---|---|
| Day 1 | 1000 ml | 5 | 200 ml |
| ... | ... | ... | ... |
| Day 15 | 640 ml | 4 | 160 ml |
| ... | ... | ... | ... |
| Day 30 | 420 ml | 3 | 140 ml |

In some implementations, the step of acquiring configuration information includes following steps.
(1). Preconfiguration information is acquired.
(2). When the preconfiguration information is a total number of weaning days, historical milk expressing data is acquired, and an average daily milk expressing volume is determined based on the historical breast milk expressing data.
(3). A preset minimum breast milk expressing volume and a preset number of milk expressing operations are acquired.
(4). A daily total breast milk expressing volume is determined based on the total number of weaning days, the average volume of expressed milk, and the minimum breast milk expressing volume, where the daily total volume of expressed breast milk decreases as the number of completed weaning days increases.
(5). A daily volume of breast milk obtained in a single expressing operation is determined based on the daily total volume of expressed breast milk and the preset number of milk expressing operations in the weaning mode.
(6). The configuration information is determined based on the total number of weaning days, the daily total volume of expressed breast milk, and the daily volume of breast milk obtained in a single expressing operation.

In some implementations, the preset minimum breast milk expressing volume and the preset number of milk expressing operations may be set by the user, or may be default values stored in the breast pump. The preset minimum breast milk expressing volume refers to a total volume of breast milk that needs to be expressed by the breast pump on a final day of the weaning plan. The preset number of milk expressing operations refers to a number of times the breast pump performs milk expressing each day during execution of the weaning plan by the breast pump.

In some implementations, the daily total breast milk expressing volume uniformly decreases during the execution of the weaning plan by the breast pump.

In some implementations, the preset minimum breast milk expressing volume is 500 mL, and the preset number of milk expressing operations is three.

In some implementations, the preset number of milk expressing operations is a constant value, meaning that the number of times the breast pump performs milk expressing each day during the execution of the weaning plan is constant.

In some implementations, the preset number of milk expressing operations is not a constant value, but instead, varies with the number of days the breast pump executes the weaning plan. For example, the preset number of milk expressing operations may be set to 6 on day 1, 5 on day 15, and 4 on the final day.

In this way, the user only needs to input a weaning time. The breast pump can then automatically generate a weaning plan by acquiring historical milk expressing data, thereby enhancing intelligence of the breast pump.

In some implementations, the step of acquiring configuration information includes following steps.
(1). Preconfiguration information is acquired.
(2). A preset total number of weaning days and a preset daily operating parameter are acquired when the preconfiguration information is preset information.
(3). The configuration information is determined based on the preset total number of weaning days and the preset daily operating parameter.

In some implementations, the preconfiguration information is preconfigured at the factory and may also be flexibly modified by the user.

In this way, the user can be provided with a well-designed preset weaning plan, improving user experience. In addition, the user can flexibly customize a weaning plan to meet personalized needs.

In some implementations, the preset information includes first preset information and second preset information. The preset daily operating parameter includes a daily total volume of expressed breast milk. The step of acquiring a preset total number of weaning days and a preset daily operating parameter when the preconfiguration information is preset information includes following substeps.
(1). A first preset total number of weaning days and a preset daily operating parameter are acquired when the preconfiguration information is first preset information, or a second preset total number of weaning days and a preset daily operating parameter are acquired when the preset information is second preset information.
(2). The first preset total number of weaning days is greater than the second preset total number of weaning days, a total volume of breast milk expressed on day 1 corresponding to the first preset information is equal to a total volume of breast milk expressed on day 1 corresponding to the second preset information, and a total volume of breast milk expressed on a final day corresponding to the first preset information is equal to a total volume of breast milk expressed on the final day corresponding to the second preset information.

In some implementations, the first preset information corresponds to a first weaning plan, and the second preset information corresponds to a second weaning plan. Referring to Table 4 and Table 5 below, Table 4 shows an example of the first weaning plan, and Table 5 shows an example of the second weaning plan.

**(Table 4)**

| Weaning day | Total volume of expressed breast milk | Total number of completed milk expressing operations | Volume of breast milk obtained in a single expressing operation |
|---|---|---|---|
| Day 1 | 1000 ml | 5 | 200 ml |
| ... | ... | ... | ... |
| Day 15 | 640 ml | 4 | 160 ml |
| ... | ... | ... | ... |
| Day 30 | 420 ml | 3 | 140 ml |
| ... | ... | ... | ... |
| Day 60 | 300 ml | 3 | 100 ml |

**(Table 5)**

| Weaning day | Total volume of expressed breast milk | Total number of completed milk expressing operations | Volume of breast milk obtained in a single expressing operation |
|---|---|---|---|
| Day 1 | 1000 ml | 5 | 200 ml |
| ... | ... | ... | ... |
| Day 15 | 420 ml | 3 | 140 ml |
| ... | ... | ... | ... |
| Day 30 | 300 ml | 3 | 100 ml |

In some implementations, more milk expressing modes may be set for selection by the user. For example, the total number of completed milk expressing operations is reduced while leaving the volume of expressed breast milk and the volume of breast milk obtained in a single expressing operation unchanged.

In this way, various milk expressing modes can be provided for selection by users, meeting milk expressing needs of different users.

Referring then to FIG. 71, FIG. 71 is a schematic structural diagram of a system according to an embodiment of this application. As shown in FIG. 71, the system includes the breast pump and the terminal described above. The breast pump communicates with the terminal.

In some implementations, the terminal is configured to: acquire configuration information, determine a total number of weaning days and a daily operating parameter of a weaning mode based on the configuration information, and send the total number of weaning days and the daily operating parameter to the breast pump in communication with the terminal when receiving a weaning indication.

In some implementations, the breast pump is configured to: operate in the weaning mode based on the total number of weaning days after receiving the total number of weaning days; determine an operating mode of the breast pump when receiving an operation instruction; acquire a number of completed weaning days when the operating mode is a weaning mode, where the weaning mode is used to assist a mother in ceasing milk secretion; determine an operating parameter based on the number of completed weaning days; and perform milk expressing based on the operating parameter.

### Thirteenth embodiment

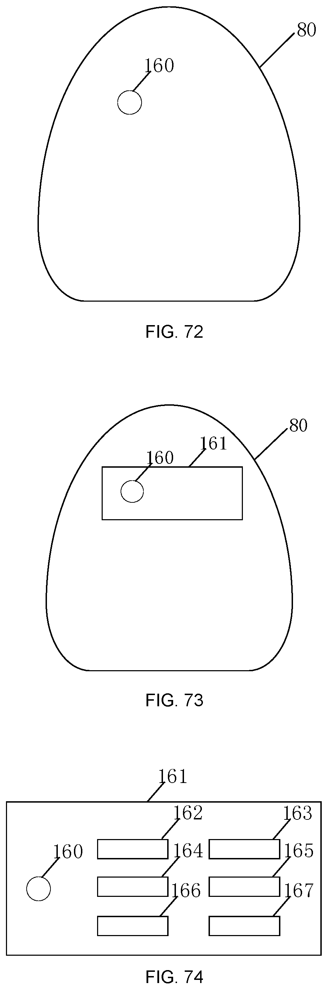

Referring to FIG. 72, as shown in FIG.72, a breast pump includes a housing 80 and a triggering unit 160. The triggering unit 160 is configured to control the breast pump to operate in a weaning mode, where the weaning mode is used to assist a mother in ceasing milk secretion.

In some implementations, the triggering unit is a physical key, a toggle, or a knob mounted on the housing.

Referring to FIG. 73, in some implementations, the triggering unit is a touch key, and the housing 80 includes a control interface 161, where the triggering unit 160 is arranged on the control interface 161.

In some implementations, the housing is provided with a display unit, such as a display screen. The control interface may be displayed on the display unit, allowing a user to trigger the triggering unit by touching the display unit.

Referring to FIG. 74, in some implementations, the control interface 161 includes at least one of a first display region 162, a second display region 163, or a third display region 164. The first display region 162 is configured to display a total number of weaning days of the weaning mode, the second display region 163 is configured to display a remaining number of weaning days of the weaning mode, and the third display region 164 is configured to display a current number of weaning days.

Exemplarily, the total number of weaning days is 30, the remaining number of weaning days is 29, and the current number of weaning days is 1.

In some implementations, the control interface 161 further includes at least one of a fourth display region 165 or a fifth display region 166. The fourth display region 165 is configured to display a number of milk expressing operations required in a day, and the fifth display region 166 is configured to display a number of milk expressing operations completed in the day.

Exemplarily, the number of milk expressing operations required in the day is 5, and the number of milk expressing operations completed in the day is 3.

In some implementations, the control interface 161 further includes a sixth display region (66) configured to display a volume of breast milk stored in a milk storage container portion.

In some implementations, a plurality of liquid level sensors are arranged at fixed intervals in the milk storage container portion to measure a liquid level of the breast milk stored in the milk storage container portion. The liquid level of the breast milk stored in the milk storage container portion can indicate the volume of the breast milk stored in the milk storage container portion. Therefore, through sensor data from the liquid level sensors, the volume of the breast milk stored in the milk storage container portion can be determined.

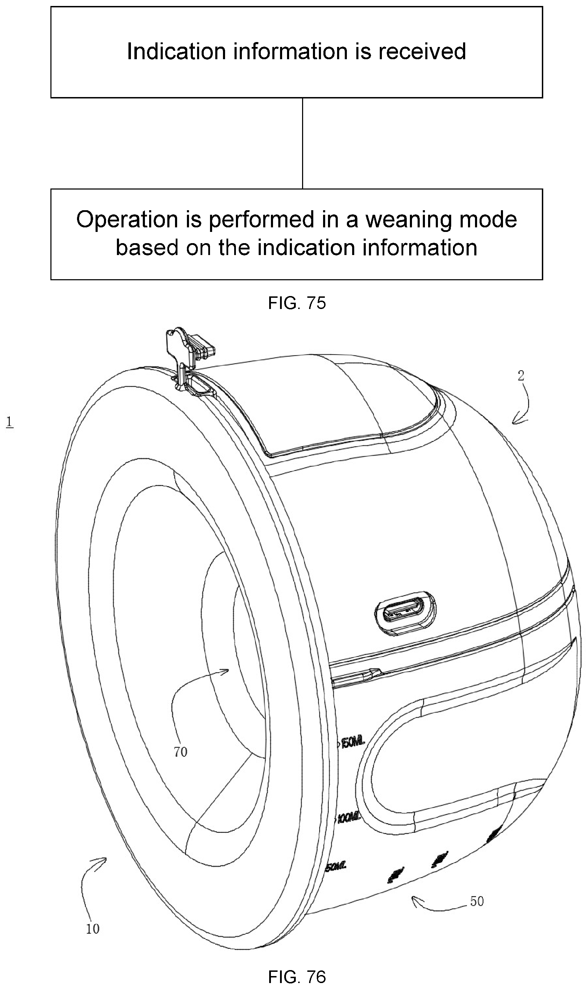

Referring to FIG. 75, FIG. 75 is a schematic flowchart of a control method according to an embodiment of this application. As shown in FIG. 75, the control method includes a first step to a second step.

First step: Weaning indication information is received.

In some implementations, generation of the weaning indication information is triggered by the triggering unit.

In some implementations, the weaning indication information is sent by a terminal in communication with the breast pump.

Second step: Operation is performed in the weaning mode based on the weaning indication information.

In some implementations, after the breast pump operates in the weaning mode, if the breast pump receives an operation instruction, the breast pump operates based on a mode parameter of the weaning mode.

Therefore, in some implementations, the control method further includes following steps:
(1). Configuration information is received.
(2). A mode parameter of the weaning mode is configured based on the configuration information, where the mode parameter includes a total number of weaning days and/or a daily operating parameter.

In some implementations, the user inputs the configuration information through a control panel on the breast pump, for receiving by the breast pump.

In some implementations, the user inputs the configuration information through the terminal, and the terminal sends the configuration information to the breast pump, for receiving by the breast pump.

In some implementations, the daily operating parameter includes at least one of a daily volume of expressed breast milk or a volume of breast milk obtained in a single expressing operation.

The daily volume of expressed breast milk refers to a total volume of breast milk that needs to be expressed by the user using the breast pump in a day, the volume of breast milk obtained in a single expressing operation refers to a breast milk volume expressed each operation by the user by using the breast pump in a day; and a daily number of completed milk expressing operations refers to a total number of times the user needs to use the breast pump in a day.

In some implementations, the volume of breast milk obtained in a single expressing operation multiplied by the daily number of completed milk expressing operations is equal to the daily volume of expressed breast milk.

The weaning mode is described below.

In some implementations, if the breast pump operates in the weaning mode, the operation of the breast pump further includes following steps (1) to (4).
(1). An operating mode of the breast pump is determined when an operation instruction is received.

In some implementations, the breast pump is provided with a start key, such as a "Start" key or an "ON" key. When the key is triggered, the breast pump receives the operation instruction and starts to perform milk expressing.

In some implementations, the breast pump includes a communication unit, through which the breast pump can communicate with an external terminal. The external terminal may send an operation instruction to the breast pump through the communication unit.

In some implementations, the operating mode of the breast pump includes a normal mode and a weaning mode.

In some implementations, in the normal mode, the breast pump operates with a fixed configuration for milk expressing. In the weaning mode, the breast pump performs milk expressing based on a current weaning progress.

(2). A current number of weaning days is acquired when the operating mode is a weaning mode, where the weaning mode is used to assist a mother in ceasing milk secretion.

In some implementations, the weaning mode has following characteristics: lasting for consecutive days, and a daily volume of expressed breast milk overall exhibiting a declining trend as the days progress.

In some implementations, on day 1, a volume of expressed breast milk is largest, while on a final day, a volume of expressed breast milk reaches a lowest level and approaches zero. As the days progress, the daily volume of expressed breast milk overall exhibits a declining trend.

(3). An operating parameter is determined based on the current number of weaning days.

In some implementations, the operating parameter includes a total number of completed milk expressing operations and a volume of breast milk obtained in a single expressing operation in the day.

In some implementations, the total number of completed milk expressing operations in the day refers to a total number of times the breast pump needs to be used in the day, and the volume of breast milk obtained in a single expressing operation refers to a volume of breast milk that needs to be expressed each time the breast pump is used.

In some implementations, the volume of breast milk obtained in a single expressing operation may be replaced by a duration of a single expressing operation or other parameters that can define the volume of breast milk obtained in a single expressing operation.

(4). Milk expressing is performed based on the operating parameter.

In some implementations, step (4) includes steps (41) to (42).

(41): A number of completed milk expressing operations in the day is acquired.

In some implementations, in the weaning mode, each time the breast pump completes a milk expressing operation reaching a required breast milk volume, the number of completed milk expressing operations in the day is increased by one.

In some implementations, the breast pump includes a milk storage container portion configured to store the breast milk expressed by the breast pump. A plurality of liquid level sensors are arranged at fixed intervals in the milk storage container portion. Through sensor data from the liquid level sensors, a breast milk volume expressed during each milk expressing operation can be determined.

In some implementations, in the weaning mode, each time the breast pump completes a milk expressing operation not reaching the required breast milk volume, the breast pump determines a difference between the current milk expressing volume and the required volume, and stops, when a milk expressing volume of a next milk expressing operation reaches the difference, the milk expressing operation and increases the number of completed milk expressing operations in the day by one.

In some implementations, the breast pump synchronizes the number of completed milk expressing operations in the day to a terminal and/or a server.

(42): Milk expressing is performed based on the volume of breast milk obtained in a single expressing operation when the number of completed milk expressing operations in the day is less than the total number of completed milk expressing operations.

In some implementations, step (4) further includes a step (43), in which prompt information is sent to the user to indicate that the number of milk expressing operations in the day exceeds or reaches a required number when the number of completed milk expressing operations in the day greater than or equal to the total number of completed milk expressing operations.

In some implementations, "exceeding the required number" means that the number of milk expressing operations completed by the user exceeds the total number of completed milk expressing operations, and "reaching the required number" means that the number of milk expressing operations completed by the user equals the total number of completed milk expressing operations.

Sending the prompt information to the user when the number of completed milk expressing operations in the day is greater than or equal to the total number of completed milk expressing operations can prevent the milk expressing volume of the user in the day from exceeding the required volume. This avoids problems such as incomplete execution of the weaning plan and reduced effectiveness of the weaning process. Furthermore, sending the prompt information can reduce situations where mothers struggle to persist with weaning due to factors such as baby's crying.

In some implementations, the breast pump includes a voice broadcast assembly, and step (43) includes following steps.

(431). Voice prompt information is sent to the user through the voice broadcast assembly, to indicate whether the number of milk expressing operations in the day exceeds or reaches the required number.

(432). After the voice prompt information is sent, milk expressing is performed based on the volume of breast milk obtained in a single expressing operation if another operation instruction is received, or the breast pump is caused to enter a standby mode if a stop instruction is received.

In some implementations, the voice prompt information may be "Today's expressing task is complete" or "No further expressing is required".

In some implementations, the user may trigger generation of an operation instruction by pressing a start key of the breast pump, for example, the above "Start" key or "ON" key.

In some implementations, the user may trigger generation of a stop instruction by touching and holding the start key of the breast pump, for example, touching and holding the start key of the breast pump for more than 2s.

In some implementations, an embodiment of this application provides a second control method. The control method is applied to a terminal, and includes following steps: step (11) to step (12).

Step (11): Configuration information is acquired, and a total number of days of the weaning mode and a daily operating parameter are determined based on the configuration information.

In some implementations, the user may input configuration information by operating the terminal, for example, by operating a mobile APP, computer software, and/or a website.

In some implementations, the terminal may acquire the configuration information from a server.

In some implementations, the terminal may communicate with two breast pumps simultaneously. In this case, the configuration information includes device identifiers of the breast pumps. The terminal then sends the total number of weaning days and the daily operating parameter to the breast pumps corresponding to the device identifiers.

In some implementations, the device identifier is a MAC address, a factory serial number, or the like of the breast pump.

(12): The total number of weaning days and the daily operating parameter are sent to the breast pump in communication with the terminal when a weaning indication is received, so that the breast pump operates in the weaning mode based on the total number of weaning days and the daily operating parameter.

In some implementations, the operating parameter includes a daily volume of expressed breast milk, which is inversely proportional to the current number of weaning days.

Refer to Table 1 below. In Table 1, a total number of weaning days is 30, and as the number of weaning days increases in Table 1, a volume of breast milk obtained in a single expressing operation gradually decreases.

**(Table 1)**

| Weaning day | Volume of expressed breast milk | Total number of completed milk expressing operations | Volume of breast milk obtained in a single expressing operation |
|---|---|---|---|
| Day 1 | 1000 ml | 5 | 200 ml |
| ... | ... | ... | ... |
| Day 15 | 600 ml | 5 | 120 ml |
| ... | ... | ... | ... |
| Day 30 | 400 ml | 5 | 80 ml |

In some implementations, the operating parameter includes a daily total number of completed milk expressing operations, which is inversely proportional to the current number of weaning days.

Refer to Table 2 below. In Table 2, a total number of weaning days is 30, and as the number of weaning days increases in Table 2, a total number of completed milk expressing operations gradually decreases.

**(Table 2)**

| Weaning day | Volume of expressed breast milk | Total number of completed milk expressing operations | Volume of breast milk obtained in a single expressing operation |
|---|---|---|---|
| Day 1 | 800 ml | 5 | 160 ml |
| ... | ... | ... | ... |
| Day 15 | 640 ml | 4 | 160 ml |
| ... | ... | ... | ... |
| Day 30 | 480 ml | 3 | 160 ml |

In some implementations, the total number of completed milk expressing operations and the daily volume of expressed breast milk are inversely proportional to the current number of weaning days.

Refer to Table 3 below. In Table 3, a total number of weaning days is 30, and as the number of weaning days increases in Table 3, both a total number of completed milk expressing operations and a daily volume of expressed breast milk gradually decrease.

**(Table 3)**

| Weaning day | Volume of expressed breast milk | Total number of completed milk expressing operations | Volume of breast milk obtained in a single expressing operation |
|---|---|---|---|
| Day 1 | 1000 ml | 5 | 200 ml |
| ... | ... | ... | ... |
| Day 15 | 640 ml | 4 | 160ml |
| ... | ... | ... | ... |
| Day 30 | 420 ml | 3 | 140 ml |

In some implementations, the step of acquiring configuration information includes following steps.
(1). Preconfiguration information is acquired.
(2). A preset total number of weaning days and a preset daily operating parameter are acquired when the preconfiguration information is preset information.
(3). The configuration information is determined based on the preset total number of weaning days and the preset daily operating parameter.

In some implementations, the preconfiguration information is preconfigured at the factory, or may be flexibly customized by the user.

In this way, the user can be provided with a well-designed preset weaning plan, improving user experience. In addition, the user can flexibly customize a weaning plan to meet personalized needs.

In some implementations, the preset information includes first preset information and second preset information. The preset daily operating parameter includes a daily volume of expressed breast milk. The step of acquiring a preset total number of weaning days and a preset daily operating parameter when the preconfiguration information is preset information includes following substeps.
(1). A first preset total number of weaning days and a preset daily operating parameter are acquired when the preconfiguration information is first preset information, or a second preset total number of weaning days and a preset daily operating parameter are acquired when the preset information is second preset information.
(2). The first preset total number of weaning days is greater than the second preset total number of weaning days, a total volume of breast milk expressed on day 1 corresponding to the first preset information is equal to a total volume of breast milk expressed on day 1 corresponding to the second preset information, and a total volume of breast milk expressed on a final day corresponding to the first preset information is equal to a total volume of breast milk expressed on the final day corresponding to the second preset information.

In some implementations, the first preset information corresponds to a first weaning plan, and the second preset information corresponds to a second weaning plan. Referring to Table 4 and Table 5 below, Table 4 shows an example of the first weaning plan, and Table 5 shows an example of the second weaning plan.

**(Table 4)**

| Weaning day | Volume of expressed breast milk | Total number of completed milk expressing operations | Volume of breast milk obtained in a single expressing operation |
|---|---|---|---|
| Day 1 | 1000 ml | 5 | 200 ml |
| ... | ... | ... | ... |
| Day 15 | 640 ml | 4 | 160 ml |
| ... | ... | ... | ... |
| Day 30 | 420 ml | 3 | 140 ml |
| ... | ... | ... | ... |
| Day 60 | 300 ml | 3 | 100 ml |

**(Table 5)**

| Weaning day | Volume of expressed breast milk | Total number of completed milk expressing operations | Volume of breast milk obtained in a single expressing operation |
|---|---|---|---|
| Day 1 | 1000 ml | 5 | 200 ml |
| ... | ... | ... | ... |
| Day 15 | 420 ml | 3 | 140 ml |
| ... | ... | ... | ... |
| Day 30 | 300 ml | 3 | 100 ml |

In some implementations, more milk expressing modes may be set for selection by the user. For example, the total number of completed milk expressing operations is reduced while leaving the volume of expressed breast milk and the volume of breast milk obtained in a single expressing operation unchanged.

In this way, various milk expressing modes can be provided for selection by users, meeting milk expressing needs of different users.

In some implementations, an embodiment of this application further provides a system. The system includes the breast pump and the terminal described above. The breast pump communicates with the terminal.

In some implementations, the terminal is configured to: acquire configuration information, determine a total number of weaning days and a daily operating parameter of a weaning mode based on the configuration information, and send the total number of weaning days and the daily operating parameter to the breast pump in communication with the terminal when receiving a weaning indication.

In some implementations, the breast pump is configured to: operate in the weaning mode based on the total number of weaning days after receiving the total number of weaning days; determine an operating mode of the breast pump when receiving an operation instruction; acquire a current number of weaning days when the operating mode is a weaning mode, where the weaning mode is used to assist a mother in ceasing milk secretion; determine an operating parameter based on the current number of weaning days; and perform milk expressing based on the operating parameter.

In a pump unit of the breast pump in this application, a power structure configured to generate negative pressure includes but is not limited to a piezoelectric pump, a diaphragm pump, a hydraulic pump, or a mechanical pump. Negative pressure may be directly generated inside the breast shield through communication, or may be indirectly generated inside the breast shield through transmission to a liquid barrier such as a diaphragm or an airbag. The breast milk is drawn into the milk storage container portion for storage through the negative pressure.

The pump unit in this application is partially or entirely arranged in the housing. The housing may further include components such as a battery, a negative pressure air passage, a control circuit board, and a solenoid valve.

### Fourteenth embodiment

In this embodiment of this application, a detection unit is arranged on an inflow path through which breast milk flows into a milk storage container portion, so that a flow rate of breast milk that, after being expressed from a breast, passes through the detection unit and enters the milk storage container portion can be measured. This enables accurate measurement of the breast milk flow rate, saves time and efforts compared to manual measurement, ensures safety and hygiene of the measurement process, and improves user experience.

An embodiment of this application provides a breast pump configured to detect a flow rate of breast milk expressed from a breast based on a gravity sensor.

An embodiment of this application further provides a breast pump configured to detect a flow rate of breast milk expressed from a breast based on an optical emitter and an optical receiver.

An embodiment of this application further provides a breast pump configured to detect a flow rate of breast milk expressed from a breast based on a capacitive sensor.

An embodiment of this application further provides a breast pump configured to detect a flow rate of breast milk expressed from a breast based on a laser sensor.

An embodiment of this application further provides another breast pump configured to detect a flow rate of breast milk expressed from a breast based on a capacitive sensor.

An embodiment VI of this application further provides another breast pump configured to detect a flow rate of breast milk expressed from a breast based on an optical emitter and an optical receiver.

Various scenarios and mounting positions of different detection units are taken into considered to measure a flow rate of breast milk expressed from a breast. This enables accurate measurement of the breast milk flow rate, saves time and efforts compared to manual measurement, ensures safety and hygiene of the measurement process improves user experience.

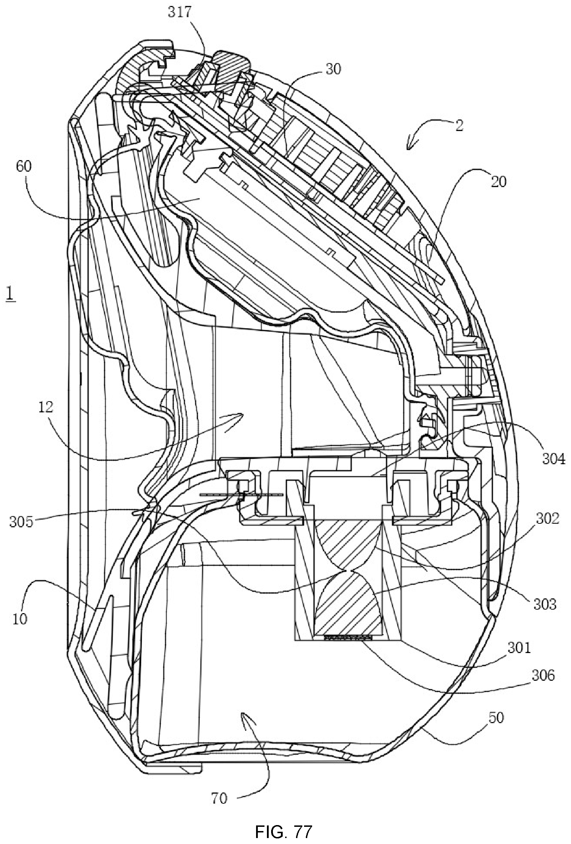

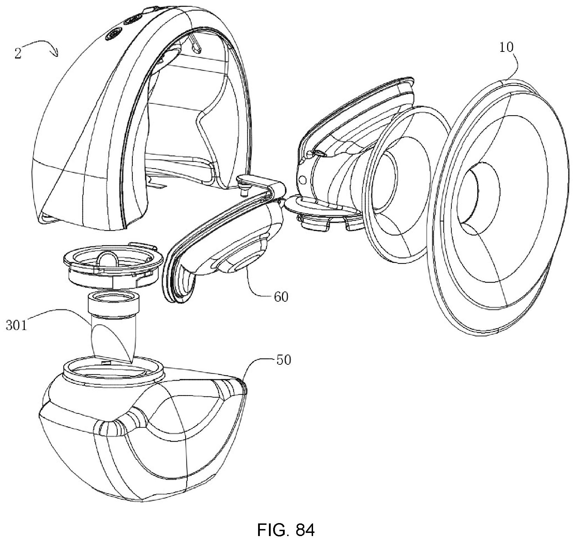

Referring to FIG. 77 and FIG. 84, an embodiment provides a breast pump 1 that can pump and store milk from a human breast, including a milk storage container portion 50, a main unit 2 mounted on the milk storage container portion 50, a breast shield 10, and a diaphragm 60. The milk storage container portion 50 is configured to hold expressed breast milk. An inflow path for circulation of breast milk exists between the breast shield 10 and the milk storage container portion 50, so that breast milk can flow from the breast shield 10 to an interior 70 of the milk storage container portion 50. The breast pump 1 further includes a detection unit arranged on or near the inflow path for breast milk to flow into the milk storage container portion 50. A flow rate monitoring element in this embodiment is arranged on the inflow path of the breast pump 1. Furthermore, the breast shield 10 is detachably mounted on a side of the milk storage container portion 50 to encapsulate the milk storage container portion 50 and to contact the human breast. A nipple channel 12 provided on the breast shield 10 communicates with the interior 70 of the milk storage container portion 50, to ensure that the breast pump 1 can effectively pump and transfer breast milk through the nipple channel 12. The diaphragm 60 is mounted on the milk storage container portion 50 and communicates with the interior 70 of the milk storage container portion 50. The diaphragm 60 communicates with a negative pressure mechanism 20 in the main unit 2 to form a negative pressure chamber in the diaphragm 60, to further pump milk from the breast. Furthermore, the detection unit is electrically connected to the main unit 2, and the main unit 2 communicates with an external terminal, to detect a flow rate of breast milk flowing through the detection unit and send breast milk flow rate data to the main unit 2, so that the main unit 2 can transmit breast milk flow rate information to the external terminal in a timely manner. In this way, a user can learn the flow rate of the expressed breast milk in a timely manner.

Preferably, in this embodiment, prompt information may be sent to the user through an external terminal device. The external device may be a mobile phone, a tablet computer, or a terminal device separately arranged for the breast pump 1, which is not limited herein. The main unit 2 further includes a display element 317, a control unit 30, and a negative pressure mechanism 20. The negative pressure mechanism 20 is electrically connected to the control unit 30, the control unit 30 controls the negative pressure mechanism 20 to directly or indirectly apply negative pressure to the breast shield 10, and the control unit 30 is electrically connected to the detection unit, to promote expressing of milk from the breast. The control unit 30 analyzes data fed back from the detection unit to obtain breast milk flow rate data, and feeds the breast milk flow rate data back to the display element 317. Furthermore, the diaphragm 60 communicates with the negative pressure mechanism 20, and the control unit 30 controls the negative pressure mechanism 20 to operate, to generate negative pressure inside the diaphragm 60 to pump milk from the breast.

Based on the above solution, preferably, the inflow path includes a check valve 301 arranged between the breast shield 10 and the milk storage container portion 50. The check valve 301 is arranged on the breast shield 10, and the nipple channel 12 communicates with the interior 70 of the milk storage container portion 50. The detection unit is arranged on the check valve 301 and is configured to detect a flow rate of breast milk that passes through the check valve 301 and enters the milk storage container portion 50.

Specifically, the check valve 301 includes a first valve portion 302 and a second valve portion 303 communicated with the first valve portion 302, where the first valve portion 302 has a first opening 304 communicated with the nipple channel 12 and a second opening 305 communicated with the second valve portion 303. The first valve portion 302 of the check valve 301 is configured to collect a breast milk flow of the nipple channel 12 through the first opening 304, and allow breast milk with a predetermined outflow characteristic to flow into the second valve portion 303 through the second opening 305. The detection unit is arranged between the first valve portion 302 and the second valve portion 303, and is configured to detect a flow rate of the breast milk with the predetermined outflow characteristic.

Based on the above solution, a breast milk flow with the predetermined outflow characteristic refers to breast milk that exhibits a stable flow rate and flow velocity in an outflow process. For example, when a droplet of breast milk accumulates to a specific weight within the check valve over a unit time before falling. These characteristics can be predicted and controlled. To achieve a breast milk flow with the predetermined outflow characteristic, it is necessary to employ appropriate devices, such as a check valve or a one-way valve, to measure and regulate the flow rate and the flow velocity in real time with accuracy, thereby better understanding and regulating the outflow characteristic of the breast milk.

Based on the above solution, preferably, the first valve portion 302 and the second valve portion 303 are integrally formed, and are combined into a figure-8-shaped structure. Optionally, in an embodiment, the first valve portion 302 and the second valve portion 303 are arranged as two conical structures combined together, while in another embodiment, the first valve portion 302 and the second valve portion 303 are arranged as two spherical structures combined together, both forming a figure-8- shaped structure. The integral design offers advantages of preventing breast milk from flowing out of the check valve 301, while also providing improved transfer and sealing performance.

Specifically, the detection unit includes a gravity sensor 306 arranged on the second valve portion 303 and configured to detect a change in gravity of the second valve portion 303 to determine the breast milk flow rate. Specifically, the gravity sensor 306 may be mounted at a bottom of an outlet of the second valve portion 303 in communication with the milk storage container portion 50. When a breast milk flow with the predetermined outflow characteristic drips from the first valve portion 302 through the second opening 305, the gravity sensor 306 detects a change in a weight of the second valve portion 303, causing the gravity sensor 306 to deform and output an electrical signal. This weight change per unit time is constant due to the determined outflow characteristic of the breast milk. Therefore, the gravity sensor 306 can measure a number of weight changes of the second valve portion 303 per unit time, thereby outputting an equivalent number of electrical signals. The actual breast milk flow rate can then be determined through the output number and/or frequency of these electrical signals.

Based on the above solution, specifically, an internal structure of the gravity sensor 306 usually includes components such as an elastic element, a damping element, and a sensing element. When breast milk drips near the gravity sensor 306, a weight of the breast milk is applied to the elastic element, causing the elastic element to deform. The damping element is configured to reduce vibration of the elastic element, to improve measurement stability. The sensing element is configured to convert the deformation of the elastic element into an electrical signal for output. The output electrical signal is proportional to the weight of the breast milk. Since the breast milk dripping into the second valve portion 303 is consistently a breast milk flow with the predetermined outflow characteristic, a breast milk flow rate can be measured by counting a number of times the electrical signal is generated.

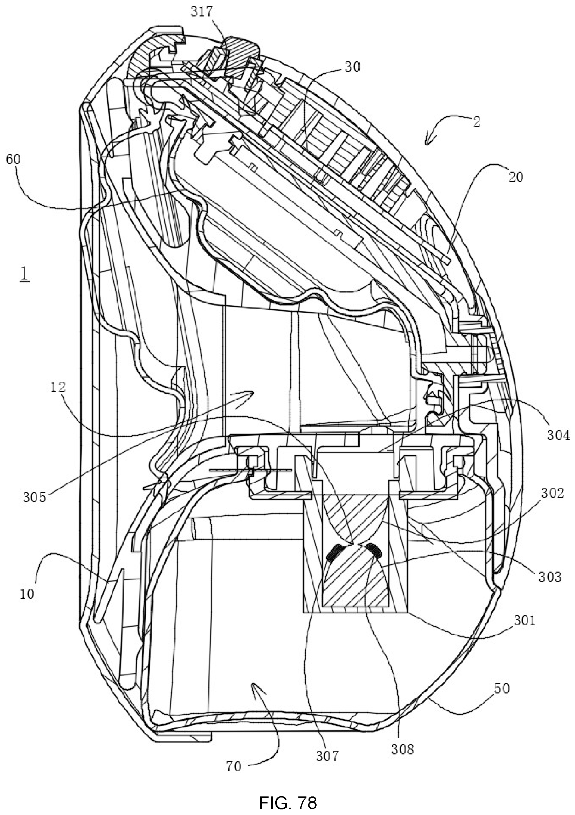

Referring to FIG. 78 and FIG. 84, specifically, the detection unit includes an optical emitter 307 and an optical receiver 308 respectively arranged on two sides of the second valve portion 303 close to the second opening 305 and configured to detect a number of drops of breast milk with the predetermined outflow characteristic that passes through, to determine the actual breast milk flow rate.

Specifically, the optical emitter 307 may be mounted on a side of the second valve portion 303 close to the second opening 305, while the optical receiver 308 is mounted on another side of the second valve portion 303 close to the second opening 305, with the optical emitter 307 and the optical receiver 308 corresponding to each other. When breast milk flows out, the breast milk blocks or alters a transmission path of light rays emitted by the optical emitter 307, causing changes in an optical signal received by the optical receiver 308. Since the breast milk dripping into the second valve portion 303 is consistently a flow with the predetermined outflow characteristic, an actual breast milk flow rate can be measured by counting a number of times the optical signal received by the optical receiver 308 is generated.

Based on the above solution, specifically, a specific principle by which the optical emitter 307 and the optical receiver 308 measure the breast milk flow rate is measuring the breast milk flow rate based on reflection and absorption properties of light. When breast milk flows out, the optical emitter 307 emits light rays onto the breast milk, a part of which is absorbed by the breast milk while another part is reflected. The reflected light ray strikes the optical receiver 308, which is received by the optical receiver 308 and is converted into an electrical signal. A magnitude of the electrical signal is proportional to a breast milk flow rate. Since the breast milk dripping into the second valve portion 303 is consistently a flow with the predetermined outflow characteristic, an actual breast milk flow rate can be measured by counting a number of times the electrical signal is generated.

In conclusion, the method for measuring a breast milk flow rate by using the optical emitter and the optical receiver offers advantages such as being non-contact and nondestructive, which can be widely applied in fields such as maternal and infant products and daily care, providing benefits in terms of hygiene, environmental friendliness, and safety.

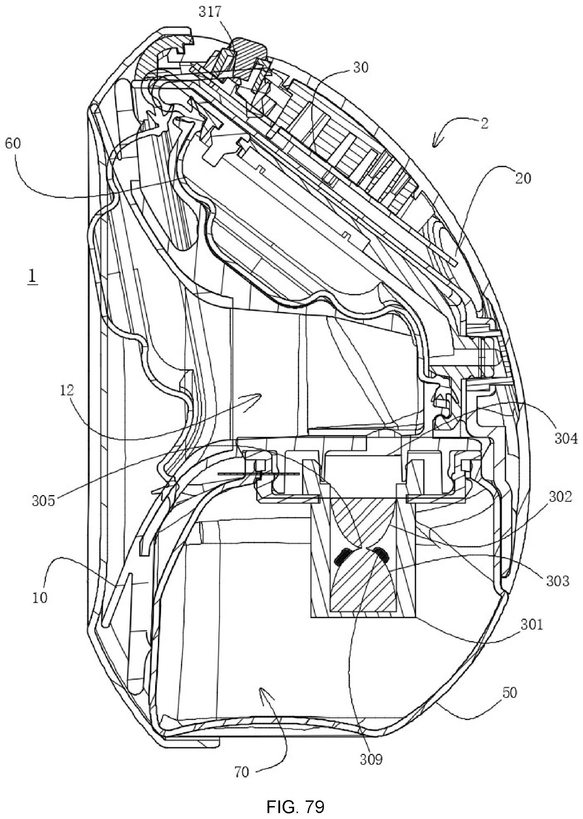

Referring to FIG. 79 and FIG. 84, specifically, the detection unit further includes a capacitive sensor 309, which includes at least one pair of electrode terminals. The at least one pair of electrode terminals are respectively arranged on the two sides of the second valve portion 303 close to the second opening 305, to detect changes in a current as breast milk with the predetermined outflow characteristics passes through, thereby determining a breast milk flow rate.

Specifically, a principle by which the capacitive sensor 309 measures the breast milk flow rate is to measure the breast milk flow rate by detecting a change in a capacitance. The capacitive sensor 309 generally includes two electrode terminals. One of the electrode terminals may be mounted on a side of the second valve portion 303 close to the second opening 305, and the other of the electrode terminals may be arranged on an opposite side of the second valve portion 303 close to the second opening 305. When breast milk flows out, the breast milk acts as a dielectric medium between the two electrode terminals, forming a capacitor and causing a change in a capacitance between the electrode terminals. This change can be detected by the capacitive sensor 309 and converted into an electrical signal for output.

Specifically, when breast milk flows between the two electrode terminals of the capacitive sensor 309, electrolyte ions in the breast milk generate an electric field between the electrodes, leading to a change in the capacitance. This change is proportional to a breast milk flow rate. Since the breast milk dripping into the second valve portion 303 is consistently a flow with the predetermined outflow characteristic, an actual breast milk flow rate can be measured by detecting the change in the capacitance.

To improve accuracy and reliability of the measurement, some measures may be taken. For example, in some other embodiments, a plurality of pairs of electrode terminals may be utilized for measurement, and signals may be averaged to reduce errors. Alternatively, high-frequency circuits may be utilized to minimize impact of electromagnetic interference. Details are not described herein.

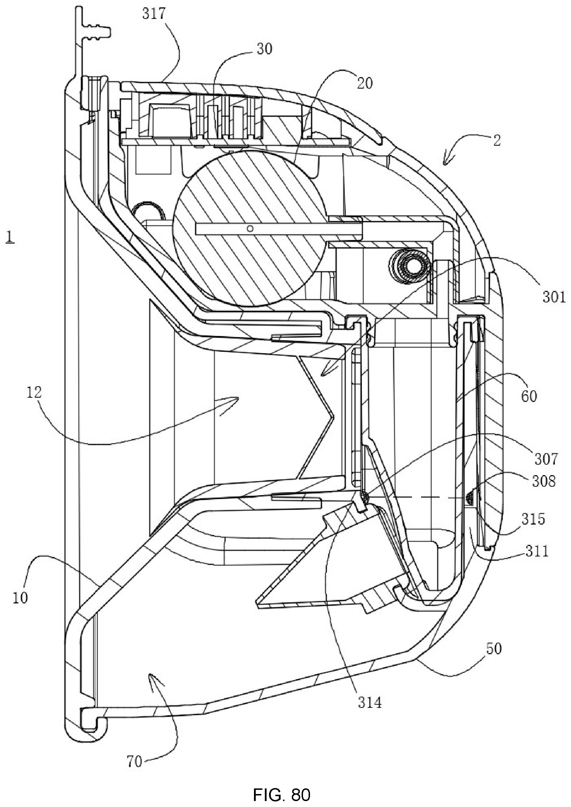

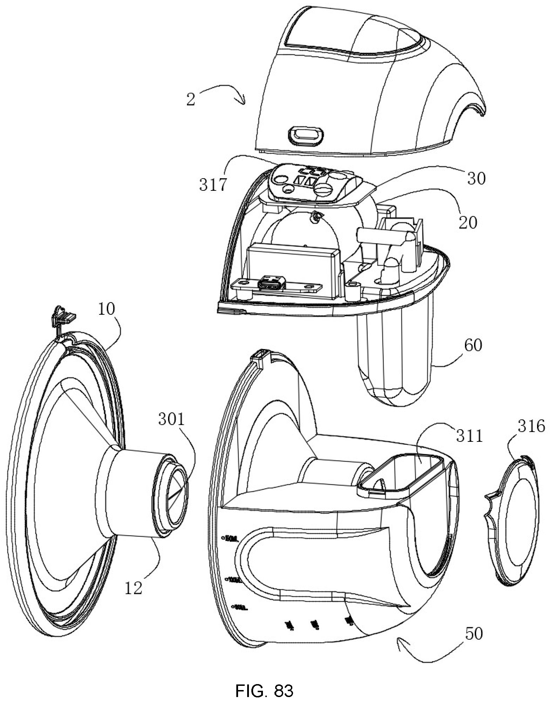

Referring to FIG. 76, FIG. 80, and FIG. 83, the milk storage container portion 50 is provided with a support 311 engaged with the diaphragm 60 to mount the diaphragm 60 on the milk storage container portion 50. Since the diaphragm 60 is a thin film located inside the milk storage container portion 50, and is mainly configured to isolate breast milk from air and prevent contamination on the breast milk, the diaphragm 60 is generally made of an elastic or flexible material. The support 311 supports the diaphragm 60, so that the diaphragm 60 is more stable after the support 311 is mounted, thereby preventing the breast milk from spilling out and ensuring that the breast milk flows smoothly to the interior 70 of the milk storage container portion 50 after being expressed from the breast. Furthermore, when the main unit 2 starts the negative pressure mechanism 20 to pump breast milk, the diaphragm 60 experiences a certain amount of pressure due to generated negative pressure. Additionally, since the support 311 is arranged on a side of the milk storage container portion 50 and integrally formed with a side wall of the milk storage container portion 50, the support 311 achieves greater stability, leading to improved support. The support 311 supports the diaphragm 60, ensuring that the diaphragm 60 does not shift or flip, and ensuring stability and correct positioning of the diaphragm 60.

Based on the above solution, furthermore, the support 311 is provided with at least one mounting position 312, and the detection unit is mounted at the mounting position 312. The mounting position 312 on the support 311 is usually used to mount the detection unit configured to measure a breast milk flow rate. Mounting the detection unit on the mounting position 312 of the support 311 can achieve accurate measurement and monitoring of the breast milk flow rate. It may be understood that, designing the mounting position 312 on the support 311 can achieve higher stability of the mounting position 312, ensuring that the detection unit can be securely mounted without displacement or loosening due to external forces or movement. In contrast, the diaphragm 60 is relatively soft and cannot provide sufficient stability to support the mounting of the detection unit. Moreover, the detection unit mounted on the support 311 can measure the breast milk flow rate more accurately. Due to the stability and security of the support 311, the detection unit can more instantly sense flowing of breast milk, thereby obtaining more precise data. In addition, designing the mounting position 312 on the support 311 facilitates cleaning and maintenance for the user. The support 311 is generally made of durable materials that can withstand cleaning with detergents and water without affecting performance of the detection unit. In contrast, the diaphragm 60 is more delicate and requires more careful handling.

Based on the above solution, preferably, the detection unit is configured to detect a flow rate of breast milk that is expressed from the nipple channel 12 and passes through a side of the diaphragm 60, and to transmit breast milk flow rate data to the main unit 2, allowing the user to learn breast milk flow rate information in a timely manner.

Preferably, the breast pump 1 further includes a diaphragm cover 316 detachably mounted on the milk storage container portion 50 and located on a side of the diaphragm 60. This structure offers an advantage that the diaphragm cover 316 can be easily removed, allowing the user to clean and disinfect the diaphragm. In this way, residue, bacteria, and other contaminants can be effectively removed, ensuring hygiene and safety of the milk storage container portion 50. In addition, the user can easily detach and replace the diaphragm 60 with a new one, facilitating subsequent maintenance, and also facilitating mounting of the detection unit during manufacturing of the breast pump 1. Furthermore, in this embodiment, the diaphragm 60 is spaced apart from the diaphragm cover 316 and also spaced apart from the nipple channel 12. This spacing design facilitates subsequent mounting of the detection unit, provides suitable positioning for the detection unit, and enables the detection unit to more effectively measure the breast milk flow rate.

Preferably, the detection unit includes an optical emitter 307 and an optical receiver 308, and the mounting position 312 includes a first mounting position 314 and a second mounting position 315 respectively arranged on two sides of the support 311. The first mounting position 314 and the second mounting position 315 are aligned with each other, with the optical emitter 307 mounted at the first mounting position 314 and the optical receiver 308 mounted at the second mounting position 315.

Based on the above solution, specifically, in this embodiment, the first mounting position 314 and the second mounting position 315 are respectively designed on the two sides of the support 311. The first mounting position 314 is arranged on a side wall of the support 311 away from the nipple channel 12, while the second mounting position 315 is arranged on a side wall of the support 311 close to the nipple channel 12. The optical emitter 307 mounted at the first mounting position 314 and the optical receiver 308 mounted at the second mounting position 315 are arranged opposite each other. In an actual milk expressing process, breast milk expressed from the breast flows into the breast pump 1 through the check valve 301 at a port of the nipple channel 12. At this point, the breast milk moves toward the milk storage container portion 50 under expressing pressure and is to pass between the optical emitter 307 and the optical receiver 308 before entering the interior 70 of the milk storage container portion through a milk inlet of the milk storage container portion 50, thereby causing light rays emitted by the optical emitter 307 to contact the breast milk flow.

First, a tube may be added at the port of the nipple channel 12, with one end connected to an opening of the check valve 301 and another end connected to the optical emitter 307 and the optical receiver 308. In addition, this embodiment designs a measurement scheme in which the tube receives breast milk flow from the nipple channel 12 and measures a breast milk flow with the predetermined outflow characteristic. Specifically, a flow rate of the breast milk with the predetermined outflow characteristic is measured by using the optical emitter 307 and the optical receiver 308, that is, by employing an optical flow meter. A primary principle of this method is to determine the flow rate by using optical properties such as scattering, absorption, or the Doppler effect caused by particles or microbubbles in a fluid to.

As previously described, in this case, the optical emitter 307 emits a light beam toward the breast milk in the breast milk tube, and the optical receiver 308 detects the light beam. When breast milk flows through the light beam, a part of the light beam is blocked by the breast milk flow and cannot reach the receiver, thereby weakening the light beam received by the optical receiver. The optical receiver 308 feeds back to the control unit 30 a duration in which the breast milk flow blocks the light beam and causes the light beam to weaken in intensity. The control unit 30 then may perform analysis through algorithms and preset programs to calculate the breast milk flow rate.

Optionally, the above tube may be replaced with a funnel or other containers, and may be adjusted based on the specific structure of the milk storage container portion 50 of the breast pump 1, which is not limited herein.

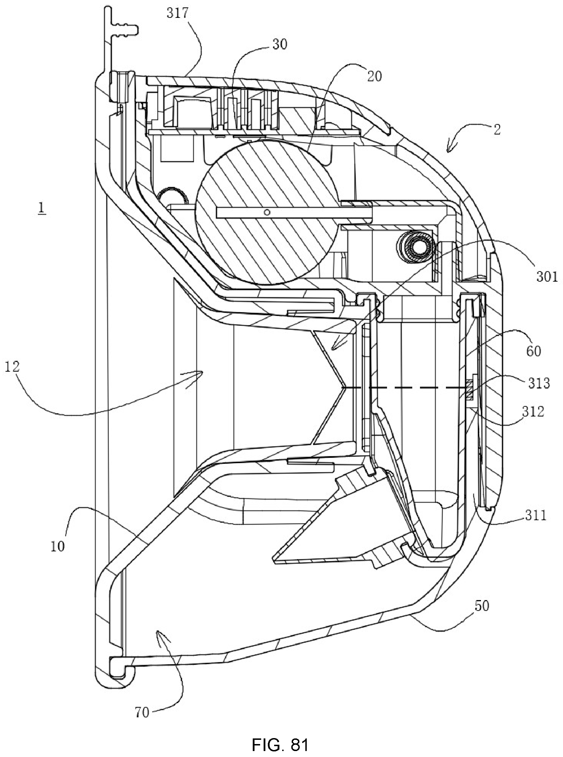

Embodiment 5: Referring to FIG. 76, FIG. 81, and FIG. 83, this embodiment further provides an implementation for measuring a breast milk flow rate. Preferably, the detection unit includes a laser sensor 313 arranged between the diaphragm cover 316 and the nipple channel 12. The laser sensor 313 is at least aligned with the opening of the check valve 301 to detect an open duration of the check valve 301.

Based on the above solution, specifically, the laser sensor 313 is arranged on a side of the support 311 close to the diaphragm cover 316 and is positioned at the mounting position 312, so that a direction of the laser sensor 313 is aligned with the opening of the check valve 301. In this embodiment, the laser sensor 313 is arranged as a laser transducer. It may be understood that, the opening of the check valve 301 is arranged as a one-way valve structure and an opening angle of the check valve 301 is configured as a consistent opening angle during milk expressing. This allows a breast milk flow rate per unit time through the opening to be quantified.

As previously described, in this case, when breast milk flows from the nipple channel 12 into the breast pump 1, the opening of the check valve 301 opens. At this point, light rays from the laser sensor, which were originally directed at a closed cover of the opening of the check valve 301 when the check valve 301 was closed, now shift to direct at the expressed breast milk flow. This change causes a change in an intensity of light rays reflected back to the laser sensor. A duration of intensity decrease of light signals is fed back by the laser sensor to the control unit 30. The control unit 30 then may perform analysis through algorithms and preset programs to calculate the breast milk flow rate.

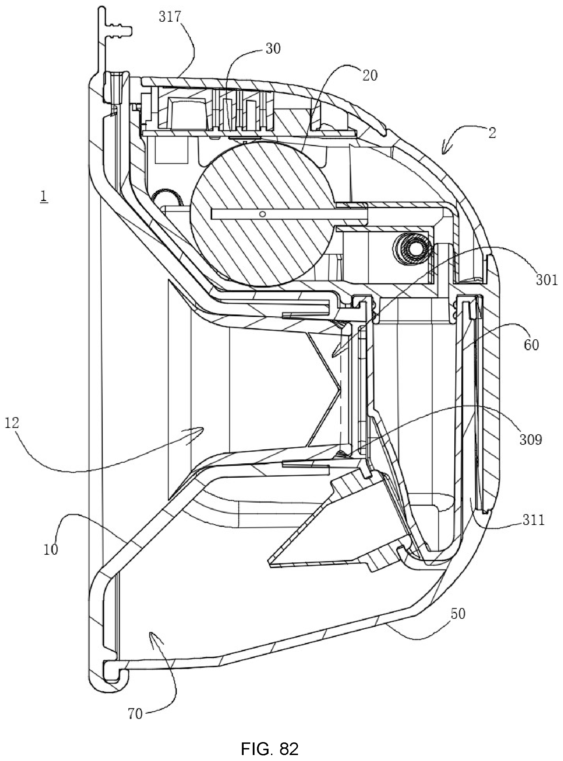

Referring to FIG. 76, FIG. 82, and FIG. 83, this embodiment further provides an implementation for measuring a breast milk flow rate. Preferably, the detection unit includes a capacitive sensor 309 arranged between the milk storage container portion 50 and the nipple channel 12. The capacitive sensor 309 includes a pair of electrode terminals respectively located on two sides of the nipple channel 12, so that breast milk expressed from the nipple channel 12 flows between the electrode terminals.

Based on the above solution, specifically, the capacitive sensor 309 is arranged on the side wall of the milk storage container portion 50 and located on two sides of the nipple channel 12. Moreover, the capacitive sensor 309 includes a pair of electrode terminals respectively arranged on the two sides of the nipple channel 12. It may be understood that, in this embodiment, the capacitive sensor 309 is implemented by an electrode sensor. The two electrode terminals are arranged as electrode plates facing each other, so that in an actual milk expressing process, breast milk expressed from the breast flows into the breast pump 1 through the opening of the check valve 301. At this point, the breast milk moves toward the milk storage container portion 50 under expressing pressure and is to pass between the two electrode terminals before entering the interior 70 of the milk storage container portion through the milk inlet of the milk storage container portion 50. This allows a current between the two electrode terminals to contact the breast milk flow, thereby increasing impedance between the electrode terminals and reducing the current. A duration of this current decrease is fed back by the electrode terminals to the control unit 30. The control unit 30 then may perform analysis through algorithms and preset programs to calculate the breast milk flow rate.

In the above embodiments, it may be understood that at least portions of the diaphragm 60 opposite the optical emitter 307, the optical receiver 308, and the laser sensor 313 are made transparent, so that light rays from the optical emitter 307 in Embodiment 1 and light rays from the laser sensor in Embodiment 2 can pass through the diaphragm 60 for irradiation, thereby achieving measurement of the breast milk flow rate.

In the above embodiments, regardless of whether the optical emitter 307, the optical receiver 308, the laser sensor 313, the capacitive sensor 309, or the gravity sensor 306 is used, proper signal processing and data processing need to be performed by the control unit 30 of the main unit 2, to extract accurate electrical signals. In addition, proper adjustments and optimizations need to be performed based on a specific design of the breast pump 1 and breast milk characteristics.

Based on the above embodiments, it may be understood that the detection unit is configured to detect a flow rate of breast milk passing through the inflow path. The detection unit may be arranged inside the inflow path, outside the inflow path, embedded in a physical inner wall corresponding to the inflow path, or at any position between the above. These positions may all be referred to as being on or near the inflow path. Therefore, the above embodiments may be understood as placing all flow rate monitoring elements on or near the inflow path. It should be noted that when optical sensors are used, the corresponding structural portion of the inflow path is made transparent to facilitate light transmission. When capacitive sensors are used, such limitations do not apply.

### Fifteenth embodiment

In some implementations, a detection unit 40 includes a breast milk flow rate detection unit 232.

### Implementation I

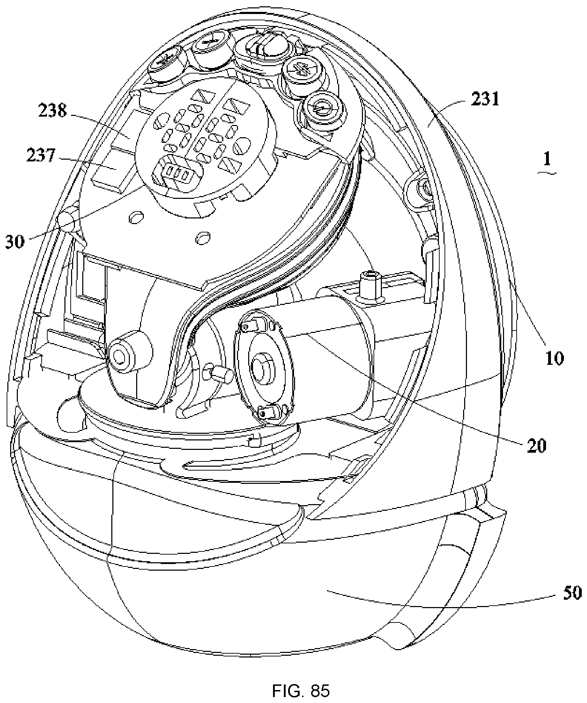

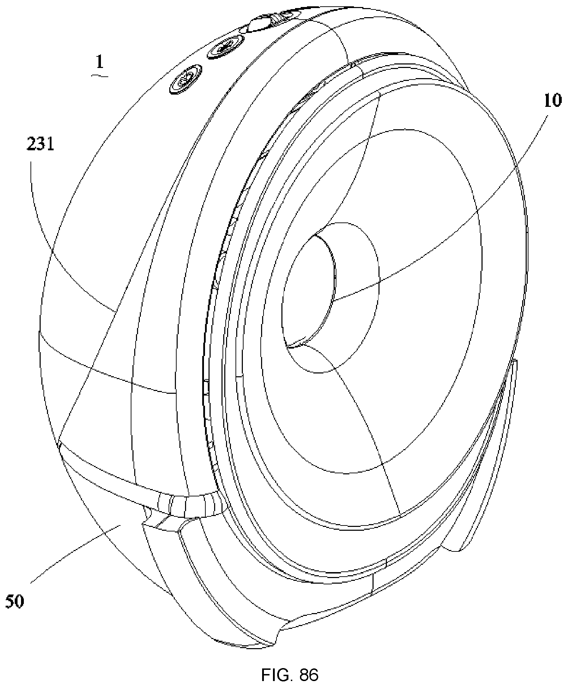

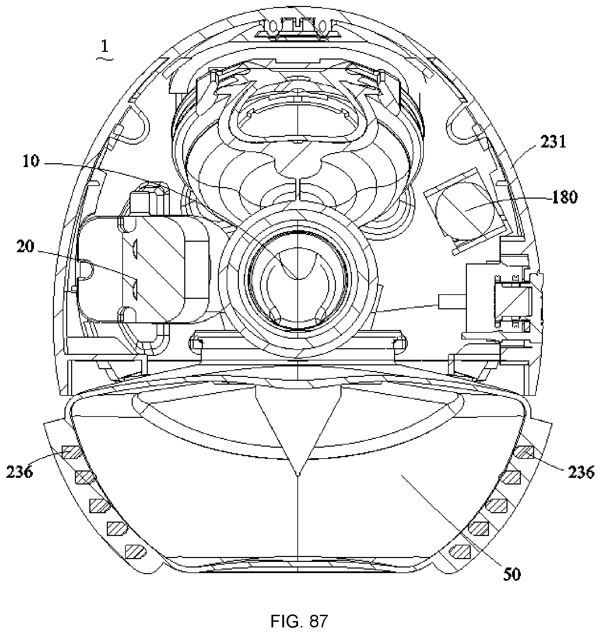

Referring to FIG. 85 to FIG. 87, in some embodiments of this application, this application provides a breast milk flow rate detection unit 232, which is applied to a breast pump 1 and includes a liquid level detection unit 233 and a control unit 30. The liquid level detection unit 233 is configured to detect a liquid level of a milk storage container portion 50. The control unit 30 is configured to determine a breast milk volume in the milk storage container portion 50 based on at least liquid level data information from the liquid level detection unit 233.

The liquid level detection unit 233 detects a liquid level of breast milk in the milk storage container portion 50. The control unit 30 acquires liquid level data information from the liquid level detection unit 233, and determines the breast milk volume in the milk storage container portion 50 based on the liquid level data information. This allows for detection of the breast milk volume in the milk storage container portion 50 during the milk expressing.

In some embodiments of this application, the liquid level detection unit 233 includes at least one through-beam sensor 234, including an emitter 235 and a receiver 236. The emitter 235 and the receiver 236 are respectively mounted on two opposite sides of the milk storage container portion 50.

A liquid level in the milk storage container portion 50 is detected by the through-beam sensor 234. When the liquid level in the milk storage container portion 50 reaches a height at which a through-beam sensor 234 is positioned, the breast milk in the milk storage container portion 50 blocks light rays emitted by the emitter 235 of the through-beam sensor 234, which prevents the receiver 236 of the same through-beam sensor 234 from receiving the light rays emitted by the corresponding emitter 235, thereby causing the through-beam sensor 234 to respond. The control unit 30 may determine a height at which a responding through-beam sensor 234 is positioned based on a serial number of the through-beam sensor 234, so as to obtain the liquid level in the milk storage container portion 50 through the through-beam sensor 234.

In some embodiments of this application, the liquid level detection unit 233 includes at least two through-beam sensors 234.

The through-beam sensors 234 are arranged in sequence linearly from a minimum liquid level boundary of the milk storage container portion 50 to a maximum liquid level boundary of the milk storage container portion 50.

Since breast milk accumulates from a bottom of the milk storage container portion 50 during milk expressing, the liquid level in the milk storage container portion 50 rises from a lower position to a higher position. As the liquid level in the milk storage container portion 50 rises, the through-beam sensors 234 respond sequentially in ascending order of heights. The control unit 30 queries serial numbers of the through-beam sensors 234 in ascending order of heights until a through-beam sensor 234 that has not responded is found. In this case, a serial number of a last responding through-beam sensor 234, that is, a through-beam sensor 234 positioned highest among all responding through-beam sensors 234 and closest to a liquid surface in the milk storage container portion 50, is acquired. Based on the serial number of the last responding through-beam sensor 234, the control unit 30 can determine a height at which the through-beam sensor 234 is positioned, thereby obtaining the liquid level in the milk storage container portion 50 through the through-beam sensor 234.

In some embodiments of this application, the milk storage container portion 50 includes a milk storage chamber defined by the side wall, and the emitter 235 and the receiver 236 of the through-beam sensor 234 are arranged opposite each other on the side wall.

The emitter 235 and the receiver 236 of the through-beam sensor 234 are arranged on the side wall of the milk storage container portion 50, so that the emitter 235 and the receiver 236 of the through-beam sensor 234 remain relatively fixed with respect to the side wall of the milk storage container portion 50, and remain relatively fixed in position and orientation. This enables the receiver 236 to properly receive the light rays from the emitter 235, thereby ensuring reliability and stability of the through-beam sensor 234.

In some embodiments of this application, at least portions of the milk storage container portion 50 for transmission between the emitter 235 and the receiver 236 are made transparent.

The emitter 235 and the receiver 236 of the through-beam sensor 234 both may be mounted on outside of the milk storage container portion 50 to prevent the through-beam sensor 234 from contacting breast milk. At least portions of the side wall of the milk storage container portion 50 corresponding to the positions of the emitter 235 and the receiver 236 of the through-beam sensor 234 are made transparent, so that the light rays emitted by the emitter 235 of the through-beam sensor 234 can penetrate the side wall of the milk storage container portion 50 and be received by the receiver 236 of the through-beam sensor 234. When the liquid level in the milk storage container portion 50 rises to the height at which the through-beam sensor 234 is positioned, the breast milk in the milk storage container portion 50 blocks the light rays between the receiver 236 and the emitter 235 of the through-beam sensor 234, which prevents the receiver 236 from receiving the light rays from the corresponding emitter 235, thereby causing the through-beam sensor 234 to respond. The control unit 30 may determine a height at which a responding through-beam sensor 234 is positioned based on a serial number of the through-beam sensor 234, so as to obtain the liquid level in the milk storage container portion 50 through the through-beam sensor 234.

In some application scenarios of this application, ten through-beam sensors 234 are arranged between the minimum liquid level boundary and the maximum liquid level boundary of the milk storage container portion 50. All of the through-beam sensors 234 are arranged in sequence in ascending order of heights linearly and equidistantly. The through-beam sensors 234 are sequentially numbered in ascending order of heights. The numbering of the through-beam sensors 234 may correspond to actual heights, such as 10 mm, 20 mm, and so on up to 100 mm; or customized identifiers, such as #1, #2, and so on up to #10, may be used. Alternatively, any other identifiable numbering method that distinguishes different through-beam sensors 234 may also be adopted.

Customized numbering is taken as an example. Since a shape of the milk storage container portion 50 varies across different models of breast pumps 1, a same liquid level in the milk storage container portions 50 of different models of breast pumps 1 may correspond to different breast milk capacities. Therefore, before the breast pump 1 leaves the factory, the breast milk capacity of the milk storage container portion 50 needs to be calibrated, and a breast milk capacity of the milk storage container portion 50 corresponding to a through-beam sensor 234 positioned at each height or of each serial number is measured and recorded. For example, a breast milk capacity of the milk storage container portion 50 corresponding to a 1# through-beam sensor 234 is 10 ml, a breast milk capacity of the milk storage container portion 50 corresponding to a 2# through-beam sensor 234 is 20 ml, a breast milk capacity of the milk storage container portion 50 corresponding to a 3# through-beam sensor 234 is 30 ml, and so on, and a breast milk capacity of the milk storage container portion 50 corresponding to a 10# through-beam sensor 234 is 100 ml.

When the liquid level in the milk storage container portion 50 rises above a position of the 3# through-beam sensor 234 but remains below a position of the 4# through-beam sensor 234, the breast milk in the milk storage container portion 50 causes the 1#, 2#, and 3# through-beam sensors 234 to respond. The 3# through-beam sensor 234 is a highest-positioned through-beam sensor 234 among the responding through-beam sensors 234. At this point, the control unit 30 queries a database based on the serial number of the 3# through-beam sensor 234 and obtains the breast milk capacity of 30 ml corresponding to the 3# through-beam sensor 234. In this way, the liquid level in the milk storage container portion 50 is detected by the through-beam sensors 234, and the breast milk volume in the milk storage container portion 50 is determined based on the liquid level data.

### Implementation II

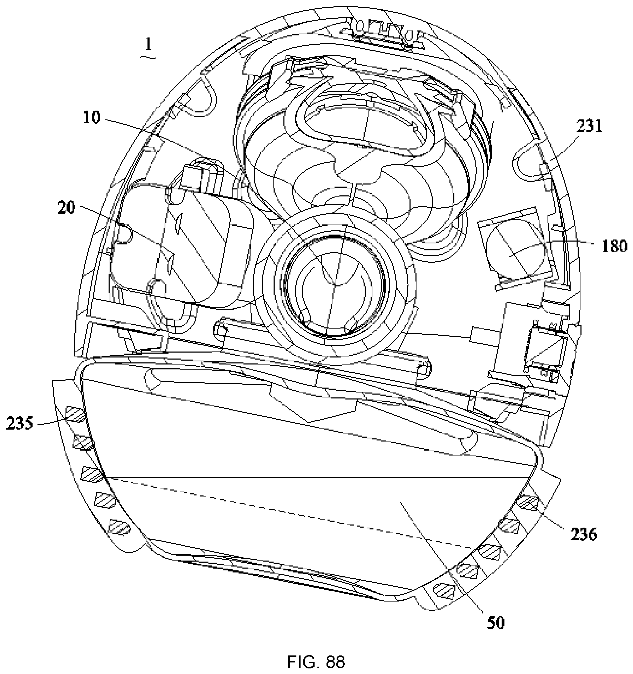

Referring to FIG. 85 to FIG. 89, in some embodiments of this application, this application provides a breast milk flow rate detection unit 232, including a liquid level detection unit 233, an angle detection unit 237, and a control unit 30.

The liquid level detection unit 233 is configured to detect a liquid level of a milk storage container portion 50. The angle detection unit 237 is configured to detect tilting data of the milk storage container portion 50. The control unit 30 is configured to determine a breast milk volume in the milk storage container portion 50 based on liquid level data information from the liquid level detection unit 233 and tilting data information from the angle detection unit 237.

A breast milk level in the milk storage container portion 50 is detected by the liquid level detection unit 233, and the tilting data of the milk storage container portion 50 is detected by the angle detection unit 237. The control unit 30 acquires the liquid level data information from the liquid level detection unit 233 and the tilting data information from the angle detection unit 237, and determines the breast milk volume in the milk storage container portion 50 based on the liquid level data information and the tilting data information. Therefore, during milk expressing, the breast milk volume in the milk storage container portion 50 is detected, and detection errors caused by tilting of the milk storage container portion 50 is reduced, ensuring intended detection accuracy.

In some embodiments of this application, a database may be preconfigured in the control unit 30. Content of the database includes at least breast milk level gradient data of the milk storage container portion 50, tilting gradient data of the milk storage container portion 50, and breast milk volume parameters corresponding to the liquid level data and the tilting data.

The control unit 30 acquires the liquid level data information from the liquid level detection unit 233 and the tilting data information from the angle detection unit 237, and then queries the database to obtain breast milk volume parameter information corresponding to the liquid level data information and the tilting data information, and determines the breast milk volume in the milk storage container portion 50 from the obtained breast milk volume parameter information.

Since a shape and a dimension of the milk storage container portion 50 vary across different models of breast pumps 1, the breast milk storage capacity of the milk storage container portion 50 is calibrated before the breast pump 1 leaves the factory. A breast milk storage capacity of the milk storage container portion 50 corresponding to each group of parameter gradients (for example, a group of parameter gradients including a breast milk level gradient of the milk storage container portion 50 and a tilting data gradient of the milk storage container portion 50) is measured and recorded, to form the breast milk volume parameters.

During actual use, the breast milk level in the milk storage container portion 50 is detected by the liquid level detection unit 233, and the tilting data of the milk storage container portion 50 is detected by the angle detection unit 237. The control unit 30 acquires the liquid level data information from the liquid level detection unit 233 and the tilting data information from the angle detection unit 237, and then queries the database to obtain breast milk volume parameter information based on the liquid level data information and the tilting data information, and determines the breast milk volume in the milk storage container portion 50.

In some usage scenarios of this application, the liquid level detection unit 233 detects that the liquid level in the milk storage container portion 50 is 10 mm, and the angle detection unit 237 detects that the tilting data of the milk storage container portion 50 is 30°. It may be learned from the database that when the liquid level is 10 mm and the tilting data is 30°, a corresponding breast milk volume parameter is 12 ml, thereby determining that the breast milk volume in the milk storage container portion 50 is 12 ml.

In some usage scenarios of this application, the liquid level detection unit 233 detects that the liquid level in the milk storage container portion 50 is 10 mm, and the angle detection unit 237 detects that the tilting data of the milk storage container portion 50 is 20°. It may be learned from the database that when the liquid level is 10 mm and the tilting data is 20°, a corresponding breast milk volume parameter is 15 ml, thereby determining that the breast milk volume in the milk storage container portion 50 is 15 ml.

It may be learned the breast milk volumes determined in the above two usage scenarios that, at the same liquid level, different tilting data of the milk storage container portion 50 corresponds to different breast milk volumes. Therefore, the tilting data of the milk storage container portion 50 is detected by the angle detection unit 237, to determine the breast milk volumes in the milk storage container portion 50 under different tilting data, thereby reducing detection errors caused by the tilting of the milk storage container portion 50 and ensuring intended detection accuracy.

Similarly, under the same tilting data of the milk storage container portion 50, different liquid levels also result in variations. A process and a principle for determining the breast milk volumes in the milk storage container portion 50 under such conditions are the same as those described above. Details are not described herein.

In some embodiments of this application, the angle detection unit 237 is a gyroscope.

Utilizing the gyroscope to detect the tilting data of the milk storage container portion 50 can ensure intended detection accuracy, with a short response time and a short detection delay, and can achieve prompt feedback of the detected tilting data to the control unit 30.

In some embodiments of this application, the liquid level detection unit 233 includes at least one through-beam sensor 234, including an emitter 235 and a receiver 236. The emitter 235 and the receiver 236 are respectively mounted on two opposite sides of the milk storage container portion 50.

A liquid level in the milk storage container portion 50 is detected by the through-beam sensor 234. When the liquid level in the milk storage container portion 50 reaches a height at which a through-beam sensor 234 is positioned, the breast milk in the milk storage container portion 50 blocks light rays emitted by the emitter 235 of the through-beam sensor 234, which prevents the receiver 236 of the same through-beam sensor 234 from receiving the light rays emitted by the corresponding emitter 235, thereby causing the through-beam sensor 234 to respond. The control unit 30 may determine a height at which a responding through-beam sensor 234 is positioned based on a serial number of the through-beam sensor 234, so as to obtain the liquid level in the milk storage container portion 50 through the through-beam sensor 234.

In some embodiments of this application, the liquid level detection unit 233 includes at least two through-beam sensors 234.

The through-beam sensors 234 are arranged in sequence linearly from a minimum liquid level boundary of the milk storage container portion 50 to a maximum liquid level boundary of the milk storage container portion 50.

Since breast milk accumulates from a bottom of the milk storage container portion 50 during milk expressing, the liquid level in the milk storage container portion 50 rises from a lower position to a higher position. As the liquid level in the milk storage container portion 50 rises, the through-beam sensors 234 respond sequentially in ascending order of heights. The control unit 30 queries serial numbers of the through-beam sensors 234 in ascending order of heights until a through-beam sensor 234 that has not responded is found. In this case, a serial number of a last responding through-beam sensor 234, that is, a through-beam sensor 234 positioned highest among all responding through-beam sensors 234 and closest to a liquid surface in the milk storage container portion 50, is acquired. Based on the serial number of the last responding through-beam sensor 234, the control unit 30 can determine a height at which the through-beam sensor 234 is positioned, thereby obtaining the liquid level in the milk storage container portion 50 through the through-beam sensor 234.

In some embodiments of this application, an alarm unit may be further arranged, which is connected to the control unit 30. When the liquid level detection unit 233 detects that the liquid level in the milk storage container portion 50 is close to a maximum liquid level boundary, an audible and visual alarm is sent through the alarm unit, and the breast pump 1 may be controlled to stop or pause the milk expressing.

The control unit 30 may further control the alarm unit to send different types of alarms based on the parameter information fed back by the liquid level detection unit 233 and the angle detection unit 237.

A liquid level warning threshold, a standard tilting data threshold, and a tilting data warning threshold may be set.

When the parameter information received by the control unit 30 indicates that the liquid level data reaches or exceeds the liquid level warning threshold while the tilting data is below the standard tilting data threshold, this signifies that the tilting data of the milk storage container portion 50 is relatively small and the milk storage container portion 50 reaches a nearly milk fullness state. In this case, the control unit 30 sends an instruction to the alarm unit to send a full-level alarm.

If the liquid level data reaches or exceeds the liquid level warning threshold, and the tilting data reaches or exceeds the tilting data warning threshold, it indicates that the tilting data of the milk storage container portion 50 is relatively large. Although a liquid surface in the milk storage container portion 50 is close to the maximum liquid level boundary, the milk storage container portion 50 is in a false fullness state. In this case, the control unit 30 sends an instruction to the alarm unit to send a spillage alarm, prompting the user to adjust a posture until the tilting data falls below the standard tilting data threshold.

In some embodiments of this application, the control unit 30 is preconfigured with a first breast milk volume parameter corresponding to the liquid level data information and the tilting data information.

During milk expressing, values detected by the liquid level detection unit 233 and the angle detection unit 237 may vary across different time points. Liquid level data and tilting data measured at each time point form a parameter pair, and each parameter pair corresponds to a first breast milk volume parameter. The first breast milk volume parameter may be either a specific numerical value of the breast milk volume corresponding to the liquid level data and the tilting data in the parameter pair, or may be a code that corresponds to a specific breast milk volume value. After acquiring the parameter pair formed by the liquid level data and the tilting data, the control unit 30 queries the database to obtain the first breast milk volume parameter, and then determines the breast milk volume in the milk storage container portion 50 through the first breast milk volume parameter.

In some embodiments of this application, the breast milk flow rate detection unit 232 provided in this application further includes an acceleration sensor 238 connected to the control unit 30. The acceleration sensor 238 is configured to detect displacement data information of the milk storage container portion 50. The control unit 30 is preconfigured with a second breast milk volume parameter corresponding to the liquid level data information, the tilting data information, and the displacement data information.

During the milk expressing, when the user performs actions such as walking or standing up, the liquid level in the milk storage container portion 50 may fluctuate or vibrate, reducing accuracy of a determined breast milk capacity.

The acceleration sensor 238 detects displacement data information of the milk storage container portion 50, to assess a fluctuation or vibration status of the liquid level in the milk storage container portion 50. Before the breast pump 1 leaves the factory, a breast milk storage capacity of the milk storage container portion 50 is calibrated. A breast milk capacity corresponding to the displacement data information under corresponding liquid level data and tilting data conditions is measured and recorded, to form the second breast milk volume parameter. During actual operation, the control unit 30 queries the second breast milk volume parameter corresponding to the liquid level data information, the tilting data information, and the displacement data information, and then determines the breast milk capacity of the milk storage container portion 50 under such parameter conditions.

In some embodiments of this application, the displacement data information includes a displacement velocity, a displacement acceleration, and a displacement direction. A motion status of the user may be comprehensively determined based on the displacement data information, and comprehensive displacement data may be fitted. The comprehensive displacement data may be obtained through measurement and assignment. For example, the acceleration sensor 238 obtains a displacement velocity of 1 m/s, a displacement acceleration of 1.5 m/s², and a displacement direction horizontally forward. It may be learned through the comprehensive assessment based on the displacement data information that, the motion status of the user is starting to walk. In this case, the comprehensive displacement data may be assigned a value of a. Motion statuses of the user under various conditions are measured and assigned values in the above manner, thereby forming a database including displacement data.

In some application scenarios of this application, the liquid level detection unit 233 detects that the liquid level in the milk storage container portion 50 is 10 mm, the angle detection unit 237 detects that the tilting data of the milk storage container portion 50 is 30°, and the acceleration sensor 238 obtains a comprehensive displacement data value assigned as a. It may be learned by querying the database that, when the liquid level is 10 mm, the tilting data is 30°, and the comprehensive displacement data value is assigned as a, the corresponding second breast milk volume parameter is 13 ml, thereby determining that the breast milk volume in the milk storage container portion 50 is 13 ml.

### Implementation III

Referring to FIG. 85 to FIG. 89, in some embodiments of this application, this application provides a breast milk flow rate detection unit 232, including a liquid level detection unit 233, an angle detection unit 237, an acceleration sensor 238, and a control unit 30.

The liquid level detection unit 233 is configured to detect a liquid level of a milk storage container portion 50. The angle detection unit 237 is configured to detect tilting data of the milk storage container portion 50. The acceleration sensor 238 is configured to detect displacement data information of the milk storage container portion 50. The control unit 30 is configured to determine whether to initiate measurement of a breast milk volume in the milk storage container portion 50 based on at least one of the tilting data information from the angle detection unit 237 or the displacement data information from the acceleration sensor 238, and determine the breast milk volume in the milk storage container portion 50 based on the liquid level data information from the liquid level detection unit 233.

In some embodiments of this application, the liquid level detection unit 233 may be a through-beam sensor 234, and the angle sensor may be a gyroscope.

In some embodiments of this application, the control unit 30 is configured with a tilting threshold and a displacement threshold. When the tilting data exceeds the tilting threshold and/or the displacement data exceeds the displacement threshold, the control unit 30 does not send an instruction and thus does not initiate the measurement of the breast milk volume in the milk storage container portion 50.

When the tilting data is within the tilting threshold and the displacement data is within the displacement threshold, the control unit 30 sends an instruction to initiate the measurement of the breast milk volume in the milk storage container portion 50. In this case, the control unit 30 determines the breast milk volume in the milk storage container portion 50 based on the liquid level data information from the liquid level detection unit 233.

It may be understood that when the tilting data exceeds the tilting threshold, it indicates that the milk storage container portion 50 is in a tilted state and conditions for measuring the breast milk volume in the milk storage container portion 50 are not met. Even if the breast milk volume in the milk storage container portion 50 is measured, it is impossible to achieve intended accuracy. When the displacement data exceeds the displacement threshold, it indicates that the milk storage container portion 50 is in a moving state (when the user is in motion, the milk storage container portion 50 is caused to be in a moving state). When the milk storage container portion 50 is in a moving state, the breast milk surface in the milk storage container portion 50 is unstable, and the conditions for measuring the breast milk volume in the milk storage container portion 50 are not met. An alarm unit may be arranged. When the tilting data exceeds the tilting threshold and/or the displacement data exceeds the displacement threshold, the control unit 30 sends an instruction to the alarm unit to send an alarm, prompting the user to adjust a posture so that the tilting data is within the tilting threshold and the displacement data is within the displacement threshold. When the tilting data is within the tilting threshold and the displacement data is within the displacement threshold, the control unit 30 sends an instruction to initiate the measurement of the breast milk volume in the milk storage container portion 50. In this case, the control unit 30 determines the breast milk volume in the milk storage container portion 50 based on the liquid level data information from the liquid level detection unit 233.

### Implementation IV

Referring to FIG. 85 to FIG. 89, in some embodiments of this application, this application provides a breast milk flow rate detection unit 232, including a liquid level detection unit 233, an angle detection unit 237, an acceleration sensor 238, and a control unit 30.

The liquid level detection unit 233 is configured to detect a liquid level of a milk storage container portion 50. The angle detection unit 237 is configured to detect tilting data of the milk storage container portion 50. The acceleration sensor 238 is configured to detect displacement data information of the milk storage container portion 50. The control unit 30 is configured to determine whether to initiate measurement of a breast milk volume in the milk storage container portion 50 based on at least one of tilting data information from the angle detection unit 237 or displacement data information from the acceleration sensor 238, and determine the breast milk volume in the milk storage container portion 50 based on liquid level data information from the liquid level detection unit 233 and the tilting data information from the angle detection unit 237.

In some embodiments of this application, the liquid level detection unit 233 may be a through-beam sensor 234, and the angle sensor may be a gyroscope.

In some embodiments of this application, the control unit 30 is configured with a tilting threshold and a displacement threshold. When the tilting data exceeds the tilting threshold and/or the displacement data exceeds the displacement threshold, the control unit 30 does not send an instruction and thus does not initiate the measurement of the breast milk volume in the milk storage container portion 50.

When the tilting data is within the tilting threshold and the displacement data is within the displacement threshold, the control unit 30 sends an instruction to initiate the measurement of the breast milk volume in the milk storage container portion 50. In this case, the control unit 30 determines the breast milk volume in the milk storage container portion 50 based on the liquid level data information from the liquid level detection unit 233 and the tilting data information from the angle detection unit 237.

It may be understood that when the tilting data exceeds the tilting threshold, it indicates that a tilting angle of the milk storage container portion 50 exceeds a measurement limit and conditions for measuring the breast milk volume in the milk storage container portion 50 are not met. Even if the breast milk volume in the milk storage container portion 50 is measured, it is impossible to achieve intended accuracy. When the displacement data exceeds the displacement threshold, it indicates that the milk storage container portion 50 is in a moving state (when the user is in motion, the milk storage container portion 50 is caused to be in a moving state). When the milk storage container portion 50 is in a moving state, the breast milk surface in the milk storage container portion 50 is unstable, and the conditions for measuring the breast milk volume in the milk storage container portion 50 are not met. An alarm unit may be arranged. When the tilting data exceeds the tilting threshold and/or the displacement data exceeds the displacement threshold, the control unit 30 sends an instruction to the alarm unit to send an alarm, prompting the user to adjust a posture so that the tilting data is within the tilting threshold and the displacement data is within the displacement threshold. When the tilting data is within the tilting threshold and the displacement data is within the displacement threshold, the control unit 30 sends an instruction to initiate the measurement of the breast milk volume in the milk storage container portion 50. In this case, the control unit 30 determines the breast milk volume in the milk storage container portion 50 based on the liquid level data information from the liquid level detection unit 233 and the tilting data information from the angle detection unit 237.

### Implementation V

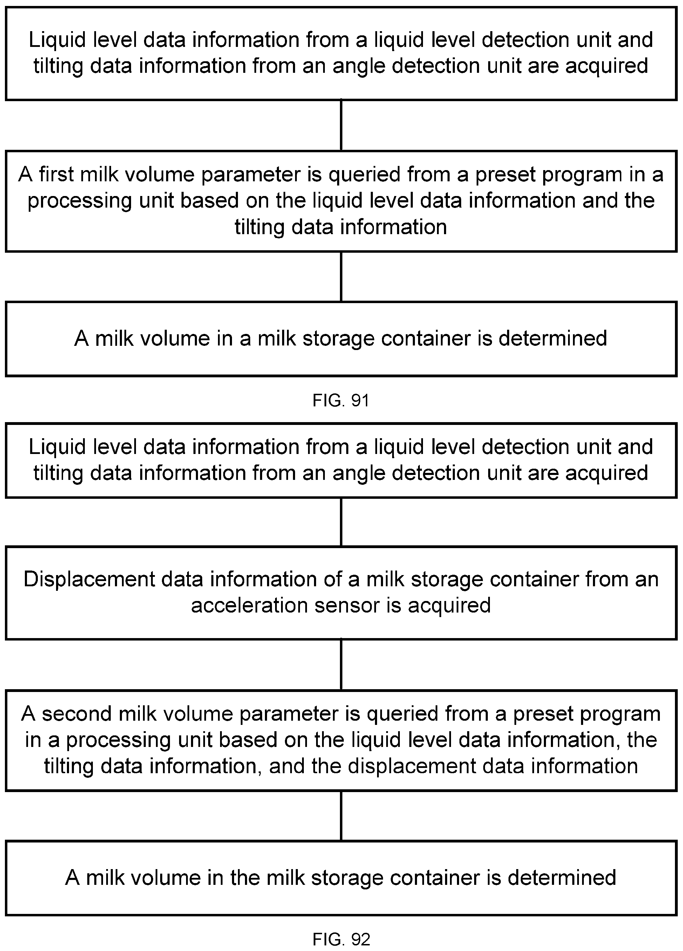

Referring to FIG. 91, in some embodiments of this application, this application provides a detection method, including following steps:
acquiring liquid level data information from the liquid level detection unit 233 and tilting data information from the angle detection unit 237; and
determining a breast milk volume in a milk storage container portion 50 based on the liquid level data information and the tilting data information.

A breast milk level in the milk storage container portion 50 is detected by the liquid level detection unit 233, and the tilting data of the milk storage container portion 50 is detected by the angle detection unit 237. A control unit 30 acquires the liquid level data information from the liquid level detection unit 233 and the tilting data information from the angle detection unit 237, and determines the breast milk volume in the milk storage container portion 50 based on the liquid level data information and the tilting data information. Therefore, during milk expressing, the breast milk volume in the milk storage container portion 50 is detected, and detection errors caused by tilting of the milk storage container portion 50 is reduced, ensuring intended detection accuracy.

In some embodiments of this application, a database may be preconfigured in the control unit 30. Content of the database includes at least breast milk level gradient data of the milk storage container portion 50, tilting gradient data of the milk storage container portion 50, and breast milk volume parameters corresponding to the liquid level data and the tilting data.

The control unit 30 acquires the liquid level data information from the liquid level detection unit 233 and the tilting data information from the angle detection unit 237, and then queries the database to obtain breast milk volume parameter information corresponding to the liquid level data information and the tilting data information, and determines the breast milk volume in the milk storage container portion 50 from the obtained breast milk volume parameter information.

In some embodiments of this application, the step of determining a breast milk volume in a milk storage container portion 50 includes:
querying a first breast milk volume parameter in a preset program within the control unit 30 based on the liquid level data information and the tilting data information.

During milk expressing, values detected by the liquid level detection unit 233 and the angle detection unit 237 may vary across different time points. Liquid level data and tilting data measured at each time point form a parameter pair, and each parameter pair corresponds to a first breast milk volume parameter. The first breast milk volume parameter is a breast milk volume corresponding to the liquid level data and the tilting data in the parameter pair. After acquiring the parameter pair formed by the liquid level data and the tilting data, the control unit 30 queries the database to obtain the first breast milk volume parameter, and then determines the breast milk volume in the milk storage container portion 50 through the first breast milk volume parameter.

Referring to FIG. 92, in some embodiments of this application, the detection method provided in this application further includes: acquiring displacement data information of the milk storage container portion 50 from an acceleration sensor 238.

The step of determining a breast milk volume in a milk storage container portion 50 includes:
querying a second breast milk volume parameter in the preset program within the control unit 30 based on the liquid level data information and the tilting data information in combination with the displacement data information.

After acquiring the liquid level data information from the liquid level detection unit 233, the tilting data information from the angle detection unit 237, and the displacement data information from the acceleration sensor 238, the control unit 30 queries the database to obtain the second breast milk volume parameter, and then determines the breast milk volume in the milk storage container portion 50 based on the second breast milk volume parameter.

In some embodiments of this application, the breast milk volume in the milk storage container portion 50 may be calculated based on an average of the first breast milk volume parameter and the second breast milk volume parameter. Calculating the average of the first breast milk volume parameter and the second breast milk volume parameter to calculate the breast milk volume in the milk storage container portion 50 can improve detection accuracy.

According to another aspect, this application further provides a computer-readable storage medium, storing computer instructions that, when run, perform the above detection method.

### Implementation VI

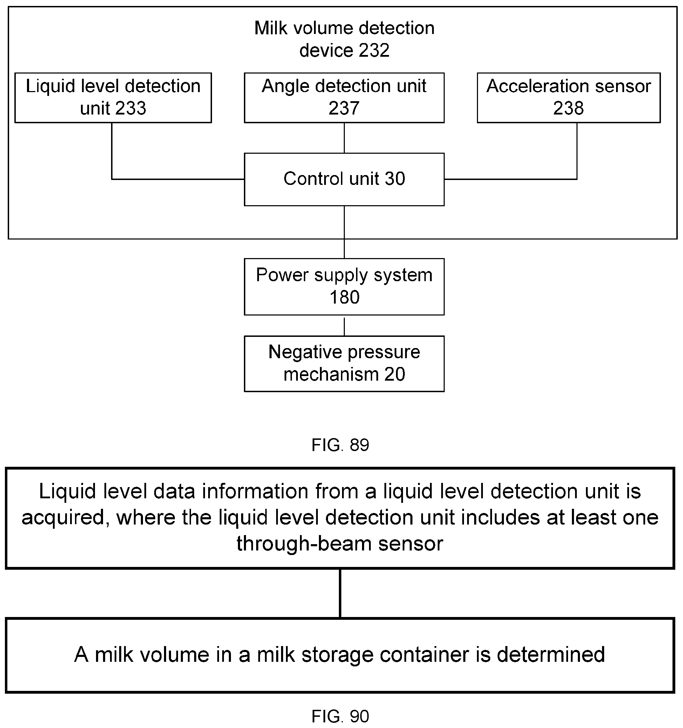

Referring to FIG. 90, in some embodiments of this application, this application provides a detection method, including following steps:
acquiring liquid level data information from a liquid level detection unit 233, where the liquid level detection unit 233 includes at least one through-beam sensor 234; and
determining a breast milk volume in a milk storage container portion 50 based on the liquid level data information.

An emitter 235 and a receiver 236 of the through-beam sensor 234 may be arranged on the side wall of the milk storage container portion 50, so that the emitter 235 and the receiver 236 of the through-beam sensor 234 remain relatively fixed with respect to the side wall of the milk storage container portion 50, and remain relatively fixed in position and orientation. This enables the receiver 236 to properly receive light rays from the emitter 235, thereby ensuring reliability and stability of the through-beam sensor 234.

The through-beam sensors 234 are arranged in sequence linearly from a minimum liquid level boundary of the milk storage container portion 50 to a maximum liquid level boundary of the milk storage container portion 50. The through-beam sensors 234 respond in ascending order of heights as a liquid level in the milk storage container portion 50 rises. The control unit 30 queries serial numbers of the through-beam sensors 234 in ascending order of heights until a through-beam sensor 234 that has not responded is found. In this case, a serial number of a last responding through-beam sensor 234, that is, a through-beam sensor 234 positioned highest among all responding through-beam sensors 234 and closest to a liquid surface in the milk storage container portion 50, is acquired. Based on the serial number of the last responding through-beam sensor 234, the control unit 30 can determine a height at which the through-beam sensor 234 is positioned, so as to obtain the liquid level in the milk storage container portion 50 through the through-beam sensor 234, thereby determining the breast milk volume in the milk storage container portion 50 by querying the database based on the obtained liquid level data.

According to another aspect, this application further provides a computer-readable storage medium, storing computer instructions that, when run, perform the above detection method.

### Implementation VII

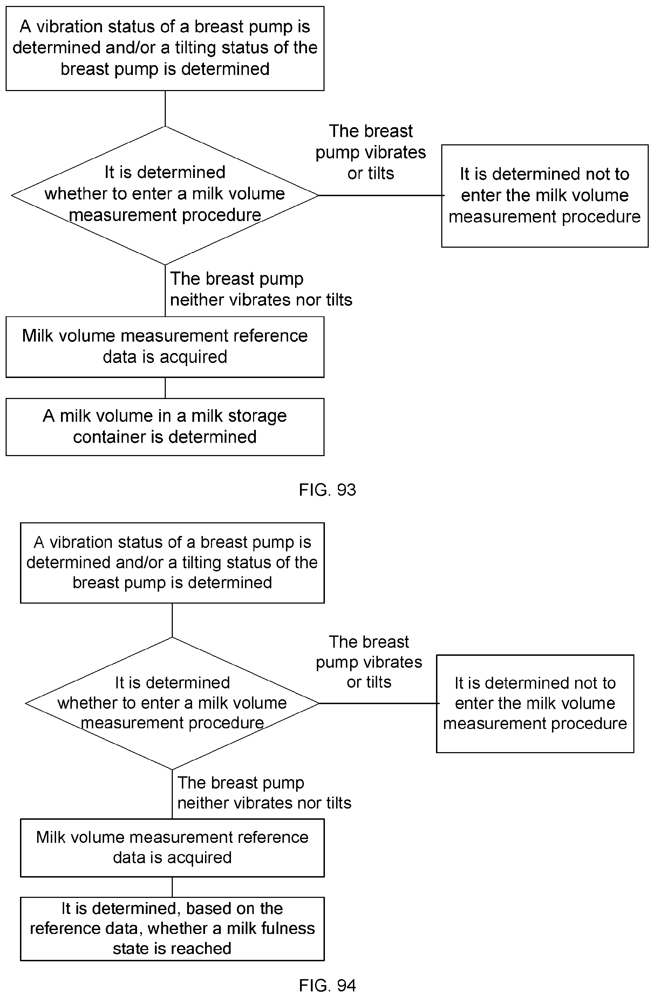

Referring to FIG. 93, in some embodiments of this application, this application provides a detection method, including following steps:
determining whether to enter a breast milk volume measurement procedure;
acquiring breast milk volume measurement reference data; and
determining a current breast milk volume.

In some embodiments of this application, the step of determining whether to enter a breast milk volume measurement procedure includes following steps:
determining a vibration status of a breast pump 1 and/or determining a tilting status of the breast pump 1.

In some embodiments of this application, the reference data includes liquid level data and/or tilting data.

In some embodiments of this application, the liquid level data is from a through-beam sensor 234, and the tilting data is from a gyroscope.

If a tilting angle and a motion status of a milk storage container portion 50 do not meet a breast milk volume measurement condition, it indicates that the tilting angle of the milk storage container portion 50 is excessively large and/or a liquid level in the milk storage container portion 50 is excessively fluctuating, making it difficult to achieve intended measurement accuracy.

The breast milk volume measurement procedure is initiated when the tilting angle and the motion status of the milk storage container portion 50 meet the breast milk volume measurement condition.

The liquid level data of the milk storage container portion 50 is detected by the through-beam sensor 234, and the tilting data of the milk storage container portion 50 is detected by the gyroscope, thereby completing acquisition of reference data for breast milk volume measurement. Furthermore, a current breast milk volume in the milk storage container portion 50 is determined based on at least one of the liquid level data or the tilting data.

Since a shape and a dimension of the milk storage container portion 50 vary across different models of breast pumps 1, the breast milk storage capacity of the milk storage container portion 50 is calibrated before the breast pump 1 leaves the factory. A breast milk storage capacity of the milk storage container portion 50 corresponding to each group of parameter gradients (for example, a group of parameter gradients including a breast milk level gradient of the milk storage container portion 50 and a tilting data gradient of the milk storage container portion 50) is measured and recorded, to form the breast milk volume parameters.

During actual use, the breast milk level in the milk storage container portion 50 is detected by the liquid level detection unit 233, and the tilting data of the milk storage container portion 50 is detected by the angle detection unit 237. The control unit 30 acquires the liquid level data information from the liquid level detection unit 233 and the tilting data information from the angle detection unit 237, and then queries the database to obtain breast milk volume parameter information based on the liquid level data information and the tilting data information, and determines the breast milk volume in the milk storage container portion 50.

According to another aspect, this application further provides a computer-readable storage medium, storing computer instructions that, when run, perform the above detection method.

### Implementation VIII

Referring to FIG. 85 to FIG. 89, in some embodiments of this application, this application provides a breast pump 1, which is configured to pump milk from a breast and store the breast milk in a milk storage container portion 50, and includes a breast shield 10, a breast milk flow rate detection unit 232, a negative pressure mechanism 20, the milk storage container portion 50, and a power supply system 230. The breast shield 10 is configured to be attached to the breast and accommodate at least a nipple. The breast milk flow rate detection unit 232 is configured to detect a breast milk volume expressed by the breast pump 1. The negative pressure mechanism 20 is configured to directly or indirectly apply negative pressure to the breast shield 10 to pump milk from the breast. The milk storage container portion 50 is in fluid communication with the breast shield 10 and can store the milk expressed from the breast. A liquid level detection unit 233 is arranged on the milk storage container portion 50. The power supply system 230 is configured to at least supply power to the negative pressure mechanism 20.

During milk expressing by using the breast pump 1, the breast shield 10 is placed over a breast of a user, and a nipple of the breast is accommodated in the breast shield 10, forming a sealed space, so that negative pressure can be generated around the nipple. The power supply system 230 provides electrical energy to cause the negative pressure mechanism 20 to operate, thereby generating negative pressure to perform milk expressing. During breast milk expressing, breast milk enters the milk storage container portion 50, which holds the expressed breast milk. The liquid level detection unit 233 detects a liquid level in the milk storage container portion 50, and then determines a breast milk volume in the milk storage container portion 50.

In some embodiments of this application, the milk storage container portion 50 may be a milk bowl or a milk bottle. The liquid level detection unit 233 may be a through-beam sensor 234. An emitter 235 and a receiver 236 of the through-beam sensor 234 may be respectively arranged on the outer sides of side walls on two sides of the milk storage container portion 50. Regions of the milk storage container portion 50 where the emitter 235 and the receiver 236 are mounted may be made transparent to ensure proper functioning of the through-beam sensor 234.

In the negative pressure mechanism 20, a power structure configured to generate negative pressure includes but is not limited to a piezoelectric pump, a diaphragm pump, a hydraulic pump, or a mechanical pump. The negative pressure mechanism 20 may directly generate negative pressure in the breast shield 10 through communication with the breast shield 10, or may indirectly generate negative pressure in the breast shield 10 by transmitting the negative pressure to a liquid barrier such as a diaphragm or an airbag. The breast milk is drawn into the milk storage container portion 50 for storage through the negative pressure.

The fluid communication between the breast shield 10 and the milk storage container portion 50 may be either direct communication or communication formed through a tube or other flow paths.

In some embodiments of this application, the breast pump 1 provided in this application further includes a housing 231. The negative pressure mechanism 20 is at least partially arranged in the housing 231. The breast shield 10 and the milk storage container portion 50 are respectively connected to the housing 231 to form an integral wearable unit, or the breast shield 10 and the milk storage container portion 50 are connected to each other and then connected as a whole to the housing 231 to form an integral wearable unit. The wearable unit, or at least part of the wearable unit, may be worn within a shoulder strap. The housing 231 may further includes components such as a battery, a negative pressure air passage, a circuit board, and a solenoid valve therein.

In some embodiments of this application, the breast pump 1 provided in this application further includes a housing 231. The negative pressure mechanism 20 is at least partially arranged in the housing 231. The breast shield 10 and the milk storage container portion 50 are connected to form an integral wearable unit, and the negative pressure system is at least partially separated from the wearable unit. The wearable unit, or at least part of the wearable unit, may be worn within a shoulder strap. The housing 231 may further includes components such as a battery, a negative pressure air passage, a circuit board, and a solenoid valve therein.

In some embodiments of this application, the breast pump 1 provided in this application further includes an angle detection unit 237 and/or an acceleration sensor 238 arranged on the wearable unit.

It may be understood that the breast pump 1 includes at least a main unit, a breast shield 10, and a milk storage container portion 50. The main unit includes a negative pressure mechanism 20, a power supply system 230, a circuit board, a control panel, and an air passage system, forming a body of the breast pump 1. The breast shield 10 is attached to the breast of the user during use, so that a breast region, including the nipple, can be sucked by negative pressure for milk expressing, and expressed breast milk is initially drained through the breast shield 10. The milk storage container portion 50 is configured to collect and store the expressed breast milk, so that the breast pump 1 can pump an intended volume of breast milk in a single operation and that the breast pump 1 can operate continuously within an intended duration. The negative pressure mechanism 20 may be connected to the breast shield 10 directly or indirectly through an air conduit to provide negative pressure to the breast shield 10 through the negative pressure mechanism 20. The breast shield 10 may be connected to the milk storage container portion 50 directly or indirectly through a liquid conduit, so that the expressed breast milk can flow from the breast shield 10 into the milk storage container portion 50.

In some embodiments of this application, the main unit, the breast shield 10, and the milk storage container portion 50 may be integrated. The negative pressure mechanism 20 may be directly in airflow communication to the breast shield 10 to reduce a volume of the breast pump 1. The breast shield 10 may be directly in fluid communication with the milk storage container portion 50 to reduce the volume of the breast pump 1. In this case, the liquid level detection unit 233 is arranged on the milk storage container portion 50 to detect liquid level data of the milk storage container portion 50. The angle detection unit 237 may be arranged on the main unit, or may be arranged on the breast shield 10, or may be arranged on the milk storage container portion 50, as long as the angle detection unit 237 can effectively detect tilting data of the milk storage container portion 50 merely by rotating synchronously with the milk storage container portion 50. The acceleration sensor 238 may be arranged on the main unit, or may be arranged on the breast shield 10, or may be arranged on the milk storage container portion 50, as long as the acceleration sensor 238 can effectively detect displacement data of the milk storage container portion 50 merely by moving synchronously with the milk storage container portion 50.

In some embodiments of this application, the breast shield 10 and the milk storage container portion 50 may form an integral wearable unit, and the main unit may be arranged separately from the wearable unit to facilitate operation of the breast pump 1. The negative pressure mechanism 20 may be in airflow communication with the breast shield 10 through an air conduit to provide negative pressure to the breast shield 10. The breast shield 10 may be directly in liquid flow communication with the milk storage container portion 50 to reduce a volume of the wearable unit. In this case, the liquid level detection unit 233 is arranged on the milk storage container portion 50 to detect liquid level data of the milk storage container portion 50. The angle detection unit 237 may be arranged on the breast shield 10, or may be arranged on the milk storage container portion 50, as long as the angle detection unit 237 can effectively detect tilting data of the milk storage container portion 50 merely by rotating synchronously with the milk storage container portion 50. The acceleration sensor 238 may be arranged on the breast shield 10, or may be arranged on the milk storage container portion 50, as long as the acceleration sensor 238 can effectively detect displacement data of the milk storage container portion 50 merely by moving synchronously with the milk storage container portion 50.

In some embodiments of this application, the breast shield 10 may separately form a wearable unit, and the main unit may be integrated with the milk storage container portion 50 to form an integral body unit, so as to facilitate operation of the breast pump 1. The negative pressure mechanism 20 may be in airflow communication with the breast shield 10 through an air conduit to provide negative pressure to the breast shield 10. The breast shield 10 may be in fluid communication with the milk storage container portion 50 through a liquid conduit, to collect breast milk from the breast shield 10 in the milk storage container portion 50. In this case, the liquid level detection unit 233 is arranged on the milk storage container portion 50 to detect liquid level data of the milk storage container portion 50. The angle detection unit 237 may be arranged on the main unit, or may be arranged on the milk storage container portion 50, as long as the angle detection unit 237 can effectively detect tilting data of the milk storage container portion 50 merely by rotating synchronously with the milk storage container portion 50. The acceleration sensor 238 may be arranged on the main unit, or may be arranged on the milk storage container portion 50, as long as the acceleration sensor 238 can effectively detect displacement data of the milk storage container portion 50 merely by moving synchronously with the milk storage container portion 50.

In some embodiments of this application, the breast shield 10 may separately form a wearable unit. The main unit may be arranged separately from the wearable unit to facilitate operation of the breast pump 1. The milk storage container portion 50 may be arranged separately from the main unit and the breast shield 10 to facilitate measurement of a breast milk volume in the milk storage container portion 50 and to reduce a volume of the wearable unit. The negative pressure mechanism 20 may be in airflow communication with the breast shield 10 through an air conduit to provide negative pressure to the breast shield 10. The breast shield 10 may be in fluid communication with the milk storage container portion 50 through a liquid conduit, to collect breast milk from the breast shield 10 in the milk storage container portion 50. In this case, the liquid level detection unit 233 is arranged on the milk storage container portion 50 to detect liquid level data of the milk storage container portion 50. The angle detection unit 237 may be arranged on the milk storage container portion 50, as long as the angle detection unit 237 can effectively detect tilting data of the milk storage container portion 50 merely by rotating synchronously with the milk storage container portion 50. The acceleration sensor 238 may be arranged on the milk storage container portion 50, as long as the acceleration sensor 238 can effectively detect displacement data of the milk storage container portion 50 merely by moving synchronously with the milk storage container portion 50.

In some embodiments of this application, the negative pressure mechanism 20 may be arranged separately from the main unit, and the negative pressure mechanism 20 may form a wearable unit with the breast shield 10. The main unit may form an integral body unit with the milk storage container portion 50, to facilitate operation of the breast pump 1 and to reduce a volume of the wearable unit. The negative pressure mechanism 20 may be directly in airflow communication with the breast shield 10, to provide negative pressure to the breast shield 10 and to ensure stability of the negative pressure at the breast shield 10. The negative pressure mechanism 20 may be electrically connected to the main unit through a wire, so that the main unit can control the negative pressure mechanism 20. The breast shield 10 may be in fluid communication with the milk storage container portion 50 through a liquid conduit, to collect breast milk from the breast shield 10 in the milk storage container portion 50. In this case, the liquid level detection unit 233 is arranged on the milk storage container portion 50 to detect liquid level data of the milk storage container portion 50. The angle detection unit 237 may be arranged on the main unit, or may be arranged on the milk storage container portion 50, as long as the angle detection unit 237 can effectively detect tilting data of the milk storage container portion 50 merely by rotating synchronously with the milk storage container portion 50. The acceleration sensor 238 may be arranged on the main unit, or may be arranged on the milk storage container portion 50, as long as the acceleration sensor 238 can effectively detect displacement data of the milk storage container portion 50 merely by moving synchronously with the milk storage container portion 50.

### Implementation IX

Referring to FIG. 85 to FIG. 89, in some embodiments of this application, this application provides a milk-fullness detection device, which is applied to a breast pump 1 and includes a liquid level detection unit 233 and a control unit 30. The liquid level detection unit 233 is a through-beam sensor 234, and the liquid level detection unit 233 is arranged at or just below a maximum liquid level boundary of the milk storage container portion 50. The control unit 30 is configured to control operation of the breast pump 1 based on liquid level data information from the liquid level detection unit 233.

The liquid level detection unit 233 may be arranged at the maximum liquid level boundary of the milk storage container portion 50, and a liquid level in the milk storage container portion 50 is detected by the liquid level detection unit 233. When the liquid level in the milk storage container portion 50 reaches the maximum liquid level boundary of the milk storage container portion 50, the liquid level detection unit 233 responds and feeds back a response signal to the control unit 30. The control unit 30 determines that the milk storage container portion 50 reaches milk fullness based on the response signal from the liquid level detection unit 233. The control unit 30 controls the operation of the breast pump 1 based on the liquid level data information from the liquid level detection unit 233. In this way, the breast pump 1 stops milk expressing or sends an alarm signal to prompt a user that the milk storage container portion 50 reaches milk fullness.

The liquid level detection unit 233 may be mounted just below the maximum liquid level boundary of the milk storage container portion 50 to detect the liquid level in the milk storage container portion 50. When the liquid level in the milk storage container portion 50 reaches a vicinity below the maximum liquid level boundary of the milk storage container portion 50, the liquid level detection unit 233 responds and feeds back a response signal to the control unit 30. The control unit 30 determines that the milk storage container portion 50 reaches nearly milk fullness based on the response signal from the liquid level detection unit 233. The control unit 30 controls the operation of the breast pump 1 based on the liquid level data information from the liquid level detection unit 233. In this way, the breast pump 1 can slow down the milk expressing, and continue operating at the reduced speed for a fixed duration to cause the milk storage container portion 50 to reach a milk fullness state, or can send an alarm signal to prompt the user that the milk storage container portion 50 reaches a nearly milk fullness state and that the user needs to adjust a posture and a motion status to prevent breast milk spillage.

In some embodiments of this application, the milk fullness detection device provided in this application further includes an angle detection unit 237 and/or an acceleration sensor 238. The angle detection unit 237 is configured to detect tilting data of the milk storage container portion 50, and the acceleration sensor 238 is configured to detect displacement data information of the milk storage container portion 50. The control unit 30 is configured to control the operation of the breast pump 1 based on at least one of the liquid level data information from the liquid level detection unit 233, tilting data information from the angle detection unit 237, or the displacement data information from the acceleration sensor 238.

In some embodiments of this application, the milk fullness detection device provided in this application further includes an alarm unit connected to the control unit 30. The control unit 30 is configured to control the alarm unit to operate based on the liquid level data information from the liquid level detection unit 233.

The angle detection unit 237 detects the tilting data of the milk storage container portion 50. If the tilting data exceeds a tilting threshold and the liquid level detection unit 233 responds, it indicates that breast milk in the milk storage container portion 50 reaches the maximum liquid level boundary of the milk storage container portion 50 due to tilting of the milk storage container portion 50. In this case, the liquid level in the milk storage container portion 50 reaching the maximum liquid level boundary is not due to milk fullness. In this case, the breast pump 1 may be paused, and an alarm signal may be sent to prompt the user to adjust a tilting angle of the milk storage container portion 50 within the tilting threshold. Measures the user can take include, but are not limited to, adjusting a posture.

The acceleration sensor 238 detects displacement data of the milk storage container portion 50, where the displacement data includes a displacement speed, a displacement acceleration, and a displacement direction. Comprehensive displacement data is fitted based on various pieces of displacement data information. When the comprehensive displacement data exceeds a displacement threshold, the breast pump 1 is controlled to stop operating, or an alarm is sent to prompt the user to reduce a range of motion to prevent breast milk from spilling from the milk storage container portion 50.

The angle detection unit 237 detects the tilting data of the milk storage container portion 50, and the acceleration sensor 238 detects the displacement data of the milk storage container portion 50. When the tilting data is within the tilting threshold and the displacement data is within the displacement threshold, the liquid level detection unit 233 responds. In this case, the liquid level in the milk storage container portion 50 reaches the maximum liquid level boundary of the milk storage container portion 50, and the liquid level detection unit 233 feeds back a response signal to the control unit 30. The control unit 30 determines that the milk storage container portion 50 reaches milk fullness based on the response signal from the liquid level detection unit 233. The control unit 30 controls the operation of the breast pump 1 based on the liquid level data information from the liquid level detection unit 233. In this way, the breast pump 1 stops milk expressing or sends an alarm signal to prompt a user that the milk storage container portion 50 reaches milk fullness.

### Implementation X

Referring to FIG. 94, in some embodiments of this application, this application provides a detection method, including following steps:
acquiring breast milk volume detection reference data; and
determining a milk fullness status based on the reference data.

In some embodiments of this application, the reference data includes liquid level data and tilting data. If the liquid level data reaches a liquid level threshold and the tilting data is less than a tilting threshold, milk fullness is determined.

In some embodiments of this application, the liquid level data is from a through-beam sensor 234, and the tilting data is from a gyroscope.

In some embodiments of this application, before the step of acquiring breast milk volume detection reference data, the detection method further includes: determining whether to enter a breast milk volume measurement procedure.

In some embodiments of this application, the step of determining whether to enter a breast milk volume measurement procedure includes a following step: determining a vibration status of a breast pump 1 and/or determining a tilting status of the breast pump 1.

A liquid level in a milk storage container portion 50 may be detected by a liquid level detection unit 233 such as a through-beam sensor 234, and then a milk fullness status of the milk storage container portion 50 may be determined based on the liquid level data. When the liquid level data detected by the liquid level detection unit 233 indicates that the liquid level in the milk storage container portion 50 reaches a maximum liquid level boundary of the milk storage container portion 50, it may be determined that the milk storage container portion 50 reaches milk fullness.

The angle detection unit 237 detects the tilting data of the milk storage container portion 50. If the tilting data exceeds a tilting threshold and the liquid level detection unit 233 responds, it indicates that breast milk in the milk storage container portion 50 reaches the maximum liquid level boundary of the milk storage container portion 50 due to tilting of the milk storage container portion 50. In this case, the liquid level in the milk storage container portion 50 reaching the maximum liquid level boundary is not due to milk fullness. In this case, the breast pump 1 may be paused, and an alarm signal may be sent to prompt the user to adjust a tilting angle of the milk storage container portion 50 within the tilting threshold. Measures the user can take include, but are not limited to, adjusting a posture.

Displacement data information of the milk storage container portion 50 may be detected by an acceleration sensor 238, and the vibration status of the breast pump 1 may be evaluated based on the displacement data information. Tilting data of the milk storage container portion 50 may be detected by an angle detection unit 237 such as a gyroscope, and a tilting status of the breast pump 1 is determined based on the tilting data. When the breast pump 1 is in at least one of a vibrating state or a tilted state, the breast milk volume measurement procedure is not initiated. When the breast pump 1 is neither in a tilted state nor in a vibrating state, the breast milk volume measurement procedure is initiated, to achieve more precise breast milk volume measurement and ensure an intended measurement result.

According to another aspect, this application further provides a computer-readable storage medium, storing computer instructions that, when run, perform the above detection method.

### Implementation XI

In some embodiments of this application, this application further provides a detection method, including following steps:
acquiring liquid level data for breast milk volume detection; and
determining a milk spillage status based on the liquid level data.

In some embodiments of this application, before the step of determining the milk spillage status based on the liquid level data, the detection method further includes a following step:
acquiring tilting data for breast milk volume detection.

In some embodiments of this application, the liquid level data is from a through-beam sensor, and the tilting data is from a gyroscope.

A liquid level in a milk storage container portion 50 may be detected by a liquid level detection unit 233 such as a through-beam sensor 234, and then it may be determined based on the liquid level data whether the liquid level in the milk storage container portion 50 reaches a maximum liquid level boundary. When the liquid level data detected by the liquid level detection unit 233 indicates that the liquid level in the milk storage container portion 50 reaches a maximum liquid level boundary of the milk storage container portion 50, it may be determined that the milk storage container portion 50 reaches milk spillage.

The angle detection unit 237 detects the tilting data of the milk storage container portion 50. If the tilting data exceeds a tilting threshold and the liquid level detection unit 233 responds, it indicates that breast milk in the milk storage container portion 50 reaches the maximum liquid level boundary of the milk storage container portion 50 due to tilting of the milk storage container portion 50. In this case, the liquid level in the milk storage container portion 50 reaching the maximum liquid level boundary is not due to milk fullness. In this case, the breast pump 1 may be paused, and an alarm signal may be sent to prompt a user to adjust a tilting angle of the milk storage container portion 50 within the tilting threshold. Measures the user can take include, but are not limited to, adjusting a posture.

The liquid level detection unit 233 in this application may alternatively be an independent unit module that may be detachably mounted to the milk storage container portion 50, so that the user can determine whether to mount the liquid level detection unit 233 and perform detection based on personal needs.

### Sixteenth embodiment

In some implementations, a breast pump 1 may include an expressing assembly, a detection unit 40, and a control unit 30.

The expressing assembly is configured to pump milk from a breast of a user.

The detection unit 40 is arranged on the expressing assembly, and may be configured to receive a light ray reflected during breast milk expressing and generate a detection signal corresponding to a light intensity of the light ray.

The control unit 30 may be electrically connected to the detection unit 40, and may be configured to determine a breast milk flow rate based on the light intensity corresponding to the detection signal.

Specifically, during milk expressing by the expressing assembly from the breast of the user, the detection unit 40 may obtain light reflected from the breast milk and generate a detection signal used to represent the intensity of the light reflected during breast milk expressing. Therefore, the control unit 30 may determine a breast milk flow rate corresponding to the light intensity based on a preset mapping relationship between light intensity and a breast milk flow rate.

In some examples, since light reflected from a breast milk surface attenuates along a transmission path, leading to deviations in a detected milk flow rate, a position of the detection unit in the breast pump in the breast pump may be set to improve accuracy of the obtained milk flow rate.

For example, the expressing assembly in the example of this specification may have a transparent side wall. The light reflected from the breast milk may be transmitted through the transparent side wall to the detection unit, so that the detection unit can be configured to receive a light ray from the transparent side wall during breast milk expressing.

That is, designing the expressing assembly with a transparent side wall can reduce attenuation of the light reflected from the breast milk surface along the transmission path, and can achieve a more accurate representation of the breast milk flow rate. Therefore, accuracy of the obtained detection signal can be improved.

For another example, the detection unit may be arranged on a breast milk flow path.

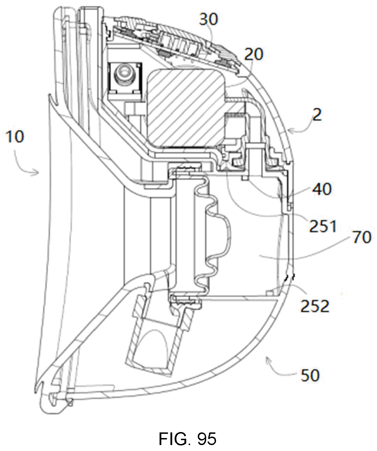

As an example, refer to a schematic diagram of a specific structure of a breast pump in FIG. 95. The expressing assembly may include a breast pump body and a milk storage container portion 50. The breast pump body may include a breast shield 10 and a negative pressure mechanism 20 arranged on the breast shield, where the negative pressure mechanism 20 may be configured to generate negative pressure; the breast shield 10 may contact the breast of the user and pump breast milk under the negative pressure; and during the milk expressing, the negative pressure mechanism 20 can prevent breast milk from flowing back to the breast, ensuring that the breast milk is smoothly expressed.

The milk storage container portion 50 may be detachably connected to the breast pump body (for example, the breast shield 10), and may be configured to store the breast milk expressed by the breast shield 10.

Specifically, when the breast shield 10 contacts the breast of the user, the negative pressure mechanism 20 can be driven to operate, generating negative pressure. Under the negative pressure, the breast shield 10 can pump breast milk from the breast and cause the breast milk to flow into the milk storage container portion 50 through a corresponding tube, so that the milk storage container portion 50 stores the expressed breast milk.

When a current expressing task is completed or a breast milk volume reaches a breast milk volume threshold, the negative pressure mechanism 20 stops operating, and the negative pressure environment disappears. In this case, the breast shield 10 no longer pumps milk from the breast. In this case, the milk storage container portion may be dismounted for feeding operations, or the breast milk in the milk storage container portion may be stored in other ways.

In some implementations, the breast shield 10 includes a flange and a nipple channel. Since the flange is a part that directly contacts the nipple and the breast, the flange may made of soft, non-toxic, and non-irritating materials to reduce friction and irritation to the nipple and the breast.

In some examples of this specification, the milk storage container portion may be a milk bowl or other containers matching a main body of the breast pump for storing liquids. For example, the milk storage container portion may alternatively be a bottle made of glass.

In some examples, the breast pump 1 may further include a main unit 2 that may be configured to control generation of a suction force, a magnitude of the suction force, a milk expressing speed, and a milk expressing duration as well as to record milk expressing data and the like.

In some optional examples, the main unit 2 includes a negative pressure mechanism 20 configured to generate negative pressure through expressing and a control unit 30 configured to control the negative pressure mechanism 20. The negative pressure mechanism 20 is configured to generate a suction force on the breast of the user through the breast shield 10, pump breast milk, and discharge the breast milk into the milk storage container portion 50. The breast shield 10 is configured to connect to the milk storage container portion 50 and fasten the milk storage container portion 50.

In some optional examples, the detection unit 40 may be arranged on or near the breast milk flow path of the expressing assembly, where the breast milk flow path includes a passage from the breast shield to the milk storage container portion.

For example, as shown in FIG. 95, a detection unit 40 is arranged between the breast shield 10 and the milk storage container portion 50. When breast milk flows from the breast shield 10 into the milk storage container portion 50, the detection unit 40 can detect the breast milk, and then can determine a breast milk flow rate.

Arranging the detection unit on or near the breast milk flow path of the expressing assembly can reduce a degree of attenuation of the light during transmission, further improving the accuracy of the obtained detection signal.

It should be noted that the breast pump provided in this embodiment of this specification may further include other components, such as a diaphragm support and a tube. To facilitate the description of the technical solutions in this specification and to highlight innovative aspects, components of the breast pump that may be implemented by the related art are omitted in this specification.

For example, in some examples, the breast pump may further include a display screen. The display screen may be arranged on the breast pump body and electrically connected to a breast milk flow rate detection unit, and is configured to display a breast milk volume detected by the breast milk flow rate detection unit.

It may be understood that the overall operation of the breast pump 1 in this embodiment is controlled by the control unit 30 in the main unit 2. The control unit 30 may be a CPU, an ASIC, or one or more integrated circuits configured to implement the embodiments of the present disclosure. This is not limited herein.

In some optional examples, the processor can simultaneously control the negative pressure generation process and the breast milk flow rate detection process, which can reduce an overall volume of the breast pump.

In some examples, the detection unit may be a component or a device that can detect reflected light.

As an example, the detection unit may include an illuminance sensor. The illuminance sensor is a component based on a photoelectric effect, and can convert optical signals into electrical signals. Moreover, the illuminance sensor has high sensitivity to light, which can more accurately acquire an intensity of light reflected during breast milk expressing, thereby improving the accuracy of the obtained breast milk flow rate.

In some other examples, the detection unit may be other components that can sense light. A specific type of the detection unit is not limited in this embodiment of this specification.

In some implementations, different light intensities indicate different breast milk flow rates during breast milk expressing. That is, a correspondence may exist between a light intensity and a breast milk flow rate. Once the light intensity corresponding to the detection signal is determined, the breast milk flow rate can be determined.

As an example, the control unit may be configured to determine a breast milk flow rate corresponding to the light intensity based on the light intensity corresponding to the detection signal and a preset mapping relationship between a light intensity and a breast milk flow rate.

Specifically, when a detection signal is obtained, a light intensity corresponding to the detection signal may be determined. Then a breast milk flow rate corresponding to the light intensity may be automatically determined based on the preset mapping relationship between a light intensity and a breast milk flow rate. The entire process requires no manual intervention, improving calculation efficiency and the accuracy of the calculated breast milk flow rate, thereby improving accuracy of an obtained current breast milk volume in the breast pump.

In some examples, the control unit may be implemented by processing chips such as CPU, a field programmable gate array (FPGA), or a micro controller unit (MCU). The processing may be implemented by an ASIC or by one or more integrated circuits configured to implement the embodiments of this specification.

In some embodiments, the mapping relationship between a light intensity and a breast milk flow rate may be obtained in a following manner:

During breast milk expressing, under otherwise identical conditions, breast milk flow rates at different moments are acquired, and intensities of light reflected from a breast milk surface at different moments are acquired. That is, when a breast milk flow rate is acquired, a corresponding light intensity is acquired. A proper calculation model may be utilized, to establish a mapping relationship between a light intensity and a breast milk flow rate.

In some optional examples, to further improve the accuracy of breast milk flow rate detection, the structure of the breast pump may be further expanded.

For example, still referring to FIG. 95, the breast pump 1 may further include a detection unit 40, which includes a first detection element 251 and a second detection element 252. The first detection element 251 may be configured to generate a first signal when the main unit 2 is not properly mounted, and trigger the second detection element 252 to generate a second signal when the breast shield 10 is not properly mounted. The control unit 30 may be further configured to detect the first signal and the second signal, and automatically generate prompt information and/or stop milk expressing and/or automatically turn off the main unit 2.

In some examples, the second detection element 252 may include a pressure sensor, which may be arranged on the negative pressure mechanism 20 between the milk storage container portion 50 and the breast shield 10. The purpose is to prompt the user in a timely manner and automatically activate a response mechanism when the breast shield 10 is not properly mounted on or deviates on the milk storage container portion 50 or when the breast shield 10 is directly removed from the milk storage container portion 50 during use.

Specifically, during mounting, the pressure sensor is arranged on the milk storage container portion 50, and is located in an internal cavity 70 formed between the milk storage container portion 50 and the negative pressure mechanism 20.

This specification further provides a detection method corresponding to the above breast pump. A detailed description is made below by using specific examples with reference to the drawings.

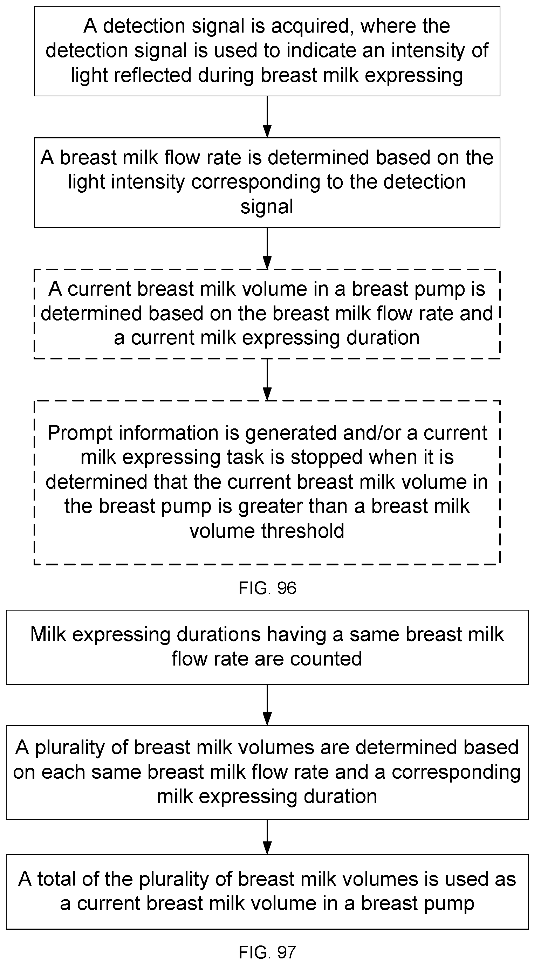

Referring to a flowchart of a detection method in an embodiment of this specification shown in FIG. 96, in some embodiments of this specification, the detection method may be applied to a breast pump. The breast pump may be the breast pump in any one of the above embodiments.

As shown in FIG. 96, the detection method includes a first step to a fourth step.

First step: A detection signal is acquired, where the detection signal is used to represent an intensity of light reflected during breast milk expressing.

Specifically, during breast milk expressing, when light is incident on breast milk, the light can be reflected by the breast milk and received by a corresponding detection unit. The light reflected from the breast milk can then be processed to obtain a light intensity corresponding to the reflected light, thereby acquiring a detection signal used to represent the intensity of the light reflected during breast milk expressing.

In some examples, the light reflected during breast milk expressing may be natural light or light that meets preset parameters (such as a wavelength or an intensity). A type of the light is not limited in this embodiment of this specification.

Second step: A breast milk flow rate is determined based on the light intensity corresponding to the detection signal.

Specifically, different light intensities indicate different breast milk flow rates during breast milk expressing. That is, a correspondence may exist between a light intensity and a breast milk flow rate. Once the light intensity corresponding to the detection signal is determined, the breast milk flow rate can be determined.

According to the milk detection method in the above example, the breast milk flow rate can be determined based on the detection signal representing the intensity of the light reflected during breast milk expressing. The entire process requires no manual intervention and can achieve automatic determination of a current breast milk flow rate in the breast pump.

During breast milk expressing, the breast milk flow rate is not entirely consistent. For example, in an initial stage, the breast milk flow rate is relatively high, but then gradually decreases over time. If the breast milk volume in the breast pump is calculated directly based on the breast milk flow rate and a total milk expressing duration, accurate results may not be obtained.

During actual use, the inventors found that during breast milk expressing, different breast milk flow rates correspond to different intensities of reflected light. Therefore, a breast milk flow rate may be determined based on a light intensity. Then a current breast milk volume in the breast pump may be calculated based on the breast milk flow rate and a corresponding milk expressing duration.

As an optional example, when a detection signal used to represent an intensity of light reflected during breast milk expressing is obtained, a breast milk flow rate corresponding to the light intensity may be determined based on the light intensity corresponding to the detection signal and the preset mapping relationship between a light intensity and a breast milk flow rate.

Specifically, when a detection signal is obtained, a light intensity corresponding to the detection signal may be determined. Then a breast milk flow rate corresponding to the light intensity may be automatically determined based on the preset mapping relationship between a light intensity and a breast milk flow rate. The entire process requires no manual intervention, improving calculation efficiency and the accuracy of the calculated breast milk flow rate, thereby improving accuracy of an obtained current breast milk volume in the breast pump.

In some embodiments, the mapping relationship between a light intensity and a breast milk flow rate may be obtained in a following manner:

During breast milk expressing, under otherwise identical conditions, breast milk flow rates at different moments are acquired, and intensities of light reflected from a breast milk surface at different moments are acquired. That is, when a breast milk flow rate is acquired, a corresponding light intensity is acquired. A proper calculation model may be utilized, to establish a mapping relationship between a light intensity and a breast milk flow rate.

In some examples, acquiring only the breast milk flow rate may not be sufficient to determine the current breast milk volume in the breast pump. In this case, breast milk may spill from the breast pump. Therefore, the current breast milk volume in the breast pump needs to be further determined.

Based on the above, still referring to FIG. 96, the detection method in this embodiment of this specification may further include a following step:

Third step: A current breast milk volume in the breast pump is determined based on the breast milk flow rate and a current milk expressing duration.

Specifically, the breast milk flow rate can indicate a rate at which breast milk is expressed, and the current milk expressing duration can indicate a duration of the breast milk expressing. Therefore, the current breast milk volume in the breast pump may be determined based on the breast milk flow rate and the current milk expressing duration.

In some examples of this specification, after the breast milk flow rate and the current milk expressing duration are determined, the current breast milk volume in the breast pump may be determined in various calculation manners. Two optional examples are provided below.

Refer to a flowchart of a method for determining a current breast milk volume in a breast pump in an embodiment of this specification in FIG. 97. As shown in FIG. 97, the third step includes following substeps:
(1). Milk expressing durations having a same breast milk flow rate are counted.

Specifically, during breast milk expressing, the breast milk flow rate is not entirely consistent. For a same breast milk flow rate, corresponding milk expressing durations may be accumulated.

For example, during a single breast milk expressing operation, a milk expressing duration may be T1. Breast milk flow rates within this period may be classified into v11, v12, and v13, where an accumulated milk expressing duration corresponding to v11 is t11, an accumulated milk expressing duration corresponding to v12 is t12, and an accumulated milk expressing duration corresponding to v13 is t13, and T1=t11+t12+t13.

(2). A plurality of breast milk volumes are determined based on each same breast milk flow rate and a corresponding milk expressing duration.

As an example, a breast milk flow rate may be multiplied by a corresponding milk expressing duration to obtain a breast milk volume.

For instance, a breast milk volume corresponding to the flow rate of v11 is V11=v11*t11, a breast milk volume corresponding to the flow rate of v12 is V12=v12*t12, and a breast milk volume corresponding to the flow rate of v13 is V13=v13*t13.

(3). A total of the plurality of breast milk volumes is used as the current breast milk volume in the breast pump.

As an example, the current breast milk volume is V1=v11*t11+v12*t12+v13*t13.

It should be noted that the breast milk flow rates and the corresponding milk expressing durations listed in the above example are for illustrative purposes only, used to explain that the current breast milk volume in the breast pump can be determined based on each same breast milk flow rate and the corresponding milk expressing duration, and shall not be construed as limiting the present disclosure.

Calculating the breast milk volume corresponding to each same flow rate to determine the current breast milk volume in the breast pump can reduce a calculation load and improve calculation efficiency.

In some examples, when a detection signal is obtained, a breast milk volume may be determined in real time to determine the current breast milk volume in the breast pump.

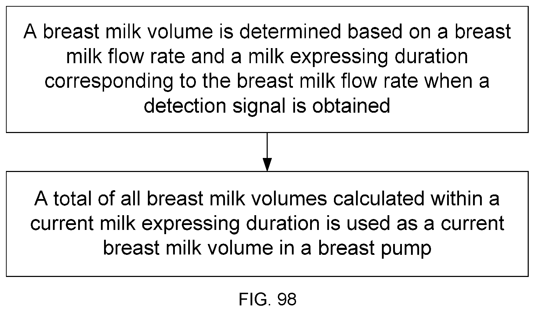

Refer to a flowchart of another method for determining a current breast milk volume in a breast pump in an embodiment of this specification in FIG. 98. As shown in FIG. 98, the third step includes following substeps:
(1). When the detection signal is obtained, a breast milk volume is determined based on the breast milk flow rate and the milk expressing duration corresponding to the breast milk flow rate.

Specifically, a time interval may exist between adjacent detection signals. Each time a detection signal is obtained, a breast milk flow rate and a corresponding milk expressing duration can be obtained, thereby determining a breast milk volume.

In some examples, a breast milk flow rate may be multiplied by a milk expressing duration corresponding to the breast milk flow rate to obtain a breast milk volume.

(2). A sum of all breast milk volumes calculated during the current milk expressing duration is used as the current breast milk volume in the breast pump.

Specifically, in a single breast milk expressing process, at least one detection signal can be obtained. Therefore, a sum of at least one breast milk volume calculated within the current milk expressing duration may be used as the current breast milk volume in the breast pump.

As an example, the milk expressing duration is T2, and within this milk expressing duration, a total of n detection signals can be obtained, corresponding to n breast milk flow rates and n milk expressing durations. For instance, the n breast milk flow rates may be v21, v22, ..., v2n. A milk expressing duration corresponding to v21 may be t21, a milk expressing duration corresponding to v22 may be t22, ..., and a milk expressing duration corresponding to v2n may be t2n, T2=t21+t22+...+t2n, and n is an integer greater than 1.

Therefore, the current breast milk volume in the breast pump is V2=v21*t21+v22*t22+...+v2n*t2n.

It should be noted that, first, the plurality of breast milk flow rates and the corresponding milk expressing durations listed in the above example are for illustrative purposes only, used to explain that the current breast milk volume in the breast pump can be determined in real-time based on each breast milk flow rate and the corresponding milk expressing duration, and shall not be construed as limiting the present disclosure. Second, for v21, v22, ..., v2n listed above, values may all be different, or some may be the same. Similarly, for t21, t22, ..., t2n listed above, values may all be different, or some may be the same. This is not limited in this embodiment of this application.

Determining the current breast milk volume in the breast pump in real-time based on the breast milk volume corresponding to each breast rate flow rate can achieve real-time acquisition of the current breast milk volume in the breast pump and improve user experience.

It may be understood that the two calculation manners provided above are merely examples for illustration. In this embodiment of this specification, a specific calculation manner for determining the current breast milk volume in the breast pump is not limited, as long as the current breast milk volume in the breast pump can be determined based on the breast milk flow rate and the current milk expressing duration.

According to the solution in the above examples, the breast milk flow rate can be determined based on the intensity of the light reflected during breast milk expressing, and then the current breast milk volume in the breast pump can be determined based on the breast milk flow rate and the current milk expressing duration. However, during the milk expressing, breast milk may overflow from the breast pump as a result of a limited capacity of the breast pump.

To avoid the above situation, still referring to FIG. 96, the breast milk volume detection method provided in this embodiment of this specification may further include a following step:
Fourth step: Prompt information is generated and/or the current milk expressing task is stopped when it is determined that the current breast milk volume in the breast pump is greater than a breast milk volume threshold.

Specifically, when the current breast milk volume in the breast pump is greater than the breast milk volume threshold, it indicates a possibility of breast milk overflowing from the breast pump. In this case, generating the prompt information can prompt the user to stop the current breast milk expressing process in a timely manner, and/or stopping the current breast milk expressing task can automatically stop the current breast milk expressing process. Either of the two manners can prevent the breast milk from overflowing from the breast pump and improve user experience.

In some examples, the breast milk volume threshold may be related to the capacity of the breast pump. For example, the breast milk volume threshold may be less than the capacity of the breast pump. The breast milk volume threshold is not limited in this embodiment of this specification, which may be set based on the capacity of the breast pump.

It may be understood that, a plurality of implementations provided in the embodiments of this specification are described above. The optional manners described in the implementations may be combined and cross referenced without conflict, thereby forming a plurality of possible implementations, all of which may be considered as disclosed implementations of this specification.

In some examples, during determining of the current breast milk volume in the breast pump, a signal may be further generated to display the current breast milk volume, to directly provide the user with a specific value of the current breast milk volume.

### Seventeenth embodiment

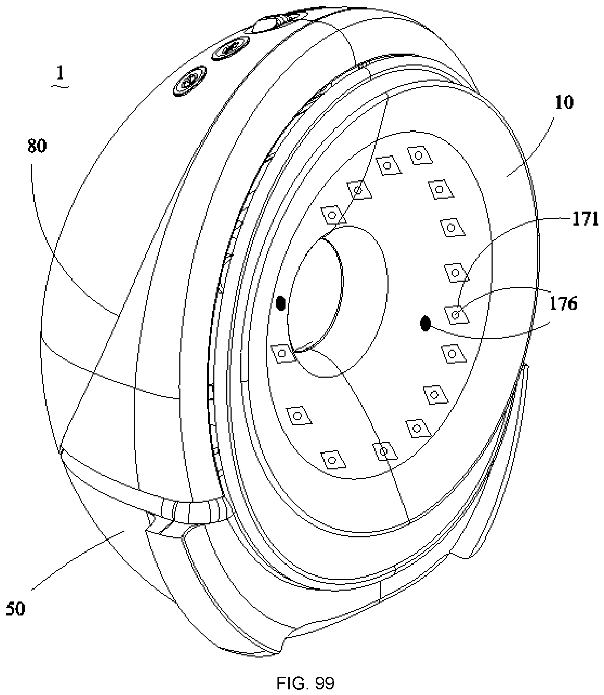

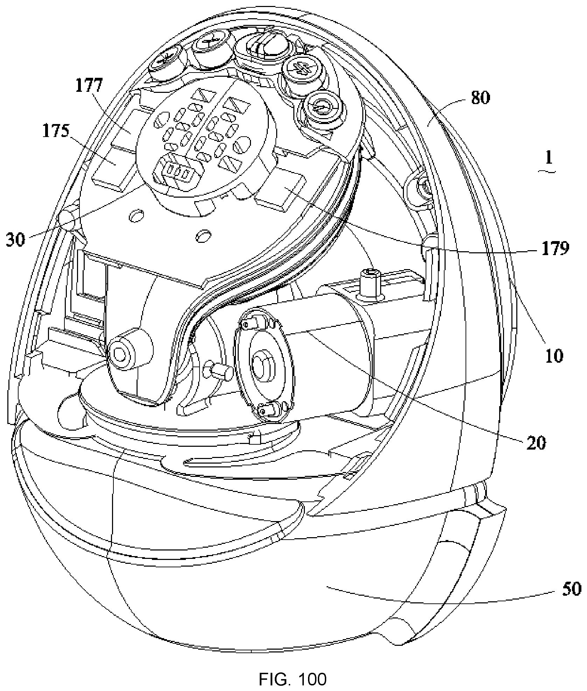

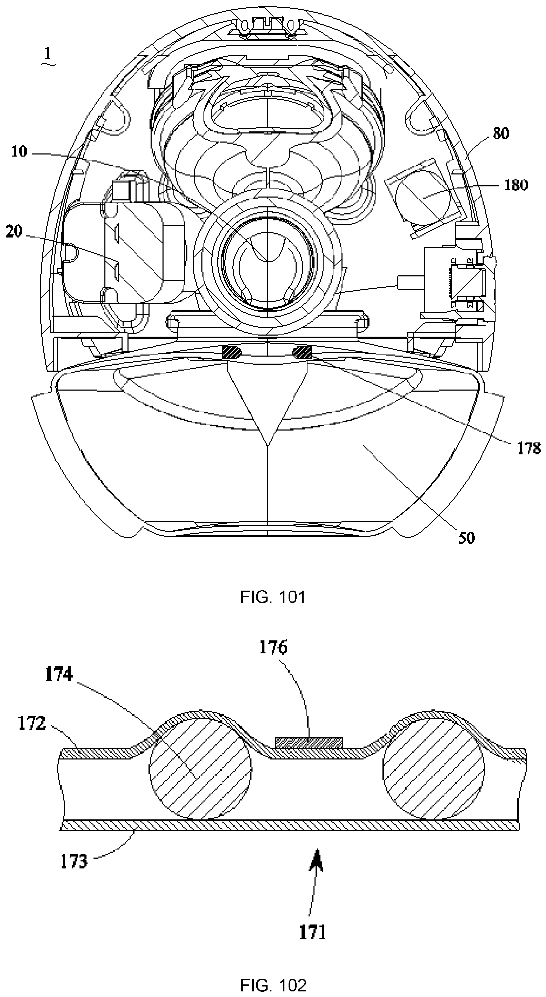

In a first implementation, referring to FIG. 99 to FIG. 102, in some embodiments of this application, a breast pump proposed in this application includes a breast massage device 170, including an attachment portion and at least one electrode portion.

The attachment portion is configured to be attached to a breast, and is arranged on a side of a breast milk collection portion close to the breast, and at least part of the attachment portion forms a part of the breast milk collection portion or is configured to be detachably mounted on or attached to the breast milk collection portion.

The at least one electrode portion is connected to the attachment portion, is directly in contact with a breast surface or is indirectly in contact with the breast surface through a conductive portion on the attachment portion, and is configured to perform electrical stimulation and massage on the breast and/or detect an impedance of the breast.

An attachment portion 171 and at least one electrode 176. The attachment portion 171 is configured to be attached to a breast. The electrode portion 176 is connected to the attachment portion 171, is directly in contact with a breast surface or is indirectly in contact with the breast surface through a conductive portion on the attachment portion 171, and is configured to perform electrical stimulation and massage on the breast and/or detect an impedance of the breast.

The electrode portion 176 is mounted on a surface of the attachment portion 171, and the attachment portion 171 is attached to the breast, so that the breast massage device 170 can provide effective electrical stimulation and massage to the breast and/or detect the impedance value of the breast during operation. The electrical stimulation and massage are performed on the breast to stimulate tissue cells of the breast, induce contraction and relaxation in related tissues of the breast, regulate flowing of bodily fluids such as blood and breast milk, stimulate internal neural regulation of the breast, improve an overall status of the breast, promote secretion and flowing of breast milk, and improve functionality of internal tissues of the breast. The impedance of the breast is detected to determine a volume of milk in the breast, which is used a basis for determining whether to perform electrical stimulation and massage on the breast, thereby further enhancing an effect of the electrical stimulation and massage.

In some embodiments of this application, the breast massage device 170 provided herein includes an impedance detection unit 177 and an electrical stimulation unit 175, both of which are connected to the electrode portion 176. The electrical stimulation unit 175 includes at least an electrical stimulation control circuit, which actively or passively performs electrical stimulation on the breast surface. The impedance detection unit 177 includes at least an impedance detection circuit, which actively or passively detects the impedance of the breast.

The impedance detection unit 177 acquires impedance data of the breast through the electrode portion 176, and the electrical stimulation unit 175 stimulates the breast through the electrode portion 176 to perform electrical stimulation and massage on the breast. The impedance of the breast is detected to determine a volume of milk in the breast, which is used a basis for determining whether to perform electrical stimulation and massage on the breast, thereby further enhancing an effect of the electrical stimulation and massage.

In some embodiments of this application, the breast massage device 170 provided in this application further includes a control unit 30. The control unit 30 is configured to acquire impedance data information from the impedance detection unit 177, and control an operating parameter of the electrical stimulation unit 175 based on the impedance data information.

It may be understood that breast milk is a liquid substance containing water. Therefore, a larger volume of breast milk in a breast indicates a lower impedance of the breast. The impedance data information of the breast is acquired by using a bioimpedance method. The impedance detection unit 177 introduces a weak alternating current signal into the human breast. The current flows through bodily fluids which have a low resistance and good conductivity. In the human breast, a breast milk volume defines a width of a path through which the current passes. Thus, impedances of the breast under different milk contents may be measured, to represent the volume of milk in the breast by using the impedance of the breast. An impedance threshold may be set to indicate whether the milk in the breast is sufficient. If the monitored impedance of the breast is greater than the impedance threshold, it indicates that the milk in the breast is insufficient, and electrical stimulation and massage need to be performed on the breast, thereby promoting breast milk secretion. The impedance of the breast may be monitored in real time, and then the operating parameter of the electrical stimulation unit 175 is adjusted based on real-time changes in the impedance of the breast. The operating parameter of the electrical stimulation unit 175 may include a current intensity, a pulse width, a frequency, a waveform, a polarity, a massage duration, and a current path.

In some embodiments of this application, the breast massage device 170 provided in this application further includes a storage unit 179 connected to the control unit 30. After acquiring the impedance data information from the impedance detection unit 177, the control unit 30 controls the operating parameter of the electrical stimulation unit 175 based on the impedance data information, and transmits the impedance data information to the storage unit 179 for storage. During control of the operating parameter of the electrical stimulation unit 175 based on the impedance data information, the control unit 30 retrieves historical impedance data information stored in the storage unit 179, and combines real-time impedance data information with the historical impedance data information to control the operating parameter of the electrical stimulation unit 175, thereby further optimizing the operating parameter of the electrical stimulation unit 175.

In some embodiments of this application, the operating parameter of the electrical stimulation unit 175 may be detected and fed back to the control unit 30, and the control unit 30 transmits the operating parameter of the electrical stimulation unit 175 to the storage unit 179 for storage. During adjustment of the operating parameter of the electrical stimulation unit 175, the control unit 30 retrieves the historical impedance data information and a historical operating parameter of the electrical stimulation unit 175 stored in the storage unit 179, to determine constitution of the user and a proper operating parameter of the electrical stimulation unit 175. The control unit 30 adjusts the operating parameter of the electrical stimulation unit 175 based on the constitution of the user and the proper operating parameter of the electrical stimulation unit 175, to further optimize the operating parameter of the electrical stimulation unit 175, which ensures an intended electrical stimulation and massage effect while improving user experience.

In a second implementation, referring to FIG. 99 to FIG. 102, in some embodiments of this application, a breast massage device 170 provided in this application includes an attachment portion 171 and an electrical stimulation unit 175. The attachment portion 171 is configured to be attached to a breast. The electrical stimulation unit 175 is connected to at least two electrode portions 176. The electrode portions 176 are mounted on a contact surface of the attachment portion 171, and are configured to perform electrical stimulation and massage on the breast.

Since the electrode portions 176 of the electrical stimulation unit 175 are mounted on the contact surface of the attachment portion 171, and the attachment portion 171 is attached to the breast, the electrical stimulation unit 175 can effectively perform electrical stimulation and massage on the breast during operation. The electrical stimulation and massage are performed on the breast to stimulate tissue cells of the breast, induce contraction and relaxation in related tissues of the breast, regulate flowing of bodily fluids such as blood and breast milk, stimulate internal neural regulation of the breast, improve an overall status of the breast, promote secretion and flowing of breast milk, and improve functionality of internal tissues of the breast.

Referring to FIG. 99 to FIG. 103, in some implementations, the breast massage device 170 further includes

an impedance detection unit 177 and/or a flow rate detection unit 178, and a control unit 30. The impedance detection unit 177 is configured to detect an impedance of the breast, and the flow rate detection unit 178 is configured to detect a flow rate of the breast milk in the breast pump 1.

The control unit 30 is configured to adjust an operating parameter of the electrical stimulation unit 175 based on impedance data information from the impedance detection unit 177 and/or flow rate data information from the flow rate detection unit 178.

In some embodiments of this application, the impedance of the breast may be detected by the impedance detection unit 177 to determine a volume of milk in the breast, and then the operating parameter of the electrical stimulation unit 175 is adjusted based on the volume of milk in the breast.

In some embodiments of this application, the flow rate of the breast milk in the breast pump 1 may be detected by the flow rate detection unit 178, to determine a volume and a speed of breast milk discharged from the breast, and then the operating parameter of the electrical stimulation unit 175 is adjusted based on the volume and the speed of the breast milk discharged from the breast.

It may be understood that, the flow rate of the breast milk in the breast pump 1 is detected by the flow rate detection unit 178. If the flow rate detection unit 178 can detect flow rate data, it indicates that breast milk is being discharged from the breast. A flow rate threshold may be set to indicate whether a volume and a speed of the milk discharged from the breast are as intended. If the monitored flow rate of the breast milk in the breast pump 1 is less than the flow rate threshold, it indicates that the volume and the speed of the milk discharged from the breast are not as intended. In this case, electrical stimulation and massage are performed, to promote breast milk secretion and unblock milk flow channels in the breast. The flow rate of the breast milk in the breast pump 1 may be monitored in real time, and then the operating parameter of the electrical stimulation unit 175 is adjusted based on real-time changes in the flow rate of the breast milk in the breast pump 1. The operating parameter of the electrical stimulation unit 175 may include a current intensity, a pulse width, a frequency, a waveform, a polarity, a massage duration, and a current path.

In some embodiments of this application, comprehensive analysis on a milk discharge status of the breast and a volume of milk in the breast may be performed in combination with the impedance of the breast and the flow rate of the breast milk in the breast pump 1, to determine an overall milk status in the breast, and then adjust the operating parameter of the electrical stimulation unit 175 based on the overall milk status in the breast.

It may be understood that both the impedance of the breast and the flow rate of the breast milk in the breast pump 1 may be monitored to determine the overall milk status in the breast.

When the impedance of the breast is greater than an impedance threshold, and the flow rate of the breast milk in the breast pump 1 is greater than zero but less than a flow rate threshold, it indicates that the milk in the breast is insufficient, but milk can still be discharged from the breast. In this case, a first overall milk status in the breast may be determined as: a small amount of milk exists in the breast and the milk flow channels in the breast are unblocked. In this case, the operating parameter of the electrical stimulation unit 175 may be adjusted based on the first overall milk status to promote breast milk secretion. The operating parameter of the electrical stimulation unit 175 may include a current intensity, a pulse width, a frequency, a waveform, a polarity, a massage duration, and a current path.

When the impedance of the breast is greater than the impedance threshold, and the flow rate of the breast milk in the breast pump 1 is greater than the flow rate threshold, it indicates that the milk in the breast is insufficient, but the flow rate detection unit 178 can still detect a considerable volume of breast milk flowing in the breast pump 1. In this case, it may be determined that the flow rate detection unit 178 is malfunctioning, or that breast milk in the breast pump 1 flows back. In this case, an alarm may be sent through an alarm unit to prompt a malfunction.

When the impedance of the breast is less than the impedance threshold, and the flow rate of the breast milk in the breast pump 1 is equal to zero or less than the flow rate threshold, it indicates that the milk in the breast is sufficient, but no milk is being discharged from the breast, or only a small amount of milk is being discharged from the breast. In this case, a first overall milk status in the breast may be determined as: a large amount of milk exists in the breast but the milk flow channels in the breast are blocked, that is, milk duct blockage occurs. In this case, the operating parameter of the electrical stimulation unit 175 may be adjusted based on the second overall milk status to promote breast milk secretion and unclog the milk flow channels in the breast.

When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

In some embodiments of this application, during the adjustment of the operating parameter of the electrical stimulation unit 175, the operating parameter of the electrical stimulation unit 175 may be automatically adjusted based on the impedance data information from the impedance detection unit 177 and/or the flow rate data information from the flow rate detection unit 178. Alternatively, corresponding operating modes may be selected based on the impedance data information from the impedance detection unit 177 and/or the flow rate data information from the flow rate detection unit 178, where each operating mode corresponds to a specific electrical stimulation procedure, and the operating parameter of the electrical stimulation unit 175 is adjusted based on the corresponding electrical stimulation procedure. The operating modes include but are not limited to a let-down mode, a lactation stimulation mode, a breast unclogging mode, a milk discharge mode, and a milk expressing mode. Since an actual operating condition varies across different models of breast pumps 1, before the breast pumps 1 leave the factory, the operating parameters of the electrical stimulation units 175 under different operating modes may be calibrated based on different models of the breast pumps 1, to provide a reference for adjusting the operating parameters of the electrical stimulation units 175.

During the adjustment of the operating parameter of the electrical stimulation unit 175, the control unit 30 adjusts the operating parameter of the electrical stimulation unit 175 in combination with historical impedance data information, historical flow rate data information, historical operating parameter information from an automatic adjustment process of the electrical stimulation unit 175, historical operating parameter data information from a manual adjustment process of the electrical stimulation unit 175, as well as real-time impedance data information, real-time flow rate data information, and real-time operating parameter data information.

The attachment portion 171 is designed with a shape similar to a shape of the breast, so that the attachment portion 171 can be better attached to the breast, thereby ensuring that the electrode portion 176 of the electrical stimulation unit 175 can be effectively attached to the breast and ensuring effectiveness of the operation of the electrical stimulation unit 175.

During the massage on the breast, vibration of a massage member 174 can assist in massaging the breast, thereby improving activity of tissues in the breast, promoting flowing of blood and milk in the breast, facilitating breast milk secretion, and improving breast milk fluidity.

A vibration device may be arranged in the massage assembly 174 to drive the massage assembly 174 to vibrate. The vibration device may be structured as a motor driving an eccentric wheel, where vibration is generated by rotating the eccentric wheel to rotate through the motor. Alternatively, the vibration device may be structured with an electromagnet and a permanent magnet, where vibration is generated by changing a direction of an electromagnetic force of the electromagnet acting on the permanent magnet by altering a polarity of the electromagnet. The vibration device may alternatively be any other device that can generate vibration.

In some embodiments of this application, a distance between a structural layer 173 and the attachment layer 172 is less than a diameter of the massage member 174, and the massage member 174 is fixedly mounted on the structural layer 173.

The attachment layer 172 may be made of flexible materials such as silicone. Since the distance between the structural layer 173 and the attachment layer 172 is less than the diameter of the massage member 174, the massage member 174 causes the attachment layer 172 to protrude and deform toward an attachment surface of the attachment layer 172, thereby enabling the attachment portion 171 to exert a specific degree of pressure on the breast after being attached to the breast. Therefore, when the massage assembly 174 vibrates, the breast can receive more intense vibration, and the vibration can propagate deeper into the breast, improving a massage effect.

Referring to FIG. 99 to FIG. 103, in some embodiments of this application, this application provides a breast pump 1, which is configured to pump milk from a breast and store the breast milk in a milk storage container portion 50, and includes a breast massage device 170, a breast shield 10, a negative pressure mechanism 20, a milk storage container portion 50, and a power supply system 180. The breast massage device 170 is configured to massage a breast. The breast shield 10 is configured to be attached to the breast and accommodate at least a nipple. The attachment portion 171 is arranged on a side of the breast shield 10 close to the breast, and at least part of the attachment portion 171 forms a part of the breast shield 10 or may be detachably mounted on or attached to the breast shield 10. The negative pressure mechanism 20 is configured to directly or indirectly apply negative pressure to the breast shield 10 to pump milk from the breast. The milk storage container portion 50 is in fluid communication with the breast shield 10 and can store the breast milk expressed from the breast. The power supply system 180 is configured to at least supply power to the negative pressure mechanism 20 and the breast massage device 170.

During milk expressing by using the breast pump 1, the breast shield 10 is placed over a breast of a user, and a nipple of the breast is accommodated in the breast shield 10, forming a sealed space, so that negative pressure can be generated around the nipple. The power supply system 180 provides electrical energy to cause the negative pressure mechanism 20 to operate, thereby generating negative pressure to perform milk expressing. During the milk expressing, breast milk enters the milk storage container portion 50, which holds the expressed breast milk.

In some embodiments of this application, the milk storage container portion 50 may be a milk bowl, a milk bottle, or a milk bag.

In the negative pressure mechanism 20, a power structure configured to generate negative pressure includes but is not limited to a piezoelectric pump, a diaphragm pump, a hydraulic pump, or a mechanical pump. The negative pressure mechanism 20 may directly generate negative pressure in the breast shield 10 through communication with the breast shield 10, or may indirectly generate negative pressure in the breast shield 10 by transmitting the negative pressure to a liquid barrier such as a diaphragm or an airbag. The breast milk is drawn into the milk storage container portion 50 for storage through the negative pressure.

The fluid communication between the breast shield 10 and the milk storage container portion 50 may be either direct communication or communication formed through a tube or other flow paths.

In some embodiments of this application, the breast pump 1 provided in this application further includes a flow rate detection unit 178, which can measure breast milk flow rate data of at least a segment of a fluid passage between the breast shield 10 and the milk storage container portion 50.

In some embodiments of this application, the breast pump 1 provided in this application further includes a housing 80. The negative pressure mechanism 20 is at least partially arranged in the housing 80. The breast shield 10 and the milk storage container portion 50 are respectively connected to the housing 80 to form an integral wearable unit, or the breast shield 10 and the milk storage container portion 50 are connected to each other and then connected as a whole to the housing 80 to form an integral wearable unit. The wearable unit, or at least part of the wearable unit, may be worn within a shoulder strap. The housing 80 may further include components such as a battery, a negative pressure air passage, a circuit board, and a solenoid valve therein.

In some embodiments of this application, the breast pump 1 provided in this application further includes a housing 80. The negative pressure mechanism 20 is at least partially arranged in the housing 80. The breast shield 10 and the milk storage container portion 50 are connected to form an integral wearable unit, and the negative pressure system is at least partially separated from the wearable unit. The wearable unit, or at least part of the wearable unit, may be worn within a shoulder strap. The housing 80 may further include components such as a battery, a negative pressure air passage, a circuit board, and a solenoid valve therein.

In some embodiments of this application, the attachment portion 171 is integrally formed with the breast shield 10.

In some embodiments of this application, preset control programs for different operating modes may be preset. Operating modes of the breast pump 1 may include a let-down mode, a lactation stimulation mode, a breast unclogging mode, a milk expressing mode, and a milk discharge mode.

In the let-down mode, a let-down operation may be performed through control of an expressing frequency, an expressing width, and a suction force of the breast pump 1, and the breast massage device 170 massages the breast, to promote milk secretion, open a breast milk flow channel, and improve a let-down effect. Massage and expressing may be performed alternately.

An instruction may be sent to a control unit 30 through a key or an external control device to enter the let-down mode. The control unit 30 adjusts an operating parameter of an electrical stimulation unit 175 based on a preset let-down mode procedure, to perform electrical stimulation and massage on the breast. During the electrical stimulation and massage, an impedance of the breast may be monitored by the impedance detection unit 177, and monitored impedance data information is fed back to the control unit 30. If it is determined based on the impedance data information that the impedance of the breast is less than an impedance threshold, the control unit 30 sends a startup instruction to the negative pressure mechanism 20 and sends a pause instruction to the electrical stimulation unit 175, to pause the massage on the breast and cause the breast pump 1 to start milk expressing. During the milk expressing, a flow rate of the breast milk in the breast pump 1 may be monitored by the flow rate detection unit 178, and flow rate data information of the breast milk in the breast pump 1 is fed back to the control unit 30. If it is determined based on the flow rate data information that the flow rate of the breast milk in the breast pump 1 is less than a flow rate threshold, it indicates that milk channels in the breast are blocked. The control unit 30 then sends an instruction to control the negative pressure mechanism 20 to pause operation, and sends a startup instruction to the electrical stimulation unit 175 to perform stimulation-based massage on the breast, and control the operating parameter of the electrical stimulation unit 175 to promote unclogging of the milk channels in the breast. When an operating duration of the electrical stimulation unit 175 reaches a first duration threshold, the control unit 30 sends a startup instruction to the negative pressure mechanism 20 and sends a pause instruction to the electrical stimulation unit 175, to pause the massage on the breast and cause the breast pump 1 to re-start milk expressing. During the milk expressing, the flow rate of the breast milk in the breast pump 1 may be monitored by the flow rate detection unit 178, and the flow rate data information of the breast milk in the breast pump 1 is fed back to the control unit 30. If it is determined based on the flow rate data information that the flow rate of the breast milk in the breast pump 1 is still less than the flow rate threshold, it indicates that milk channels in the breast are still blocked. In this case, massage is re-performed on the breast. This process is repeated until it is determined based on the flow rate data information fed back by the flow rate detection unit 178 that the flow rate of the breast milk in the breast pump 1 is greater than the flow rate threshold, which indicates that the milk channels in the breast are unclogged. In this case, the control unit 30 switches the operating mode to the milk expressing mode. In this way, the let-down process of the breast is completed. An operating parameter corresponding to the let-down mode may be preset, and the negative pressure mechanism 20 and the electrical stimulation unit 175 are controlled based on the preset operating parameter.

During the milk expressing, the impedance of the breast may be monitored in real time by the impedance detection unit 177, and the flow rate of the breast milk in the breast pump 1 may be monitored in real time by the flow rate detection unit 178. When the impedance of the breast is greater than an impedance threshold, and the flow rate of the breast milk in the breast pump 1 is greater than zero but less than a flow rate threshold, it indicates that the milk in the breast is insufficient, but milk can still be discharged from the breast. In this case, an instruction may be sent to the control unit 30 through a key or an external control device to enter the lactation stimulation mode, or the control unit 30 may automatically enter the lactation stimulation mode. The control unit 30 adjusts the operating parameter of the electrical stimulation unit 175 based on a preset lactation stimulation mode procedure, to perform electrical stimulation and massage on the breast, so as to promote breast milk secretion. The operating parameter of the electrical stimulation unit 175 may include a current intensity, a pulse width, a frequency, a waveform, a polarity, a massage duration, and a current path. During the massage on the breast, the operating parameter of the electrical stimulation unit 175 may be adjusted based on real-time breast impedance data information. When it is detected that the impedance of the breast is less than the impedance threshold, the control unit 30 sends an instruction to the electrical stimulation unit 175 to stop operating, to cease the massage on the breast. The control unit 30 then sends a startup instruction to the negative pressure mechanism 20 to cause the breast pump 1 to start operating. The flow rate of the breast milk in the breast pump 1 is monitored in real time by the flow rate detection unit 178. If the flow rate of the breast milk in the breast pump 1 is greater than the flow rate threshold, it indicates that the lactation stimulation is completed. In this case, the control unit 30 automatically enters the milk expressing mode.

During the milk expressing, the impedance of the breast may be monitored in real time by the impedance detection unit 177, and the flow rate of the breast milk in the breast pump 1 may be monitored in real time by the flow rate detection unit 178. When the impedance of the breast is less than the impedance threshold, and the flow rate of the breast milk in the breast pump 1 is equal to zero or less than the flow rate threshold, it indicates that the milk in the breast is sufficient, but no milk is being discharged from the breast, or only a small amount of milk is being discharged from the breast. In this case, an instruction may be sent to the control unit 30 through a key or an external control device to enter the breast unclogging mode, or the control unit 30 may automatically enter the breast unclogging mode. The control unit 30 adjusts the operating parameter of the electrical stimulation unit 175 based on a preset breast unclogging mode procedure, to perform electrical stimulation massage on the breast, so as to promote unclogging of milk channels in the breast. The operating parameter of the electrical stimulation unit 175 may include a current intensity, a pulse width, a frequency, a waveform, a polarity, a massage duration, and a current path. During the massage on the breast, the operating parameter of the electrical stimulation unit 175 may be adjusted based on real-time flow rate data information of the breast milk in the breast pump 1. When the operating duration of the electrical stimulation unit 175 reaches a second duration threshold, the control unit 30 sends a startup instruction to the negative pressure mechanism 20 and sends a pause instruction to the electrical stimulation unit 175, to pause the massage on the breast and cause the breast pump 1 to re-start milk expressing. During the milk expressing, the flow rate of the breast milk in the breast pump 1 may be monitored by the flow rate detection unit 178, and the flow rate data information of the breast milk in the breast pump 1 is fed back to the control unit 30. If it is determined based on the flow rate data information that the flow rate of the breast milk in the breast pump 1 is still less than the flow rate threshold, it indicates that milk channels in the breast are still blocked. In this case, massage is re-performed on the breast. This process is repeated until it is determined based on the flow rate data information fed back by the flow rate detection unit 178 that the flow rate of the breast milk in the breast pump 1 is greater than the flow rate threshold, which indicates that the milk channels in the breast are unclogged. In this case, the control unit 30 switches the operating mode to the milk expressing mode. In this way, the unclogging of the breast is completed.

During the milk expressing, the impedance of the breast may be monitored in real time by the impedance detection unit 177, and the flow rate of the breast milk in the breast pump 1 may be monitored in real time by the flow rate detection unit 178. When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

When a milk discharge operation needs to be performed in a specific time period of a day, an instruction may be sent to the control unit 30 through a key or an external control device to enter the milk discharge mode, so that the breast pump 1 enters the milk discharge mode. During the milk discharge, the impedance of the breast may be monitored in real time by the impedance detection unit 177, and the flow rate of the breast milk in the breast pump 1 may be monitored in real time by the flow rate detection unit 178. When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing operation is performed. During the milk expressing by the breast pump 1, a milk discharge status is monitored based on impedance data information and flow rate data information monitored in real time. When the impedance of the breast is greater than the impedance threshold, and the flow rate of the breast milk in the breast pump 1 is zero or less than the flow rate threshold, it indicates that a majority of the milk in the breast has been expressed. In this case, the control unit 30 sends a startup signal to the electrical stimulation unit 175 and controls the electrical stimulation unit 175 to massage the breast by using the operating parameter preset for the milk discharge mode, so as to stimulate tissue cells of the breast, induce contraction and relaxation in related tissues of the breast, regulate flowing of bodily fluids such as blood and breast milk, stimulate internal neural regulation of the breast, improve an overall status of the breast, promote secretion and flowing of breast milk, and improve functionality of internal tissues of the breast. When the operating duration of the electrical stimulation unit 175 reaches a third duration threshold, the control unit 30 sends a startup instruction to the negative pressure mechanism 20 and a pause instruction to the electrical stimulation unit 175, to pause the massage on the breast and cause the breast pump 1 to resume milk expressing. This process continues until the impedance of the breast is greater than the impedance threshold and the flow rate of breast milk in the breast pump 1 is less than the flow rate threshold. At this point, the control unit 30 determines that the milk discharge is completed and sends a stop instruction to the negative pressure mechanism 20, causing the breast pump 1 to stop operation.

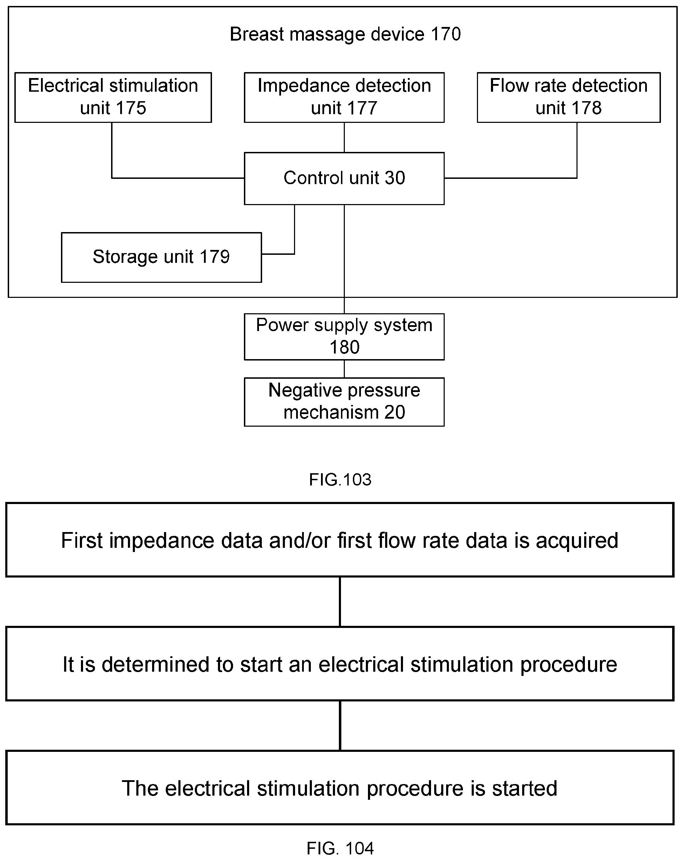

Referring to FIG. 104, in some embodiments of this application, this application provides a control method, including following steps:
acquiring first breast status information and/or first breast milk status information of a breast pump;
determining to start an electrical stimulation procedure; and
starting the electrical stimulation procedure.

In some embodiments of this application, an impedance of a breast may be monitored in real time by an impedance detection unit 177, and a flow rate of breast milk in the breast pump 1 may be monitored in real time by a flow rate detection unit 178. Then the first breast status information is acquired based on the impedance of the breast, and the first breast milk status information of the breast pump is acquired based on the flow rate of the breast milk in the breast pump 1. Comprehensive analysis on a milk discharge status of the breast and a volume of milk in the breast may be performed in combination with the first breast status information and the first breast milk status information of the breast pump, to determine an overall milk status in the breast, and then the operating parameter of the electrical stimulation unit 175 is adjusted based on the overall status of the milk in the breast.

When the impedance of the breast is greater than an impedance threshold, and the flow rate of the breast milk in the breast pump 1 is greater than zero but less than a flow rate threshold, it indicates that the milk in the breast is insufficient, but milk can still be discharged from the breast. In this case, the operating parameter of the electrical stimulation unit 175 may be adjusted to promote breast milk secretion. The operating parameter of the electrical stimulation unit 175 may include a current intensity, a pulse width, a frequency, a waveform, a polarity, a massage duration, and a current path.

When the impedance of the breast is greater than the impedance threshold, and the flow rate of the breast milk in the breast pump 1 is greater than the flow rate threshold, it indicates that the milk in the breast is insufficient, but the flow rate detection unit 178 can still detect a considerable volume of breast milk flowing in the breast pump 1. In this case, it may be determined that the flow rate detection unit 178 is malfunctioning, or that breast milk in the breast pump 1 flows back. In this case, an alarm may be sent through an alarm unit to prompt a malfunction.

When the impedance of the breast is less than the impedance threshold, and the flow rate of the breast milk in the breast pump 1 is equal to zero or less than the flow rate threshold, it indicates that the milk in the breast is sufficient, but no milk is being discharged from the breast, or only a small amount of milk is being discharged from the breast, meaning that milk duct blockage occurs. In this case, the operating parameter of the electrical stimulation unit 175 may be adjusted based on the second overall milk status to promote breast milk secretion and unclog the milk flow channels in the breast.

When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

In some embodiments of this application, the impedance of the breast may be monitored in real time by the impedance detection unit 177, and then the first breast status information is acquired based on the impedance of the breast. When the impedance of the breast is greater than the impedance threshold, it indicates that the milk in the breast is insufficient. In this case, the operating parameter of the electrical stimulation unit 175 may be adjusted to promote breast milk secretion. The operating parameter of the electrical stimulation unit 175 may include a current intensity, a pulse width, a frequency, a waveform, a polarity, a massage duration, and a current path.

In some embodiments of this application, the flow rate of the breast milk in the breast pump 1 may be monitored in real time by the flow rate detection unit 178, and then the first breast milk status information of the breast pump is acquired based on the flow rate of the breast milk in the breast pump 1. If the flow rate of the breast milk in the breast pump 1 is less than the flow rate threshold, it indicates that the milk in the breast is insufficient. In this case, the operating parameter of the electrical stimulation unit 175 may be adjusted to promote breast milk secretion. The operating parameter of the electrical stimulation unit 175 may include a current intensity, a pulse width, a frequency, a waveform, a polarity, a massage duration, and a current path.

In some embodiments of this application, the first breast status information includes first impedance data of the breast, and the first breast milk status information of the breast pump includes first flow rate data of the breast milk in the breast pump 1.

The step of determining to start an electrical stimulation procedure includes: determining that the first impedance data is greater than the impedance threshold and/or determining that the first flow rate data is less than a flow rate threshold.

When at least one of these conditions occurs, that is, the first impedance data is greater than the impedance threshold and/or the first flow rate data is less than the flow rate threshold, it indicates that the milk in the breast is insufficient or that milk duct blockage occurs. In this case, the control unit 30 sends a startup instruction to the electrical stimulation unit 175 to start the electrical stimulation procedure, thereby massaging the breast, promoting breast milk secretion, and unclogging the breast milk channels.

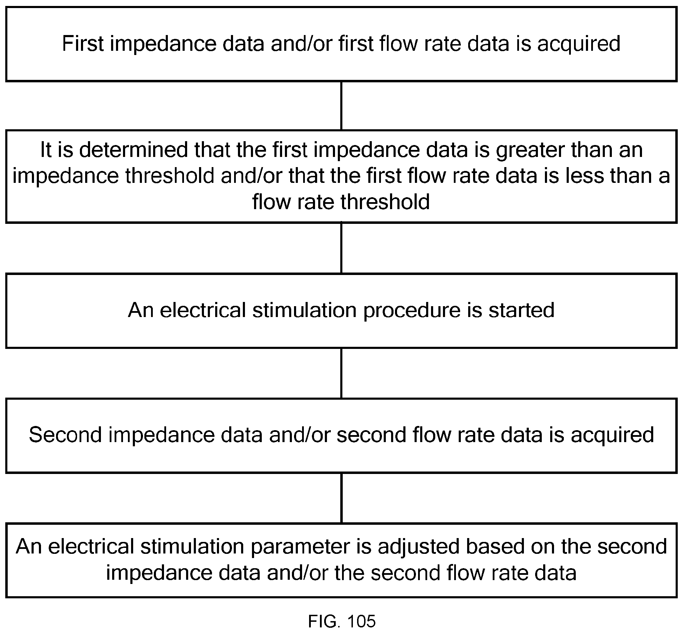

Referring to FIG. 105, in some embodiments of this application, after the step of starting the electrical stimulation procedure, the control method further includes following steps:
acquiring second breast status information and/or second breast milk status information of the breast pump; and
adjusting the electrical stimulation parameter based on the second breast status information and/or the second breast milk status information of the breast pump.

The second breast status information includes second impedance data of the breast, and the second breast milk status information of the breast pump includes second flow rate data of the breast milk in the breast pump 1.

When at least one of the following conditions occurs, that is, the second impedance data is less than the first impedance data and greater than the impedance threshold, and/or the second flow rate data is greater than the first flow rate data and less than the flow rate threshold, it indicates that the milk in the breast has increased and that the breast milk channels have been unclogged to some extent. In this case, the control unit 30 sends an instruction to adjust the electrical stimulation parameter of the electrical stimulation unit 175, so as to moderate the massage on the breast and promote the breast to secrete milk naturally, thereby reducing dependence on the massage.

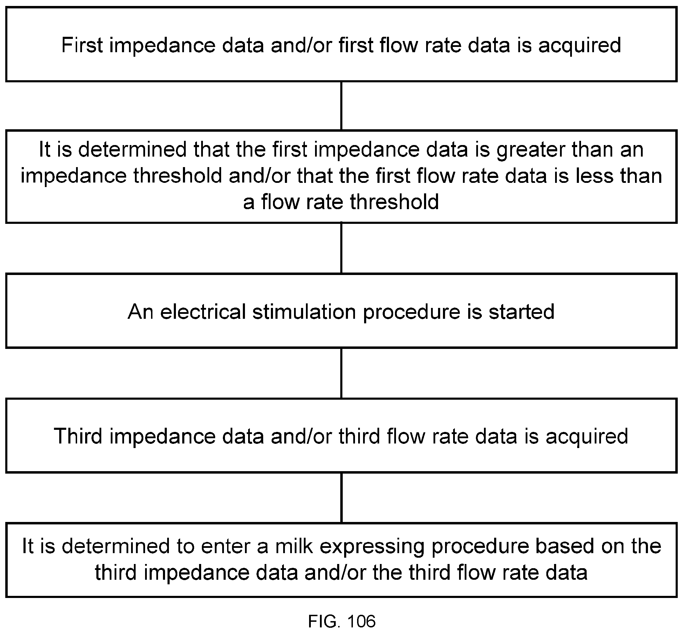

Referring to FIG. 106, in some embodiments of this application, after the step of starting the electrical stimulation procedure, the control method further includes following steps:
acquiring third breast status information and/or third breast milk status information of the breast pump; and
determining to enter a milk expressing procedure based on the third breast status information and/or the third breast milk status information of the breast pump.

During the milk expressing, the impedance of the breast may be monitored in real time by the impedance detection unit 177, and then the third breast status information is acquired. The flow rate of the breast milk in the breast pump 1 may be monitored in real time by the flow rate detection unit 178, and then the third breast milk status information of the breast pump is acquired. When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

In some embodiments of this application, the third breast status information includes third impedance data of the breast, and the third breast milk status information of the breast pump includes third flow rate data of the breast milk in the breast pump 1.

The step of determining to enter a milk expressing procedure based on the third breast status information and/or the third breast milk status information of the breast pump includes: determining that the third impedance data is less than the impedance threshold and/or determining that the third flow rate data is greater than the flow rate threshold.

During the milk expressing, the impedance of the breast may be monitored in real time by the impedance detection unit 177, and the flow rate of the breast milk in the breast pump 1 may be monitored in real time by the flow rate detection unit 178. When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

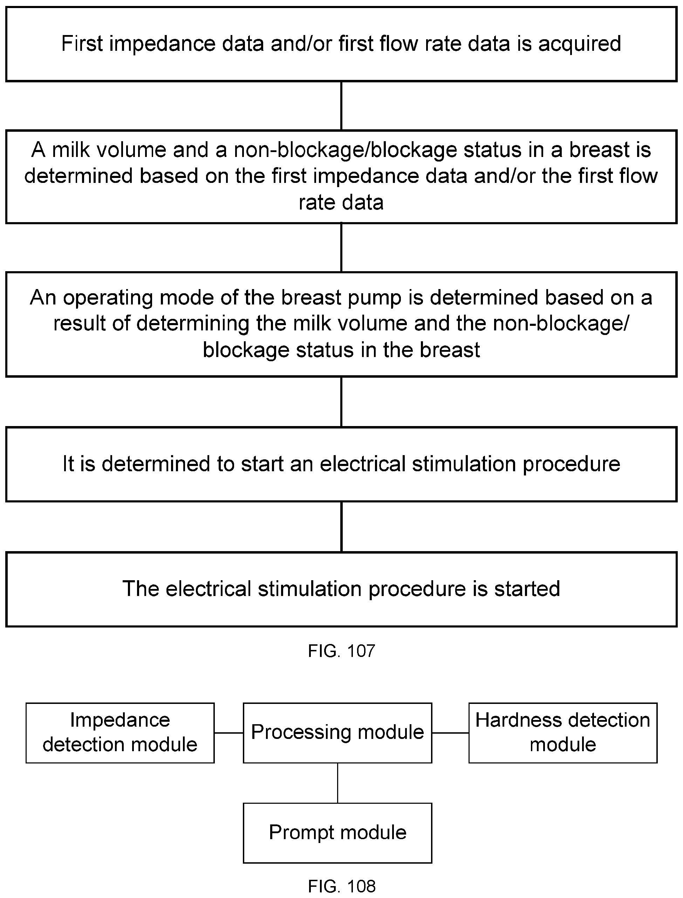

Referring to FIG. 107, in some embodiments of this application, before the step of determining to start an electrical stimulation procedure, the control method further includes following steps:
determining a milk volume and a non-blockage/blockage status in the breast based on the first impedance data and/or the first flow rate data; and
determining an operating mode of the breast pump 1 based on a result of determining the milk volume and the non-blockage/blockage status in the breast.

In some embodiments of this application, after the first impedance data of the breast and the first flow rate data of the breast milk in the breast pump 1 are acquired, the milk volume and the non-blockage/blockage status in the breast are determined based on the first impedance data and the first flow rate data. If the first impedance data is less than the impedance threshold and the first flow rate data is less than the flow rate threshold, it indicates that the milk in the breast is sufficient, but a breast milk expressing volume of the breast pump 1 is not as intended. Therefore, it may be determined that the breast is in a blocked state. At this point, the control unit 30 sends an instruction to cause the breast pump 1 to enter the breast unclogging mode. If the first impedance data is less than the impedance threshold and the first flow rate data is greater than the flow rate threshold, it indicates that the milk in the breast is sufficient, and the breast milk expressing volume of the breast pump 1 is as intended. Therefore, it may be determined that milk discharge channels in the breast are unblocked. At this point, the control unit 30 sends an instruction to cause the breast pump 1 to enter the milk expressing mode. If the first impedance data is greater than the impedance threshold, it indicates that the volume of milk in the breast is low. At this point, the control unit 30 sends an instruction to cause the breast pump 1 to enter the lactation stimulation mode.

If the breast pump enters the breast unclogging mode or the lactation stimulation mode, it is determined that the electrical stimulation procedure needs to be started, and then the electrical stimulation procedure is started depending on different modes. In different modes, an adjustment rule for the operating parameter of the electrical stimulation unit 175 varies to meet a demand for electrical stimulation and massage of a corresponding mode.

According to another aspect, this application further provides a computer-readable storage medium, storing computer instructions that, when run, perform the above control method.

In some implementations, this application provides a control method. The acquiring a control parameter includes:
acquiring breast status data and/or breast milk flow rate status data.

The performing a preset operation based on the control parameter includes:
determining a breast state based on the breast status data and/or the breast milk flow rate status data; and
starting a proper milk expressing procedure based on the breast state.

According to another aspect, in some embodiments of this application, this application provides a control method, including following steps:
acquiring breast status data and/or breast milk flow rate status data;
determining a breast state based on the breast status data and/or the breast milk flow rate status data; and
starting a proper milk expressing procedure based on the breast state.

It is determined whether milk in the breast is sufficient based on the breast status data, and a flow rate of the milk from the breast is determined based on a breast milk flow rate state. A breast state is determined based on the breast status data and/or the breast milk flow rate status data. If the milk in the breast is sufficient but the flow rate of the milk from the breast is low, it is determined that the breast state is blocked. If the milk in the breast is sufficient and the flow rate of the milk from the breast is normal, it is determined that the breast state is normal milk expressing. If the milk in the breast is insufficient, it is determined that the breast state is milk-deficiency. A milk expressing procedure matching the determined breast state is started based on the breast state. If the breast state is blocked, a milk expressing procedure in a breast unclogging mode is started. If the determined breast state is normal milk expressing, a milk expressing procedure in a milk expressing mode is started. If the determined breast state is milk-deficiency, a milk expressing procedure in a lactation stimulation mode is started.

In some embodiments of this application, the breast status data includes breast milk sufficiency status data or breast milk deficiency status data.

In some embodiments of this application, a step of acquiring a breast state includes:
acquiring breast impedance data;
comparing the breast impedance data with an impedance threshold; and
determining a breast state.

If the breast impedance data is less than the impedance threshold, it is determined that the breast state is milk-sufficiency.

If the breast impedance data is greater than or equal to the impedance threshold, it is determined that the breast state is milk-deficiency.

When the impedance data is less than the impedance threshold, which indicates that a moisture content in the breast is high, it is determined that the breast state is milk-sufficiency. When the impedance data is greater than or equal to the impedance threshold, which indicates that the moisture content in the breast is low, it is determined that the breast state is milk-deficiency.

In some embodiments of this application, the breast milk flow rate status data includes normal flow rate status data, low flow rate status data, or high flow rate status data.

In some embodiments of this application, a step of acquiring a breast milk flow rate state includes:
acquiring breast milk flow rate data in a fluid pathway of the breast pump;
comparing the flow rate data with a flow rate threshold; and
determining a flow rate state.

If the flow rate data is greater than or equal to a first flow rate threshold and less than a second flow rate threshold, it is determined that the breast milk flow rate state is normal.

If the flow rate data is less than or equal to the first flow rate threshold, it is determined that the breast milk flow rate state is low.

If the flow rate data is greater than or equal to the second flow rate threshold, it is determined that the breast milk flow rate state is high.

If the flow rate data is greater than or equal to the first flow rate threshold and less than the second flow rate threshold, which indicates that milk can flow out of the breast smoothly, that is, the breast milk flow rate is normal, it is determined that the breast milk flow rate state is normal. If the flow rate data is less than or equal to the first flow rate threshold, which indicates that milk cannot easily flow out of the breast, it is determined that the breast milk flow rate state is low. If the flow rate data is greater than or equal to the second flow rate threshold, which indicates that excessive milk flows out of the breast, it is determined that the breast milk flow rate state is high.

In some embodiments of this application, the breast state includes blocked, normal milk flow, or milk deficiency.

In some embodiments of this application, the step of determining a breast state includes:
acquiring breast impedance data and breast milk flow rate data in a fluid pathway of the breast pump;
comparing the breast impedance data with an impedance threshold and the flow rate data with a flow rate threshold; and
determining a breast state.

If the breast impedance data is less than the impedance threshold and the flow rate data is less than the flow rate threshold, it is determined that the breast state is blocked.

If the breast impedance data is less than the impedance threshold and the flow rate data is greater than the flow rate threshold, it is determined that the breast state is normal milk flow.

If the breast impedance data is greater than the impedance threshold, it is determined that the breast state is milk-deficiency.

If the impedance data is less than the impedance threshold and the flow rate data is less than the flow rate threshold, which indicates that the milk in the breast is sufficient but only a small amount of milk flows out of the breast, it is determined the breast state is blocked. If the impedance data is less than the impedance threshold and the flow rate data is greater than or equal to the flow rate threshold, which indicates that the milk in the breast is sufficient and sufficient milk flows out of the breast, it is determined the breast state is normal milk flow. If the impedance data is greater than the impedance threshold, which indicates that the moisture content in the breast is low, it is determined that the breast state is milk-deficiency.

In some embodiments of this application, the starting a proper milk expressing procedure includes initiating a stimulation mode, initiating a breast unclogging mode, initiating an expressing intensity matching mode, or initiating a relief mode.

In some embodiments of this application, the breast status data is derived from one or more of breast composition detection or breast size detection.

In some embodiments of this application, the breast composition includes a volume of milk in the breast. The breast composition may further include a distribution and a status of various tissues in the breast, such as a breast hardness, a breast fat content, a breast muscle distribution, and a breast gland distribution.

In some embodiments of this application, the composition detection is achieved through breast impedance detection.

In some embodiments of this application, the initiating a stimulation mode includes initiating one or more of electrical stimulation, vibration, or kneading massage.

In some embodiments of this application, the initiating a breast unclogging mode includes initiating a hot compress mode.

In some embodiments of this application, the initiating an expressing intensity matching mode includes selecting a comfortable expressing intensity matching a current breast state of the user based on historical data.

In some embodiments of this application, initiating the relief mode includes initiating either a cold compress mode or a hot compress mode.

### Eighteenth embodiment

Referring to FIG. 108, FIG. 110, FIG. 112, and FIG. 113, in a breast pump of this embodiment, a detection unit includes an impedance detection module and a hardness detection module. The impedance detection module is configured to acquire an impedance signal of a human breast, and the hardness detection module is configured to acquire a hardness signal of the human breast. A control unit 30 is electrically connected to both the impedance detection module and the hardness detection module, so that the control unit 30 can receive the impedance signal from the impedance detection module and the hardness signal from the hardness detection module, and can determine at least a distribution of milk in the human breast based on the impedance signal and the hardness signal, thereby further determining whether to prompt a user to perform a milk expressing operation. In addition, the control unit 30 can further evaluate and determine, based on the hardness signal, a milk volume in the human breast and/or whether milk duct blockage occurs, thereby further prompting the user whether to perform a milk expressing operation or prompting the user of the milk duct blockage, so that the user can respond in a timely manner.

It may be understood that an impedance of the human breast can indicate a distribution of milk in the breast. A principle is to measure a content of milk in the breast through a bioelectrical impedance (BI-impedance) analysis. This method infers the breast milk content by measuring a resistance to a current in breast tissues. Since breast milk has high resistance, when a large amount of milk exists in the breast, a current encounters a large resistance when passing through the breast, resulting in an increase in an impedance of the breast. Therefore, when the impedance of the breast received by the control unit 30 is high and exceeds a first threshold preset by the control unit 30, the control unit 30 determines that a proper milk expressing time point is reached and sends a prompt to prompt the user to pump milk in a timely manner. Conversely, if the milk in the breast is insufficient, the resistance encountered by the current when passing through the breast is lower. Therefore, the impedance of the breast is correspondingly lower, falling below the first threshold preset by the control unit 30. The control unit 30 then determines that the proper milk expressing time point is not reached and therefore does not send a prompt.

Based on the above solution, further, when the impedance signal received by the control unit 30 does not meet a criteria for prompting the user to pump milk, due to the various factors affecting the impedance of the human breast, the detection device in this embodiment further acquires the hardness signal of the human breast through the hardness detection module, to more accurately indicate a status of the human breast, thereby determining whether to pump milk or whether milk duct blockage occurs. It may be understood that a principle of determining the milk content in the breast based on a hardness of the human breast is mainly performing determination based on physical characteristics of the breast tissues. When the breast is full of milk, the hardness of the breast increases, because breast milk makes the breast tissues fuller and firmer. Moreover, when the breast is full of milk, if a mother does not pump milk in a timely manner, the breast may become increasingly hard as the breast milk volume continuously increases, leading to engorgement or milk duct blockage. Conversely, if the milk content in the breast is low, the hardness of the breast is low. Therefore, a change in the breast hardness can assist in estimating the milk volume in the human breast and determining whether milk duct blockage occurs. Therefore, when the milk volume in the breast gradually increases, the hardness of the breast increases. Thus, when the hardness signal of the breast received by the control unit 30 is high and exceeds a second threshold preset by the control unit 30, the control unit 30 determines that a proper milk expressing time point is reached and sends a prompt to prompt the user to pump milk in a timely manner. Conversely, if the milk in the breast is insufficient, the hardness of the breast is correspondingly low, falling below the second threshold preset by the control unit 30. The control unit 30 then determines that the proper milk expressing time point is not reached and therefore does not send a prompt. Moreover, when the hardness signal of the breast received by the control unit 30 is such high that it exceeds the second threshold and reaches a relatively high value, the control unit 30 determines that the breast of the user may encounter milk duct blockage and sends a prompt to prompt the user to respond in a timely manner.

Preferably, this embodiment further includes a prompt module configured to send prompt information to the user based on the impedance signal or the hardness signal, to prompt the user of a breast milk distribution and a determined milk duct blockage status in a timely manner. The detection unit is caused to contact the human breast, and transmits the impedance signal or the hardness signal of the breast to the control unit in a timely manner. The control unit determines the milk volume in the breast in a timely manner based on the impedance signal and the hardness signal, so that the user can select a more proper milk expressing time point, and the mother can pump a larger volume of breast milk within a fixed duration, thereby improving milk expressing efficiency, and improving user experience.

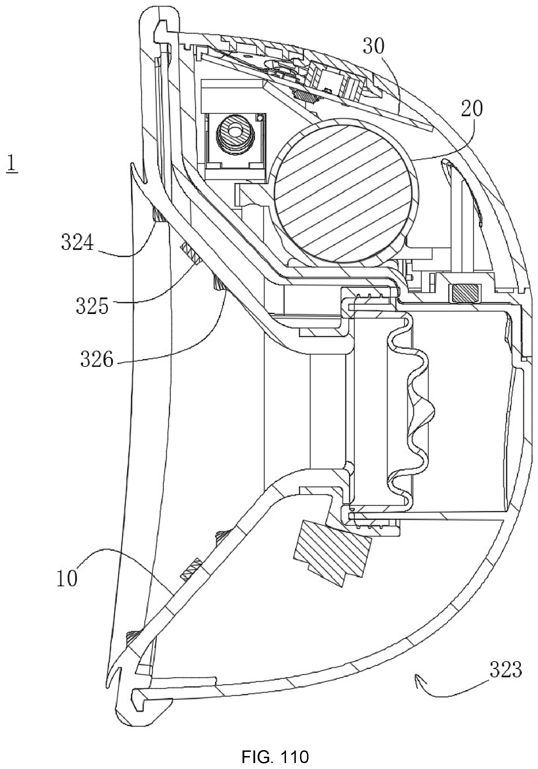

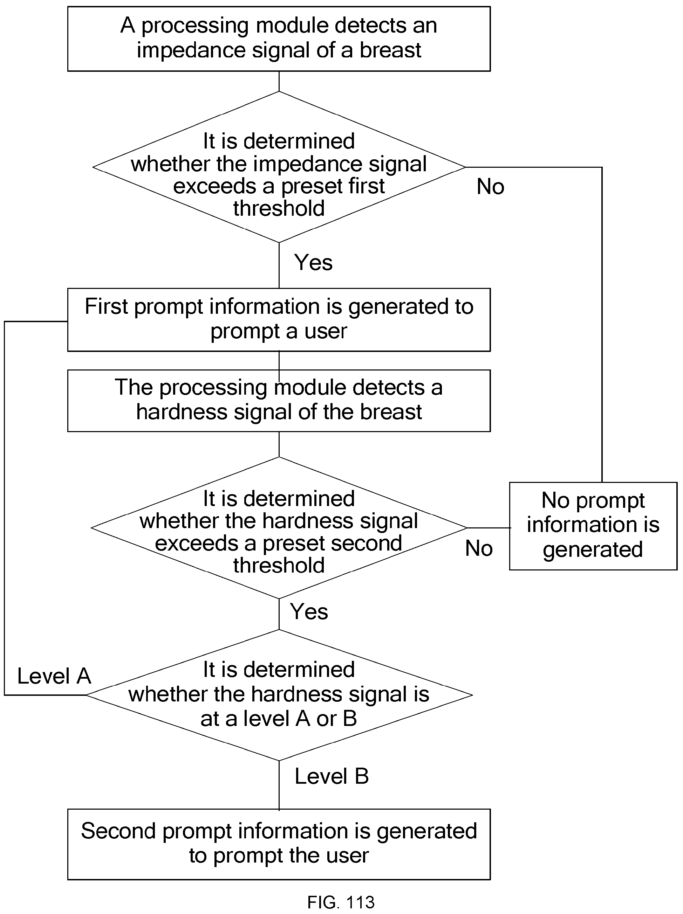

Referring to FIG. 110 and FIG. 113, a breast pump 1 includes a body 2, a breast shield 10 mounted on the body 2, and a stimulation element 324 arranged on a side of the breast shield 10. The body 2 includes an air pump 211 configured to generate negative pressure and a control unit 30 configured to control the air pump 211. The breast shield 10 is configured to cover a human breast and be snugly attached to the human breast. The control unit 30 is configured to receive an impedance signal from an electrode module and a hardness signal from a hardness detection module. The control unit 30 is configured to control the air pump 211 to enter different operating modes based on the impedance signal or the hardness signal.

Optionally, the stimulation element 324 is controlled by the control unit 30 to apply stimulation to the human breast, triggering milk-ejection reflex. In an embodiment, the stimulation element 324 includes an infrared sensor, which stimulates the breast to heat up by radiating infrared rays to the breast. In another embodiment, the stimulation element 324 further includes a massage head arranged on a side of the breast shield 10 and can simulate manual techniques to massage the breast so as to promote blood circulation and relieve breast hardness and engorgement.

It may be understood that, in actual use, when a mother has just finished breast-feeding or can predict that a breast milk volume is low and therefore milk expressing is not needed within a specific time period, it is unnecessary to wear the breast pump 1 all the time. It may be understood that, because the breast pump 1 is heavy, a control unit 30 of a detection device may be electrically connected to the control unit 30 of the breast pump 1 through a wire, so that the user can avoid wearing the breast pump 1 to reduce a breast weight, and an impedance signal and a hardness signal detected by the control unit 30 can be sent to the control unit 30 in a timely manner. Therefore, the breast pump 1 or a terminal device can send prompt information. The terminal device may be a mobile phone, a tablet computer, or a terminal device specifically designed for a breast pump. The breast pump 1 may operate in a corresponding mode when the breast milk volume increases to reach the proper milk expressing time point or milk duct blockage is detected.

Optionally, in actual use, the detection unit may be directly mounted on the breast pump 1, the impedance detection module is arranged on an inner side of the breast shield 10 for contact with the human breast, and the hardness detection module 216 is arranged on the inner side of the breast shield 10 for contact with the human breast.

Furthermore, the impedance detection module 215 includes at least one pair of electrodes, which are arranged on the breast shield 10 so as to be respectively attached to opposite sides of the breast. The hardness detection module 216 is a pressure sensor, including at least one pair of contact terminals, which are arranged on the breast shield 10 so as to be respectively attached to opposite sides of the breast.

It may be understood that the overall operation of the breast pump 1 in this embodiment is controlled by the control unit 30 in the main unit. The control unit 30 may be a CPU, an ASIC, or one or more integrated circuits configured to implement the embodiments of the present disclosure. This is not limited herein.

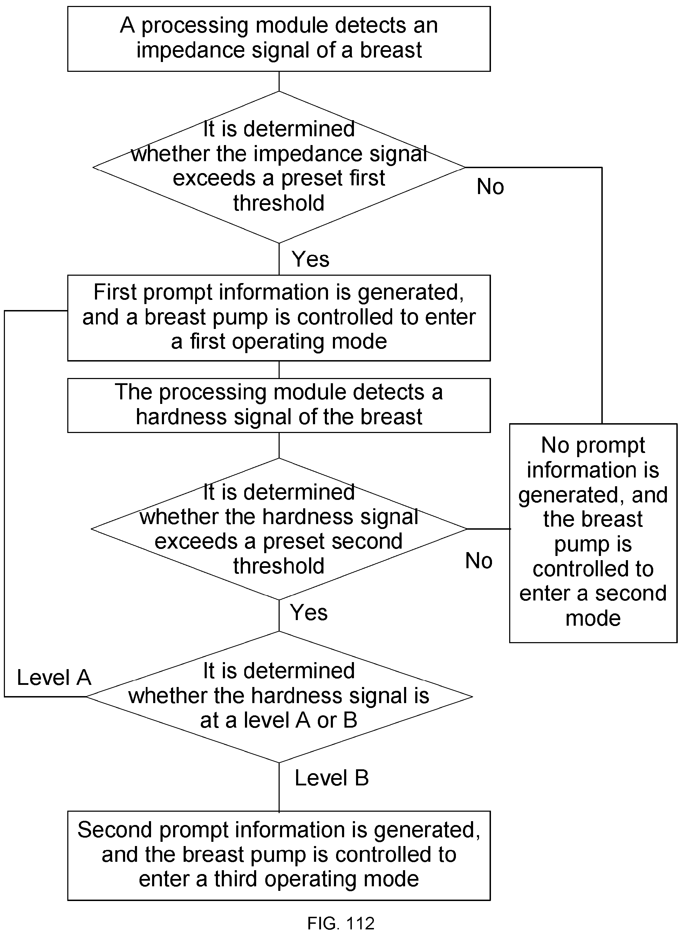

This embodiment is based on acquiring the detection parameter and performing the preset operation based on the detection parameter. Specifically, the impedance signal and/or the hardness signal of the breast is acquired, and then analysis is performed based on the received impedance signal and/or hardness signal. As shown in FIG. 112 and FIG. 113, specific steps in this embodiment are as follows:

The detection unit acquires the impedance signal of the breast, and sends the impedance signal to the control unit; and the control unit determines whether the impedance signal of the breast exceeds a preset first threshold.

When it is determined that the impedance signal of the breast exceeds the preset first threshold, the prompt module is controlled to generate first prompt information to prompt the user to pump milk.

When it is determined that the impedance signal of the breast does not exceed the preset first threshold, the prompt module is controlled to skip generating prompt information, and the breast pump is controlled to enter a second operating mode. The second operating mode is a mode in which the user can stimulate the breast in a timely manner to promote milk-ejection reflex of the breast or can promote lactation for the breast.

The detection unit obtains the hardness signal of the breast and sends the hardness signal to the control unit. The control unit determines whether the hardness signal of the breast exceeds a preset second threshold. When it is determined that the hardness signal of the breast exceeds the preset second threshold, a hardness signal level of the breast is determined as "A" or "B". When it is determined that the hardness signal of the breast does not exceed the preset second threshold, no prompt information is generated, and the control unit controls the breast pump to enter the second operating mode, to promote milk-ejection reflex of the breast or to promote lactation for the breast.

Furthermore, specific steps through which the control unit determines the hardness signal level of the breast as "A" or "B" are as follows: When it is determined that the hardness signal level of the breast is "A", the prompt module generates first prompt information to prompt the user to pump milk, and the control unit controls the breast pump to enter a first operating mode, so that the user can pump milk from the breast in a timely manner through the first operating mode of the breast pump, where the first operating mode is a milk expressing mode.

When it is determined that the hardness signal level of the breast is "B", second prompt information is generated to prompt the user to pump milk, and the breast pump is controlled to enter a third operating mode, so that the user can pump milk from the breast in a timely manner and care for the breast.

Preferably, in the above steps of the method, in addition to the control unit, determining whether the impedance signal exceeds the first threshold, determining whether the hardness signal exceeds the second threshold, and determining the hardness signal level further require the control unit to perform analysis and conclusion.

Preferably, in the above steps of the control method, both the first operating mode and the third operating mode of the breast pump involve the control unit performing milk expressing on the breast by controlling the air pump to generate negative pressure. However, an air pump power is set differently in the first operating mode and the third operating mode. Moreover, in the third operating mode, the user can care for and unclog the breast through the stimulation element. That is, the stimulation element massages and unclogs the breast during milk expressing, to alleviate conditions such as milk duct blockage and engorgement of the breast. Optionally, an intensity of negative pressure generated under the power of the air pump in the first operating mode is less than or equal to -0.032 MPa, and an intensity of negative pressure generated under the power of the air pump in the third operating mode is less than or equal to -0.024 MPa. In this way, a gentle suction force with low negative pressure is utilized to pump milk from the breast when it is determined that the breast hardness is high, thereby avoiding pain during use and improving comfort during the milk expressing.

Preferably, in the above steps of the control method for a breast pump, the second operating mode of the breast pump is to massage and/or heat the breast. The massage and/or heating is implemented through the stimulation element arranged on the breast shield. When the user wants to pump milk, but the impedance signal does not exceed the first threshold and the hardness signal does not exceed the second threshold, which indicates that the user needs stimulation such as lactation stimulation or massage to promote milk production or the milk-ejection reflex in the breast, a milk volume in the breast of the user may be increased within a relatively short time through the second operating mode of the breast pump.

In this embodiment, the breast pump may alternatively be controlled from a perspective of determining whether to prompt the user. Specific steps are as follows:

The detection unit receives the impedance signal of the breast and determines whether the impedance signal of the breast exceeds the preset first threshold; and generates, when determining that the impedance signal of the breast exceeds the preset first threshold, first prompt information to prompt the user to pump milk and controls the breast pump to enter the first operating mode, so that the user can pump milk from the breast in a timely manner; or generates no prompt information when determining that the impedance signal of the breast does not exceed the preset first threshold.

The detection unit receives the hardness signal of the breast; determines whether the hardness signal of the breast exceeds the preset second threshold; further determines the hardness signal level of the breast as "A" or "B" when determining that the hardness signal of the breast exceeds the preset second threshold; and generates, when determining that the hardness signal level of the breast is "A", first prompt information to prompt the user to pump milk and controls the breast pump to enter the first operating mode, so that the user can pump milk from the breast in a timely manner; or generates, when determining that the hardness signal level of the breast is "B", second prompt information to prompt the user of milk duct blockage, so that the user can care for the breast in a timely manner.

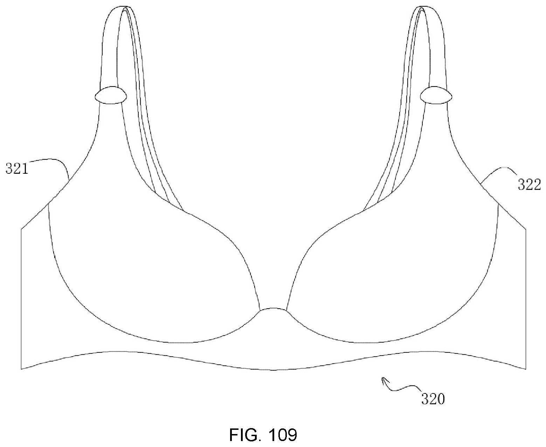

In other applications of this embodiment, the detection unit may be applied to a wearable device. As shown in FIG. 109, a wearable device 320 includes a left wearable portion 321 configured to be worn on a left human breast, a right wearable portion 322 configured to be worn on a right human breast, and a control unit 310. The detection unit is electrically connected to the control unit 310.

Optionally, the wearable device 320 is implemented as a women's undergarment, which includes a left bra cup and a right bra cup, that is, the left wearable portion 321 and the right wearable portion 322. The impedance detection module in the detection unit includes a first impedance detection module 328 mounted on an inner side of the left bra cup and a second impedance detection module 329 mounted on an inner side of the right bra cup. Arranging the first impedance detection module 328 and the second impedance detection module 329 on the inner side of the women's undergarment causes the first impedance detection module 328 to contact the left human breast and the second impedance detection module 329 to contact the right human breast, thereby enabling simultaneous acquisition of impedance signals from the left breast and the right breast.

Moreover, the first impedance detection module 328 includes at least one pair of electrodes respectively attached to opposite sides of the left breast, and the second impedance detection module 329 includes at least one pair of electrodes respectively attached to opposite sides of the right breast.

Optionally, the first impedance detection module 328 may include one pair or a plurality of pairs of electrodes, and the second impedance detection module 329 may include one pair or a plurality of pairs of electrodes. In practical use, women's undergarment products with different numbers of electrode pairs may be designed based on actual human breast sizes of users. In this way, the control unit 30 can provide more accurate information based on data from a plurality of pairs of electrode pairs when serving users with different breast sizes, thereby improving user experience.

Optionally, the hardness detection module in the detection unit includes a first sensor 330 mounted on the inner side of the left bra cup and a second sensor 331 mounted on the inner side of the right bra cup. Arranging the first sensor 330 and the second sensor 331 on the inner side of the women's undergarment causes the first sensor 330 to contact the left human breast and the second sensor 331 to contact the right human breast, thereby enabling simultaneous acquisition of hardness signals from the left breast and the right breast.

Moreover, the first sensor 330 includes at least one pair of contact terminals respectively attached to opposite sides of the left breast, and the second sensor 331 includes at least one pair of contact terminals respectively attached to opposite sides of the right breast.

Specifically, in actual of the above solution, the first sensor 330 and the second sensor 331 may be pressure sensors. Contact terminals of the pressure sensor are caused to abut against the human breast to detect the hardness of the breast. Optionally, the first sensor 330 and the second sensor 331 may be any other sensors that can detect a hardness.

### Nineteenth embodiment

In a first implementation, referring to FIG. 99 to FIG. 102, in some embodiments of this application, a breast pump proposed in this application includes a breast massage device 170, including an attachment portion and at least one electrode portion.

The attachment portion is configured to be attached to a breast, and is arranged on a side of a breast milk collection portion close to the breast, and at least part of the attachment portion forms a part of the breast milk collection portion or is configured to be detachably mounted on or attached to the breast milk collection portion.

The at least one electrode portion is connected to the attachment portion, is directly in contact with a breast surface or is indirectly in contact with the breast surface through a conductive portion on the attachment portion, and is configured to perform electrical stimulation and massage on the breast and/or detect an impedance of the breast.

An attachment portion 171 and at least one electrode 176. The attachment portion 171 is configured to be attached to a breast. The electrode portion 176 is connected to the attachment portion 171, is directly in contact with a breast surface or is indirectly in contact with the breast surface through a conductive portion on the attachment portion 171, and is configured to perform electrical stimulation and massage on the breast and/or detect an impedance of the breast.

The electrode portion 176 is mounted on a surface of the attachment portion 171, and the attachment portion 171 is attached to the breast, so that the breast massage device 170 can provide effective electrical stimulation and massage to the breast and/or detect the impedance value of the breast during operation. The electrical stimulation and massage are performed on the breast to stimulate tissue cells of the breast, induce contraction and relaxation in related tissues of the breast, regulate flowing of bodily fluids such as blood and breast milk, stimulate internal neural regulation of the breast, improve an overall status of the breast, promote secretion and flowing of breast milk, and improve functionality of internal tissues of the breast. The impedance of the breast is detected to determine a volume of milk in the breast, which is used a basis for determining whether to perform electrical stimulation and massage on the breast, thereby further enhancing an effect of the electrical stimulation and massage.

In some embodiments of this application, the breast massage device 170 provided herein includes an impedance detection unit 177 and an electrical stimulation unit 175, both of which are connected to the electrode portion 176. The electrical stimulation unit 175 includes at least an electrical stimulation control circuit, which actively or passively performs electrical stimulation on the breast surface. The impedance detection unit 177 includes at least an impedance detection circuit, which actively or passively detects the impedance of the breast.

The impedance detection unit 177 acquires impedance data of the breast through the electrode portion 176, and the electrical stimulation unit 175 stimulates the breast through the electrode portion 176 to perform electrical stimulation and massage on the breast. The impedance of the breast is detected to determine a volume of milk in the breast, which is used a basis for determining whether to perform electrical stimulation and massage on the breast, thereby further enhancing an effect of the electrical stimulation and massage.

In some embodiments of this application, the breast massage device 170 provided in this application further includes a control unit 30. The control unit 30 is configured to acquire impedance data information from the impedance detection unit 177, and control the operating parameter of the electrical stimulation unit 175 based on the impedance data information.

It may be understood that breast milk is a liquid substance containing water. Therefore, a larger volume of breast milk in a breast indicates a lower impedance of the breast. The impedance data information of the breast is acquired by using a bioimpedance method. The impedance detection unit 177 introduces a weak alternating current signal into the human breast. The current flows through bodily fluids which have a low resistance and good conductivity. In the human breast, a breast milk volume defines a width of a path through which the current passes. Thus, impedances of the breast under different milk contents may be measured, to represent the volume of milk in the breast by using the impedance of the breast. An impedance threshold may be set to indicate whether the milk in the breast is sufficient. If the monitored impedance of the breast is greater than the impedance threshold, it indicates that the milk in the breast is insufficient, and electrical stimulation and massage need to be performed on the breast, thereby promoting breast milk secretion. The impedance of the breast may be monitored in real time, and then the operating parameter of the electrical stimulation unit 175 is adjusted based on real-time changes in the impedance of the breast. The operating parameter of the electrical stimulation unit 175 may include a current intensity, a pulse width, a frequency, a waveform, a polarity, a massage duration, and a current path.

In some embodiments of this application, the breast massage device 170 provided in this application further includes a storage unit 179 connected to the control unit 30. After acquiring the impedance data information from the impedance detection unit 177, the control unit 30 controls the operating parameter of the electrical stimulation unit 175 based on the impedance data information, and transmits the impedance data information to the storage unit 179 for storage. During control of the operating parameter of the electrical stimulation unit 175 based on the impedance data information, the control unit 30 retrieves historical impedance data information stored in the storage unit 179, and combines real-time impedance data information with the historical impedance data information to control the operating parameter of the electrical stimulation unit 175, thereby further optimizing the operating parameter of the electrical stimulation unit 175.

In some embodiments of this application, the operating parameter of the electrical stimulation unit 175 may be detected and fed back to the control unit 30, and the control unit 30 transmits the operating parameter of the electrical stimulation unit 175 to the storage unit 179 for storage. During adjustment of the operating parameter of the electrical stimulation unit 175, the control unit 30 retrieves the historical impedance data information and a historical operating parameter of the electrical stimulation unit 175 stored in the storage unit 179, to determine constitution of the user and a proper operating parameter of the electrical stimulation unit 175. The control unit 30 adjusts the operating parameter of the electrical stimulation unit 175 based on the constitution of the user and the proper operating parameter of the electrical stimulation unit 175, to further optimize the operating parameter of the electrical stimulation unit 175, which ensures an intended electrical stimulation and massage effect while improving user experience.

In a second implementation, referring to FIG. 99 to FIG. 102, in some embodiments of this application, a breast massage device 170 provided in this application includes an attachment portion 171 and an electrical stimulation unit 175. The attachment portion 171 is configured to be attached to a breast. The electrical stimulation unit 175 is connected to at least two electrode portions 176. The electrode portions 176 are mounted on a contact surface of the attachment portion 171, and are configured to perform electrical stimulation and massage on the breast.

Since the electrode portions 176 of the electrical stimulation unit 175 are mounted on the contact surface of the attachment portion 171, and the attachment portion 171 is attached to the breast, the electrical stimulation unit 175 can effectively perform electrical stimulation and massage on the breast during operation. The electrical stimulation and massage are performed on the breast to stimulate tissue cells of the breast, induce contraction and relaxation in related tissues of the breast, regulate flowing of bodily fluids such as blood and breast milk, stimulate internal neural regulation of the breast, improve an overall status of the breast, promote secretion and flowing of breast milk, and improve functionality of internal tissues of the breast.

Referring to FIG. 99 to FIG. 103. In some implementations, the breast massage device 170 further includes:
an impedance detection unit 177 and/or a flow rate detection unit 178, and a control unit 30. The impedance detection unit 177 is configured to detect an impedance of the breast, and the flow rate detection unit 178 is configured to detect a flow rate of the breast milk in the breast pump 1.

The control unit 30 is configured to adjust an operating parameter of the electrical stimulation unit 175 based on impedance data information from the impedance detection unit 177 and/or flow rate data information from the flow rate detection unit 178.

In some embodiments of this application, the impedance of the breast may be detected by the impedance detection unit 177 to determine a volume of milk in the breast, and then the operating parameter of the electrical stimulation unit 175 is adjusted based on the volume of milk in the breast.

In some embodiments of this application, the flow rate of the breast milk in the breast pump 1 may be detected by the flow rate detection unit 178, to determine a volume and a speed of breast milk discharged from the breast, and then the operating parameter of the electrical stimulation unit 175 is adjusted based on the volume and the speed of the breast milk discharged from the breast.

It may be understood that, the flow rate of the breast milk in the breast pump 1 is detected by the flow rate detection unit 178. If the flow rate detection unit 178 can detect flow rate data, it indicates that breast milk is being discharged from the breast. A flow rate threshold may be set to indicate whether a volume and a speed of the milk discharged from the breast are as intended. If the monitored flow rate of the breast milk in the breast pump 1 is less than the flow rate threshold, it indicates that the volume and the speed of the milk discharged from the breast are not as intended. In this case, electrical stimulation and massage are performed, to promote breast milk secretion and unblock milk flow channels in the breast. The flow rate of the breast milk in the breast pump 1 may be monitored in real time, and then the operating parameter of the electrical stimulation unit 175 is adjusted based on real-time changes in the flow rate of the breast milk in the breast pump 1. The operating parameter of the electrical stimulation unit 175 may include a current intensity, a pulse width, a frequency, a waveform, a polarity, a massage duration, and a current path.

In some embodiments of this application, comprehensive analysis on a milk discharge status of the breast and a volume of milk in the breast may be performed in combination with the impedance of the breast and the flow rate of the breast milk in the breast pump 1, to determine an overall milk status in the breast, and then adjust the operating parameter of the electrical stimulation unit 175 based on the overall milk status in the breast.

It may be understood that both the impedance of the breast and the flow rate of the breast milk in the breast pump 1 may be monitored to determine the overall milk status in the breast.

When the impedance of the breast is greater than an impedance threshold, and the flow rate of the breast milk in the breast pump 1 is greater than zero but less than a flow rate threshold, it indicates that the milk in the breast is insufficient, but milk can still be discharged from the breast. In this case, a first overall milk status in the breast may be determined as: a small amount of milk exists in the breast and the milk flow channels in the breast are unblocked. In this case, the operating parameter of the electrical stimulation unit 175 may be adjusted based on the first overall milk status to promote breast milk secretion. The operating parameter of the electrical stimulation unit 175 may include a current intensity, a pulse width, a frequency, a waveform, a polarity, a massage duration, and a current path.

When the impedance of the breast is greater than the impedance threshold, and the flow rate of the breast milk in the breast pump 1 is greater than the flow rate threshold, it indicates that the milk in the breast is insufficient, but the flow rate detection unit 178 can still detect a considerable volume of breast milk flowing in the breast pump 1. In this case, it may be determined that the flow rate detection unit 178 is malfunctioning, or that breast milk in the breast pump 1 flows back. In this case, an alarm may be sent through an alarm unit to prompt a malfunction.

When the impedance of the breast is less than the impedance threshold, and the flow rate of the breast milk in the breast pump 1 is equal to zero or less than the flow rate threshold, it indicates that the milk in the breast is sufficient, but no milk is being discharged from the breast, or only a small amount of milk is being discharged from the breast. In this case, a first overall milk status in the breast may be determined as: a large amount of milk exists in the breast but the milk flow channels in the breast are blocked, that is, milk duct blockage occurs. In this case, the operating parameter of the electrical stimulation unit 175 may be adjusted based on the second overall milk status to promote breast milk secretion and unclog the milk flow channels in the breast.

When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

In some embodiments of this application, during the adjustment of the operating parameter of the electrical stimulation unit 175, the operating parameter of the electrical stimulation unit 175 may be automatically adjusted based on the impedance data information from the impedance detection unit 177 and/or the flow rate data information from the flow rate detection unit 178. Alternatively, corresponding operating modes may be selected based on the impedance data information from the impedance detection unit 177 and/or the flow rate data information from the flow rate detection unit 178, where each operating mode corresponds to a specific electrical stimulation procedure, and the operating parameter of the electrical stimulation unit 175 is adjusted based on the corresponding electrical stimulation procedure. The operating modes include but are not limited to a let-down mode, a lactation stimulation mode, a breast unclogging mode, a milk discharge mode, and a milk expressing mode. Since an actual operating condition varies across different models of breast pumps 1, before the breast pumps 1 leave the factory, the operating parameters of the electrical stimulation units 175 under different operating modes may be calibrated based on different models of the breast pumps 1, to provide a reference for adjusting the operating parameters of the electrical stimulation units 175.

During the adjustment of the operating parameter of the electrical stimulation unit 175, the control unit 30 adjusts the operating parameter of the electrical stimulation unit 175 in combination with historical impedance data information, historical flow rate data information, historical operating parameter information from an automatic adjustment process of the electrical stimulation unit 175, historical operating parameter data information from a manual adjustment process of the electrical stimulation unit 175, as well as real-time impedance data information, real-time flow rate data information, and real-time operating parameter data information.

The attachment portion 171 is designed with a shape similar to a shape of the breast, so that the attachment portion 171 can be better attached to the breast, thereby ensuring that the electrode portion 176 of the electrical stimulation unit 175 can be effectively attached to the breast and ensuring effectiveness of the operation of the electrical stimulation unit 175.

During the massage on the breast, vibration of a massage member 174 can assist in massaging the breast, thereby improving activity of tissues in the breast, promoting flowing of blood and milk in the breast, facilitating breast milk secretion, and improving breast milk fluidity.

A vibration device may be arranged in the massage assembly 174 to drive the massage assembly 174 to vibrate. The vibration device may be structured as a motor driving an eccentric wheel, where vibration is generated by rotating the eccentric wheel to rotate through the motor. Alternatively, the vibration device may be structured with an electromagnet and a permanent magnet, where vibration is generated by changing a direction of an electromagnetic force of the electromagnet acting on the permanent magnet by altering a polarity of the electromagnet. The vibration device may alternatively be any other device that can generate vibration.

In some embodiments of this application, a distance between a structural layer 173 and the attachment layer 172 is less than a diameter of the massage member 174, and the massage member 174 is fixedly mounted on the structural layer 173.

The attachment layer 172 may be made of flexible materials such as silicone. Since the distance between the structural layer 173 and the attachment layer 172 is less than the diameter of the massage member 174, the massage member 174 causes the attachment layer 172 to protrude and deform toward an attachment surface of the attachment layer 172, thereby enabling the attachment portion 171 to exert a specific degree of pressure on the breast after being attached to the breast. Therefore, when the massage assembly 174 vibrates, the breast can receive more intense vibration, and the vibration can propagate deeper into the breast, improving a massage effect.

Referring to FIG. 99 to FIG. 103, in some embodiments of this application, this application provides a breast pump 1, which is configured to pump milk from a breast and store the breast milk in a milk storage container portion 50, and includes a breast massage device 170, a breast shield 10, a negative pressure mechanism 20, a milk storage container portion 50, and a power supply system 180. The breast massage device 170 is configured to massage a breast. The breast shield 10 is configured to be attached to the breast and accommodate at least a nipple. The attachment portion 171 is arranged on a side of the breast shield 10 close to the breast, and at least part of the attachment portion 171 forms a part of the breast shield 10 or may be detachably mounted on or attached to the breast shield 10. The negative pressure mechanism 20 is configured to directly or indirectly apply negative pressure to the breast shield 10 to pump milk from the breast. The milk storage container portion 50 is in fluid communication with the breast shield 10 and can store the breast milk expressed from the breast. The power supply system 180 is configured to at least supply power to the negative pressure mechanism 20 and the breast massage device 170.

During milk expressing by using the breast pump 1, the breast shield 10 is placed over a breast of a user, and a nipple of the breast is accommodated in the breast shield 10, forming a sealed space, so that negative pressure can be generated around the nipple. The power supply system 180 provides electrical energy to cause the negative pressure mechanism 20 to operate, thereby generating negative pressure to perform milk expressing. During the milk expressing, breast milk enters the milk storage container portion 50, which holds the expressed breast milk.

In some embodiments of this application, the milk storage container portion 50 may be a milk bowl, a milk bottle, or a milk bag.

In the negative pressure mechanism 20, a power structure configured to generate negative pressure includes but is not limited to a piezoelectric pump, a diaphragm pump, a hydraulic pump, or a mechanical pump. The negative pressure mechanism 20 may directly generate negative pressure in the breast shield 10 through communication with the breast shield 10, or may indirectly generate negative pressure in the breast shield 10 by transmitting the negative pressure to a liquid barrier such as a diaphragm or an airbag. The breast milk is drawn into the milk storage container portion 50 for storage through the negative pressure.

The fluid communication between the breast shield 10 and the milk storage container portion 50 may be either direct communication or communication formed through a tube or other flow paths.

In some embodiments of this application, the breast pump 1 provided in this application further includes a flow rate detection unit 178, which can measure breast milk flow rate data of at least a segment of a fluid passage between the breast shield 10 and the milk storage container portion 50.

In some embodiments of this application, the breast pump 1 provided in this application further includes a housing 80. The negative pressure mechanism 20 is at least partially arranged in the housing 80. The breast shield 10 and the milk storage container portion 50 are respectively connected to the housing 80 to form an integral wearable unit, or the breast shield 10 and the milk storage container portion 50 are connected to each other and then connected as a whole to the housing 80 to form an integral wearable unit. The wearable unit, or at least part of the wearable unit, may be worn within a shoulder strap. The housing 80 may further include components such as a battery, a negative pressure air passage, a circuit board, and a solenoid valve therein.

In some embodiments of this application, the breast pump 1 provided in this application further includes a housing 80. The negative pressure mechanism 20 is at least partially arranged in the housing 80. The breast shield 10 and the milk storage container portion 50 are connected to form an integral wearable unit, and the negative pressure system is at least partially separated from the wearable unit. The wearable unit, or at least part of the wearable unit, may be worn within a shoulder strap. The housing 80 may further include components such as a battery, a negative pressure air passage, a circuit board, and a solenoid valve therein.

In some embodiments of this application, the attachment portion 171 is integrally formed with the breast shield 10.

In some embodiments of this application, preset control programs for different operating modes may be preset. Operating modes of the breast pump 1 may include a let-down mode, a lactation stimulation mode, a breast unclogging mode, a milk expressing mode, and a milk discharge mode.

In the let-down mode, a let-down operation may be performed through control of an expressing frequency, an expressing width, and a suction force of the breast pump 1, and the breast massage device 170 massages the breast, to promote milk secretion, open a breast milk flow channel, and improve a let-down effect. Massage and expressing may be performed alternately.

An instruction may be sent to a control unit 30 through a key or an external control device to enter the let-down mode. The control unit 30 adjusts an operating parameter of an electrical stimulation unit 175 based on a preset let-down mode procedure, to perform electrical stimulation and massage on the breast. During the electrical stimulation and massage, an impedance of the breast may be monitored by the impedance detection unit 177, and monitored impedance data information is fed back to the control unit 30. If it is determined based on the impedance data information that the impedance of the breast is less than an impedance threshold, the control unit 30 sends a startup instruction to the negative pressure mechanism 20 and sends a pause instruction to the electrical stimulation unit 175, to pause the massage on the breast and cause the breast pump 1 to start milk expressing. During the milk expressing, a flow rate of the breast milk in the breast pump 1 may be monitored by the flow rate detection unit 178, and flow rate data information of the breast milk in the breast pump 1 is fed back to the control unit 30. If it is determined based on the flow rate data information that the flow rate of the breast milk in the breast pump 1 is less than a flow rate threshold, it indicates that milk channels in the breast are blocked. The control unit 30 then sends an instruction to control the negative pressure mechanism 20 to pause operation, and sends a startup instruction to the electrical stimulation unit 175 to perform stimulation-based massage on the breast, and control the operating parameter of the electrical stimulation unit 175 to promote unclogging of the milk channels in the breast. When an operating duration of the electrical stimulation unit 175 reaches a first duration threshold, the control unit 30 sends a startup instruction to the negative pressure mechanism 20 and sends a pause instruction to the electrical stimulation unit 175, to pause the massage on the breast and cause the breast pump 1 to re-start milk expressing. During the milk expressing, the flow rate of the breast milk in the breast pump 1 may be monitored by the flow rate detection unit 178, and the flow rate data information of the breast milk in the breast pump 1 is fed back to the control unit 30. If it is determined based on the flow rate data information that the flow rate of the breast milk in the breast pump 1 is still less than the flow rate threshold, it indicates that milk channels in the breast are still blocked. In this case, massage is re-performed on the breast. This process is repeated until it is determined based on the flow rate data information fed back by the flow rate detection unit 178 that the flow rate of the breast milk in the breast pump 1 is greater than the flow rate threshold, which indicates that the milk channels in the breast are unclogged. In this case, the control unit 30 switches the operating mode to the milk expressing mode. In this way, the let-down process of the breast is completed. An operating parameter corresponding to the let-down mode may be preset, and the negative pressure mechanism 20 and the electrical stimulation unit 175 are controlled based on the preset operating parameter.

During the milk expressing, the impedance of the breast may be monitored in real time by the impedance detection unit 177, and the flow rate of the breast milk in the breast pump 1 may be monitored in real time by the flow rate detection unit 178. When the impedance of the breast is greater than an impedance threshold, and the flow rate of the breast milk in the breast pump 1 is greater than zero but less than a flow rate threshold, it indicates that the milk in the breast is insufficient, but milk can still be discharged from the breast. In this case, an instruction may be sent to the control unit 30 through a key or an external control device to enter the lactation stimulation mode, or the control unit 30 may automatically enter the lactation stimulation mode. The control unit 30 adjusts the operating parameter of the electrical stimulation unit 175 based on a preset lactation stimulation mode procedure, to perform electrical stimulation and massage on the breast, so as to promote breast milk secretion. The operating parameter of the electrical stimulation unit 175 may include a current intensity, a pulse width, a frequency, a waveform, a polarity, a massage duration, and a current path. During the massage on the breast, the operating parameter of the electrical stimulation unit 175 may be adjusted based on real-time breast impedance data information. When it is detected that the impedance of the breast is less than the impedance threshold, the control unit 30 sends an instruction to the electrical stimulation unit 175 to stop operating, to cease the massage on the breast. The control unit 30 then sends a startup instruction to the negative pressure mechanism 20 to cause the breast pump 1 to start operating. The flow rate of the breast milk in the breast pump 1 is monitored in real time by the flow rate detection unit 178. If the flow rate of the breast milk in the breast pump 1 is greater than the flow rate threshold, it indicates that the lactation stimulation is completed. In this case, the control unit 30 automatically enters the milk expressing mode.

During the milk expressing, the impedance of the breast may be monitored in real time by the impedance detection unit 177, and the flow rate of the breast milk in the breast pump 1 may be monitored in real time by the flow rate detection unit 178. When the impedance of the breast is less than the impedance threshold, and the flow rate of the breast milk in the breast pump 1 is equal to zero or less than the flow rate threshold, it indicates that the milk in the breast is sufficient, but no milk is being discharged from the breast, or only a small amount of milk is being discharged from the breast. In this case, an instruction may be sent to the control unit 30 through a key or an external control device to enter the breast unclogging mode, or the control unit 30 may automatically enter the breast unclogging mode. The control unit 30 adjusts the operating parameter of the electrical stimulation unit 175 based on a preset breast unclogging mode procedure, to perform electrical stimulation massage on the breast, so as to promote unclogging of milk channels in the breast. The operating parameter of the electrical stimulation unit 175 may include a current intensity, a pulse width, a frequency, a waveform, a polarity, a massage duration, and a current path. During the massage on the breast, the operating parameter of the electrical stimulation unit 175 may be adjusted based on real-time flow rate data information of the breast milk in the breast pump 1. When the operating duration of the electrical stimulation unit 175 reaches a second duration threshold, the control unit 30 sends a startup instruction to the negative pressure mechanism 20 and sends a pause instruction to the electrical stimulation unit 175, to pause the massage on the breast and cause the breast pump 1 to re-start milk expressing. During the milk expressing, the flow rate of the breast milk in the breast pump 1 may be monitored by the flow rate detection unit 178, and the flow rate data information of the breast milk in the breast pump 1 is fed back to the control unit 30. If it is determined based on the flow rate data information that the flow rate of the breast milk in the breast pump 1 is still less than the flow rate threshold, it indicates that milk channels in the breast are still blocked. In this case, massage is re-performed on the breast. This process is repeated until it is determined based on the flow rate data information fed back by the flow rate detection unit 178 that the flow rate of the breast milk in the breast pump 1 is greater than the flow rate threshold, which indicates that the milk channels in the breast are unclogged. In this case, the control unit 30 switches the operating mode to the milk expressing mode. In this way, the unclogging of the breast is completed.

During the milk expressing, the impedance of the breast may be monitored in real time by the impedance detection unit 177, and the flow rate of the breast milk in the breast pump 1 may be monitored in real time by the flow rate detection unit 178. When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

When a milk discharge operation needs to be performed in a specific time period of a day, an instruction may be sent to the control unit 30 through a key or an external control device to enter the milk discharge mode, so that the breast pump 1 enters the milk discharge mode. During the milk discharge, the impedance of the breast may be monitored in real time by the impedance detection unit 177, and the flow rate of the breast milk in the breast pump 1 may be monitored in real time by the flow rate detection unit 178. When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing operation is performed. During the milk expressing by the breast pump 1, a milk discharge status is monitored based on impedance data information and flow rate data information monitored in real time. When the impedance of the breast is greater than the impedance threshold, and the flow rate of the breast milk in the breast pump 1 is zero or less than the flow rate threshold, it indicates that a majority of the milk in the breast has been expressed. In this case, the control unit 30 sends a startup signal to the electrical stimulation unit 175 and controls the electrical stimulation unit 175 to massage the breast by using the operating parameter preset for the milk discharge mode, so as to stimulate tissue cells of the breast, induce contraction and relaxation in related tissues of the breast, regulate flowing of bodily fluids such as blood and breast milk, stimulate internal neural regulation of the breast, improve an overall status of the breast, promote secretion and flowing of breast milk, and improve functionality of internal tissues of the breast. When the operating duration of the electrical stimulation unit 175 reaches a third duration threshold, the control unit 30 sends a startup instruction to the negative pressure mechanism 20 and a pause instruction to the electrical stimulation unit 175, to pause the massage on the breast and cause the breast pump 1 to resume milk expressing. This process continues until the impedance of the breast is greater than the impedance threshold and the flow rate of breast milk in the breast pump 1 is less than the flow rate threshold. At this point, the control unit 30 determines that the milk discharge is completed and sends a stop instruction to the negative pressure mechanism 20, causing the breast pump 1 to stop operation.

Referring to FIG. 104, in some embodiments of this application, this application provides a control method, including following steps:
acquiring first breast status information and/or first breast milk status information of a breast pump;
determining to start an electrical stimulation procedure; and
starting the electrical stimulation procedure.

In some embodiments of this application, an impedance of a breast may be monitored in real time by an impedance detection unit 177, and a flow rate of breast milk in the breast pump 1 may be monitored in real time by a flow rate detection unit 178. Then the first breast status information is acquired based on the impedance of the breast, and the first breast milk status information of the breast pump is acquired based on the flow rate of the breast milk in the breast pump 1. Comprehensive analysis on a milk discharge status of the breast and a volume of milk in the breast may be performed in combination with the first breast status information and the first breast milk status information of the breast pump, to determine an overall milk status in the breast, and then the operating parameter of the electrical stimulation unit 175 is adjusted based on the overall status of the milk in the breast.

When the impedance of the breast is greater than an impedance threshold, and the flow rate of the breast milk in the breast pump 1 is greater than zero but less than a flow rate threshold, it indicates that the milk in the breast is insufficient, but milk can still be discharged from the breast. In this case, the operating parameter of the electrical stimulation unit 175 may be adjusted to promote breast milk secretion. The operating parameter of the electrical stimulation unit 175 may include a current intensity, a pulse width, a frequency, a waveform, a polarity, a massage duration, and a current path.

When the impedance of the breast is greater than the impedance threshold, and the flow rate of the breast milk in the breast pump 1 is greater than the flow rate threshold, it indicates that the milk in the breast is insufficient, but the flow rate detection unit 178 can still detect a considerable volume of breast milk flowing in the breast pump 1. In this case, it may be determined that the flow rate detection unit 178 is malfunctioning, or that breast milk in the breast pump 1 flows back. In this case, an alarm may be sent through an alarm unit to prompt a malfunction.

When the impedance of the breast is less than the impedance threshold, and the flow rate of the breast milk in the breast pump 1 is equal to zero or less than the flow rate threshold, it indicates that the milk in the breast is sufficient, but no milk is being discharged from the breast, or only a small amount of milk is being discharged from the breast, meaning that milk duct blockage occurs. In this case, the operating parameter of the electrical stimulation unit 175 may be adjusted based on the second overall milk status to promote breast milk secretion and unclog the milk flow channels in the breast.

When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

In some embodiments of this application, the impedance of the breast may be monitored in real time by the impedance detection unit 177, and then the first breast status information is acquired based on the impedance of the breast. When the impedance of the breast is greater than the impedance threshold, it indicates that the milk in the breast is insufficient. In this case, the operating parameter of the electrical stimulation unit 175 may be adjusted to promote breast milk secretion. The operating parameter of the electrical stimulation unit 175 may include a current intensity, a pulse width, a frequency, a waveform, a polarity, a massage duration, and a current path.

In some embodiments of this application, the flow rate of the breast milk in the breast pump 1 may be monitored in real time by the flow rate detection unit 178, and then the first breast milk status information of the breast pump is acquired based on the flow rate of the breast milk in the breast pump 1. If the flow rate of the breast milk in the breast pump 1 is less than the flow rate threshold, it indicates that the milk in the breast is insufficient. In this case, the operating parameter of the electrical stimulation unit 175 may be adjusted to promote breast milk secretion. The operating parameter of the electrical stimulation unit 175 may include a current intensity, a pulse width, a frequency, a waveform, a polarity, a massage duration, and a current path.

In some embodiments of this application, the first breast status information includes first impedance data of the breast, and the first breast milk status information of the breast pump includes first flow rate data of the breast milk in the breast pump 1.

The step of determining to start an electrical stimulation procedure includes: determining that the first impedance data is greater than the impedance threshold and/or determining that the first flow rate data is less than a flow rate threshold.

When at least one of these conditions occurs, that is, the first impedance data is greater than the impedance threshold and/or the first flow rate data is less than the flow rate threshold, it indicates that the milk in the breast is insufficient or that milk duct blockage occurs. In this case, the control unit 30 sends a startup instruction to the electrical stimulation unit 175 to start the electrical stimulation procedure, thereby massaging the breast, promoting breast milk secretion, and unclogging the breast milk channels.

Referring to FIG. 105, in some embodiments of this application, after the step of starting the electrical stimulation procedure, the control method further includes following steps:
acquiring second breast status information and/or second breast milk status information of the breast pump; and
adjusting the electrical stimulation parameter based on the second breast status information and/or the second breast milk status information of the breast pump.

The second breast status information includes second impedance data of the breast, and the second breast milk status information of the breast pump includes second flow rate data of the breast milk in the breast pump 1.

When at least one of the following conditions occurs, that is, the second impedance data is less than the first impedance data and greater than the impedance threshold, and/or the second flow rate data is greater than the first flow rate data and less than the flow rate threshold, it indicates that the milk in the breast has increased and that the breast milk channels have been unclogged to some extent. In this case, the control unit 30 sends an instruction to adjust the electrical stimulation parameter of the electrical stimulation unit 175, so as to moderate the massage on the breast and promote the breast to secrete milk naturally, thereby reducing dependence on the massage.

Referring to FIG. 106, in some embodiments of this application, after the step of starting the electrical stimulation procedure, the control method further includes following steps:
acquiring third breast status information and/or third breast milk status information of the breast pump; and
determining to enter a milk expressing procedure based on the third breast status information and/or the third breast milk status information of the breast pump.

During the milk expressing, the impedance of the breast may be monitored in real time by the impedance detection unit 177, and then the third breast status information is acquired. The flow rate of the breast milk in the breast pump 1 may be monitored in real time by the flow rate detection unit 178, and then the third breast milk status information of the breast pump is acquired. When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

In some embodiments of this application, the third breast status information includes third impedance data of the breast, and the third breast milk status information of the breast pump includes third flow rate data of the breast milk in the breast pump 1.

The step of determining to enter a milk expressing procedure based on the third breast status information and/or the third breast milk status information of the breast pump includes: determining that the third impedance data is less than the impedance threshold and/or determining that the third flow rate data is greater than the flow rate threshold.

During the milk expressing, the impedance of the breast may be monitored in real time by the impedance detection unit 177, and the flow rate of the breast milk in the breast pump 1 may be monitored in real time by the flow rate detection unit 178. When the impedance of the breast is less than the impedance threshold, and the flow rate of the breast milk in the breast pump 1 is greater than the flow rate threshold, it indicates that the milk in the breast is sufficient and the milk flow channels in the breast are unblocked. In this case, the electrical stimulation unit 175 may not be started, and a normal milk expressing mode proceeds.

Referring to FIG. 107, in some embodiments of this application, before the step of determining to start an electrical stimulation procedure, the control method further includes following steps:
determining a milk volume and a non-blockage/blockage status in the breast based on the first impedance data and/or the first flow rate data; and
determining an operating mode of the breast pump 1 based on a result of determining the milk volume and the non-blockage/blockage status in the breast.

In some embodiments of this application, after the first impedance data of the breast and the first flow rate data of the breast milk in the breast pump 1 are acquired, the milk volume and the non-blockage/blockage status in the breast are determined based on the first impedance data and the first flow rate data. If the first impedance data is less than the impedance threshold and the first flow rate data is less than the flow rate threshold, it indicates that the milk in the breast is sufficient, but a breast milk expressing volume of the breast pump 1 is not as intended. Therefore, it may be determined that the breast is in a blocked state. At this point, the control unit 30 sends an instruction to cause the breast pump 1 to enter the breast unclogging mode. If the first impedance data is less than the impedance threshold and the first flow rate data is greater than the flow rate threshold, it indicates that the milk in the breast is sufficient, and the breast milk expressing volume of the breast pump 1 is as intended. Therefore, it may be determined that milk discharge channels in the breast are unblocked. At this point, the control unit 30 sends an instruction to cause the breast pump 1 to enter the milk expressing mode. If the first impedance data is greater than the impedance threshold, it indicates that the volume of milk in the breast is low. At this point, the control unit 30 sends an instruction to cause the breast pump 1 to enter the lactation stimulation mode.

If the breast pump enters the breast unclogging mode or the lactation stimulation mode, it is determined that the electrical stimulation procedure needs to be started, and then the electrical stimulation procedure is started depending on different modes. In different modes, an adjustment rule for the operating parameter of the electrical stimulation unit 175 varies to meet a demand for electrical stimulation and massage of a corresponding mode.

According to another aspect, this application further provides a computer-readable storage medium, storing computer instructions that, when run, perform the above control method.

In some implementations, this application provides a control method. The acquiring a control parameter includes:
acquiring breast status data and/or breast milk flow rate status data.

The performing a preset operation based on the control parameter includes:
determining a breast state based on the breast status data and/or the breast milk flow rate status data; and
starting a proper milk expressing procedure based on the breast state.

According to another aspect, in some embodiments of this application, this application provides a control method, including following steps:
acquiring breast status data and/or breast milk flow rate status data;
determining a breast state based on the breast status data and/or the breast milk flow rate status data; and
starting a proper milk expressing procedure based on the breast state.

It is determined whether milk in the breast is sufficient based on the breast status data, and a flow rate of the milk from the breast is determined based on a breast milk flow rate state. A breast state is determined based on the breast status data and/or the breast milk flow rate status data. If the milk in the breast is sufficient but the flow rate of the milk from the breast is low, it is determined that the breast state is blocked. If the milk in the breast is sufficient and the flow rate of the milk from the breast is normal, it is determined that the breast state is normal milk expressing. If the milk in the breast is insufficient, it is determined that the breast state is milk-deficiency. A milk expressing procedure matching the determined breast state is started based on the breast state. If the breast state is blocked, a milk expressing procedure in a breast unclogging mode is started. If the determined breast state is normal milk expressing, a milk expressing procedure in a milk expressing mode is started. If the determined breast state is milk-deficiency, a milk expressing procedure in a lactation stimulation mode is started.

In some embodiments of this application, the breast status data includes breast milk sufficiency status data or breast milk deficiency status data.

In some embodiments of this application, a step of acquiring a breast state includes:
acquiring breast impedance data;
comparing the breast impedance data with an impedance threshold; and
determining a breast state.

If the breast impedance data is less than the impedance threshold, it is determined that the breast state is milk-sufficiency.

If the breast impedance data is greater than or equal to the impedance threshold, it is determined that the breast state is milk-deficiency.

When the impedance data is less than the impedance threshold, which indicates that a moisture content in the breast is high, it is determined that the breast state is milk-sufficiency. When the impedance data is greater than or equal to the impedance threshold, which indicates that the moisture content in the breast is low, it is determined that the breast state is milk-deficiency.

In some embodiments of this application, the breast milk flow rate status data includes normal flow rate status data, low flow rate status data, or high flow rate status data.

In some embodiments of this application, a step of acquiring a breast milk flow rate state includes:
acquiring breast milk flow rate data in a fluid pathway of the breast pump;
comparing the flow rate data with a flow rate threshold; and
determining a flow rate state.

If the flow rate data is greater than or equal to a first flow rate threshold and less than a second flow rate threshold, it is determined that the breast milk flow rate state is normal.

If the flow rate data is less than or equal to the first flow rate threshold, it is determined that the breast milk flow rate state is low.

If the flow rate data is greater than or equal to the second flow rate threshold, it is determined that the breast milk flow rate state is high.

If the flow rate data is greater than or equal to the first flow rate threshold and less than the second flow rate threshold, which indicates that milk can flow out of the breast smoothly, that is, the breast milk flow rate is normal, it is determined that the breast milk flow rate state is normal. If the flow rate data is less than or equal to the first flow rate threshold, which indicates that milk cannot easily flow out of the breast, it is determined that the breast milk flow rate state is low. If the flow rate data is greater than or equal to the second flow rate threshold, which indicates that excessive milk flows out of the breast, it is determined that the breast milk flow rate state is high.

In some embodiments of this application, the breast state includes blocked, normal milk flow, or milk deficiency.

In some embodiments of this application, the step of determining a breast state includes:
acquiring breast impedance data and breast milk flow rate data in a fluid pathway of the breast pump;
comparing the breast impedance data with an impedance threshold and the flow rate data with a flow rate threshold; and
determining a breast state.

If the breast impedance data is less than the impedance threshold and the flow rate data is less than the flow rate threshold, it is determined that the breast state is blocked.

If the breast impedance data is less than the impedance threshold and the flow rate data is greater than the flow rate threshold, it is determined that the breast state is normal milk flow.

If the breast impedance data is greater than the impedance threshold, it is determined that the breast state is milk-deficiency.

If the impedance data is less than the impedance threshold and the flow rate data is less than the flow rate threshold, which indicates that the milk in the breast is sufficient but only a small amount of milk flows out of the breast, it is determined the breast state is blocked. If the impedance data is less than the impedance threshold and the flow rate data is greater than or equal to the flow rate threshold, which indicates that the milk in the breast is sufficient and sufficient milk flows out of the breast, it is determined the breast state is normal milk flow. If the impedance data is greater than the impedance threshold, which indicates that the moisture content in the breast is low, it is determined that the breast state is milk-deficiency.

In some embodiments of this application, the starting a proper milk expressing procedure includes initiating a stimulation mode, initiating a breast unclogging mode, initiating an expressing intensity matching mode, or initiating a relief mode.

In some embodiments of this application, the breast status data is derived from one or more of breast composition detection or breast size detection.

In some embodiments of this application, the breast composition includes a volume of milk in the breast. The breast composition may further include a distribution and a status of various tissues in the breast, such as a breast hardness, a breast fat content, a breast muscle distribution, and a breast gland distribution.

In some embodiments of this application, the composition detection is achieved through breast impedance detection.

In some embodiments of this application, the initiating a stimulation mode includes initiating one or more of electrical stimulation, vibration, or kneading massage.

In some embodiments of this application, the initiating a breast unclogging mode includes initiating a hot compress mode.

In some embodiments of this application, the initiating an expressing intensity matching mode includes selecting a comfortable expressing intensity matching a current breast state of the user based on historical data.

In some embodiments of this application, initiating the relief mode includes initiating either a cold compress mode or a hot compress mode.

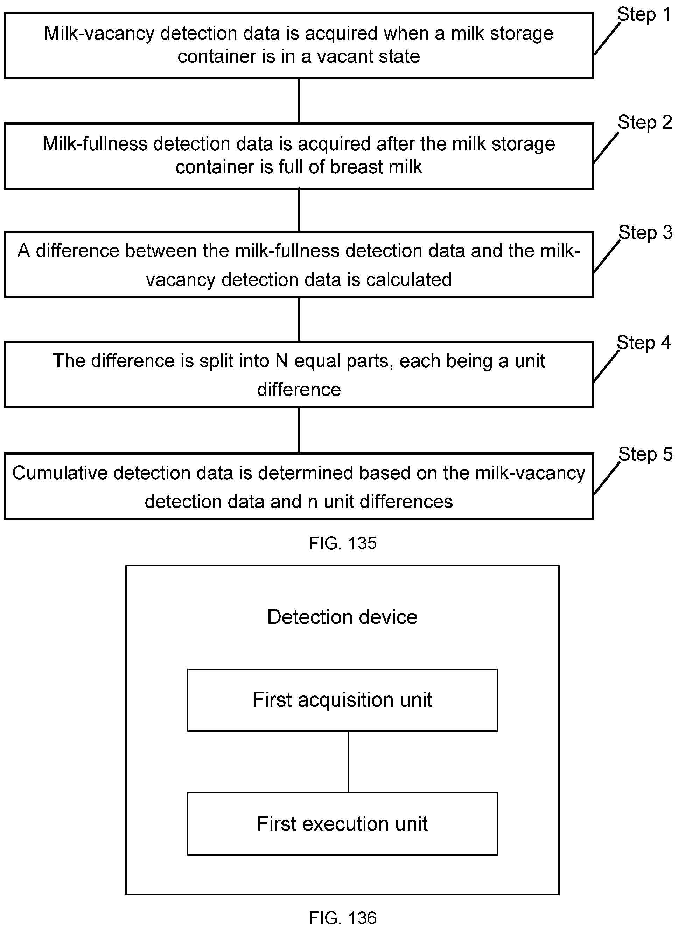

Referring to FIG. 136, FIG. 136 is a schematic structural diagram of a detection device according to an embodiment of this application. As shown in FIG. 136, a detection device 136 includes: a first acquisition unit and a first execution unit.

The first acquisition unit is configured to acquire a detection parameter detected by a detection unit.

The first execution unit is configured to perform a preset operation based on the detection parameter.

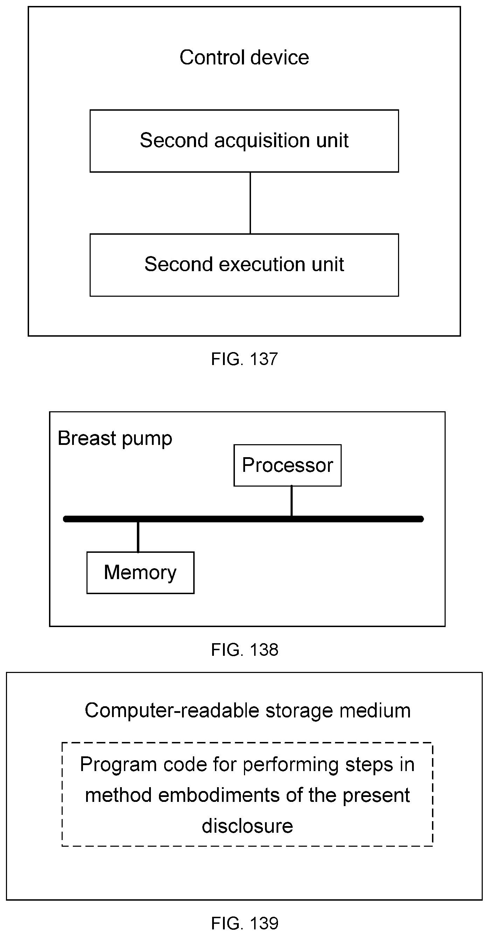

Referring to FIG. 137, FIG. 137 is a schematic structural diagram of a control device according to an embodiment of this application. As shown in FIG. 137, a control device 137 includes: a second acquisition unit and a second execution unit.

The second acquisition unit is configured to acquire a control parameter.

The second execution unit is configured to perform a preset operation based on the control parameter.

Referring to FIG. 138, FIG. 138 is a schematic structural diagram of a breast pump according to an embodiment of this application. As shown in FIG. 138, the breast pump includes one or more processors and a memory. In FIG. 138, a single processor is used as an example.

In some implementations, the processor may be connected to the memory by a bus or in other manners. In FIG. 138, the processor is connected to the memory by a bus, for example.

In some implementations, the processor is configured to acquire a detection parameter detected by a detection unit, and perform a preset operation based on the detection parameter.

In some implementations, the processor is configured to acquire a control parameter, and perform a preset operation based on the control parameter.

In some implementations, the memory is used as a non-transitory computer-readable storage medium that may be configured to store non-transitory software programs, non-transitory computer-executable programs, and modules, such as program instructions/modules for the control method and/or the detection method in the embodiments of this application. The processor executes various functional applications and data processing of the breast pump by running the non-transitory software programs, instructions, and modules stored in the memory, to implement the control method and/or the detection method in the above method embodiments.

In some implementations, the memory may include a program storage region and a data storage region, where the program storage region can store an operating system and at least one application program required for a function, and the data storage region can store data created based on the use of the breast pump. In addition, the memory may include a high speed random access memory, and may further include a non-volatile memory, such as at least one magnetic disk memory device, a flash memory device, or other non-volatile solid state memory devices. In some embodiments, the memory may optionally include memories remotely arranged relative to the processor. The remote memories may be connected to a controller by a network. Examples of the network include, but are not limited to, the Internet, an intranet, a local area network, a mobile communication network, and a combination thereof.

In some implementations, one or more modules are stored in the memory, which, when executed by one or more processors, perform the display control method for a breast pump in any one of the above method embodiments, for example, perform the control method and/or the detection method described above.

Referring to FIG. 139, FIG. 139 is a structural block diagram of a computer-readable storage medium according to an embodiment of this application. The computer-readable storage medium stores program code, which may be called by a processor to perform the control method and/or the detection method described in the above method embodiments.

The computer-readable storage medium may be an electronic memory such as a flash memory, an electrically erasable programmable read-only memory (EEPROM), an erasable programmable read-only memory (EPROM), a hard disk, or a read-only memory (ROM). Optionally, the computer-readable storage medium includes a non-transitory computer-readable storage medium. The computer-readable storage medium has a space for storing program code for performing any control operation in the above method. The program code may be read from or written into one or more computer program products. The program code may be, for example, compressed in an appropriate form.

In conclusion, this application provides a breast pump, a storage medium, a detection method, and a control method and device. The breast pump includes a breast milk collection portion, a milk storage container portion, and a negative pressure mechanism, and further includes one or both of a detection unit and a control unit. One end of the breast milk collection portion is attached to a breast, and another end of the breast milk collection portion communicates with the milk storage container portion. The milk storage container portion is configured to store breast milk collected by the breast milk collection portion, and the breast milk collection portion forms an internal cavity with an interior of the milk storage container portion for circulation or storage of the breast milk. The negative pressure mechanism is configured to directly or indirectly apply negative pressure to the internal cavity, causing the breast milk to be expressed to the milk storage container portion for storage. The detection unit is configured to acquire a detection parameter. The control unit is arranged on the breast pump. This application utilizes the detection unit and the control unit to perform intelligent control on the breast pump, to resolve at least one of problems of breast milk spillage, limited functionality, and improper mounting through intelligent control during use of the breast pump.

Finally, it should be noted that the above embodiments are merely used to describe the technical solutions of this application, and are not intended to limit this application. Although this application has been described in detail with reference to the above embodiments, it should be understood by a person of ordinary skill in the art that the technical solutions described in the above embodiments can still be modified, or some or all of technical features can be replaced by equivalents. However, these modifications or substitutions do not cause the essence of corresponding technical solutions to depart from the spirit and scope of the technical solutions in the embodiments of this application.

## Claims

1. A breast pump, wherein the breast pump comprises a breast milk collection portion, a milk storage container portion, and a negative pressure mechanism, and further comprising a detection unit and a control unit, wherein
one end of the breast milk collection portion is attached to a breast, and another end of the breast milk collection portion communicates with the milk storage container portion;
the milk storage container portion is configured to store breast milk collected by the breast milk collection portion, and the breast milk collection portion forms an internal cavity with an interior of the milk storage container portion for circulation or storage of the breast milk;
the negative pressure mechanism is configured to directly or indirectly apply negative pressure to the internal cavity, causing the breast milk to be expressed to the milk storage container portion for storage;
the detection unit is configured to acquire a detection parameter;
the control unit is configured to perform a selection operation based on the detection parameter; and
the detection parameter comprises at least one of following: a signal indicating whether a component of the breast pump is properly mounted, data on a volume of breast milk expressed by the breast milk collection portion in a single expressing operation, breast milk flow rate data, a current milk expressing status, data information of a volume of expressed breast milk, breast impedance data, breast hardness data, a user parameter, indication information indicating whether the breast milk stored in the breast pump is expressed from a left breast or a right breast, preset user parameter information, a startup signal, weaning indication information, a weaning parameter, breast status data, breast milk flow rate status data, preset breast pump operating parameter information, or milk expressing status data.

2. The breast pump according to claim 1, wherein the breast milk collection portion comprises a flange detachably mounted on the milk storage container portion;
the detection unit comprises a first detection unit configured to generate a first signal when the flange is not properly mounted on the milk storage container portion; and the control unit is configured to control, based on the first signal, the breast pump to operate.

3. The breast pump according to claim 2, further comprising a diaphragm mounted on the flange and detachably mounted on the milk storage container portion along with the flange, wherein
the detection unit further comprises a second detection unit configured to generate a second signal when the diaphragm is not properly mounted on the milk storage container portion along with the flange; and
the control unit is configured to control, based on the second signal, the breast pump to operate.

4. The breast pump according to any one of the preceding claims, further comprising a main unit, wherein the negative pressure mechanism and the control unit are arranged inside the main unit, and the main unit is detachably mounted on the milk storage container portion;
the detection unit further comprises a third detection unit configured to generate a third signal when the main unit is not properly mounted on the milk storage container portion; and
the control unit is configured to control, based on the third signal, the breast pump to operate.

5. The breast pump according to any one of claims 2 to 4, wherein
the detection unit further comprises a fourth detection unit configured to generate a fourth signal when the one end of the breast milk collection portion does not meet an attachment condition with the breast; and
the control unit is configured to perform following control based on the fourth signal:
controlling the negative pressure mechanism to stop operating;
controlling a power source of the breast pump to turn off when a duration of the fourth signal exceeds a preset duration threshold; and
controlling the negative pressure mechanism to resume operation when the duration of the fourth signal does not exceed the preset duration threshold.

6. The breast pump according to any one of the preceding claims, further comprising:
a measurement container configured to receive breast milk expressed by the breast milk collection portion in at least a single expressing operation, wherein
the detection unit comprises a fifth detection unit configured to detect a volume of the breast milk that is expressed by the breast milk collection portion in at least a single expressing operation and that is received in the measurement container; and
the control unit is configured to control a processing task of the breast pump based on the volume of the breast milk expressed by the breast milk collection portion in at least a single expressing operation, wherein the controlling a processing task of the breast pump comprises adjusting an operating parameter of the negative pressure mechanism and/or adjusting an operating mode of the breast pump.

7. The breast pump according to any one of the preceding claims, wherein the control unit is configured to:
acquire a user parameter;
determine an operating parameter of the negative pressure mechanism during operation based on the user parameter; and
control the negative pressure mechanism to operate based on the operating parameter, wherein
the user parameter comprises a pressure tolerance, and the operating parameter comprises a milk expressing intensity and a milk expressing duration, wherein the milk expressing intensity is positively correlated with the pressure tolerance, and the milk expressing duration is negatively correlated with the pressure tolerance.

8. The breast pump according to claim 7, wherein the breast pump further comprises an input unit electrically connected to the control unit and configured to generate the user parameter in response to an input instruction of a user; and/or the breast pump further comprises a communication unit electrically connected to the control unit and configured to receive the user parameter from an external terminal.

9. The breast pump according to claim 7 or 8, further comprising a display unit electrically connected to the control unit, wherein the display unit comprises a display region configured to display the user parameter and/or a display region configured to display the operating parameter.

10. The breast pump according to any one of the preceding claims, further comprising a control interface, wherein the control interface comprises a first display region and a second display region, wherein the first display region is configured to display information indicating whether breast milk is expressed from the left breast, and the second display region is configured to display information indicating whether breast milk is expressed from the right breast; and
the control unit is configured to: control the first display region to display a first display state and the second display region to display a second display state when the breast pump operates on the right breast, and control the first display region to display a third display state and the second display region to display a fourth display state when the breast pump operates on the left breast, wherein the first display state is different from the third display state, and the second display state is different from the fourth display state.

11. The breast pump according to claim 10, wherein the first display region is located on a right side of the second display region; and
the control unit is configured to:
control the first display region to shrink and the second display region to expand when the breast pump operates on the right breast, causing the first display region to form the first display state and the second display region to form the second display state; and
control the first display region to expand and the second display region to shrink when the breast pump operates on the left breast, causing the first display region to form the third display state and the second display region to form the fourth display state.

12. The breast pump according to claim 10, wherein the first display region is located above the second display region; and
the control unit is configured to:
control the first display region to display a third color and the second display region to display a fourth color when the breast pump operates on the right breast, causing the first display region to form the first display state and the second display region to form the second display state; and
control the first display region to display the fourth color and the second display region to display the third color when the breast pump operates on the left breast,
causing the first display region to form the third display state and the second display region to form the fourth display state.

13. The breast pump according to any one of the preceding claims, further comprising: a lactation stimulation assembly embedded in the breast milk collection portion, wherein the control unit is configured to control the lactation stimulation assembly to perform lactation stimulation on the breast.

14. The breast pump according to claim 13, wherein the lactation stimulation assembly comprises:
a stimulation unit electrically connected to the control unit, wherein the control unit is configured to generate a corresponding startup signal in response to a startup instruction input by the user or based on detection data of a sensor, and control the stimulation unit to start operating in a preset mode, wherein the preset mode comprises a first mode for stimulating the breast to heat up.

15. The breast pump according to claim 14, wherein the stimulation unit comprises an infrared generator, and the control unit is configured to control, after receiving the corresponding startup signal, the infrared generator to radiate infrared rays to the breast to stimulate the breast to heat up; and
the stimulation unit further comprises a massage assembly arranged on the breast milk collection portion.

16. The breast pump according to claim 13, wherein the lactation stimulation assembly comprises a plurality of oscillators, wherein
the plurality of oscillators are embedded in an inner side wall of the breast milk collection portion in a preset arrangement, causing the plurality of oscillators to contact the breast and perform lactation stimulation on the breast under the control of the control unit when the breast milk collection portion is attached to the breast.

17. The breast pump according to any one of the preceding claims, wherein
the detection unit comprises a sixth detection unit configured to detect a current milk expressing status of the user; and
the control unit is configured to:
generate a mode adjustment signal based on the current milk expressing status and a preset milk expressing status of the user;
determine a milk expressing mode based on the mode adjustment signal; and
control the breast pump to perform milk expressing in the milk expressing mode.

18. The breast pump according to any one of the preceding claims, further comprising a triggering unit configured to generate weaning indication information, wherein
the control unit is configured to control the breast pump to operate in a weaning mode based on the weaning indication information, wherein the weaning mode is used to assist a mother in ceasing milk secretion.

19. The breast pump according to claim 18, wherein the triggering unit is a physical key, a toggle, or a knob mounted on a housing; and/or
the triggering unit is a touch key, and the housing comprises a control interface, wherein the touch key is arranged on the control interface.

20. The breast pump according to any one of the preceding claims, wherein
the internal cavity comprises an inflow path for breast milk circulation, causing the breast milk to flow from the breast milk collection portion to the milk storage container portion; and
the detection unit comprises a breast milk flow rate detection unit configured to detect a breast milk flow on the inflow path.

21. The breast pump according to claim 20, further comprising a diaphragm mounted on and communicated with the milk storage container portion, wherein
the breast milk collection portion has a milk expressing channel communicated with the milk storage container portion to form the inflow path; and
the breast milk flow rate detection unit is arranged adjacent to the diaphragm, and is configured to detect a flow rate of breast milk entering the milk storage container portion after passing through a side of the diaphragm.

22. The breast pump according to claim 21, wherein the milk storage container portion is provided with a support engaged with the diaphragm to mount the diaphragm on the milk storage container portion; and
at least one mounting position is arranged on the support, wherein the breast milk flow rate detection unit is mounted in the mounting position.

23. The breast pump according to claim 20, wherein
the breast milk flow rate detection unit is configured to receive a light ray reflected during breast milk expressing and generate a detection signal corresponding to a light intensity of the light ray; and
the control unit is configured to determine a breast milk flow rate based on the light intensity corresponding to the detection signal.

24. The breast pump according to claim 23, wherein the breast pump has a transparent side wall, and
the breast milk flow rate detection unit is configured to receive a light ray from the transparent side wall during breast milk expressing and generate a detection signal corresponding to a light intensity of the light ray.

25. The breast pump according to claim 23 or 24, wherein the control unit is configured to determine a breast milk flow rate corresponding to the light intensity based on the light intensity corresponding to the detection signal and a preset mapping relationship between a light intensity and a breast milk flow rate.

26. The breast pump according to any one of claims 20 to 25, wherein the control unit is further configured to:
acquire, in real time, a current milk expressing flow rate of the breast pump driven at a current milk expressing frequency;
determine whether the current milk expressing flow rate is less than a current preset flow rate threshold; and
stop, when it is determined that the current milk expressing flow rate is less than the current preset flow rate threshold, driving the breast pump at the current milk expressing frequency, and output a lactation stimulation and milk expression-mixed control mode to drive the breast pump based on the current milk expressing flow rate,
causing the breast pump to perform lactation stimulation and milk expression on the user; or
stop, when it is determined that the current milk expressing flow rate is greater than or equal to the current preset flow rate threshold, driving the breast pump at the current milk expressing frequency, and output a target milk expressing frequency based on a preset milk expressing frequency adjustment manner to drive the breast pump to operate.

27. The breast pump according to any one of the preceding claims, wherein the detection unit further comprises a breast milk volume detection unit configured to detect data information of a breast milk volume expressed by the breast pump; and
the control unit is configured to determine a breast milk volume based on the data information of the breast milk volume.

28. The breast pump according to claim 27, wherein a side wall of the milk storage container portion has N capacity positions having increasing capacities from a first capacity position to an N^{th} capacity position, wherein N is a positive integer;
the breast milk volume detection unit comprises at least one detection element arranged at each capacity position; and
the control unit is configured to determine a current breast milk volume in the milk storage container portion based on a detection signal of the detection element.

29. The breast pump according to claim 27 or 28, wherein the breast milk volume detection unit comprises:
a flow rate sensor arranged in a nipple channel and configured to measure flow velocity data of the breast milk expressed by the breast pump, wherein the breast milk expressed by the breast pump is transferred to the milk storage container portion through the nipple channel; and
a timing subunit configured to record an operating duration of the negative pressure mechanism and generate timing data, and
the control unit is configured to calculate a volume of the breast milk expressed by the breast pump as the breast milk volume based on the flow velocity data and the timing data.

30. The breast pump according to any one of claims 27 to 29, wherein
the breast milk volume detection unit comprises a liquid level detection unit configured to detect a liquid level of the milk storage container portion; and
the control unit is configured to determine the breast milk volume in the milk storage container portion based on at least liquid level data from the liquid level detection unit.

31. The breast pump according to claim 30, wherein the breast milk flow rate detection unit further comprises an angle detection unit configured to detect tilting data of the milk storage container portion, wherein the angle detection unit is a gyroscope; and the control unit is configured to determine the breast milk volume in the milk storage container portion based on the liquid level data from the liquid level detection unit and the tilting data from the angle detection unit.

32. The breast pump according to claim 30 or 31, wherein the liquid level detection unit comprises at least one through-beam sensor, comprising an emitter and a receiver respectively mounted on two opposite sides of the milk storage container portion.

33. The breast pump according to any one of claims 27 to 32, wherein the breast milk volume detection unit comprises a weight detection module, and the data information of the breast milk volume is breast milk weight data.

34. The breast pump according to claim 27, wherein the milk storage container portion is hung on a housing of the breast pump; and
the breast milk volume detection unit is arranged at a joint of the milk storage container portion and the housing to acquire the data information of the breast milk volume after breast milk enters the milk storage container portion.

35. The breast pump according to claim 34, wherein the housing is provided with a supporting portion having a hanging port, and the milk storage container portion is provided with a hanging portion; and
the milk storage container portion is hung on the housing through support of the supporting portion for the hanging portion.

36. The breast pump according to claim 35, wherein the hanging portion is arranged at an inlet of the milk storage container portion, and the hanging portion is flange-shaped;
an inlet diameter of the milk storage container portion is less than or equal to a diameter of the hanging port; and
a diameter of the hanging portion is greater than the diameter of the hanging port, the breast milk volume detection unit is located between the supporting portion and the hanging portion, one end of the breast milk volume detection unit is connected to the supporting portion, and another end of the detection unit is connected to the hanging portion.

37. The breast pump according to claim 35, wherein the breast milk volume detection unit comprises at least one elastic member and at least one distance sensor, wherein the elastic member and the distance sensor are both located between the supporting portion and the hanging portion, and the distance sensor is connected to the control unit;
one end of the elastic member is connected to the supporting portion, and another end of the elastic member is connected to the hanging portion; and
the distance sensor is configured to detect distance data between the supporting portion and the hanging portion, wherein
the data information of the breast milk volume is the distance data.

38. The breast pump according to claim 35, wherein the breast milk flow rate detection unit comprises at least one elastic member and at least one capacitive sensor, wherein the elastic member and the capacitive sensor are both located between the supporting portion and the hanging portion, and the capacitive sensor is connected to the control unit;
one end of the elastic member is connected to the supporting portion, and another end of the elastic member is connected to the hanging portion; and
a distance change between the hanging portion and the supporting portion causes a change in capacitance data of the capacitive sensor, wherein
the data information of the breast milk volume is the capacitance data.

39. The breast pump according to claim 35, wherein the breast milk flow rate detection unit comprises at least one pressure sensor, wherein the pressure sensor is connected to the control unit and is configured to detect pressure data between the hanging portion and the supporting portion, wherein
the data information of the breast milk volume is the pressure data.

40. The breast pump according to claim 35, wherein the breast milk flow rate detection unit comprises an air chamber assembly and an air pressure sensor, wherein
the air chamber assembly is provided with a closed space, and abuts against both the supporting portion and the hanging portion, wherein a distance change between the supporting portion and the hanging portion causes a change in a volume of the closed space; and
the air pressure sensor is configured to detect air pressure data of the air chamber assembly, wherein
the data information of the breast milk volume data is the air pressure data.

41. The breast pump according to any one of the preceding claims, further comprising a breast massage device that comprises an attachment portion and at least one electrode portion, wherein
the attachment portion is configured to be attached to the breast, and is arranged on an end of the breast milk collection portion close to the breast, and at least part of the attachment portion forms a part of the breast milk collection portion or is configured to be detachably mounted on or attached to the breast milk collection portion; and
the at least one electrode portion is connected to the attachment portion, is directly in contact with a breast surface or is indirectly in contact with the breast surface through a conductive portion on the attachment portion, and is configured to perform electrical stimulation and massage on the breast and/or detect an impedance of the breast.

42. The breast pump according to claim 41, wherein the breast massage device further comprises:
an electrical stimulation unit connected to the electrode portion and comprising at least an electrical stimulation control circuit, wherein the electrical stimulation control circuit is configured to actively or passively apply electrical stimulation to the breast surface.

43. The breast pump according to claim 42, wherein
the detection unit comprises:
an impedance detection unit connected to the electrode portion and comprising at least an impedance detection circuit, wherein the impedance detection circuit is configured to actively or passively detect breast impedance data; and/or a flow rate detection unit configured to detect a breast milk flow rate, wherein
the control unit is configured to adjust an operating parameter of the electrical stimulation unit based on the breast impedance data and/or the breast milk flow rate.

44. The breast pump according to any one of claims 41 to 43, wherein the attachment portion comprises:
an attachment layer, wherein the attachment portion is attached to the breast through the attachment layer; and
a structural layer, wherein the structural layer and the attachment layer form a double-layer structure having an intermediate accommodating space, and
the attachment layer is made of a flexible material, and at least one massage member is arranged in the accommodating space.

45. The breast pump according to any one of the preceding claims, wherein
the detection unit comprises:
an impedance detection unit configured to detect breast impedance data; and/or a hardness detection unit configured to detect breast hardness data, wherein
the control unit is configured to control the breast pump to operate in different modes based on the breast impedance data and/or the breast hardness data.

46. The breast pump according to claim 45, further comprising a prompt module configured to generate prompt information based on impedance data information or hardness data information.

47. A method for a breast pump, comprising:
acquiring a parameter related to the breast pump, wherein the parameter related to the breast pump comprises at least a detection parameter detected by a detection unit; and
performing a preset operation based on the parameter, wherein
the detection parameter comprises at least one of following: a signal indicating whether a component of the breast pump is properly mounted, data on a volume of breast milk expressed by a breast milk collection portion in a single expressing operation, breast milk flow rate data, a current milk expressing status, data information of a volume of expressed breast milk, breast impedance data, breast hardness data, a user parameter, indication information indicating whether the breast milk stored in the breast pump is expressed from a left breast or a right breast, preset user parameter information, a startup signal, weaning indication information, a weaning parameter, breast status data, breast milk flow rate status data, preset breast pump operating parameter information, or milk expressing status data.

48. The method according to claim 47, wherein
the acquiring a detection parameter detected by a detection unit comprises: acquiring a first signal generated by a first detection unit when improper mounting of a flange is detected; and
the performing a preset operation based on the parameter comprises: based on the first signal, controlling to automatically generate first prompt information and/or stop milk expressing.

49. The method according to claim 48, wherein the acquiring a detection parameter detected by a detection unit further comprises: acquiring a second signal generated by a second detection unit when improper mounting of a main unit is detected; and the performing a preset operation based on the parameter further comprises: based on the second signal, controlling to automatically generate second prompt information and/or stop milk expressing.

50. The method according to any one of claims 47 to 49, wherein
the acquiring a detection parameter detected by a detection unit comprises: acquiring a third signal generated when a diaphragm is not properly mounted; and
the performing a preset operation based on the parameter further comprises: based on the third signal, controlling the breast pump to operate.

51. The method according to any one of claims 47 to 50, wherein
the acquiring a detection parameter detected by a detection unit comprises: acquiring feature triggering information associated with a triggering status of a detection element; and
the performing a preset operation based on the parameter comprises: determining current breast milk volume information based on the feature triggering information and a preset relationship, wherein the preset relationship is used to represent a mapping relationship and/or a function relationship between feature triggering information and breast milk volume information.

52. The method according to claim 51, wherein before the step of acquiring feature triggering information associated with a triggering status of a detection element, the method further comprises:
detecting an operating parameter of the detection element when the detection element senses breast milk in a milk storage container portion; and
determining, when an operating parameter of any detection element is greater than or equal to a preset trigger threshold, that the detection element is triggered.

53. The method according to claim 51, wherein when the detection element is a capacitive sensor, the operating parameter is a capacitance of the detection element, and the preset trigger threshold is a preset capacitance threshold.

54. The method according to any one of claims 47 to 54, wherein the acquiring a detection parameter detected by a detection unit comprises: measuring a volume of breast milk expressed by the breast pump in at least a single expressing operation; and
the performing a preset operation based on the parameter comprises: determining a current breast milk volume expressed by the breast pump based on a total of the volumes of breast milk expressed by the breast pump in at least respective single expressing operations.

55. The method according to claim 54, wherein the volume of the breast milk expressed by the breast pump in at least a single expressing operation is a volume of breast milk that is obtained by the breast pump in a single expressing operation and that is received between two adjacent expressing operations of a negative pressure mechanism of the breast pump.

56. The method according to any one of claims 47 to 55, wherein the data information of the breast milk volume comprises liquid level data information of the milk storage container portion, and the acquiring a detection parameter detected by a detection unit comprises:
acquiring liquid level data from a liquid level detection unit, wherein the liquid level detection unit comprises at least one through-beam sensor; and
the performing a preset operation based on the parameter comprises:
determining a breast milk volume in the milk storage container portion based on the liquid level data.

57. The method according to claim 56, wherein the data information of the breast milk volume further comprises tilting data of the milk storage container portion, and the acquiring a parameter related to the breast pump comprises: acquiring tilting data from an angle detection unit; and
the performing a preset operation based on the parameter comprises: determining the breast milk volume in the milk storage container portion based on the liquid level data and the tilting data.

58. The method according to any one of claims 47 to 57, wherein
the acquiring a detection parameter detected by a detection unit comprises:
determining to enter a breast milk volume measurement procedure when a tilting angle and a motion status of the milk storage container portion meet a breast milk volume measurement condition; and
acquiring breast milk volume measurement reference data, wherein the breast milk volume measurement reference data comprises the liquid level data and/or the tilting data of the milk storage container portion; and
the performing a preset operation based on the parameter comprises:
determining the breast milk volume in the milk storage container portion based on the breast milk volume measurement reference data.

59. The method according to any one of claims 47 to 58, wherein the data information of the breast milk volume comprises a detection signal used to represent an intensity of light reflected during breast milk expressing;
the acquiring a parameter related to the breast pump comprises: acquiring the detection signal; and
the performing a preset operation based on the parameter comprises: determining a breast milk flow rate based on the light intensity corresponding to the detection signal.

60. The method according to claim 59, wherein the determining a breast milk flow rate based on the light intensity corresponding to the detection signal comprises:
determining a breast milk flow rate corresponding to the light intensity based on the light intensity corresponding to the detection signal and a preset mapping relationship between a light intensity and a breast milk flow rate.

61. The method according to claim 59, further comprising:
determining a current breast milk volume in the breast pump based on the breast milk flow rate and a current milk expressing duration.

62. The method according to claim 61, wherein the determining a current breast milk volume in the breast pump based on the breast milk flow rate and a current milk expressing duration comprises:
counting milk expressing durations having a same breast milk flow rate;
determining a plurality of breast milk volumes based on each same breast milk flow rate and a corresponding milk expressing duration; and
using a total of the plurality of breast milk volumes as the current breast milk volume in the breast pump.

63. The method according to any one of claims 47 to 62, wherein
the acquiring a detection parameter detected by a detection unit comprises: acquiring breast impedance data and/or breast hardness data; and
the performing a preset operation based on the detection parameter comprises:
controlling the breast pump to operate in different modes based on the breast impedance data and/or the breast hardness data.

64. The method according to any one of claims 47 to 63, wherein the acquiring a detection parameter detected by a detection unit comprises: acquiring the data information of the breast milk volume after breast milk enters the milk storage container portion; and
the performing a preset operation based on the detection parameter comprises:
determining breast milk volume data of the milk storage container portion based on the data information of the breast milk volume.

65. The method according to claim 64, wherein the data information of the breast milk volume is one or more of distance data, capacitance data, pressure data, or air pressure data.

66. The method according to claim 64 or 65, wherein before the step of acquiring detection data after breast milk enters the milk storage container portion, the method further comprises a following step:
calibrating the detection data.

67. The method according to any one of claims 64 to 66, wherein the step of acquiring detection data after breast milk enters the milk storage container portion comprises a following step:
acquiring two or more pieces of detection data at a same time point after breast milk enters the milk storage container portion.

68. The method according to any one of claims 47 to 67, wherein the parameter related to the breast pump further comprises a control parameter that comprises one or more of following: a volume of breast milk expressed by the breast pump in a single expressing operation, a user parameter, indication information indicating whether the breast milk stored in the breast pump is expressed from a left breast or a right breast, preset user parameter information, a startup signal, a volume of the breast milk stored in the milk storage container portion, current milk expressing flow rate data information, a current milk expressing status, a signal indicating whether a component of the breast pump is properly mounted, weaning indication information, a weaning parameter, breast status data, or breast milk flow rate status data; and the performing a preset operation based on the parameter comprises: performing the preset operation based on the control parameter.

69. The method according to claim 68, wherein the acquiring a control parameter comprises: measuring a volume of breast milk expressed by the breast pump in at least a single expressing operation; and
the performing the preset operation based on the control parameter comprises: controlling a processing task of the breast pump based on a volume of breast milk obtained in at least a single expressing operation within a preset time, wherein the controlling a processing task of the breast pump comprises adjusting an operating parameter of the negative pressure mechanism and/or adjusting an operating mode of the breast pump.

70. The method according to claim 68 or 69, wherein the acquiring a control parameter comprises: acquiring the user parameter, wherein the user parameter comprises a pressure tolerance; and
the performing the preset operation based on the control parameter comprises:
determining an operating parameter of the breast pump during operation based on the user parameter, wherein the operating parameter comprises a milk expressing parameter that comprises a milk expressing intensity and a milk expressing duration, wherein the milk expressing intensity is positively correlated with the pressure tolerance, and the milk expressing duration is negatively correlated with the pressure tolerance; and
controlling the breast pump to operate based on the operating parameter.

71. The method according to claim 70, wherein the operating parameter further comprises at least one of a milk expressing frequency or a lactation stimulation parameter, and the determining an operating parameter of the breast pump during operation based on the user parameter comprises:
determining the milk expressing frequency of the breast pump during operation based on the pressure tolerance, wherein the pressure tolerance is positively correlated with the milk expressing frequency; or
determining the operating parameter of the breast pump during operation based on the lactation stimulation parameter.

72. The method according to claim 70 or 71, wherein the determining an operating parameter of the breast pump during operation based on the user parameter comprises:
acquiring a reference operating parameter;
determining a deviation operating parameter based on the user parameter; and
determining the operating parameter of the breast pump during operation based on the reference operating parameter and the deviation operating parameter.

73. The method according to any one of claims 68 to 72, wherein
the acquiring a control parameter comprises: acquiring indication information; and the performing the preset operation based on the control parameter comprises:
controlling display states of a first display region and a second display region based on the indication information, wherein the first display region is controlled to display a first display state and the second display region is controlled to display a second display state when the breast pump operates on the right breast, and the first display region is controlled to display a third display state and the second display region is controlled to display a fourth display state when the breast pump operates on the left breast, wherein the first display state is different from the third display state, and the second display state is different from the fourth display state.

74. The method according to claim 73, wherein
the acquiring indication information comprises: acquiring the indication information sent by a terminal in communication with the breast pump; or
the acquiring indication information comprises:
acquiring detection information detected by a detection sensor, wherein the detection sensor is configured to detect an operating position of the breast pump; and
determining the indication information based on the detection information.

75. The method according to claim 73, wherein when the first display region is located on a right side of the second display region, the controlling display states of a first display region and a second display region based on the indication information comprises:
controlling the first display region to shrink and the second display region to expand when it is determined based on the indication information that the breast pump operates on the right breast, causing the first display region to form the first display state and the second display region to form the second display state; and
controlling the first display region to expand and the second display region to shrink when it is determined based on the indication information that the breast pump operates on the left breast, causing the first display region to form the third display state and the second display region to form the fourth display state.

76. The method according to claim 73, wherein when the first display region is located above the second display region, the controlling display states of a first display region and a second display region based on the indication information comprises:
controlling the first display region to display a third color and the second display region to display a fourth color when it is determined based on the indication information that the breast pump operates on the right breast, causing the first display region to form the first display state and the second display region to form the second display state; and
controlling the first display region to display the fourth color and the second display region to display the third color when it is determined based on the indication information that the breast pump operates on the left breast, causing the first display region to form the third display state and the second display region to form the fourth display state.

77. The method according to any one of claims 68 to 76, wherein the acquiring a control parameter comprises: receiving the preset user parameter information of a current user; and
the performing the preset operation based on the control parameter comprises:
inputting the preset user parameter information into a milk expression control model for matching, to obtain an initial milk expression control mode; and
outputting initial control data information based on the initial milk expression control mode, to drive, based on the initial control data information, a target breast pump to operate.

78. The method according to claim 77, further comprising:
receiving historical milk expressing data information of the current user;
generating a target milk expression control mode based on the historical milk expressing data information and the initial milk expression control mode; and
outputting target control data information based on the target milk expression control mode, to drive, based on the target control data information, the target breast pump to operate.

79. The method according to claim 78, wherein the historical milk expressing data information is data information collected in the initial milk expression control mode; and
the generating a target milk expression control mode based on the historical milk expressing data information and the initial milk expression control mode comprises:
acquiring an average volume of expressed milk based on a plurality of pieces of historical milk expressing data information; and
determining whether the average volume of expressed milk is within or greater than a milk expressing volume range corresponding to the initial milk expression control mode; and
determining that the target milk expression control mode is the same as the initial milk expression control mode if the average volume of expressed milk is within or
greater than the milk expressing volume range corresponding to the initial milk expression control mode; or
modifying the initial milk expression control mode based on a preset adjustment mechanism to generate the target milk expression control mode.

80. The method according to any one of claims 77 to 79, wherein the preset user parameter information comprises one or more of age data information, breast size data information, usage season information, or usage region information; and
the preset user parameter information is received by a device through manual input or selection performed by the current user on the device.

81. The method according to any one of claims 68 to 80, wherein
the acquiring a control parameter comprises: generating a corresponding startup signal in response to a startup instruction input by a user or based on the detection data of the detection sensor; and
the performing the preset operation based on the control parameter comprises:
starting, based on the startup signal, a stimulation unit to operate in a preset mode, wherein the preset mode comprises at least a first mode for stimulating the breast to heat up.

82. The method according to claim 81, wherein the starting, based on the startup signal, a stimulation unit to operate in a preset mode comprises:
controlling an infrared generator of the stimulation unit to start operating to enter the first mode; or
controlling the stimulation unit to enter a second mode, wherein in the second mode, the infrared generator stops operating, and a massage assembly of the stimulation unit starts operating to massage the breast; or
controlling the stimulation unit to enter a third mode, wherein in the third mode, the infrared generator and the massage assembly of the stimulation unit start to operate to massage the breast while stimulating the breast to heat up.

83. The method according to claim 81, wherein the breast pump further comprises a flow rate sensor configured to detect a flow rate during breast milk expressing by the breast pump, and the generating a corresponding startup signal based on the detection data of the detection sensor comprises:
generating a first startup signal when the flow rate is less than a first flow rate threshold and greater than or equal to a second flow rate threshold;
generating a second startup signal when the flow rate is less than the second flow rate threshold and greater than or equal to a third flow rate threshold; or
generating, by the flow rate sensor, a third startup signal when the flow rate is less than the third flow rate threshold; and
the starting, based on the startup signal, a stimulation unit to operate in a preset mode comprises:
controlling, based on the first startup signal, an infrared generator of the stimulation unit to start operating to enter the first mode;
controlling, based on the second startup signal, the stimulation unit to enter a second mode, wherein in the second mode, the infrared generator stops operating, and a massage assembly of the stimulation unit starts operating to massage the breast; or
controlling, based on the third startup signal, the stimulation unit to enter a third mode, wherein in the third mode, the infrared generator and the massage assembly of the stimulation unit start to operate to massage the breast while stimulating the breast to heat up.

84. The method according to claim 81, wherein after the starting, based on the startup signal, a stimulation unit to operate in a preset mode, the method further comprises:
acquiring a continuous operating duration of the stimulation unit;
determining whether the continuous operating duration is greater than a preset duration threshold; and
controlling, after it is determined that the continuous operating duration is greater than the preset duration threshold, an infrared generator and/or a massage assembly of the stimulation unit in an operating state to stop operating, and starting the negative pressure mechanism of the breast pump to perform milk expressing.

85. The method according to claim 81, wherein after the starting, based on the startup signal, a stimulation unit to operate in a preset mode, the method further comprises:
controlling a temperature sensor to detect a temperature of a breast surface;
determining whether the temperature is greater than a preset temperature threshold; and
controlling, after it is determined that the temperature of the breast surface is greater than the preset temperature threshold, an infrared generator and/or a massage assembly of the stimulation unit in an operating state to stop operating, and starting the negative pressure mechanism of the breast pump to perform milk expressing.

86. The method according to any one of claims 68 to 85, wherein
the acquiring a control parameter comprises:
controlling a first measurement unit to measure a liquid level of the milk storage container portion;
controlling a second measurement unit to measure a tilting angle of the milk storage container portion; and
determining the volume of the breast milk stored in the milk storage container portion based on the liquid level and the tilting angle; and
the performing the preset operation based on the control parameter comprises:
performing the preset operation based on the volume of the breast milk stored in the milk storage container portion.

87. The method according to claim 86, wherein the determining the volume of the breast milk stored in the milk storage container portion based on the liquid level and the tilting angle comprises:
determining a first volume of the breast milk stored in the milk storage container portion based on the liquid level;
determining a correction parameter based on the liquid level and the tilting angle; and
correcting the first volume based on the correction parameter, to obtain a second volume of the breast milk stored in the milk storage container portion.

88. The method according to claim 86, wherein the determining the volume of the breast milk stored in the milk storage container portion based on the liquid level and the tilting angle comprises:
acquiring a calculation formula, wherein the calculation formula is determined based on a capacity and a shape of the milk storage container portion; and
determining the volume of the breast milk stored in the milk storage container portion based on the liquid level, the tilting angle, and the calculation formula.

89. The method according to claim 86, further comprising:
controlling the second measurement unit to measure a tilting direction of the milk storage container portion; and
the determining the volume of the breast milk stored in the milk storage container portion based on the liquid level and the tilting angle comprises:
determining the volume of the breast milk stored in the milk storage container portion based on the liquid level, the tilting angle, and the tilting direction.

90. The method according to claim 89, wherein the determining the volume of the breast milk stored in the milk storage container portion based on the liquid level, the tilting angle, and the tilting direction comprises:
determining a region in a first plane of the tilting direction;
determining a calculation formula based on the region in the first plane of the tilting direction; and
determining the volume of the breast milk stored in the milk storage container portion based on the liquid level, the tilting angle, and the calculation formula.

91. The method according to claim 89, wherein the determining the volume of the breast milk stored in the milk storage container portion based on the liquid level, the tilting angle, and the tilting direction comprises:
determining a region in a first plane of the tilting direction;
determining a correspondence table based on the region in the first plane of the tilting direction;
searching the correspondence table for a correction parameter based on the liquid level and the tilting angle;
determining a first volume of the breast milk stored in the milk storage container portion based on the liquid level; and
correcting the first volume based on the correction parameter, to obtain a second volume of the breast milk stored in the milk storage container portion.

92. The method according to any one of claims 68 to 91, wherein the acquiring a control parameter comprises: acquiring, in real time, a current milk expressing flow rate of the breast pump driven at a current milk expressing frequency; and
the performing the preset operation based on the control parameter comprises:
determining whether the current milk expressing flow rate is less than a current preset flow rate threshold; and
stopping, when it is determined that the current milk expressing flow rate is less than the current preset flow rate threshold, driving the breast pump at the current milk expressing frequency, and outputting a lactation stimulation and milk expression-mixed control mode to drive the breast pump based on the current milk expressing flow rate data information, causing the breast pump to perform lactation stimulation and milk expression on the user; or
stopping, when it is determined that the current milk expressing flow rate is greater than or equal to the current preset flow rate threshold, driving the breast pump at the current milk expressing frequency, and outputting a target milk expressing frequency based on a preset milk expressing frequency adjustment manner to drive the breast pump to operate.

93. The method according to claim 92, wherein the outputting a target milk expressing frequency based on a preset milk expressing frequency adjustment manner to drive the breast pump to operate comprises:
setting a milk expressing frequency adjustment factor;
calculating an adjusted milk expressing frequency based on the milk expressing frequency adjustment factor and the current milk expressing frequency;
updating the current milk expressing frequency to the adjusted milk expressing frequency and applying the adjusted milk expressing frequency to the breast pump to obtain adjusted milk expressing flow rate data information;
continuously updating the current milk expressing frequency to obtain a plurality of pieces of adjusted milk expressing flow rate data information; and
acquiring a current milk expressing frequency as the target milk expressing frequency if the adjusted milk expressing flow rate data information no longer increases with adjustment of the current milk expressing frequency.

94. The method according to claim 92 or 93, wherein the lactation stimulation and milk expression-mixed control mode comprises a plurality of levels of lactation stimulation and milk expression-mixed control modes; and
the outputting a lactation stimulation and milk expression-mixed control mode to drive the breast pump based on the current milk expressing flow rate data information, causing the breast pump to perform lactation stimulation and milk expression on the user comprises:
acquiring a lactation stimulation and milk expression-mixed control mode of a corresponding level based on the current milk expressing flow rate data information; and
driving the breast pump based on the lactation stimulation and milk expression-mixed control mode of the corresponding level, causing the breast pump to perform lactation stimulation and milk expression on the user.

95. The method according to any one of claims 68 to 94, wherein the acquiring a control parameter comprises:
determining a current milk expressing status of the user; and
the performing the preset operation based on the control parameter comprises:
generating a mode adjustment signal based on the current milk expressing status and a preset milk expressing status of the user, to adjust a milk expressing mode of the breast pump.

96. The method according to claim 95, wherein the determining a current milk expressing status of the user comprises:
determining the current milk expressing status of the user based on a milk expressing flow rate and breast feature information of the user, wherein
the breast feature information comprises at least one of following: breast plumpness; breast composition; or
breast hardness.

97. The method according to claim 95 or 96, wherein the generating a mode adjustment signal based on the current milk expressing status and a preset milk expressing status of the user, to adjust a milk expressing mode of the breast pump comprises:
determining a breast milk volume status based on milk expressing efficiency corresponding to the current milk expressing status of the user and milk expressing efficiency corresponding to the preset milk expressing status; and
determining the mode adjustment signal based on the breast milk volume status.

98. The method according to any one of claims 68 to 97, comprising following steps:
the acquiring a control parameter comprises: acquiring a first signal generated when it is detected that the breast is not attached to a breast shield; and
the performing the preset operation based on the control parameter comprises:
controlling the negative pressure mechanism to stop operating and/or generating prompt information and/or turning off the breast pump based on the first signal.

99. The method according to any one of claims 68 to 97, wherein
the acquiring a control parameter comprises:
determining an operating mode of the breast pump when an operation instruction is received;
acquiring a number of completed weaning days when the operating mode is a weaning mode, wherein the weaning mode is used to assist a mother in ceasing milk secretion; and
the performing the preset operation based on the control parameter comprises:
determining an operating parameter based on the number of completed weaning days; and
performing milk expressing based on the operating parameter.

100. The method according to claim 99, wherein the determining an operating parameter based on the number of completed weaning days comprises:
determining a total number of completed milk expressing operations and/or a volume of breast milk obtained in a single expressing operation in a day based on the number of completed weaning days.

101. The method according to claim 100, wherein the performing milk expressing based on the operating parameter comprises: acquiring a number of completed milk expressing operations in the day; and
performing milk expressing based on the volume of breast milk obtained in a single expressing operation when the number of completed milk expressing operations in the day is less than the total number of completed milk expressing operations.

102. The method according to any one of claims 68 to 101, wherein the acquiring a control parameter comprises: receiving weaning indication information; and
the performing the preset operation based on the control parameter comprises: operating in the weaning mode based on the weaning indication information.

103. The method according to claim 102, wherein the weaning indication information is triggered and generated by a triggering unit, or is sent by the terminal in communication with the breast pump.

104. The method according to claim 102, further comprising:
receiving configuration information; and
configuring a mode parameter of the weaning mode based on the configuration information, wherein the mode parameter comprises a total number of weaning days and/or a daily operating parameter.

105. The method according to claim 104, wherein the daily operating parameter comprises at least one of a daily volume of expressed breast milk, a volume of breast milk obtained in a single expressing operation, or a daily number of completed milk expressing operations.

106. The method according to any one of claims 68 to 75, wherein the acquiring a control parameter comprises: acquiring breast status data and/or breast milk flow rate status data; and
the performing the preset operation based on the control parameter comprises:
determining a breast state based on the breast status data and/or the breast milk flow rate status data; and
starting a proper milk expressing procedure based on the breast state.

107. The method according to claim 106, wherein a step of acquiring a breast state comprises:
acquiring breast impedance data;
comparing the breast impedance data with an impedance threshold; and
determining a breast state, wherein
if the breast impedance data is less than the impedance threshold, it is determined that the breast state is milk-sufficiency, or if the breast impedance data is greater than or equal to the impedance threshold, it is determined that the breast state is milk-deficiency.

108. The method according to claim 107, wherein a step of acquiring a breast milk flow rate state comprises:
acquiring breast milk flow rate data in a fluid pathway of the breast pump;
comparing a breast milk flow rate with a flow rate threshold; and
determining a flow rate state, wherein
if the breast milk flow rate is greater than or equal to a first flow rate threshold and
less than a second flow rate threshold, it is determined that the breast milk flow rate state is normal;
if the breast milk flow rate is less than or equal to the first flow rate threshold, it is determined that the breast milk flow rate state is low; or
if the breast milk flow rate is greater than or equal to the second flow rate threshold,
it is determined that the breast milk flow rate state is high.

109. The method according to any one of claims 106 to 108, wherein the starting a proper milk expressing procedure comprises initiating a stimulation mode, initiating a breast unclogging mode, initiating a expressing intensity matching mode, or initiating a relief mode.

110. A breast pump, comprising:
at least one processor; and
a memory, communicatively connected to the at least one processor, wherein
the memory stores instructions executable by the at least one processor, wherein
the instructions are executed by the at least one processor, causing the at least one processor to perform the method according to any one of claims 47 to 109.

111. A breast pump, comprising:
a breast milk collection portion, a milk storage chamber, and a negative pressure mechanism, and further comprising at least one detector and a control unit, wherein
one end of the breast milk collection portion is attached to a breast, and another end of the breast milk collection portion communicates with the milk storage chamber through a nipple channel;
the milk storage chamber is configured to store breast milk collected by the breast milk collection portion;
the negative pressure mechanism is configured to directly or indirectly apply negative pressure to the nipple channel or the milk storage chamber, causing breast milk to be expressed to the milk storage chamber for storage;
the detector is configured to acquire a detection parameter;
the control unit is configured to perform an operation based on the detection parameter; and
the detection parameter comprises at least one of following: a signal indicating whether a component of the breast pump is properly mounted, data on a volume of breast milk expressed by the breast milk collection portion in a single expressing operation, breast milk flow rate data, a current milk expressing status, data information of a volume of expressed breast milk, breast impedance data, breast hardness data, a user parameter, indication information indicating whether the breast milk stored in the breast pump is expressed from a left breast or a right breast, preset user parameter information, a startup signal, weaning indication information, a weaning parameter, breast status data, breast milk flow rate status data, preset breast pump operating parameter information, or milk expressing status data.

112. The breast pump according to claim 111, wherein
the detector comprises a breast milk flow rate detector configured to detect a flow rate of the breast milk along a flow path in the breast pump.

113. The breast pump according to claim 112, wherein
an inflow channel for breast milk circulation is provided between the breast milk collection portion and the milk storage chamber, causing the breast milk to flow from the breast milk collection portion into the milk storage chamber; and
the detector comprises a breast milk flow rate detector configured to detect a breast milk flow rate on the inflow channel.

114. The breast pump according to claim 113, wherein
the inflow channel comprises a check valve arranged between the breast milk collection portion and the milk storage chamber, and the breast milk flow rate detector is arranged on the check valve to detect a flow rate of breast milk passing through the check valve.

115. The breast pump according to claim 114, wherein
the check valve comprises a first valve portion and a second valve portion communicated with the first valve portion, wherein
the first valve portion has a first opening communicated with the nipple channel and a second opening communicated with the second valve portion, wherein
the first valve portion of the check valve is configured to collect a breast milk flow of a milk expressing channel through the first opening, and allow breast milk with a predetermined outflow characteristic to flow into the second valve portion through the second opening; and
a flow rate detection element is arranged between the first valve portion and the second valve portion, and is configured to detect a flow rate of the breast milk with the predetermined outflow characteristic.

116. The breast pump according to claim 115, wherein
the first valve portion and the second valve portion are integrally formed, and are combined into a figure-8-shaped structure.

117. The breast pump according to claim 116, wherein
the flow rate detection element comprises a gravity sensor arranged on the second valve portion and configured to detect a change in gravity of the second valve portion to determine the flow rate of the breast milk.

118. The breast pump according to any one of claims 112 to 117, wherein the control unit is further configured to:
acquire, in real time, a current milk expressing flow rate of the breast pump driven at a current milk expressing frequency;
stop, when the current milk expressing flow rate is less than a current preset flow rate threshold, driving the breast pump at the current milk expressing frequency, and output a lactation stimulation and milk expression-mixed control mode to drive the breast pump based on the current milk expressing flow rate, causing the breast pump to perform lactation stimulation and milk expression on a user; or
stop, when the current milk expressing flow rate is greater than or equal to the current preset flow rate threshold, driving the breast pump at the current milk expressing frequency, and output a target milk expressing frequency based on a preset milk expressing frequency adjustment manner to drive the breast pump to operate.

119. The breast pump according to any one of claims 111 to 118, wherein the detector further comprises a breast milk volume detector configured to detect data information of a volume of acquired breast milk expressed by the breast pump, and the control unit is configured to determine a breast milk volume based on the data information of the breast milk volume.

120. The breast pump according to any one of the preceding claims, wherein
the breast milk volume detector comprises a liquid level detector configured to detect a liquid level of the milk storage chamber; and
the control unit is configured to determine a breast milk volume in the milk storage chamber based on at least liquid level data from the liquid level detector.

121. The breast pump according to claim 120, wherein the breast milk flow rate detector further comprises an angle detector configured to detect tilting data of the milk storage chamber; and
the control unit is configured to determine the breast milk volume in the milk storage chamber based on the liquid level data from the liquid level detector and the tilting data from the angle detector.

122. The breast pump according to claim 121, wherein the angle detector is a gyroscope or a three-axis acceleration sensor.

123. The breast pump according to any one of claims 111 to 122, wherein
the detector further comprises:
an impedance detector configured to detect breast impedance data; and/or a hardness detector configured to detect breast hardness data, wherein
the control unit is configured to control the breast pump to operate in different modes based on the breast impedance data and/or the breast hardness data.

124. The breast pump according to claim 123, further comprising a prompt module configured to generate prompt information based on impedance data information or hardness data information.

125. A method for a breast pump, comprising:
acquiring a parameter related to the breast pump, wherein the parameter related to the breast pump comprises at least a detection parameter detected by a detector and/or a control parameter acquired by a control unit; and
performing a preset operation based on the detection parameter and/or the control parameter, wherein
the detection parameter and/or the control parameter comprises one or more of following: a signal indicating whether a component of the breast pump is properly mounted, data on a volume of breast milk expressed by the breast milk collection portion in a single expressing operation, breast milk flow rate data, a current milk expressing status, data information of a volume of expressed breast milk, breast impedance data, breast hardness data, a user parameter, indication information indicating whether the breast milk stored in the breast pump is expressed from a left breast or a right breast, preset user parameter information, a startup signal, weaning indication information, a weaning parameter, breast status data, breast milk flow rate status data, or milk expressing status data.

126. The method according to claim 125, wherein
the data information of the breast milk volume comprises liquid level data information of a milk storage chamber, and the acquiring a detection parameter detected by a detector comprises:
determining a breast milk volume in the milk storage chamber based on the liquid level data; and
the data information of the breast milk volume further comprises tilting data of the milk storage chamber, and the acquiring a parameter related to the breast pump comprises: acquiring tilting data from an angle detector; and
the performing a preset operation based on the parameter comprises: determining the breast milk volume in the milk storage chamber based on the liquid level data and the tilting data.

127. The method according to claim 126, wherein
the acquiring a detection parameter detected by a detector comprises:
determining to enter a breast milk volume measurement procedure when a tilting angle and a motion status of the milk storage chamber meet a breast milk volume measurement condition; and
acquiring breast milk volume measurement reference data, wherein the breast milk volume measurement reference data comprises the liquid level data and/or the tilting data of the milk storage chamber; and
the performing a preset operation based on the parameter comprises:
determining the breast milk volume in the milk storage chamber based on the breast milk volume measurement reference data.

128. The method according to claim 127, wherein
the step of determining to enter a breast milk volume measurement procedure comprises a following step:
determining a vibration status of the breast pump and/or determining a tilting status of the breast pump.

129. The method according to any one of claims 125 to 128, wherein
the acquiring a detection parameter detected by a detector comprises: acquiring breast impedance data and/or breast hardness data; and
the performing a preset operation based on the detection parameter comprises:
controlling the breast pump to operate in different modes based on the breast impedance data and/or the breast hardness data.

130. The method according to any one of claims 125 to 129, comprising:
acquiring a breast pump parameter; and
acquiring an initial milk expression control mode based on the breast pump parameter.

131. The method according to claim 130, comprising: controlling the breast pump to operate based on the initial milk expression control mode.

132. The method according to claim 130, comprising:
generating a target milk expression control mode based on the historical milk expressing data information and the initial milk expression control mode; and
outputting target control data information based on the target milk expression control mode, to drive, based on the target control data information, the target breast pump to operate.

133. The method according to claim 132, comprising:
receiving historical milk expressing data information of a current user;
generating a target milk expression control mode based on the historical milk expressing data information and the initial milk expression control mode; and
outputting target control data information based on the target milk expression control mode, to drive, based on the target control data information, the target breast pump to operate.

134. The method according to claim 133, wherein
the historical milk expressing data information is data information collected in the initial milk expression control mode; and
the generating a target milk expression control mode based on the historical milk expressing data information and the initial milk expression control mode comprises:
acquiring an average volume of expressed milk based on a plurality of pieces of historical milk expressing data information; and
determining whether the average volume of expressed milk is within or greater than a milk expressing volume range corresponding to the initial milk expression control mode; and
determining that the target milk expression control mode is the same as the initial milk expression control mode if the average volume of expressed milk is within or greater than the milk expressing volume range corresponding to the initial milk expression control mode; or
modifying the initial milk expression control mode based on a preset adjustment mechanism to generate the target milk expression control mode.

135. The method according to any one of claims 130 to 134, wherein
the preset user parameter information comprises one or more of age data information, breast size data information, usage season information, or usage region information; and
the preset user parameter information is received by a device through manual input or selection performed by the current user on the device.

136. The method according to any one of claims 130 to 135, wherein
the acquiring a breast pump parameter comprises:
obtaining a liquid level in the milk storage chamber measured by a first measurement unit;
obtaining a tilting angle in the milk storage chamber measured by a second measurement unit; and
determining a volume of breast milk stored in the milk storage chamber based on the liquid level and the tilting angle.

137. The method according to claim 136, wherein
the determining a volume of breast milk stored in the milk storage chamber based on the liquid level and the tilting angle comprises:
determining a first volume of the breast milk stored in the milk storage chamber based on the liquid level;
determining a correction parameter based on the liquid level and the tilting angle; and
correcting the first volume based on the correction parameter, to obtain a second volume of the breast milk stored in the milk storage chamber.

138. The method according to claim 136, wherein the determining a volume of breast milk stored in the milk storage chamber based on the liquid level and the tilting angle comprises:
acquiring a calculation formula, wherein the calculation formula is determined based on a capacity and a shape of the milk storage chamber; and
determining the volume of the breast milk stored in the milk storage chamber based on the liquid level, the tilting angle, and the calculation formula.

139. The method according to claim 136, further comprising:
controlling the second measurement unit to measure a tilting direction of the milk storage chamber, wherein
the determining a volume of breast milk stored in the milk storage chamber based on the liquid level and the tilting angle comprises:
determining the volume of the breast milk stored in the milk storage chamber based on the liquid level, the tilting angle, and the tilting direction.

140. The method according to claim 139, wherein the determining the volume of the breast milk stored in the milk storage chamber based on the liquid level, the tilting angle, and the tilting direction comprises:
determining a region in a first plane of the tilting direction;
determining a calculation formula based on the region in the first plane of the tilting direction; and
determining the volume of the breast milk stored in the milk storage chamber based on the liquid level, the tilting angle, and the calculation formula.

141. The method according to claim 139, wherein the determining the volume of the breast milk stored in the milk storage chamber based on the liquid level, the tilting angle, and the tilting direction comprises:
determining a region in a first plane of the tilting direction;
determining a correspondence table based on the region in the first plane of the tilting direction;
searching the correspondence table for a correction parameter based on the liquid level and the tilting angle;
determining a first volume of the breast milk stored in the milk storage chamber based on the liquid level; and
correcting the first volume based on the correction parameter, to obtain a second volume of the breast milk stored in the milk storage chamber.

142. The method according to any one of claims 130 to 141, wherein
the detection parameter comprises: acquiring current milk expressing flow rate data of the breast pump in real time; and
the performing a preset operation based on the detection parameter comprises:
stopping, when the current milk expressing flow rate is less than a current preset flow rate threshold, driving the breast pump at a current milk expressing frequency, and
outputting a lactation stimulation and milk expression-mixed control mode to drive the breast pump based on current milk expressing flow rate data information, causing the breast pump to perform lactation stimulation and milk expression on the user; or
stopping, when the current milk expressing flow rate is greater than or equal to the current preset flow rate threshold, driving the breast pump at the current milk expressing frequency, and outputting a target milk expressing frequency based on a preset milk expressing frequency adjustment manner to drive the breast pump to operate.

143. The method according to claim 142, wherein the outputting a target milk expressing frequency based on a preset milk expressing frequency adjustment manner to drive the breast pump to operate comprises:
setting a milk expressing frequency adjustment factor;
calculating an adjusted milk expressing frequency based on the milk expressing frequency adjustment factor and the current milk expressing frequency;
updating the current milk expressing frequency to the adjusted milk expressing frequency and applying the adjusted milk expressing frequency to the breast pump to obtain adjusted milk expressing flow rate data information;
continuously updating the current milk expressing frequency to obtain a plurality of pieces of adjusted milk expressing flow rate data information; and
acquiring a current milk expressing frequency as the target milk expressing frequency if the adjusted milk expressing flow rate data information no longer increases with adjustment of the current milk expressing frequency.

144. The method according to claim 142 or 143, wherein the lactation stimulation and milk expression-mixed control mode comprises a plurality of levels of lactation stimulation and milk expression-mixed control modes; and
the outputting a lactation stimulation and milk expression-mixed control mode to drive the breast pump based on the current milk expressing flow rate data information, causing the breast pump to perform lactation stimulation and milk expression on the user comprises:
acquiring a lactation stimulation and milk expression-mixed control mode of a corresponding level based on the current milk expressing flow rate data information; and
driving the breast pump based on the lactation stimulation and milk expression-mixed control mode of the corresponding level, causing the breast pump to perform lactation stimulation and milk expression on the user.

145. The method according to any one of claims 125 to 144, wherein the acquiring a control parameter comprises: determining current milk expressing status data of the user; and
the performing a preset operation based on the control parameter comprises:
generating a mode adjustment signal based on the current milk expressing status data and preset milk expressing status data of the user, to adjust a control mode of the breast pump.

146. The method according to claim 145, wherein the determining a current milk expressing status of the user comprises:
determining the current milk expressing status of the user based on a breast milk flow rate state and breast status data, wherein
the breast status data comprises at least one of following:
breast plumpness;
breast composition; or
breast hardness.

147. The method according to claim 145 or 146, wherein the generating a mode adjustment signal based on the current milk expressing status and a preset milk expressing status of the user, to adjust a milk expressing mode of the breast pump comprises:
determining a breast milk volume status based on milk expressing efficiency corresponding to the current milk expressing status of the user and milk expressing efficiency corresponding to the preset milk expressing status; and
determining the mode adjustment signal based on the breast milk volume status.

148. The method according to any one of claims 125 to 144, wherein the acquiring a control parameter comprises: acquiring breast status data and/or breast milk flow rate status data; and
the performing a preset operation based on the control parameter comprises:
determining a breast pump operating mode and/or a milk expressing procedure based on the breast status data and/or the breast milk flow rate status data.

149. The method according to any one of claims 125 to 144, wherein the performing a preset operation based on the control parameter comprises:
determining a milk expressing status based on the breast status data and/or the breast milk flow rate status data; and
determining the breast pump operating mode and/or the milk expressing procedure based on the milk expressing status.

150. The method according to claim 148 or 149, wherein the step of acquiring breast status data comprises:
acquiring breast impedance data;
comparing the breast impedance data with an impedance threshold; and
determining a breast state.
if the breast impedance data is less than the impedance threshold, it is determined that the breast state is milk-sufficiency, or if the breast impedance data is greater than or equal to the impedance threshold, it is determined that the breast state is milk-deficiency.

151. The method according to any one of claims 148 to 150, wherein the step of acquiring breast milk flow rate status data comprises:
acquiring breast milk flow rate data in the breast milk flow rate detector, wherein
if the breast milk flow rate is greater than or equal to a first flow rate threshold and
less than a second flow rate threshold, it is determined that the breast milk flow rate state is normal;
if the breast milk flow rate is less than or equal to the first flow rate threshold, it is determined that the breast milk flow rate state is low; or
if the breast milk flow rate is greater than or equal to the second flow rate threshold,
it is determined that the breast milk flow rate state is high.

152. The method according to any one of claims 148 to 151, wherein the starting a proper milk expressing procedure comprises initiating a stimulation mode, initiating a breast unclogging mode, initiating a expressing intensity matching mode, or initiating a relief mode.

153. A computer-readable storage medium, storing an executable program, wherein the executable program is executed by a processor to implement the method according to any one of claims 125 to 152.
